(19) Europäisches Patentamt
European Patent Office
Office européen des brevets

(11) EP 4 105 203 A1

(12) **EUROPEAN PATENT APPLICATION**

(43) Date of publication:
**21.12.2022 Bulletin 2022/51**

(21) Application number: **22173440.3**

(22) Date of filing: **29.09.2017**

(51) International Patent Classification (IPC):
***C07D 235/30*** (2006.01) ***C07D 405/04*** (2006.01)
***C07D 471/04*** (2006.01) ***C07D 487/04*** (2006.01)
***C07D 491/10*** (2006.01) ***C07D 277/82*** (2006.01)
***C07D 209/40*** (2006.01) ***A61K 31/4184*** (2006.01)
***A61K 31/4045*** (2006.01) ***A61P 7/06*** (2006.01)
***A61P 35/02*** (2006.01)

(52) Cooperative Patent Classification (CPC):
**C07D 235/30; A61P 7/00; A61P 7/06; A61P 35/02; A61P 43/00; C07D 209/40; C07D 277/82; C07D 405/04; C07D 471/04; C07D 487/04; C07D 491/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2016 US 201662402863 P**
**22.05.2017 US 201762509620 P**

(62) Document number(s) of the earlier application(s) in accordance with Art. 76 EPC:
**17781368.0 / 3 519 393**

(71) Applicant: **Epizyme Inc**
**Cambridge, MA 02139 (US)**

(72) Inventors:
- **CAMPBELL, John Emmerson**
  **Cambridge, 02138 (US)**
- **DUNCAN, Kenneth William**
  **Westwood, 02090 (US)**

(74) Representative: **Russell, Tim et al**
**Venner Shipley LLP**
**200 Aldersgate**
**London EC1A 4HD (GB)**

Remarks:
•This application was filed on 16-05-2022 as a divisional application to the application mentioned under INID code 62.
•Claims filed after the date of filing of the application / after the date of receipt of the divisional application (Rule 68(4) EPC).

(54) **SPIRO-CONDENSED INDOLE COMPOUNDS AS EHMT2 INHIBITORS**

(57) The present disclosure relates to substituted fused bi- or tri- heterocyclic compounds of formula (Va) to (Vf). The present disclosure also relates to pharmaceutical compositions containing these compounds. Said compounds are for use in methods of treating a disorder (e.g., sickle cell anemia) via inhibition of a methyltransferase enzyme selected from EHMT1 and EHMT2.

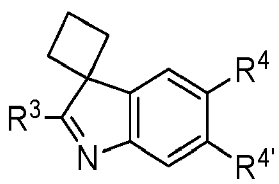 (Va), 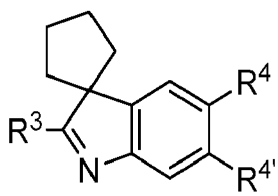 (Vb), 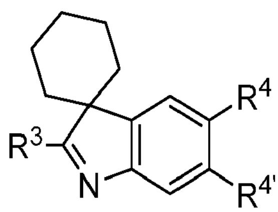 (Vc),

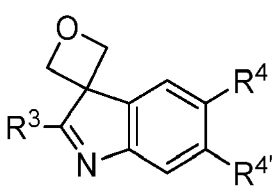 (Vd), 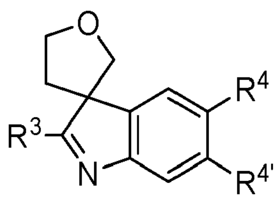 (Ve), 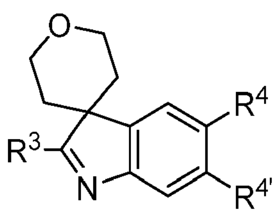 (Vf),

EP 4 105 203 A1

## Description

RELATED APPLICATIONS

This application claims priority to, and the benefit of, U.S. Provisional Application Nos. 62/402,863, filed September 30, 2016, and 62/509,620, filed May 22, 2017, the entire contents of each of which are incorporated herein by reference.

BACKGROUND

Methylation of protein lysine residues is an important signaling mechanism in eukaryotic cells, and the methylation state of histone lysines encodes signals that are recognized by a multitude of proteins and protein complexes in the context of epigenetic gene regulation.

Histone methylation is catalyzed by histone methyltransferases (HMTs), and HMTs have been implicated in various human diseases. HMTs can play a role in either activating or repressing gene expression, and certain HMTs (e.g., euchromatic histone-lysine N-methyltransferase 2 or EHMT2, also called G9a) may methylate many nonhistone proteins, such as tumor suppressor proteins *(see, e.g.,* Liu et al., Journal of Medicinal Chemistry 56:8931-8942, 2013 and Krivega et al., Blood 126(5):665-672, 2015).

Two related HMTs, EHMT1 and EHMT2, are overexpressed or play a role in diseases and disorders such as sickle cell anemia *(see, e.g.,* Renneville et al., Blood 126(16): 1930-1939, 2015) and proliferative disorders (e.g., cancers), and other blood disorders.

SUMMARY

In one aspect, the present disclosure features a substituted fused bi- or tri- heterocyclic compound of Formula (I) below:

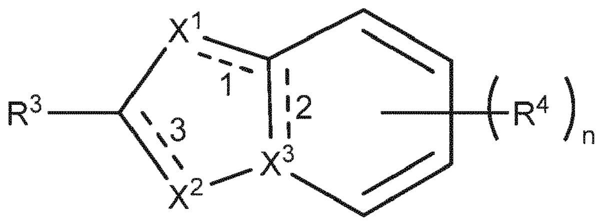

(I),

or a tautomer thereof, or a pharmaceutically acceptable salt of the compound or the tautomer, wherein

$X^1$ is O, S, $CR^1R^{11}$, or $NR^1$ when $\overset{1}{\text{-----}}$ is a single bond, or $X^1$ is N when $\overset{1}{\text{-----}}$ is a double bond;

$X^2$ is N or $CR^2$ when $\overset{3}{\text{-----}}$ is a double bond, or $X^2$ is $NR^{2'}$ when $\overset{3}{\text{-----}}$ is a single bond;

$X^3$ is N or C; when $X^3$ is N, $\overset{1}{\text{-----}}$ is a double bond and $\overset{2}{\text{-----}}$ is a single bond, and when $X^3$ is C, $\overset{1}{\text{-----}}$ is a single bond and $\overset{2}{\text{-----}}$ is a double bond;

each of $R^1$, $R^2$ and $R^{11}$, independently, is $-Q^1-T^1$, in which each $Q^1$ independently is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and each $T^1$ independently is H, halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$, $-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, $-SO_2N(R^5)_2$, $-NR^5C(O)R^6$,amino, mono- or di- alkylamino, or $C_1$-$C_6$ alkoxyl; or

$R^1$ and $R^{11}$ together with the carbon atom to which they are attached form a $C_3$-$C_{12}$ cycloalkyl or 4- to 12-membered heterocycloalkyl containing 1-4 heteroatoms selected from N, O, and S, wherein the $C_3$-$C_{12}$ cycloalkyl or 4- to 12-membered heterocycloalkyl is optionally substituted with one or more of halo, $C_1$-$C_6$ alkyl, hydroxyl, oxo, amino, mono- or di- alkylamino, or $C_1$-$C_6$ alkoxyl;

each of $R^{1'}$ and $R^{2'}$, independently, is $-Q^2-T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_{12}$ cycloalkyl, phenyl, 4- to 12-membered heterocycloalkyl containing 1-4

heteroatoms selected from N, O, and S, or a 5- or 6-membered heteroaryl and $R^{S2}$ is optionally substituted with one or more of halo, Ci-$C_6$ alkyl, hydroxyl, oxo, -C(O)$R^6$, -$SO_2R^5$, -$SO_2N(R^5)_2$, -$NR^5C(O)R^6$,amino, mono- or di-alkylamino, or $C_1$-$C_6$ alkoxyl;

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, $C_3$-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or;

$R^3$ and one of $R^{1'}$, $R^{2'}$, $R^1$, $R^2$ and $R^{11}$, together with the atoms to which they are attached, form a 5- or 6-membered heteroaryl that is optionally substituted with one or more of halo, $C_1$-$C_3$ alkyl, hydroxyl or $C_1$-$C_3$alkoxyl; or

$R^3$ is oxo and 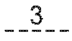 is a single bond;

each $R^4$ independently is -$Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di-alkylamino, or $C_1$-$C_6$ alkoxyl, and each $T^3$ independently is H, halo, cyano, $OR^7$, $OR^8$, C(O)$R^8$, $NR^7R^8$, C(O)$NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl;

$R^8$ is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, C(O)$R^c$, $NR^cR^d$, C(O)$NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo; and

n is 1, 2, 3, or 4, and wherein the compound is not

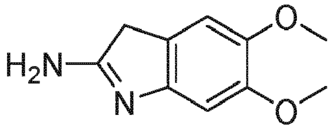 , 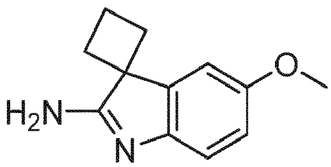 , 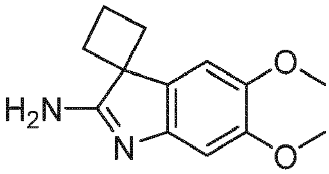 ,

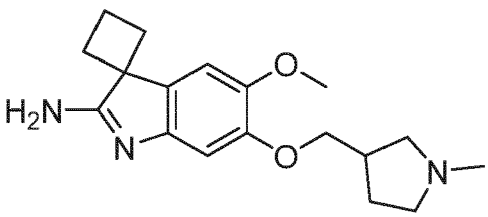 , 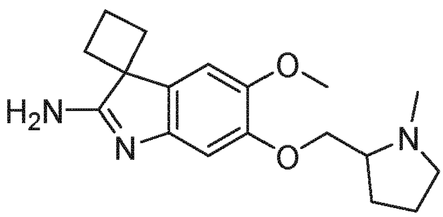 ,

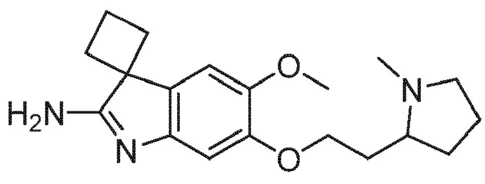, 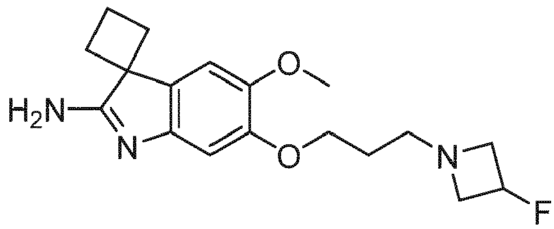,

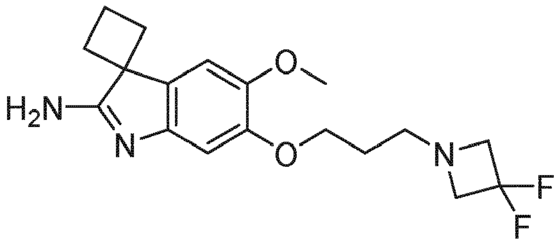, 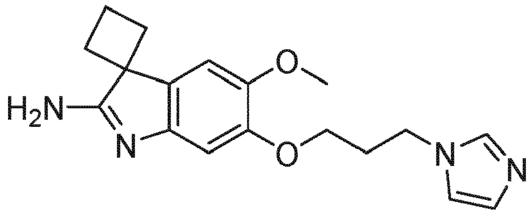,

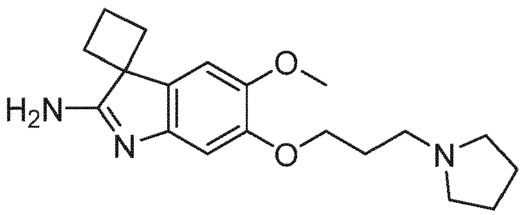, 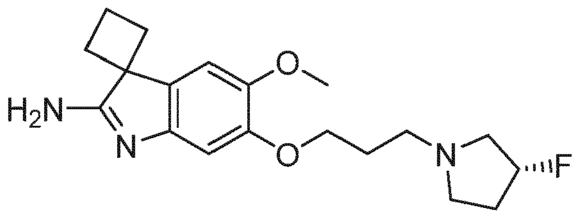,

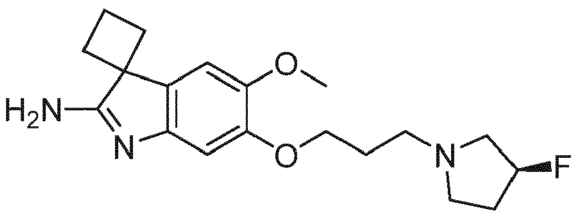, 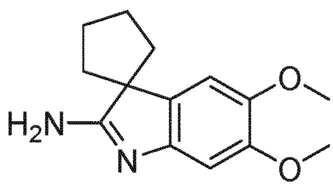,

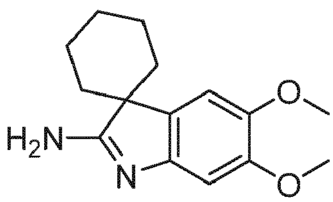, 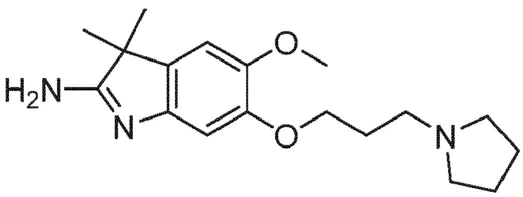, or

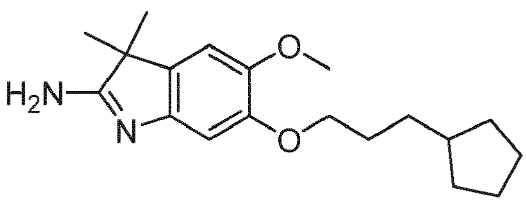.

In some embodiments, when n is 2, $X^1$ is $CR^1R^{11}$, $X^2$ is N, $X^3$ is C, $R^3$ is $NH_2$, and at least one $R^4$ is $OR^7$, then one of (1)-(4) below applies:

(1) at least one of $R^1$ and $R^{11}$ is $-Q^1-T^1$, in which $Q^1$ is a $C_1$-$C_6$ alkylene linker optionally substituted with one or more of halo, cyano, hydroxyl, or $C_1$-$C_6$ alkoxyl, and $T^1$ is cyano, $NR^5R^6$, $C(O)NR^5R^6$, $-OC(O)NR^5R^6$, $C(O)OR^5$,

-OC(O)$R^5$, C(O)$R^5$, -N$R^5$C(O)$R^6$,-N$R^5$C(O)O$R^6$, O$R^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, -C(O)$R^6$, -$SO_2R^5$, -$SO_2N(R^5)_2$, - N$R^5$C(O)$R^6$, amino, mono- or di- alkylamino, or $C_1$-$C_6$ alkoxyl; or

(2) at least one of $R^1$ and $R^{11}$ is -$Q^1$-$T^1$, in which $Q^1$ is a $C_2$-$C_6$ alkenylene or $C_2$-$C_6$ alkynylene linker optionally substituted with one or more of halo, cyano, hydroxyl, or $C_1$-$C_6$ alkoxyl, and $T^1$ is H, halo, cyano, N$R^5R^6$, C(O)N$R^5R^6$, -OC(O)N$R^5R^6$, C(O)O$R^5$, -OC(O)$R^5$, C(O)$R^5$, -N$R^5$C(O)$R^6$,-N$R^5$C(O)O$R^6$, O$R^5$, or $R^{S1}$, in which $R^{S1}$ is $C_3$-$C_{12}$ cycloalkyl, phenyl, 4-to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, -C(O)$R^6$, -$SO_2R^5$, -$SO_2N(R^5)_2$, -N$R^5$C(O)$R^6$,amino, mono- or di- alkylamino, or $C_1$-$C_6$ alkoxyl; or

(3) at least one of $R^1$ and $R^{11}$ is -$Q^1$-$T^1$, in which $Q^1$ is a bond, and $T^1$ is halo, cyano, N$R^5R^6$, C(O)N$R^5R^6$, -OC(O)N$R^5R^6$, C(O)O$R^5$, -OC(O)$R^5$, C(O)$R^5$, -N$R^5$C(O)$R^6$,-N$R^5$C(O)O$R^6$, O$R^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, -C(O)$R^6$, -$SO_2R^5$, - $SO_2N(R^5)_2$, -N$R^5$C(O)$R^6$,amino, mono- or di- alkylamino, or $C_1$-$C_6$ alkoxyl; or

(4) $R^1$ and $R^{11}$ together with the carbon atom to which they are attached form a $C_7$-$C_{12}$ cycloalkyl or 4- to 12-membered heterocycloalkyl containing 1-4 heteroatoms selected from N, O, and S, wherein the $C_7$-$C_{12}$ cycloalkyl or 4- to 12-membered heterocycloalkyl is optionally substituted with one or more of halo, $C_1$-$C_6$ alkyl, hydroxyl, oxo, amino, mono- or di- alkylamino, or $C_1$-$C_6$ alkoxyl.

Subsets of the compounds of Formula (I) include those of Formulae (IIa), (IIb), (IIc), (IId), (IIe), (IIIa), (IIIb), (IIIc), (IIId), (IIIe), (IIIf), (IVa), and (IVb):

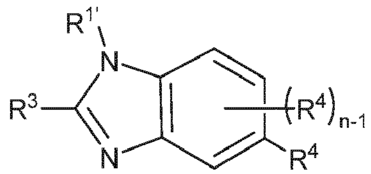
(IIa),
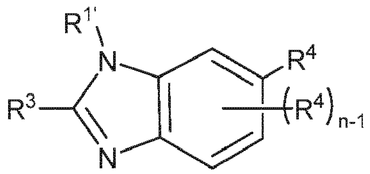
(IIb),

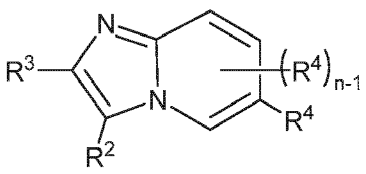
(IIc),
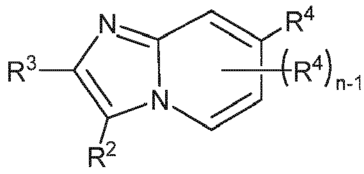
(IId),
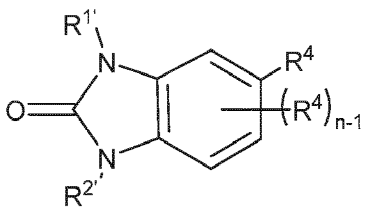

(IIe),

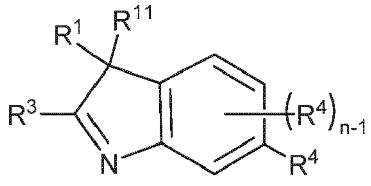
(IIIa),
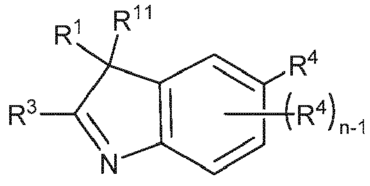
(IIIb),

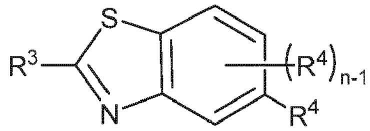
(IIIc),
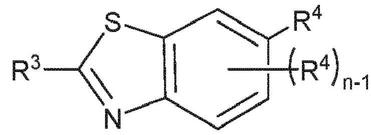
(IIId),

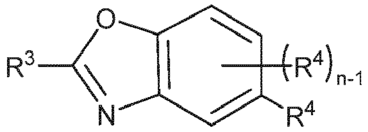 (IIIe), 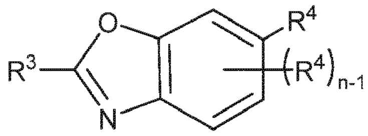 (IIIf),

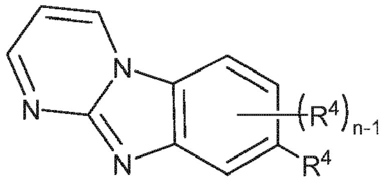 (IVa), or 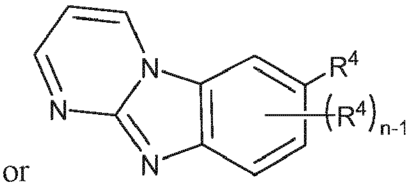 (IVb),

and tautomers thereof, and pharmaceutically acceptable salts of the compounds or the tautomers.

Subsets of the compounds of Formula (I) also include those of Formulae (IIf), (IIg), (IIh), (IIIi), (IIIj), (IIIk), and (IIIl):

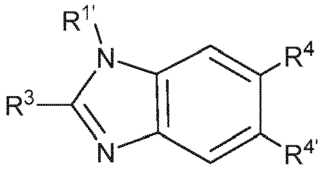 (IIf), 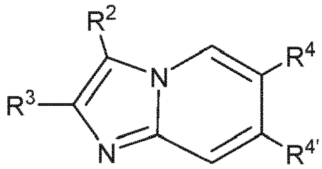 (IIg), 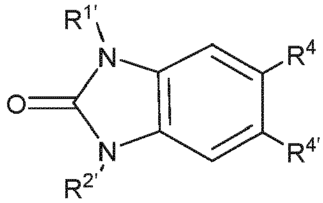 (IIh),

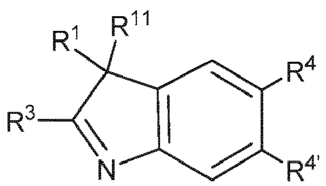 (IIIi), 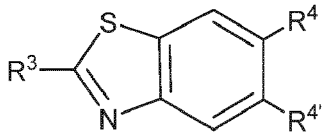 (IIIj),

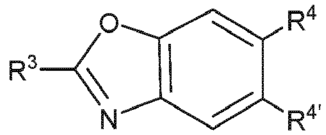 (IIIk), or 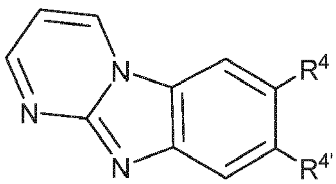 (IIIl),

and tautomers thereof, and pharmaceutically acceptable salts of the compounds or the tautomers.

Subsets of the compounds of Formula (I) also include those of Formulae (Va), (Vb), (Vc), (Vd), (Ve), and (Vf):

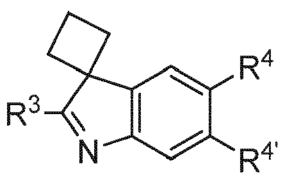 (Va), 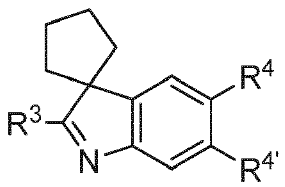 (Vb), 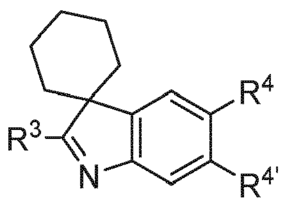 (Vc),

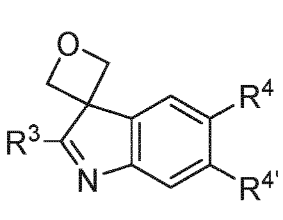 (Vd), 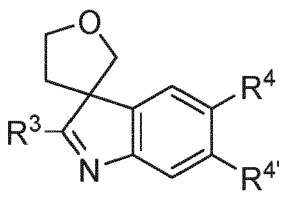 (Ve), 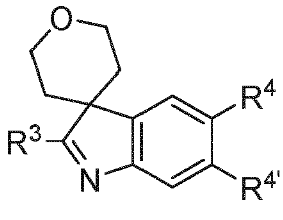 (Vf),

and tautomers thereof, and pharmaceutically acceptable salts of the compounds or the tautomers.

Subsets of the compounds of Formula (I) also include those of Formulae (VIa), (VIb), (VIc), (VId), (VIe), and (VIf):

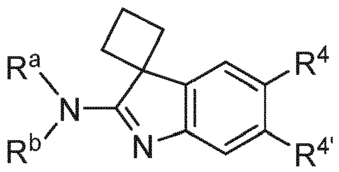 (VIa), 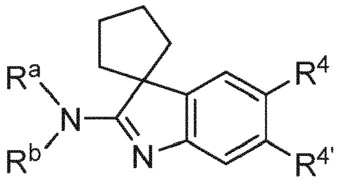 (VIb),

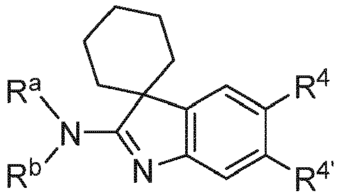 (VIc), 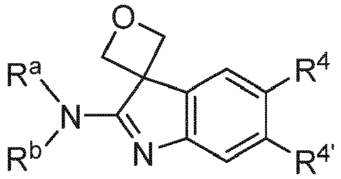 (VId),

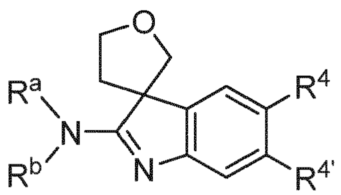 (VIe), 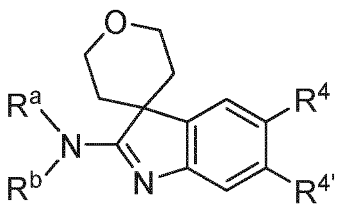 (VIf),

and tautomers thereof, and pharmaceutically acceptable salts of the compounds or the tautomers.

Subsets of the compounds of Formula (I) also include those of Formulae (VIIa), (VIIb), (VIIc), (VIId), (Vile), and (VIIf):

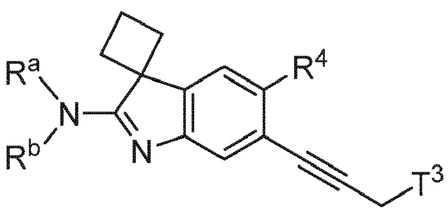 (VIIa), 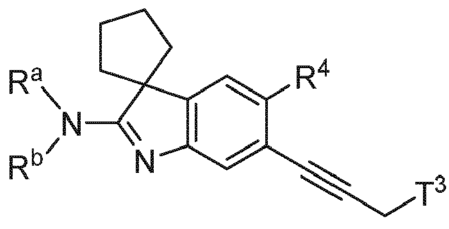 (VIIb),

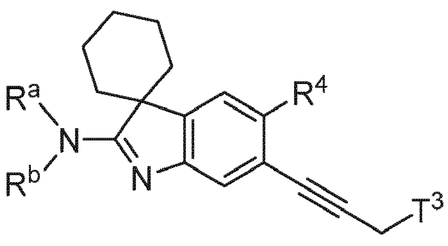 (VIIc), 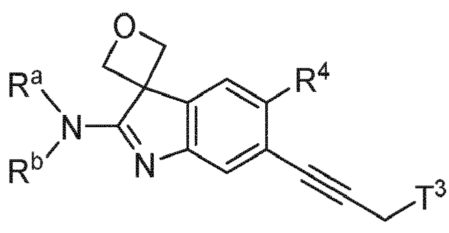 (VIId),

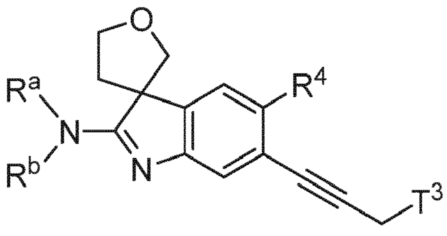 (VIIe), 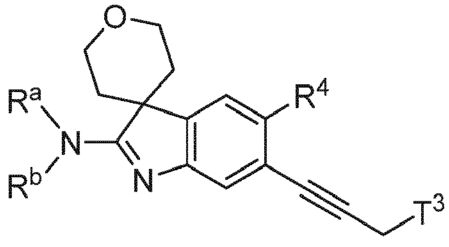 (VIIf),

and tautomers thereof, and pharmaceutically acceptable salts of the compounds or the tautomers.

Subsets of the compounds of Formula (I) also include those of Formulae (Villa), (VIIIb), (VIIIc), (VIIId), (VIIIe), and (VIIIf):

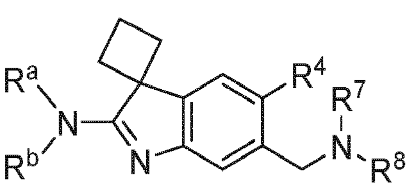 (VIIIa), 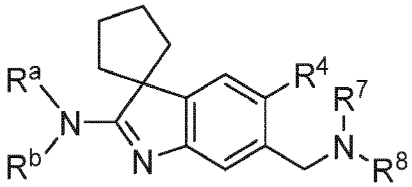 (VIIIb),

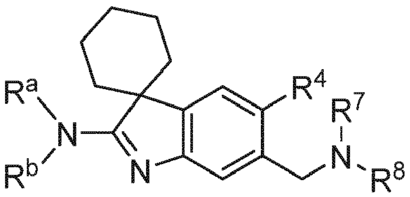 (VIIIc), 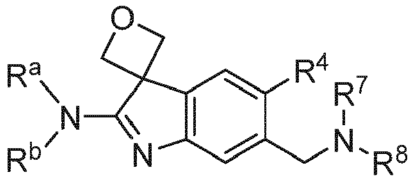 (VIIId),

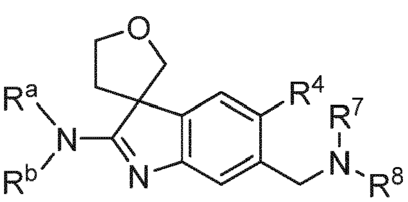 (VIIIe), 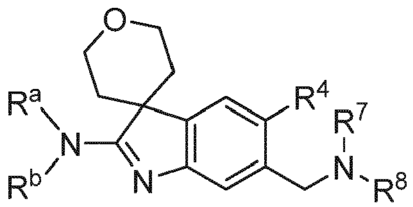 (VIIIf),

and tautomers thereof, and pharmaceutically acceptable salts of the compounds or the tautomers.

Subsets of the compounds of Formula (I) also include those of Formulae (IXa), (IXb), (IXc), (IXd), (IXe), and (IXf):

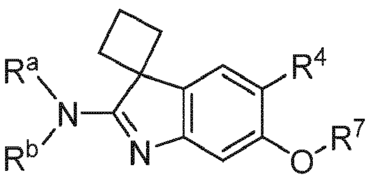 (IXa), 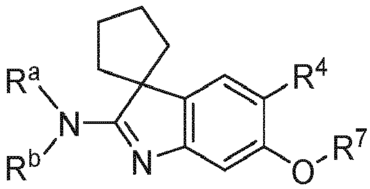 (IXb),

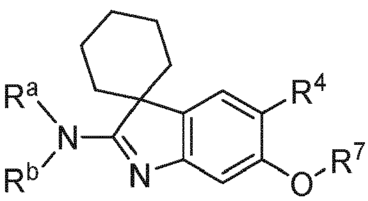 (IXc), 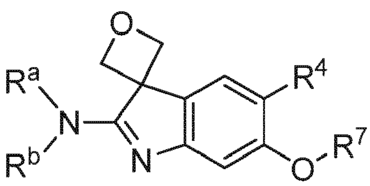 (IXd),

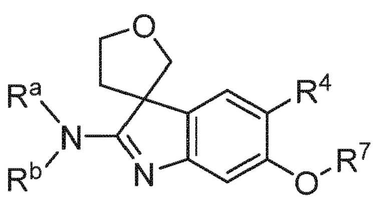 (IXe), 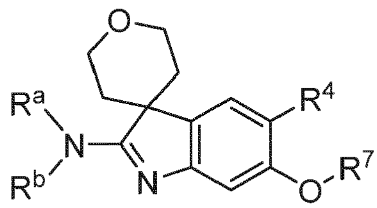 (IXf),

and tautomers thereof, and pharmaceutically acceptable salts of the compounds or the tautomers.

Subsets of the compounds of Formula (I) also include those of Formulae (Xa), (Xb), (Xc), (Xd), (Xe), and (Xf):

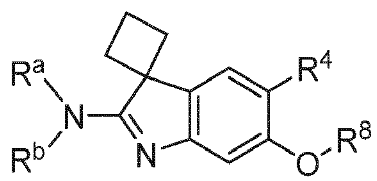 (Xa), 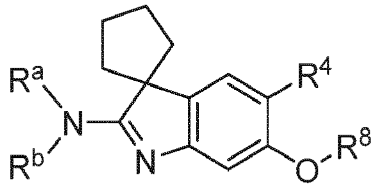 (Xb),

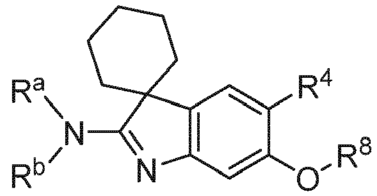 (Xc), 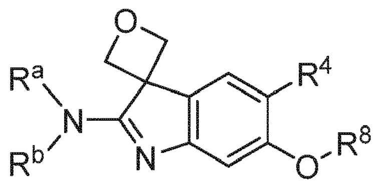 (Xd),

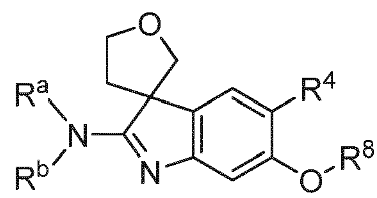 (Xe), 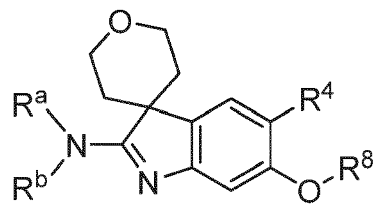 (Xf),

and tautomers thereof, and pharmaceutically acceptable salts of the compounds or the tautomers.

In certain embodiments, the compound is selected from the compounds of any of Formulae (I)-(Xf) that are selective inhibitors of EHMT2. For example, in some embodiments, the compound is selected from the compounds that inhibit EHMT2 with an enzyme inhibition $IC_{50}$ value of about 1 μM or less, about 500 nM or less, about 200 nM or less, about 100 nM or less, or about 50 nM or less.

In certain embodiments, the compound does not exhibit kinase inhibition activity, or exhibits only minimal kinase inhibition activity. For example, in some embodiments, the compound exhibits a kinase activity below measurable levels, or at a level corresponding to an $IC_{50}$ value that is not associated with significant enzyme inhibition.

In certain embodiments, the compound is selected from the compounds of any of Formulae (I)-(Xf) that inhibit a kinase with an enzyme inhibition $IC_{50}$ value of about 100 nM or greater, 1 μM or greater, 10 μM or greater, 100 μM or greater, or 1000 μM or greater.

In certain embodiments, the compound is selected from the compounds of any of Formulae (I)-(Xf) that inhibit a kinase with an enzyme inhibition $IC_{50}$ value of about 1 mM or greater.

In certain embodiments, the compound is selected from the compounds of any of Formulae (I)-(Xf) that inhibit a kinase with an enzyme inhibition $IC_{50}$ value of 1 μM or greater, 2 μM or greater, 5 μM or greater, or 10 μM or greater, wherein the kinase is one or more of the following: Abl, AurA, CHK1, MAP4K, IRAK4, JAK3, EphA2, FGFR3, KDR, Lck, MARK1, MNK2, PKCb2, SIK, and Src.

Also provided herein are pharmaceutical compositions comprising one or more pharmaceutically acceptable carriers and one or more compounds of any of the Formulae (I)-(Xf) described herein.

Another aspect of this disclosure is a method of preventing or treating an EHMT-mediated disorder. The method includes administering to a subject in need thereof a therapeutically effective amount of a compound of any of Formulae (I)-(Xf), or a tautomer thereof, or a pharmaceutically acceptable salt of the compound or the tautomer. The EHMT-mediated disorder is a disease, disorder, or condition that is mediated at least in part by the activity of EHMT1 or EHMT2

or both. In some embodiments, the EHMT-mediated disorder is a blood disease or disorder. In certain embodiments, the EHMT-mediated disorder is selected from proliferative disorders (e.g., cancers such as leukemia, hepatocellular carcinoma, prostate carcinoma, and lung cancer), addiction (e.g., cocaine addiction), and mental retardation.

Unless otherwise stated, any description of a method of treatment includes use of the compounds to provide such treatment or prophylaxis as is described herein, as well as use of the compounds to prepare a medicament to treat or prevent such condition. The treatment includes treatment of human or non-human animals including rodents and other disease models. Methods described herein may be used to identify suitable candidates for treating or preventing EHMT-mediated disorders. For example, the disclosure also provides methods of identifying an inhibitor of EHMT1 or EHMT2 or both.

For example, in some embodiments, the EHMT-mediated disease or disorder comprises a disorder that is associated with gene silencing by EHMT1 or EHMT2, e.g., blood diseases or disorders associated with gene silencing by EHMT2.

For example, in some embodiments, the method comprises the step of administering to a subject having a disease or disorder associated with gene silencing by EHMT1 or EHMT2 a therapeutically effective amount of one or more compounds of the Formulae described herein, wherein the compound(s) inhibits histone methyltransferase activity of EHMT1 or EHMT2, thereby treating the disease or disorder.

For example, in some embodiments, the blood disease or disorder is selected from the group consisting of sickle cell anemia and beta-thalassemia.

For example, in some embodiments, the blood disease or disorder is hematological cancer.

For example, in some embodiments, the hematological cancer is acute myeloid leukemia (AML) or chronic lymphocytic leukemia (CLL).

For example, in some embodiments, the method further comprises the steps of performing an assay to detect the degree of histone methylation by EHMT1 or EHMT2 in a sample comprising blood cells from a subject in need thereof.

In some embodiments, performing the assay to detect methylation of H3-K9 in the histone substrate comprises measuring incorporation of labeled methyl groups.

In some embodiments, the labeled methyl groups are isotopically labeled methyl groups.

In some embodiments, performing the assay to detect methylation of H3-K9 in the histone substrate comprises contacting the histone substrate with an antibody that binds specifically to dimethylated H3-K9.

Still another aspect of the disclosure is a method of inhibiting conversion of H3-K9 to dimethylated H3-K9. The method comprises the step of contacting a mutant EHMT, the wild-type EHMT, or both, with a histone substrate comprising H3-K9 and an effective amount of a compound of the present disclosure, wherein the compound inhibits histone methyltransferase activity of EHMT, thereby inhibiting conversion of H3-K9 to dimethylated H3-K9.

Further, the compounds or methods described herein can be used for research (e.g., studying epigenetic enzymes) and other non-therapeutic purposes.

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar or equivalent to those described herein can be used in the practice or testing of the present disclosure, suitable methods and materials are described below. All publications, patent applications, patents and other references mentioned herein are incorporated by reference. The references cited herein are not admitted to be prior art to the claimed invention. In the case of conflict, the present specification, including definitions, will control. In addition, the materials, methods and examples are illustrative only and are not intended to be limiting. In the case of conflict between the chemical structures and names of the compounds disclosed herein, the chemical structures will control.

Other features and advantages of the disclosure will be apparent from the following detailed description and claims.

## DETAILED DESCRIPTION

The present disclosure provides novel substituted fused bi- or tri- heterocyclic compounds, synthetic methods for making the compounds, pharmaceutical compositions containing them and various uses of the compounds.

In one aspect, the compounds disclosed herein may be used to treat a blood disorder, e.g., sickle-cell anemia (i.e., sickle-cell disease). Non-limiting examples of sickle-cell anemia forms that may be treated using the contemplated compounds include hemoglobin SS disease, hemoglobin SC disease, hemoglobin $S\beta^0$ thalassemia disease, hemoglobin $S\beta^+$ thalassemia disease, hemoglobin SD disease, and hemoglobin SE disease.

Without wishing to be bound by any theory, it is believed that sickle-cell anemia describes a group of inherited red blood cell disorders in which at least some of the red blood cells of a subject having sickle-cell anemia contain hemoglobin S ("HbS"). Hemoglobin S is a mutated, abnormal form of adult hemoglobin. Without wishing to be bound by any theory, it is believed that the contemplated compounds may treat sickle cell anemia by inducing fetal hemoglobin

("HbF") expression. *See, e.g.,* Renneville et al., Blood 126(16): 1930-1939, 2015, the content of which is incorporated herein by reference in its entirety.

In some embodiments, one or more complications of sickle-cell anemia may be treated or prevented using the contemplated compounds disclosed herein. Non-limiting examples of complications that may be treated or prevented using the contemplated compounds include anemia (e.g., severe anemia), hand-foot syndrome, splenic sequestration, delayed developmental growth, eye disorders (e.g., vision loss caused by, e.g., blockages in blood vessels supplying the eyes), skin ulcers (e.g., leg ulcers), heart disease, chest syndrome (e.g., acute chest syndrome), priapism, and pain.

The present disclosure provides compounds of Formula (I):

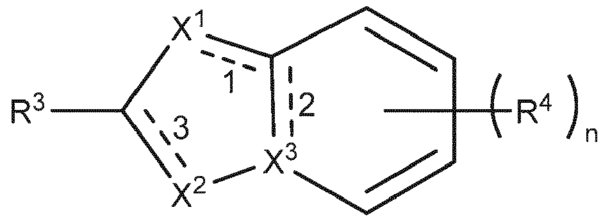

(I),

or a tautomer thereof, or a pharmaceutically acceptable salt of the compound or the tautomer, wherein

$X^1$ is O, S, $CR^1R^{11}$, or $NR^{1'}$ when $\overset{1}{-----}$ is a single bond, or $X^1$ is N when $\overset{1}{-----}$ is a double bond;

$X^2$ is N or $CR^2$ when $\overset{3}{-----}$ is a double bond, or $X^2$ is $NR^{2'}$ when $\overset{3}{-----}$ is a single bond;

$X^3$ is N or C; when $X^3$ is N, $\overset{1}{-----}$ is a double bond and $\overset{2}{-----}$ is a single bond, and when $X^3$ is C, $\overset{1}{-----}$ is a single bond and $\overset{2}{-----}$ is a double bond;

each of $R^1$, $R^2$ and $R^{11}$, independently, is $-Q^1-T^1$, in which each $Q^1$ independently is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and each $T^1$ independently is H, halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$, $-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, $-SO_2N(R^5)_2$, $-NR^5C(O)R^6$,amino, mono- or di- alkylamino, or $C_1$-$C_6$ alkoxyl; or

$R^1$ and $R^{11}$ together with the carbon atom to which they are attached form a $C_3$-$C_{12}$ cycloalkyl or 4- to 12-membered heterocycloalkyl containing 1-4 heteroatoms selected from N, O, and S, wherein the $C_3$-$C_{12}$ cycloalkyl or 4- to 12-membered heterocycloalkyl is optionally substituted with one or more of halo, $C_1$-$C_6$ alkyl, hydroxyl, oxo, amino, mono- or di- alkylamino, or $C_1$-$C_6$ alkoxyl;

each of $R^{1'}$ and $R^{2'}$, independently, is $-Q^2-T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S2}$ is optionally substituted with one or more of halo, Ci-$C_6$ alkyl, hydroxyl, oxo, $-C(O)R^6$, $-SO_2R^5$, $-SO_2N(R^5)_2$, $-NR^5C(O)R^6$,amino, mono- or di-alkylamino, or $C_1$-$C_6$ alkoxyl;

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, $C_3$-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively;

$R^3$ and one of $R^{1'}$, $R^{2'}$, $R^1$, $R^2$ and $R^{11}$, together with the atoms to which they are attached, form a 5- or 6-membered heteroaryl that is optionally substituted with one or more of halo, $C_1$-$C_3$ alkyl, hydroxyl or $C_1$-$C_3$alkoxyl; or

$R^3$ is oxo and $\overset{3}{-----}$ is a single bond;

each $R^4$ independently is $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di-

alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl;

$R^8$ is $-Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5$-$T^5$, wherein each $Q^5$ independently is a bond or $C_1$-$C_3$alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5$-$T^5$ is oxo; and

n is 1, 2, 3, or 4.

In some embodiments, the compound is not

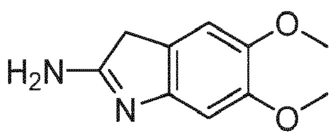,
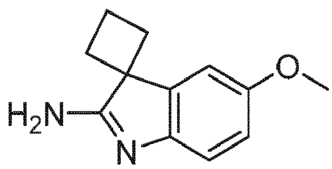,
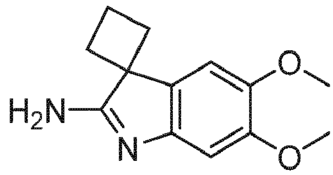,

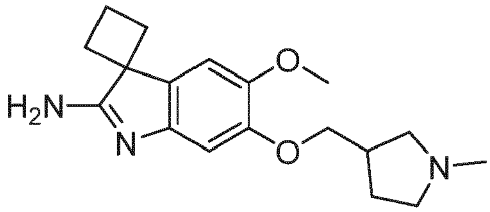,
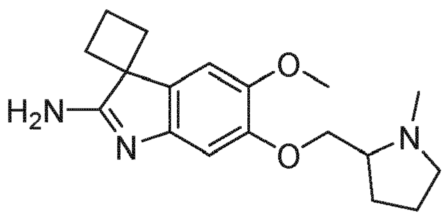,

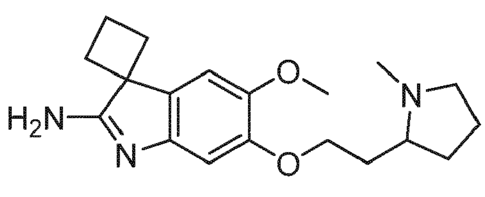,
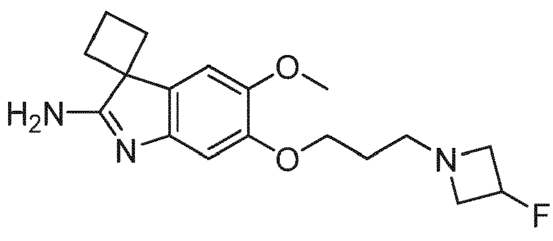,

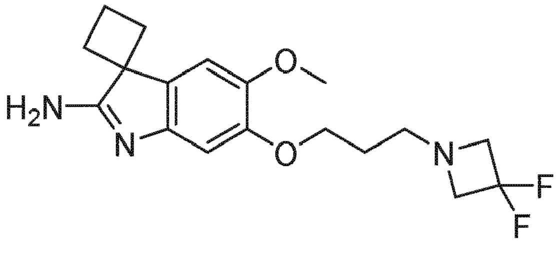,
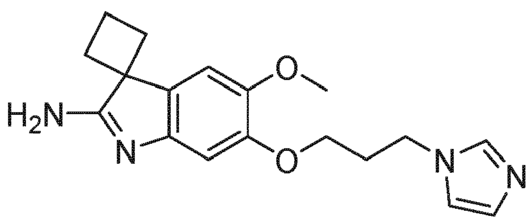,

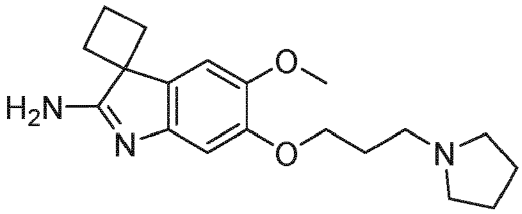
,
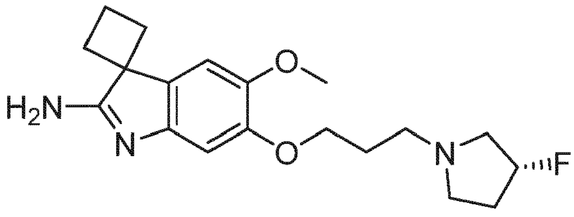
,

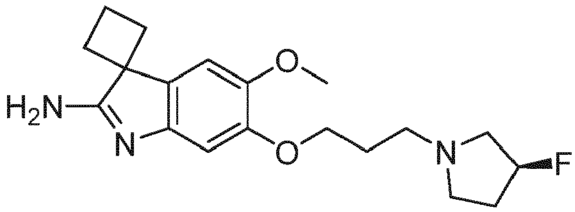
,
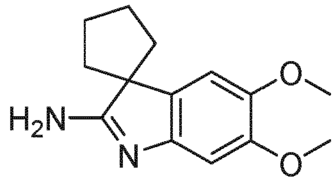
,

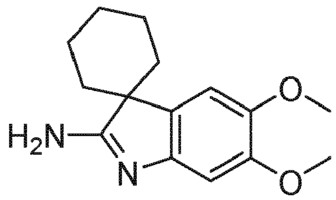
,
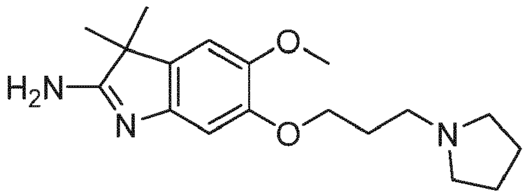
, or

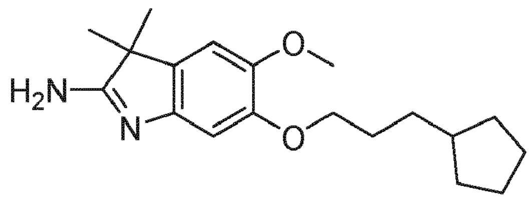
.

In some embodiments, when n is 2, $X^1$ is $CR^1R^{11}$, $X^2$ is N , $X^3$ is C, $R^3$ is $NH_2$, and at least one $R^4$ is $OR^7$, then one of (1)-(4) below applies:

(1) at least one of $R^1$ and $R^{11}$ is $-Q^1-T^1$, in which $Q^1$ is a $C_1$-$C_6$ alkylene linker optionally substituted with one or more of halo, cyano, hydroxyl, or $C_1$-$C_6$ alkoxyl, and $T^1$ is cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$,$-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, $-SO_2N(R^5)_2$, - $NR^5C(O)R^6$, amino, mono- or di- alkylamino, or $C_1$-$C_6$ alkoxyl; or

(2) at least one of $R^1$ and $R^{11}$ is $-Q^1-T^1$, in which $Q^1$ is a $C_2$-$C_6$ alkenylene or $C_2$-$C_6$ alkynylene linker optionally substituted with one or more of halo, cyano, hydroxyl, or $C_1$-$C_6$ alkoxyl, and $T^1$ is H, halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$,$-NR^5C(O)OR^6$, $OR^5$, or $R^{S1}$, in which $R^{S1}$ is $C_3$-$C_{12}$ cycloalkyl, phenyl, 4-to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, $-SO_2N(R^5)_2$, $-NR^5C(O)R^6$,amino, mono- or di- alkylamino, or $C_1$-$C_6$ alkoxyl; or

(3) at least one of $R^1$ and $R^{11}$ is $-Q^1-T^1$, in which $Q^1$ is a bond, and $T^1$ is halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$,$-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, - $SO_2N(R^5)_2$, $-NR^5C(O)R^6$,amino, mono- or di- alkylamino, or $C_1$-$C_6$ alkoxyl; or

(4) $R^1$ and $R^{11}$ together with the carbon atom to which they are attached form a $C_7$-$C_{12}$ cycloalkyl or 4- to 12-membered heterocycloalkyl containing 1-4 heteroatoms selected from N, O, and S, wherein the $C_7$-$C_{12}$ cycloalkyl or 4- to 12-membered heterocycloalkyl is optionally substituted with one or more of halo, $C_1$-$C_6$ alkyl, hydroxyl, oxo, amino, mono- or di- alkylamino, or $C_1$-$C_6$ alkoxyl.

For example, in some embodiments, at least one of $X^2$ and $X^3$ is N.

For example, in some embodiments, at least two of $X^1$, $X^2$, and $X^3$ comprise N.

For example, in some embodiments, at least one of $\overset{1}{-----}$ , $\overset{2}{-----}$ and $\overset{3}{-----}$ is a double bond.

For example, in some embodiments, $\overset{3}{-----}$ is a double bond.

For example, in some embodiments, $\overset{3}{-----}$ is a single bond.

For example, in some embodiments, $X^2$ is $NR^{2'}$ and $R^3$ is oxo.

For example, in some embodiments, $X^2$ is N and $X^3$ is C.

For example, in some embodiments, $X^2$ is $CR^2$ and $X^3$ is N.

For example, in some embodiments, $X^1$ is S.

For example, in some embodiments, $X^1$ is $NR^{1'}$.

For example, in some embodiments, $X^1$ is $CR^1R^{11}$.

For example, in some embodiments, $R^1$ and $R^{11}$ together with the carbon atom to which they are attached form a 4- to 7-membered heterocycloalkyl containing 1-4 heteroatoms selected from N, O, and S, wherein the 4- to 7-membered heterocycloalkyl is optionally substituted with one or more of halo, $C_1$-$C_6$ alkyl, hydroxyl, oxo, amino, mono- or di-alkylamino, or $C_1$-$C_6$ alkoxyl.

For example, in some embodiments, n is 1 or 2.

For example, in some embodiments, n is 2.

For example, in some embodiments, the compounds of Formula (I) include those of any of Formulae (IIa), (IIb), (IIc), (IId), (IIe), (IIIa), (IIIb), (IIIc), (IIId), (IIIe), (IIIf), (IVa), and (IVb) :

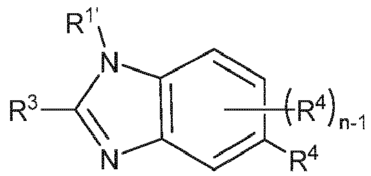 (IIa), 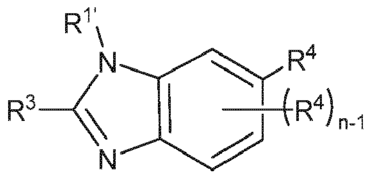 (IIb),

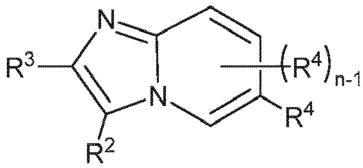 (IIc), 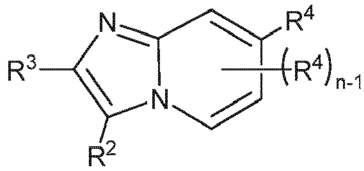 (IId), 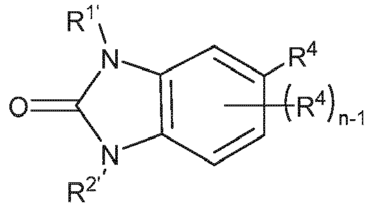

(IIe),

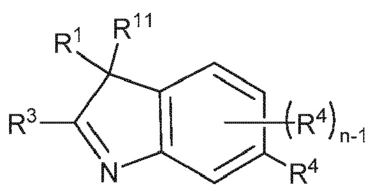 (IIIa), 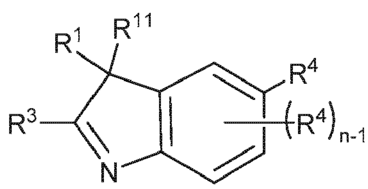 (IIIb),

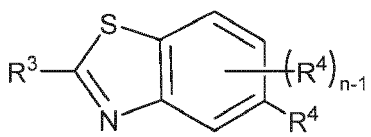 (IIIc), 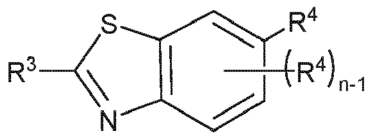 (IIId),

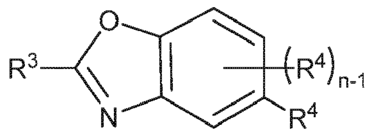 (IIIe), 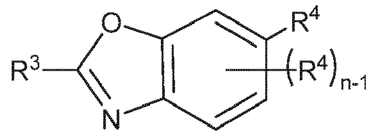 (IIIf),

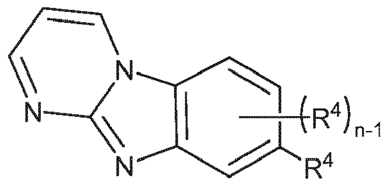 (IVa), or 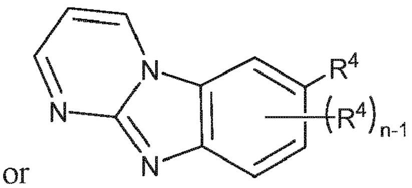 (IVb),

and tautomers thereof, and pharmaceutically acceptable salts of the compounds or the tautomers.

In some embodiments, the compounds of Formula (I) include those of any of Formulae (IIf), (IIg), (IIh), (IIIi), (IIIj), (IIIk), and (IIIl):

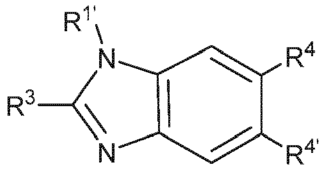 (IIf), 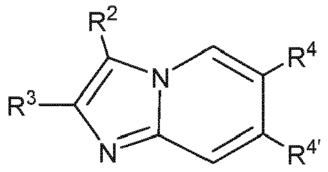 (IIg), 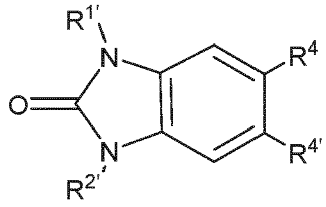 (IIh),

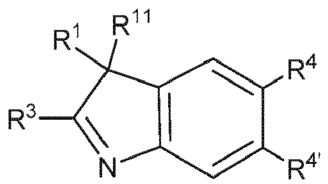 (IIIi), 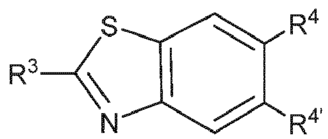 (IIIj),

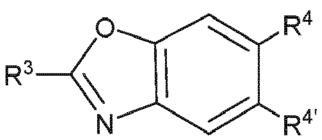 (IIIk), or 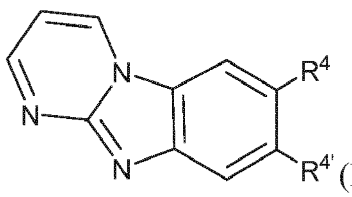 (IIIl),

and tautomers thereof, and pharmaceutically acceptable salts of the compounds or the tautomers, wherein

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, $C_3$-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S;

each of $R^4$ and $R^{4'}$ independently is $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-

membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl;

$R^8$ is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

For example, in some embodiments, the compounds of Formula (I) do not include those described in EP 0356234; US 5,106,862; US 6,025,379; US 9,284,272; WO2002/059088; and/or WO2015/200329.

For example, in some embodiments, when n is 2, $X^1$ is $CR^1R^{11}$, $X^2$ is N, $X^3$ is C, $R^3$ is $NH_2$, and at least one $R^4$ is $OR^7$, then at least one of $R^1$ and $R^{11}$ is -$Q^1$-$T^1$, in which $Q^1$ is a $C_1$-$C_6$ alkylene linker optionally substituted with one or more of halo, cyano, hydroxyl, or $C_1$-$C_6$ alkoxyl, and $T^1$ is cyano, $NR^5R^6$, $C(O)NR^5R^6$, -$OC(O)NR^5R^6$, $C(O)OR^5$, -$OC(O)R^5$, $C(O)R^5$, -$NR^5C(O)R^6$, -$NR^5C(O)OR^6$, $OR^5$, or $R^{S1}$, in which $R^{S1}$ is $C_3$-$C_{12}$ cycloalkyl, phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, -$C(O)R^6$, -$SO_2R^5$, -$SO_2N(R^5)_2$, -$NR^5C(O)R^6$,amino, mono- or di- alkylamino, or $C_1$-$C_6$ alkoxyl.

For example, in some embodiments, when n is 2, $X^1$ is $CR^1R^{11}$, $X^2$ is N, $X^3$ is C, $R^3$ is $NH_2$, and at least one $R^4$ is $OR^7$, then at least one of $R^1$ and $R^{11}$ is -$Q^1$-$T^1$, in which $Q^1$ is a $C_2$-$C_6$ alkenylene or $C_2$-$C_6$ alkynylene linker optionally substituted with one or more of halo, cyano, hydroxyl, or $C_1$-$C_6$ alkoxyl, and $T^1$ is H, halo, cyano, $NR^5R^6$, $C(O)NR^5R^6$, -$OC(O)NR^5R^6$, $C(O)OR^5$, -$OC(O)R^5$, $C(O)R^5$, -$NR^5C(O)R^6$,-$NR^5C(O)OR^6$, $OR^5$, or $R^{S1}$, in which $R^{S1}$ is $C_3$-$C_{12}$ cycloalkyl, phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, -$C(O)R^6$, -$SO_2R^5$, - $SO_2N(R^5)_2$, -$NR^5C(O)R^6$,amino, mono- or di- alkylamino, or $C_1$-$C_6$ alkoxyl.

For example, in some embodiments, when n is 2, $X^1$ is $CR^1R^{11}$, $X^2$ is N, $X^3$ is C, $R^3$ is NH2, and at least one $R^4$ is $OR^7$, then at least one of $R^1$ and $R^{11}$ is -$Q^1$-$T^1$, in which $Q^1$ is a bond, and $T^1$ is halo, cyano, $NR^5R^6$, $C(O)NR^5R^6$, -$OC(O)NR^5R^6$, $C(O)OR^5$, -$OC(O)R^5$, $C(O)R^5$, - $NR^5C(O)R^6$, -$NR^5C(O)OR^6$, $OR^5$, or $R^{S1}$, in which $R^{S1}$ is $C_3$-$C_{12}$ cycloalkyl, phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, -$C(O)R^6$, -$SO_2R^5$, -$SO_2N(R^5)_2$, -$NR^5C(O)R^6$, amino, mono- or di- alkylamino, or $C_1$-$C_6$ alkoxyl.

For example, in some embodiments, when n is 2, $X^1$ is $CR^1R^{11}$, $X^2$ is N, $X^3$ is C, $R^3$ is $NH_2$, and at least one $R^4$ is $OR^7$, then $R^1$ and $R^{11}$ together with the carbon atom to which they are attached form a $C_7$-$C_{12}$ cycloalkyl or 4- to 12-membered heterocycloalkyl (e.g., 4- to 7-membered heterocycloalkyl) containing 1-4 heteroatoms selected from N, O, and S, wherein the $C_7$-$C_{12}$ cycloalkyl or 4- to 12-membered heterocycloalkyl (e.g., 4- to 7-membered heterocycloalkyl) is optionally substituted with one or more of halo, $C_1$-$C_6$ alkyl, hydroxyl, oxo, amino, mono- or dialkylamino, or $C_1$-$C_6$ alkoxyl.

For example, in some embodiments, $R^2$ is -$Q^1$-$T^1$, in which $Q^1$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^1$ is H, halo, cyano, or $R^{S1}$, in which $R^{S1}$ is $C_3$-$C_{12}$ cycloalkyl (e.g., $C_3$-$C_8$ cycloalkyl), phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, amino, mono- or di- alkylamino, or $C_1$-$C_6$ alkoxyl.

For example, in some embodiments, $R^2$ is $C_1$-$C_6$ alkyl optionally substituted with one or more of halo, cyano, hydroxyl, or $C_1$-$C_6$ alkoxyl. For example, $R^2$ is unsubstituted $C_1$-$C_6$ alkyl.

For example, in some embodiments, $Q^1$ is a bond or $C_1$-$C_6$ alkylene linker optionally substituted with one or more of halo, cyano, hydroxyl, or $C_1$-$C_6$ alkoxyl, and $T^1$ is H, halo, cyano, or $R^{S1}$, in which $R^{S1}$ is $C_3$-$C_{12}$ cycloalkyl (e.g., $C_3$-$C_8$ cycloalkyl), phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S1}$ is optionally substituted with one or

more of halo, $C_1$-$C_6$ alkyl, hydroxyl, oxo, amino, mono- or di- alkylamino, or $C_1$-$C_6$ alkoxyl.

For example, in some embodiments, $Q^1$ is a $C_2$-$C_6$ alkenylene or $C_2$-$C_6$ alkynylene linker optionally substituted with one or more of halo, cyano, hydroxyl, or $C_1$-$C_6$ alkoxyl, and $T^1$ is H, halo, cyano, or $R^{S1}$, in which $R^{S1}$ is $C_3$-$C_{12}$ cycloalkyl *(e.g.,* $C_3$-$C_8$ cycloalkyl), phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, amino, mono- or di- alkylamino, or $C_1$-$C_6$ alkoxyl.

For example, in some embodiments, $R^{1'}$ is -$Q^2$-$T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_{12}$ cycloalkyl (e.g., $C_3$-$C_8$ cycloalkyl), phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S2}$ is optionally substituted with one or more of halo, $C_1$-$C_6$ alkyl, hydroxyl, oxo, amino, mono- or di- alkylamino, or $C_1$-$C_6$ alkoxyl.

For example, in some embodiments, $R^{2'}$ is -$Q^2$-$T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_{12}$ cycloalkyl (e.g., $C_3$-$C_8$ cycloalkyl), phenyl, 4- to 12-membered heterocycloalkyl (e.g., 4- to 7-membered heterocycloalkyl) containing 1-4 heteroatoms selected from N, O, and S, or a 5- or 6-membered heteroaryl and $R^{S2}$ is optionally substituted with one or more of halo, $C_1$-$C_6$ alkyl, hydroxyl, oxo, amino, mono- or di- alkylamino, or $C_1$-$C_6$ alkoxyl.

For example, in some embodiments, each $Q^2$ independently is a bond or $C_1$-$C_6$ alkylene linker optionally substituted with one or more of halo and each $T^2$ independently is H, halo, $C_3$-$C_{12}$ cycloalkyl *(e.g.,* $C_3$-$C_8$ cycloalkyl), or a 4- to 7-membered heterocycloalkyl.

For example, in some embodiments, each $Q^2$ independently is $C_2$-$C_6$ alkenylene or $C_2$-$C_6$ alkynylene linker optionally substituted with one or more of halo, cyano, hydroxyl, or $C_1$-$C_6$ alkoxyl.

For example, in some embodiments, $R^{2'}$ is H or $C_1$-$C_6$ alkyl.

For example, in some embodiments, $R^3$ is H.

For example, in some embodiments, $R^3$ is $NR^aR^b$ or $OR^a$, wherein each of $R^a$ and $R^b$ independently is H or $C_1$-$C_6$ alkyl optionally substituted with one or more of halo, hydroxyl, CN, amino, mono- or di- alkylamino, or $C_1$-$C_6$ alkoxyl.

For example, in some embodiments, $R^3$ is $NR^aR^b$ or $OR^a$, wherein each of $R^a$ and $R^b$ independently is H or $C_1$-$C_6$ alkyl optionally substituted with one or more of halo, hydroxyl, amino, mono- or di- alkylamino, $C_1$-$C_6$ alkoxyl, $C_3$-$C_{12}$ cycloalkyl, phenyl, 5- or 6-membered heteroaryl, or 4- to 12-membered heterocycloalkyl (e.g., 4- to 7-membered heterocycloalkyl) containing 1-4 heteroatoms selected from N, O, and S.

For example, in some embodiments, $R^3$ is $NR^aR^b$.

For example, in some embodiments, each of $R^a$ and $R^b$ independently is H or $R^{S5}$.

For example, in some embodiments, one of $R^a$ and $R^b$ is H and the other is $R^{S5}$.

For example, in some embodiments, $R^a$ and $R^b$ together with the nitrogen atom to which they are attached form a 4- to 12-membered heterocycloalkyl (e.g., 4- to 7-membered heterocycloalkyl), which is optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl (e.g., 4- to 7-membered heterocycloalkyl).

For example, in some embodiments, $R^a$ and $R^b$ together with the nitrogen atom to which they are attached form a 4- to 12-membered heterocycloalkyl (e.g., 4- to 7-membered heterocycloalkyl), which is optionally substituted with one or more of halo, hydroxyl, oxo, CN, amino, mono- or di- alkylamino, $C_1$-$C_6$ alkyl, or $C_1$-$C_6$ alkoxyl.

For example, in some embodiments, $R^{S5}$ is $C_1$-$C_6$ alkyl, and $R^{S5}$ is optionally substituted with one or more of halo, hydroxyl, CN, amino, mono- or di- alkylamino, $C_1$-$C_6$ alkoxyl, $C_3$-$C_{12}$ cycloalkyl, phenyl, 5- or 6-membered heteroaryl, or 4- to 12-membered heterocycloalkyl (e.g., 4-to 7-membered heterocycloalkyl).

For example, in some embodiments, $R^{S5}$ is phenyl, 5- or 6-membered heteroaryl, or 4- to 12-membered heterocycloalkyl (e.g., 4- to 7-membered heterocycloalkyl), and $R^{S5}$ is optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl (e.g., 4- to 7-membered heterocycloalkyl).

In some embodiments, the compounds of Formula (I) include those of any of Formulae (Va), (Vb), (Vc), (Vd), (Ve), and (Vf):

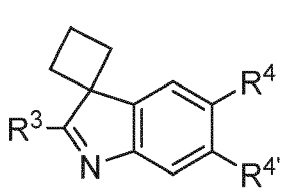 (Va), 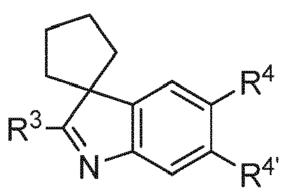 (Vb), 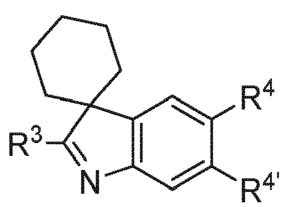 (Vc),

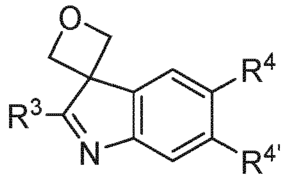 (Vd), 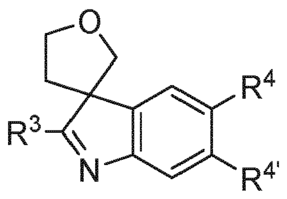 (Ve), 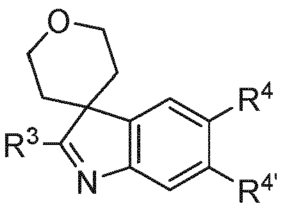 (Vf),

and tautomers thereof, and pharmaceutically acceptable salts of the compounds or the tautomers, wherein

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, $C_3$-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S;

each of $R^4$ and $R^{4'}$ independently is -$Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

$R^8$ is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

In some embodiments, when $R^3$ is -$NH_2$, then $R^4$ is not -$OCH_3$.

In some embodiments, when $R^3$ is -$NH_2$, and $R^4$ is not -$OCH_3$, then $R^{4'}$ is not $OR^8$.

For example, in some embodiments, $R^3$ is $C_1$-$C_6$ alkyl, $C_2$-$C_6$ alkenyl, or $C_2$-$C_6$ alkynyl, each of which is optionally substituted with one or more of halo, hydroxyl, oxo, CN, amino, mono- or di- alkylamino, $C_1$-$C_6$ alkoxyl, $C_3$-$C_{12}$ cycloalkyl, phenyl, 5- or 6-membered heteroaryl, or 4- to 12-membered heterocycloalkyl (e.g., 4- to 7-membered heterocycloalkyl) containing 1-4 heteroatoms selected from N, O, and S; in which each of the $C_3$-$C_{12}$ cycloalkyl, phenyl, 5- or 6-membered heteroaryl, and 4- to 12-membered heterocycloalkyl (e.g., 4- to 7-membered heterocycloalkyl) is independently optionally substituted with one or more of halo, hydroxyl, oxo, CN, amino, mono- or di- alkylamino, $C_1$-$C_6$ alkyl, or $C_1$-$C_6$ alkoxyl.

For example, in some embodiments, $R^3$ is $C_3$-$C_{12}$ cycloalkyl or 4- to 12-membered heterocycloalkyl (e.g., 4- to 7-membered heterocycloalkyl) containing 1-4 heteroatoms selected from N, O, and S, wherein each of the $C_3$-$C_{12}$ cycloalkyl and 4- to 12-membered heterocycloalkyl (e.g., 4- to 7-membered heterocycloalkyl) is independently optionally substituted with one or more of halo, hydroxyl, oxo, CN, amino, mono- or di- alkylamino, $C_1$-$C_6$ alkyl, or $C_1$-$C_6$ alkoxyl.

For example, in some embodiments, $R^3$ is

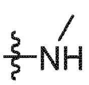, 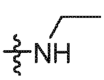, 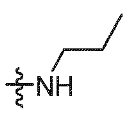, 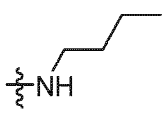,

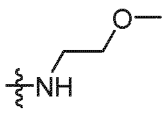, 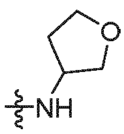, 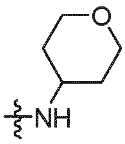, 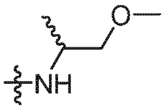, 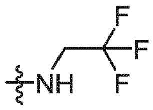, 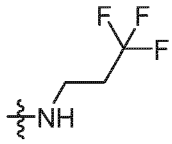, 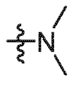, 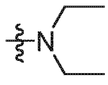,

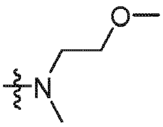, 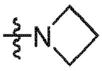, 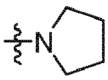, 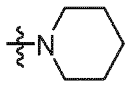, 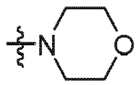, 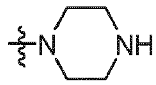, 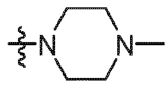, 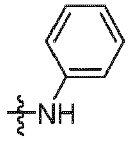,

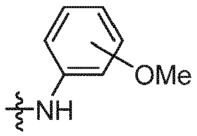, 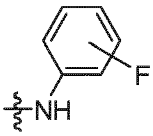, 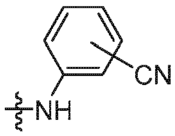, 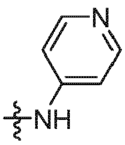, 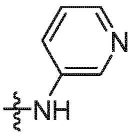, 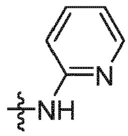, 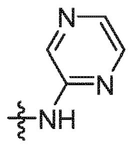,

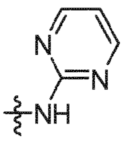, 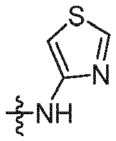, 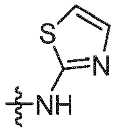, 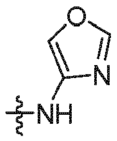, 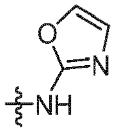, 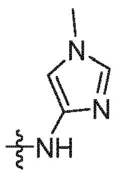, 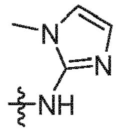, 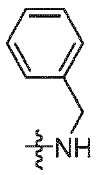, 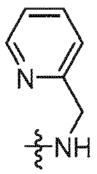, 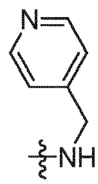,

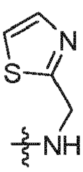, or 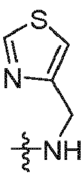.

For example, in some embodiments, $R^3$ is $NH_2$.

For example, in some embodiments, $R^3$ is $NR^aR^b$, in which one of $R^a$ and $R^b$ is H and the other is $C_1$-$C_6$ alkyl optionally substituted with one or more of halo or $C_1$-$C_6$ alkoxyl.

For example, in some embodiments, $R^3$ is oxo and $\overset{3}{----}$ is a single bond.

For example, in some embodiments, $R^3$ is OH.

For example, in some embodiments, $R^3$ is $C_1$-$C_6$ alkoxyl.

For example, in some embodiments, $R^3$ and one of $R^{1'}$, $R^{2'}$, $R^1$, $R^2$ and $R^{11}$, together with the atoms to which they are attached, form a 6-membered heteroaryl that is optionally substituted with one or more of halo, $C_1$-$C_3$ alkyl, hydroxyl or $C_1$-$C_3$ alkoxyl.

For example, in some embodiments, $R^3$ and one of $R^{1'}$, $R^{2'}$, $R^1$, $R^2$ and $R^{11}$, together with the atoms to which they are attached, form a 5-membered heteroaryl that is optionally substituted with one or more of halo, $C_1$-$C_3$ alkyl, hydroxyl or $C_1$-$C_3$ alkoxyl.

In some embodiments, the compounds of Formula (I) include those of any of Formulae (VIa), (VIb), (VIc), (VId), (VIe), and (VIf):

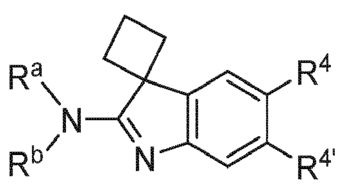 (VIa), 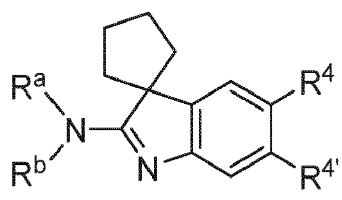 (VIb),

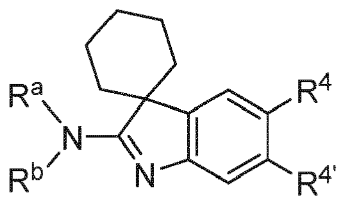 (VIc), 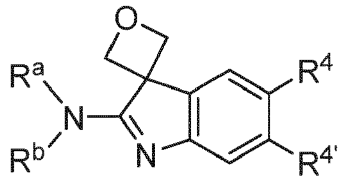 (VId),

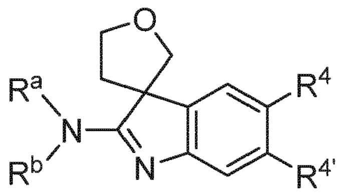 (VIe), 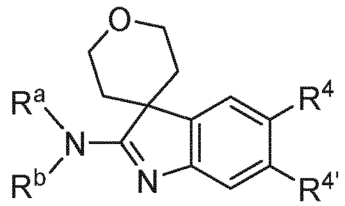 (VIf),

and tautomers thereof, and pharmaceutically acceptable salts of the compounds or the tautomers, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

each of $R^4$ and $R^{4'}$ independently is -$Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

$R^8$ is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

In some embodiments, at least one of $R^a$ and $R^b$ is $R^{S5}$.

In some embodiments, when both of $R^a$ and $R^b$ are H, then $R^4$ is not

In some embodiments, when both of $R^a$ and $R^b$ are H, and $R^4$ is -$OCH_3$, then $R^{4'}$ is not $OR^8$.

For example, in some embodiments, each of $R^4$ and $R^{4'}$ is independently -$Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, $OR^7$, $OR^8$, $NR^7R^8$, $C_6$-$C_{10}$ aryl, 5- to 10-membered heteroaryl, $C_3$-$C_{12}$ cycloalkyl, or 4- to 12-membered heterocycloalkyl.

In some embodiments, $R^4$ is -$Q^3$-$T^3$, in which $Q^3$ is a bond or $C_1$-$C_6$ alkylene linker, and $T^3$ is H, halo, $OR^7$, $C_6$-$C_{10}$ aryl, or 5- to 10-membered heteroaryl.

In some embodiments, $R^{4'}$ is -$Q^3$-$T^3$, in which $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, $OR^7$, $OR^8$, $NR^7R^8$, $C_3$-$C_{12}$ cycloalkyl, or 4- to 12-membered heterocycloalkyl.

In some embodiments, at least one of $R^4$ and $R^{4'}$ is $C_1$-$C_6$ alkyl. In some embodiments, $R^4$ is $C_1$-$C_6$ alkyl.

In some embodiments, at least one of $R^4$ and $R^{4'}$ is $CH_3$. In some embodiments, $R^4$ is $CH_3$.

In some embodiments, at least one of $R^4$ and $R^{4'}$ is halo. In some embodiments, $R^4$ is halo.

In some embodiments, at least one of $R^4$ and $R^{4'}$ is F or Cl. In some embodiments, $R^4$ is F or Cl.

In some embodiments, at least one of $R^4$ and $R^{4'}$ is $C_6$-$C_{10}$ aryl. In some embodiments, $R^4$ is $C_6$-$C_{10}$ aryl.

In some embodiments, at least one of $R^4$ and $R^{4'}$ is

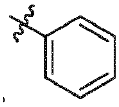 .

In some embodiments, $R^4$ is

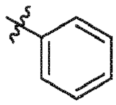 .

In some embodiments, at least one of $R^4$ and $R^{4'}$ is 5- to 10-membered heteroaryl. For example, $R^4$ is 5- to 10-membered heteroaryl.

In some embodiments, at least one of $R^4$ and $R^{4'}$ is

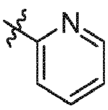 , 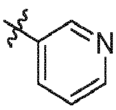 , or 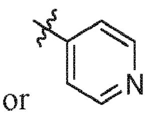 .

For example, in some embodiments, $R^4$ is

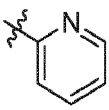 , 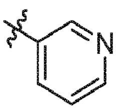 , or 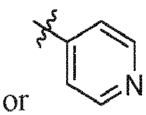 .

In some embodiments, at least one of $R^4$ and $R^{4'}$ is

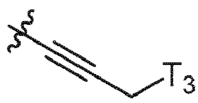 ,

wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$.

In some embodiments, $R^{4'}$ is

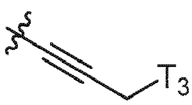 ,

wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$.

In some embodiments, at least one of $R^4$ and $R^{4'}$ is

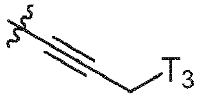,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

In some embodiments, $R^{4'}$ is

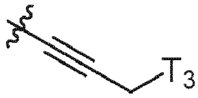,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

In some embodiments, at least one of $R^4$ and $R^{4'}$ is

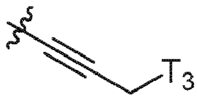,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl and the other of $R^4$ and $R^{4'}$ is halo, $C_1$-$C_6$ alkyl, or $OR^7$. For example, in some embodiments, $R^7$ is H or $C_1$-$C_6$ alkyl optionally substituted with one or more of hydroxyl, amino or mono- or di-alkylamino.

For example, in some embodiments, at least one of $R^4$ and $R^{4'}$ is -OCH3, -$OCH_2CH_3$, or - $OCH(CH_3)_2$. In some embodiments, at least one of $R^4$ and $R^{4'}$ is

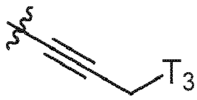,

wherein $T^3$ is 5-to 10-membered heteroaryl or 4- to 12-membered heterocycloalkyl optionally substituted with one or more of halo, hydroxyl, $C_1$-$C_6$ alkoxyl or $C_1$-$C_6$ alkyl and the other of $R^4$ and $R^{4'}$ is $OCH_3$, -$OCH_2CH_3$, or -$OCH(CH_3)_2$.

For example, in some embodiments, at least one of $R^4$ and $R^{4'}$ is

For example, in some embodiments, at least one of $R^4$ and $R^{4'}$ is

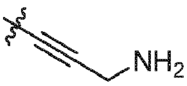,

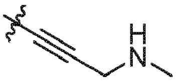, 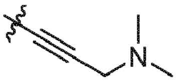, 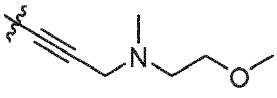, 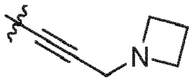, 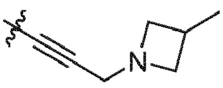,

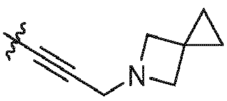, 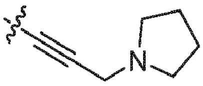, 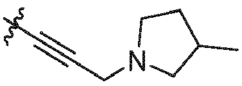, 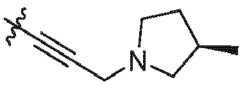, 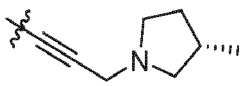,

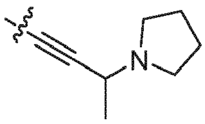, 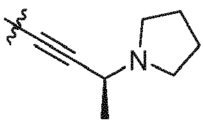, 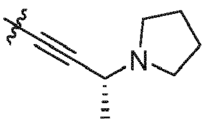, 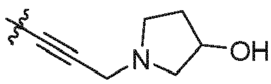, 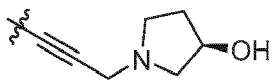,

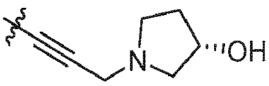, 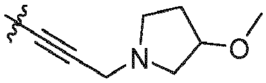, 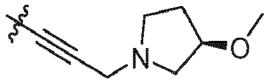, 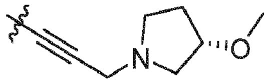,

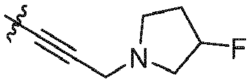, 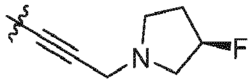, 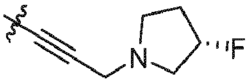, 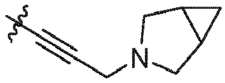,

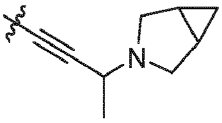, 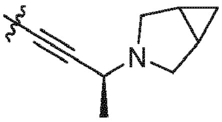, 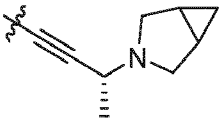, 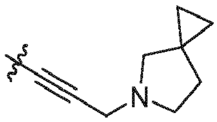, 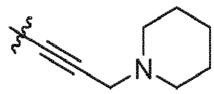,

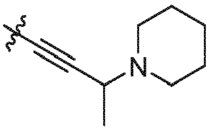, 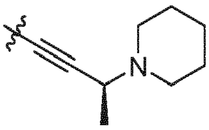, 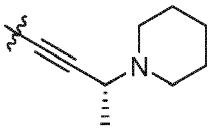, 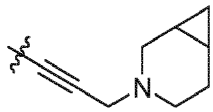, 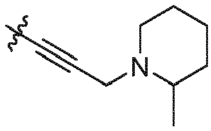,

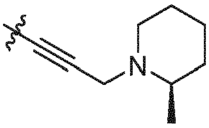, 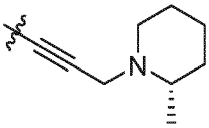, 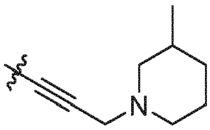, 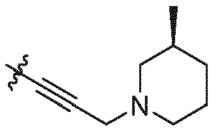, 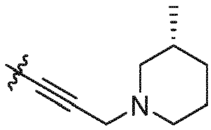,

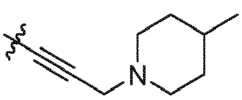, 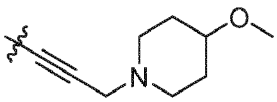, 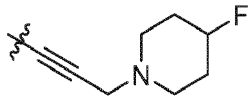, 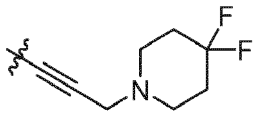,

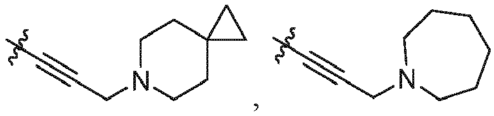, .

For example, in some embodiments, $R^{4'}$ is

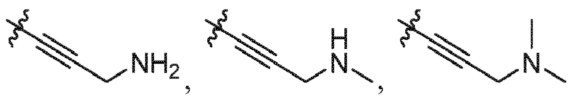, , ,

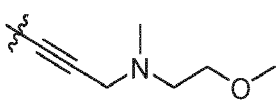, 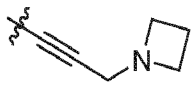, 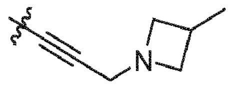, 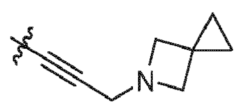, 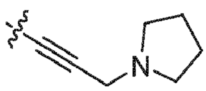,

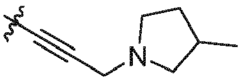, 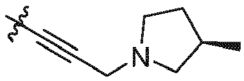, 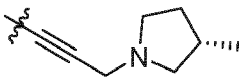, 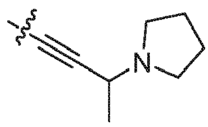, 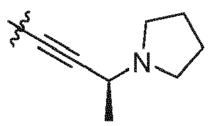,

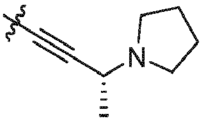, 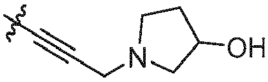, 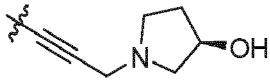, 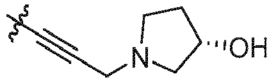,

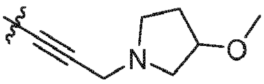, 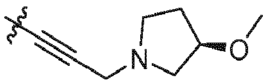, 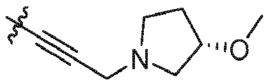, 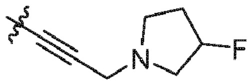,

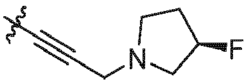, 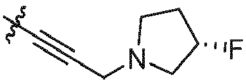, 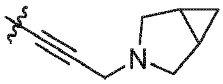, 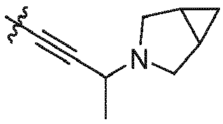, 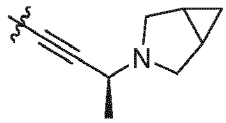,

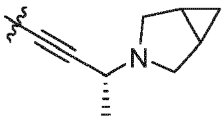, 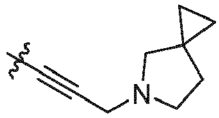, 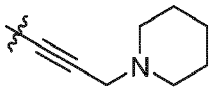, 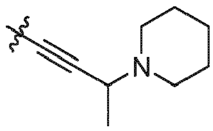, 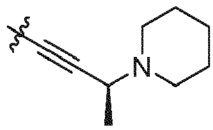,

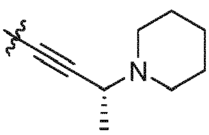, 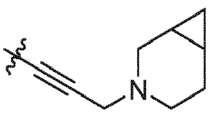, 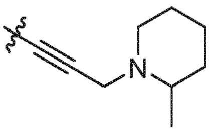, 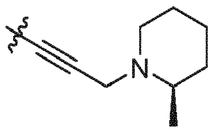, 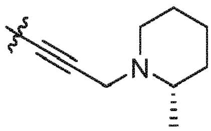,

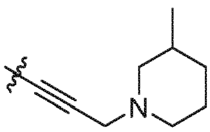, 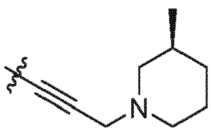, 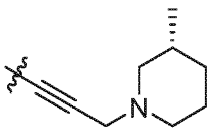, 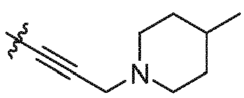, 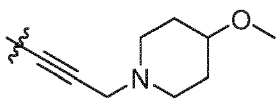,

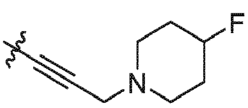, 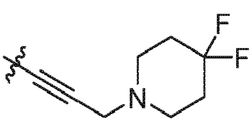, 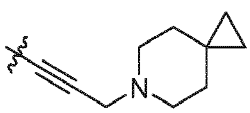, 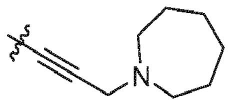.

For example, in some embodiments, at least one of $R^4$ and $R^{4'}$ is $OR^7$. In some embodiments, $R^4$ is $OR^7$. In some embodiments, $R^{4'}$ is $OR^7$

For example, in some embodiments, at least one of $R^4$ and $R^{4'}$ is $OR^8$. In some embodiments, $R^{4'}$ is $OR^8$.

In some embodiments, at least one of $R^4$ and $R^{4'}$ is $-CH_2-T^3$, wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$.

In some embodiments, $R^{4'}$ is $-CH_2-T^3$, wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl,

$C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$.

In some embodiments, at least one of $R^4$ and $R^{4'}$ is -$CH_2$-$OR_8$. In some embodiments, $R^{4'}$ is -$CH_2$-$OR_8$.

In some embodiments, at least one of $R^4$ and $R^{4'}$ is -$CH_2$-$NR_7R_8$. In some embodiments, $R^{4'}$ is -$CH_2$-$NR_7R_8$.

In some embodiments, at least one of $R^4$ and $R^{4'}$ is halo, $C_1$-$C_6$ alkyl, or $OR^7$. In some embodiments, $R^4$ is halo, $C_1$-$C_6$ alkyl, or $OR^7$.

In some embodiments, at least one of $R^4$ and $R^{4'}$ is $C_1$-$C_6$ alkoxyl. In some embodiments, $R^4$ is $C_1$-$C_6$ alkoxyl.

For example, in some embodiments, at least one of $R^4$ and $R^{4'}$ is -$OCH_3$, -$OCH_2CH_3$, or - $OCH(CH_3)_2$. In some embodiments, $R^4$ is -$OCH_3$, -$OCH_2CH_3$, or -$OCH(CH_3)_2$.

For example, in some embodiments, at least one of $R^4$ and $R^{4'}$ is In some embodiments, $R^4$ is

For example, in some embodiments, $R^7$ is H or $C_1$-$C_6$ alkyl optionally substituted with one or more of hydroxyl, amino or mono- or di- alkylamino.

For example, in some embodiments, $R^8$ is -$Q^4$-$T^4$, in which $Q^4$ is a $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is $C_3$-$C_{12}$ cycloalkyl, $C_6$-$C_{10}$ aryl, or 4- to 12-membered heterocycloalkyl (e.g., 4- to 7-membered heterocycloalkyl) containing 1-4 heteroatoms selected from N, O and S which is optionally substituted with one or more -$Q^5$-$T^5$.

For example, in some embodiments, each 4- to 12-membered heterocycloalkyl described herein include, e.g., a 4 to 7-membered monocyclic heterocycloalkyl or 7 to 12-membered bicyclic heterocycloalkyl such as azetidinyl, oxetanyl, thietanyl, pyrrolidinyl, imidazolidinyl, pyrazolidinyl, oxazolidinyl, isoxazolidinyl, triazolidinyl, tetrahyrofuranyl, piperidinyl, 1,2,3,6-tetrahydropyridinyl, piperazinyl, tetrahydro-2H-pyranyl, 3,6-dihydro-2H-pyranyl, tetrahydro-2H-thiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, 3-azabicyclo[3.1.0]hexanyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like.

For example, in some embodiments, $R^8$ is -$Q^4$-$R^{S3}$, in which $Q^4$ is a bond or a $C_1$-$C_6$ alkylene linker (e.g., $C_2$-$C_6$ alkylene linker) optionally substituted with a hydroxyl and $R^{S3}$ is 4- to 12-membered heterocycloalkyl (e.g., a 4 to 7-membered monocyclic heterocycloalkyl or 7 to 12-membered bicyclic heterocycloalkyl such as azetidinyl, oxetanyl, thietanyl, pyrrolidinyl, imidazolidinyl, pyrazolidinyl, oxazolidinyl, isoxazolidinyl, triazolidinyl, tetrahyrofuranyl, piperidinyl, 1,2,3,6-tetrahydropyridinyl, piperazinyl, tetrahydro-2H-pyranyl, 3,6-dihydro-2H-pyranyl, tetrahydro-2H-thiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, 3-azabicyclo[3.1.0]hexanyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like), which is optionally substituted with one or more -$Q^5$-$T^5$.

For example, in some embodiments, $Q^4$ is $C_1$-$C_6$ alkylene linker optionally substituted with a hydroxyl and $R^{S3}$ is $C_3$-$C_6$ cycloalkyl optionally substituted with one or more -$Q^5$-$T^5$.

For example, in some embodiments, $Q^4$ is an optionally substituted $C_2$-$C_6$ alkenylene or $C_2$-$C_6$ alkynylene linker and $R^{S3}$ is 4- to 12-membered heterocycloalkyl (e.g., a 4 to 7-membered monocyclic heterocycloalkyl or 7 to 12-membered bicyclic heterocycloalkyl such as azetidinyl, oxetanyl, thietanyl, pyrrolidinyl, imidazolidinyl, pyrazolidinyl, oxazolidinyl, isoxazolidinyl, triazolidinyl, tetrahyrofuranyl, piperidinyl, 1,2,3,6-tetrahydropyridinyl, piperazinyl, tetrahydro-2H-pyranyl, 3,6-dihydro-2H-pyranyl, tetrahydro-2H-thiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, 3-azabicyclo[3.1.0]hexanyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like), which is optionally substituted with one or more -$Q^5$-$T^5$.

For example, in some embodiments, $Q^4$ is an optionally substituted $C_2$-$C_6$ alkenylene or $C_2$-$C_6$ alkynylene linker and $R^{S3}$ is $C_3$-$C_6$ cycloalkyl optionally substituted with one or more -$Q^5$-$T^5$.

For example, in some embodiments, each $Q^5$ independently is a bond or $C_1$-$C_3$ alkylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$cycloalkyl (*e.g.,* $C_3$-$C_8$ cycloalkyl), or 4- to 7-membered heterocycloalkyl containing 1-4 heteroatoms selected from N, O, and S.

For example, in some embodiments, each $Q^5$ independently is a $C_2$-$C_3$ alkenylene, or $C_2$-$C_3$ alkynylene linker

each optionally substituted with one or more of halo, cyano, hydroxyl, or Ci-$C_6$ alkoxy, and each $T^5$ independently is selected from the group consisting of H, halo, cyano, $C_1$-$C_6$ alkyl, $C_3$-$C_{12}$cycloalkyl (*e.g.,* $C_3$-$C_8$ cycloalkyl), or 4- to 7-membered heterocycloalkyl containing 1-4 heteroatoms selected from N, O, and S.

For example, in some embodiments, -$Q^5$-$T^5$ is oxo.

In some embodiments, at least one of $R^4$ and $R^{4'}$ is

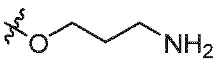, 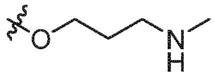,

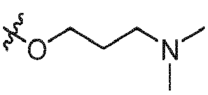, 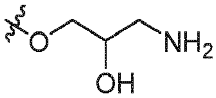, 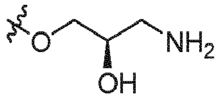, 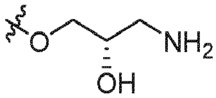, 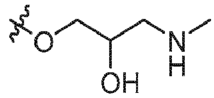,

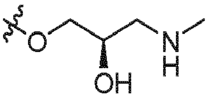, 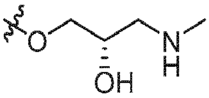, 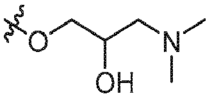, 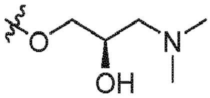, or 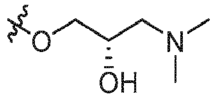.

In some embodiments, $R^{4'}$ is

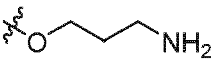, 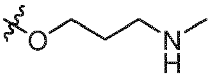, 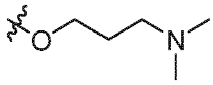,

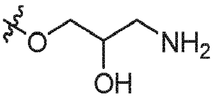, 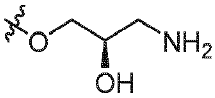, 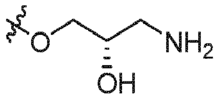, 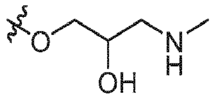, 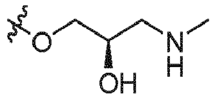,

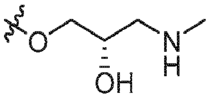, 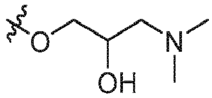, 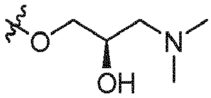, or 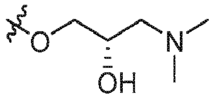.

In some embodiments, at least one of $R^4$ and $R^{4'}$ is

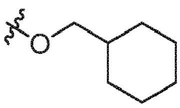, 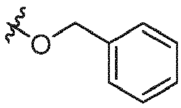,

or

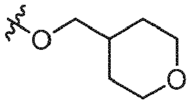.

In some embodiments, $R^{4'}$ is

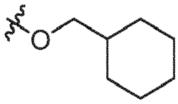, 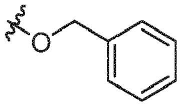, or 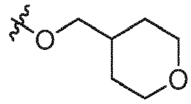.

For example, in some embodiments, at least one of $R^4$ and $R^{4'}$ is

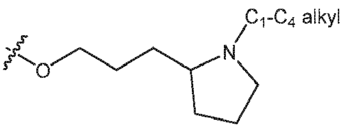, 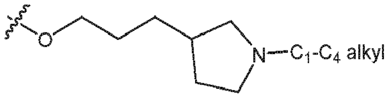, 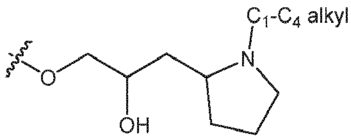,

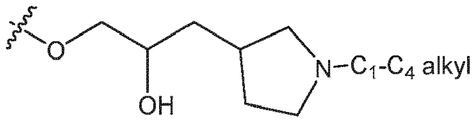, 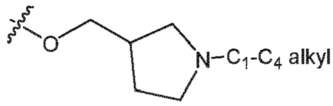, 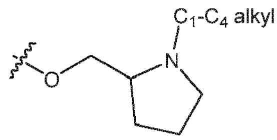,

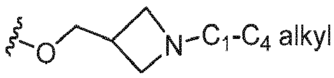, 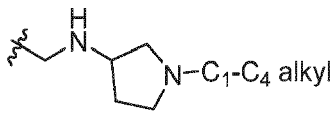, or 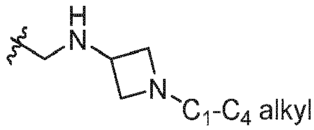.

In some embodiments, $R^{4'}$ is

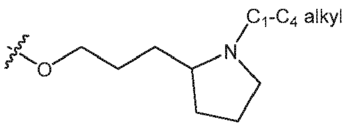, 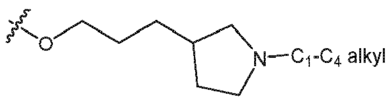,

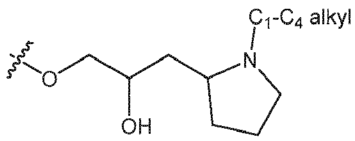, 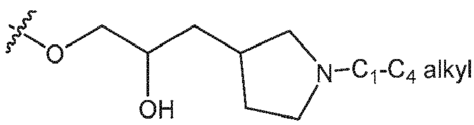, 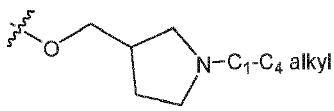,

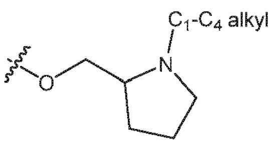, 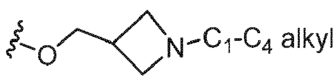, 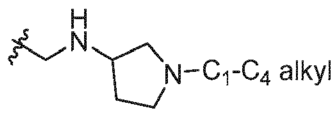,

or

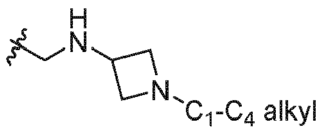.

For example, in some embodiments, at least one of $R^4$ and $R^{4'}$ is

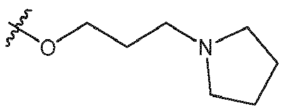,

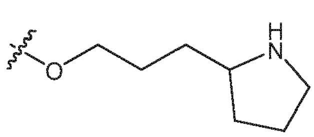, 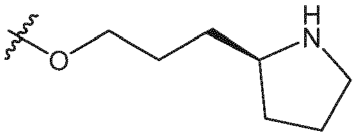, 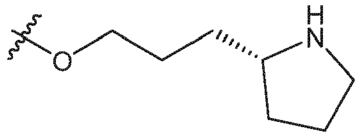,

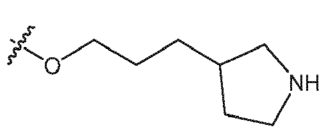, 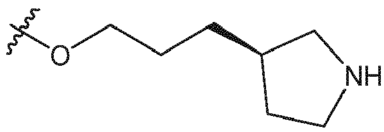, 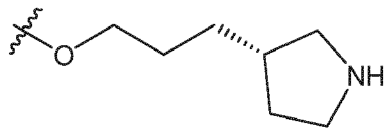,

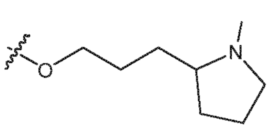, 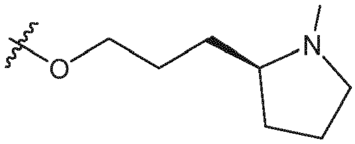, 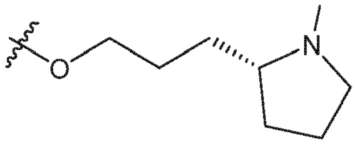,

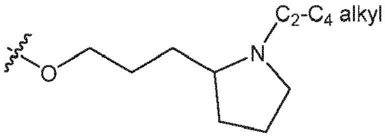, 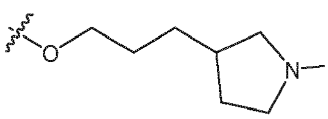, 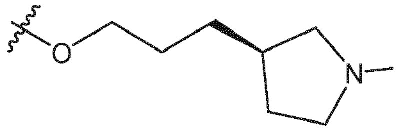,

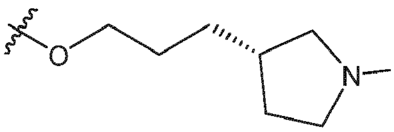, 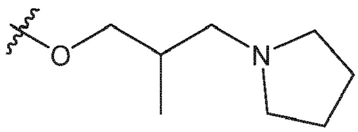, 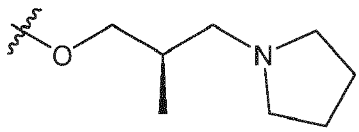,

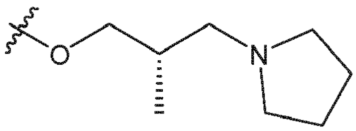, 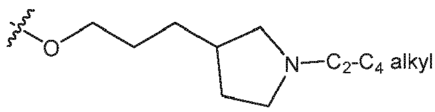, 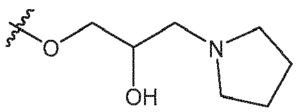,

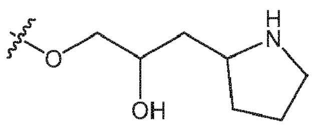, 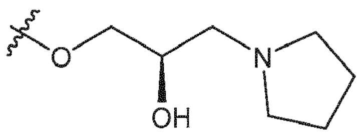, 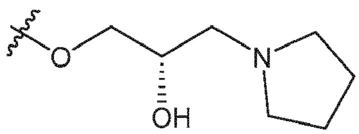,

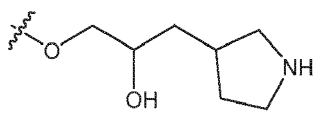, 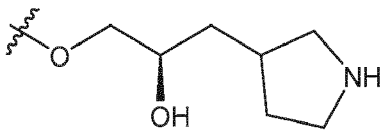, 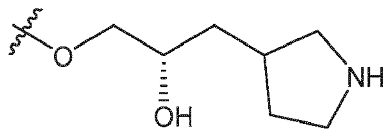,

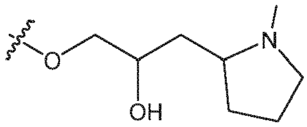, 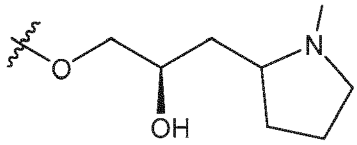, 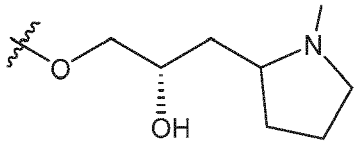,

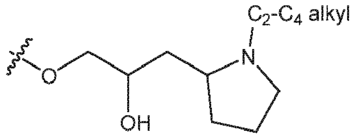, 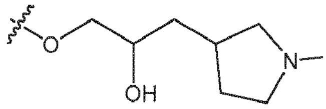, 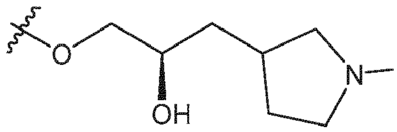,

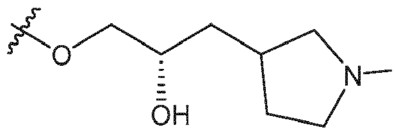, 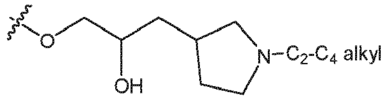, 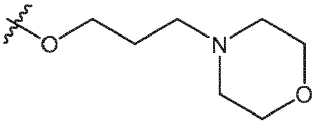,

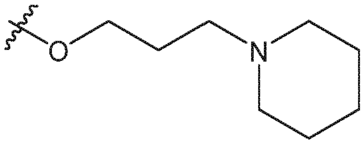, 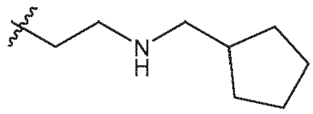, 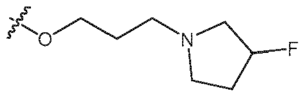,

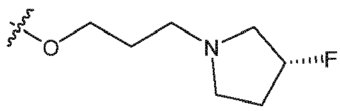, 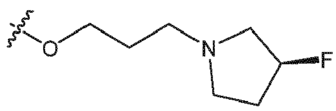, 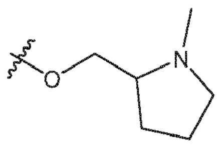, 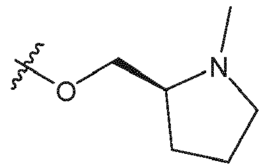,

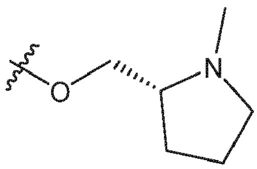, 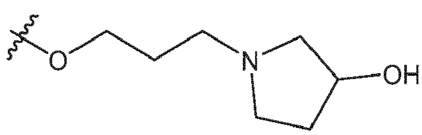, 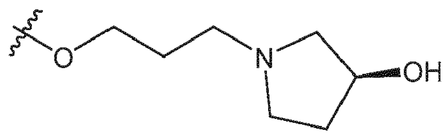,

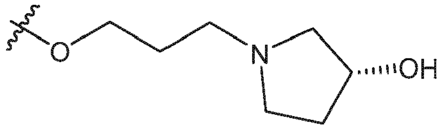, 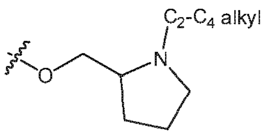, 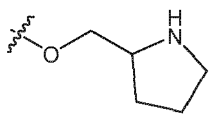, 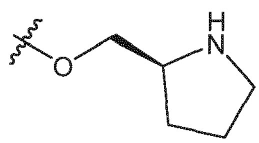,

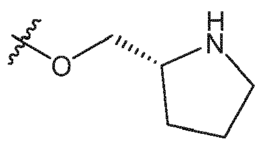, 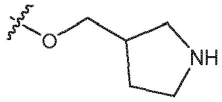, 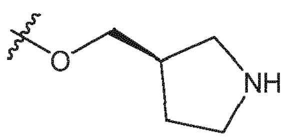, 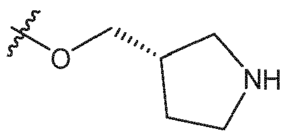,

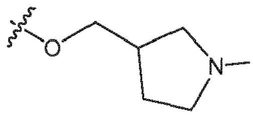, 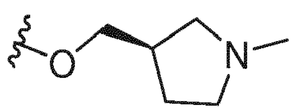, 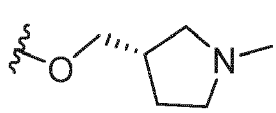, 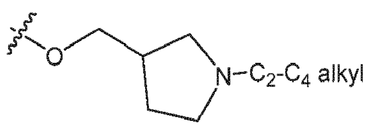,

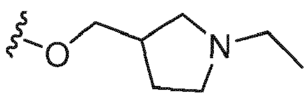, 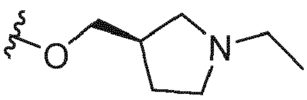, 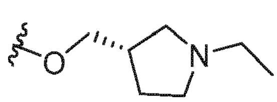,

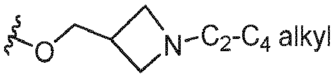, 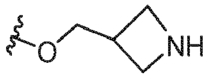, 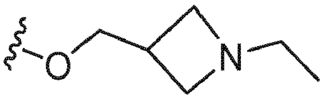, 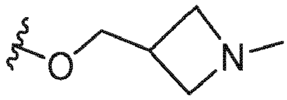,

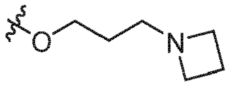, 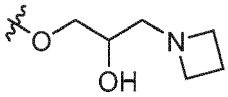, 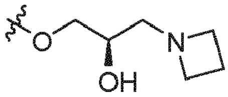, or 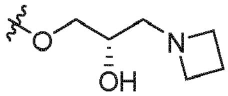.

In some embodiments, $R^{4'}$ is

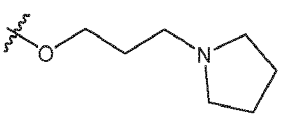, 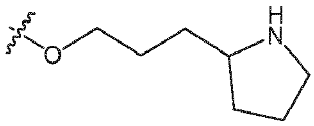,

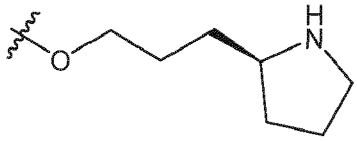, 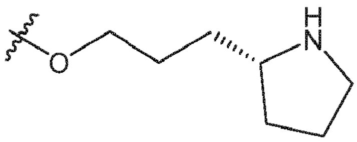, 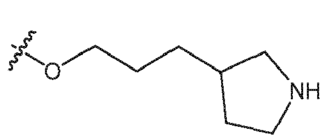,

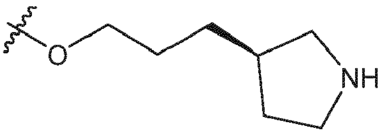, 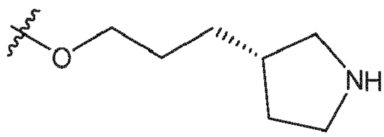, 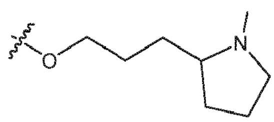,

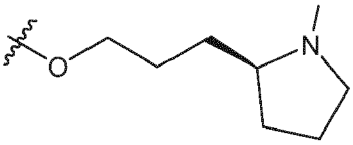, 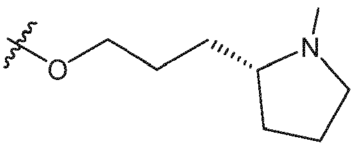, 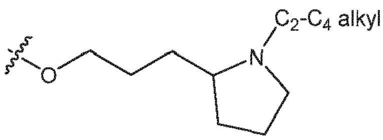,

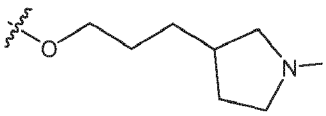, 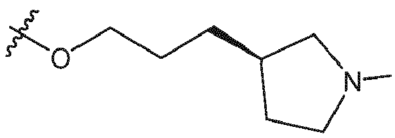, 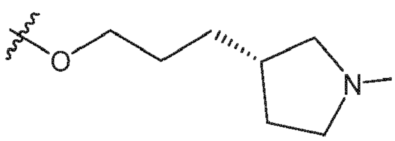,

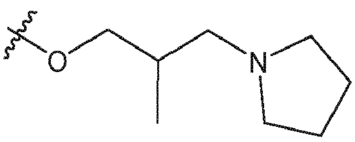, 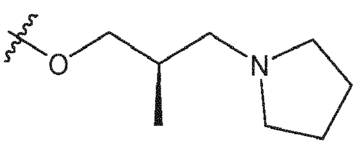, 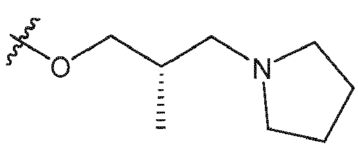,

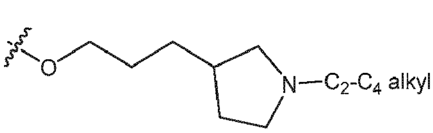, 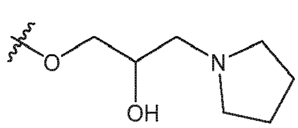, 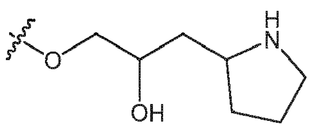,

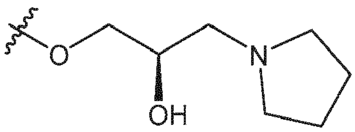, 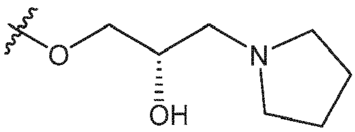, 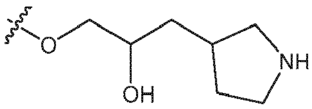,

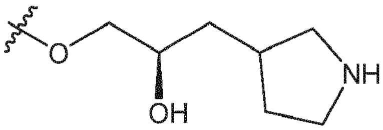, 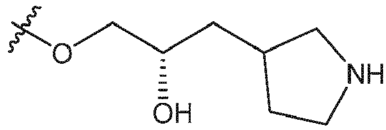, 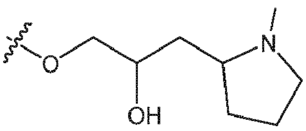,

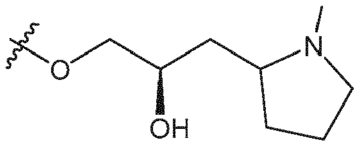, 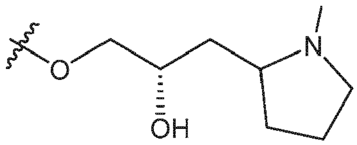, 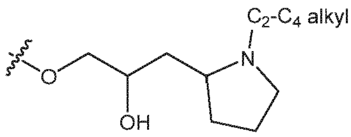,

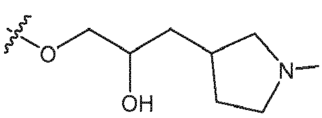, 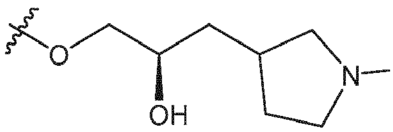, 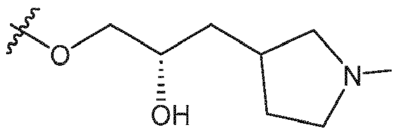,

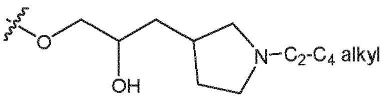, 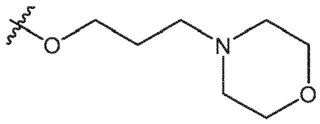, 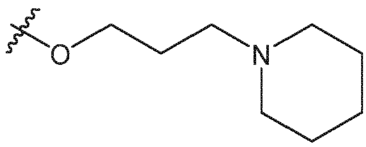,

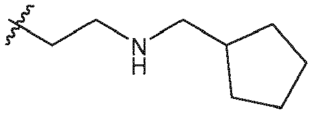, 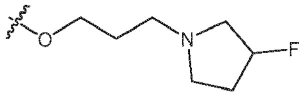, 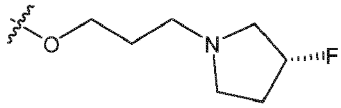,

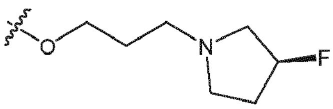, 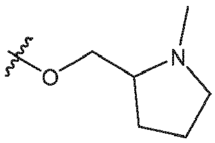, 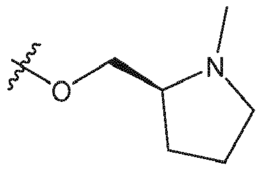, 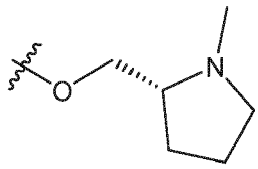,

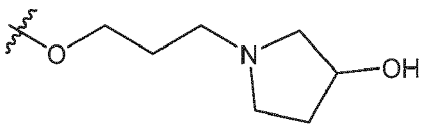, 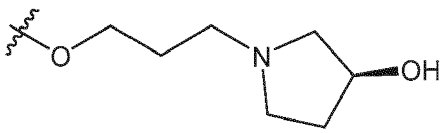,

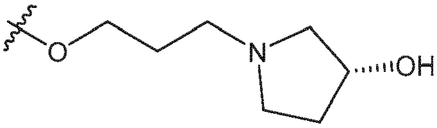, 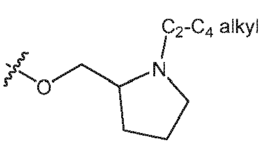, 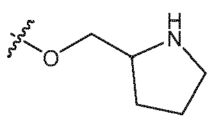, 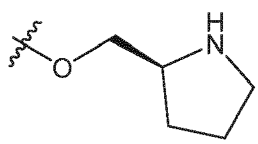,

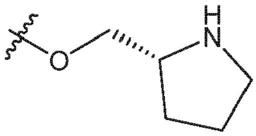, 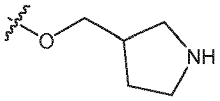, 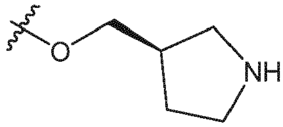, 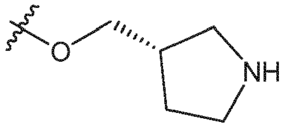,

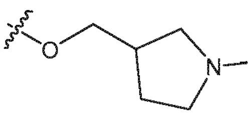, 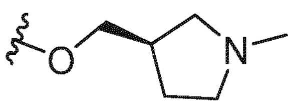, 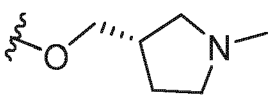, 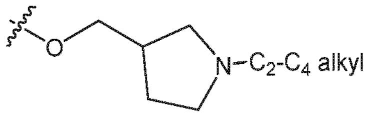,

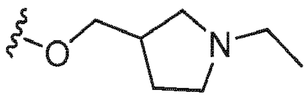, 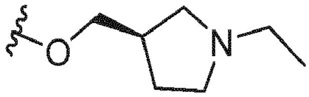, 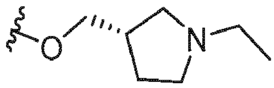,

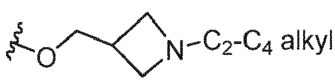, 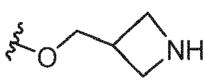, 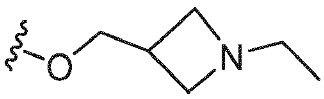, 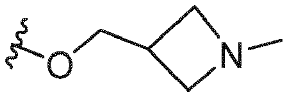,

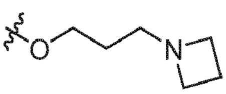, 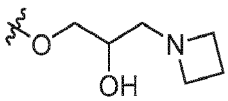, 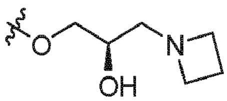, or 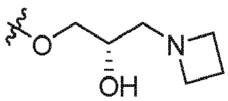.

In some embodiments, wherein at least one of $R^4$ and $R^{4'}$ is

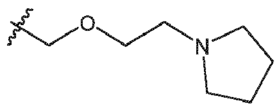.

In some embodiments, $R^{4'}$ is

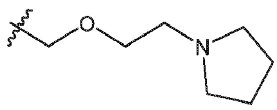.

In some embodiments, wherein at least one of $R^4$ and $R^{4'}$ is

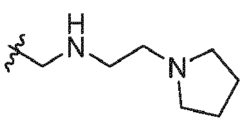,

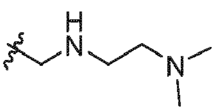, 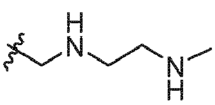, 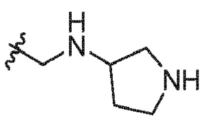, 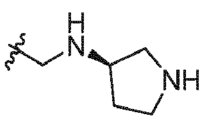, 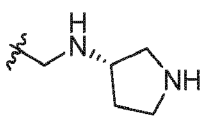,

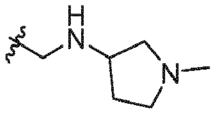, 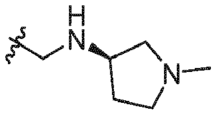, 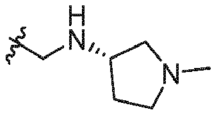, 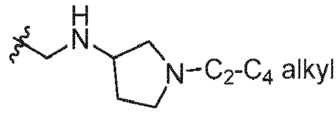,

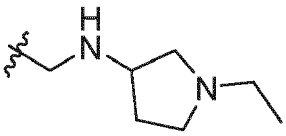, 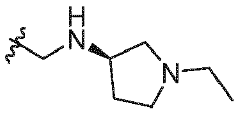, 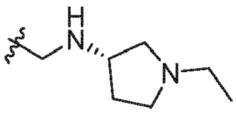, 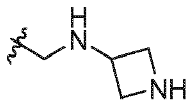, 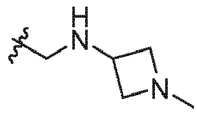,

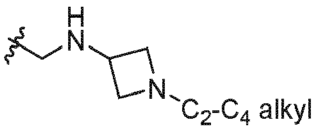, 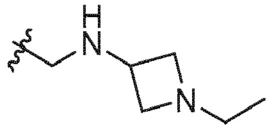, or 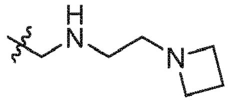.

In some embodiments, $R^{4'}$ is

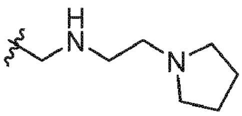, 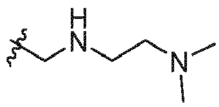, 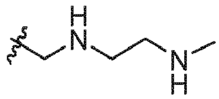,

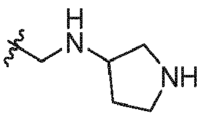, 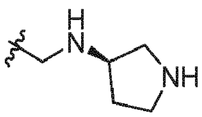, 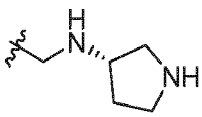, 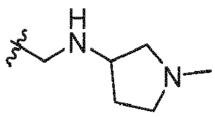, 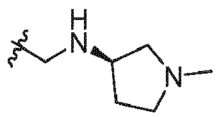,

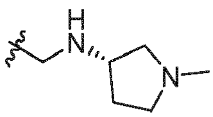, 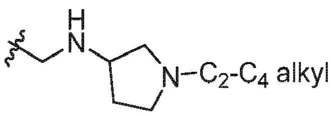, 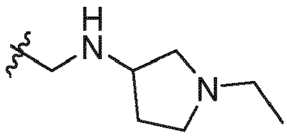, 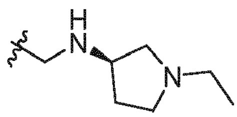,

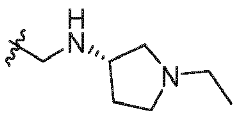, 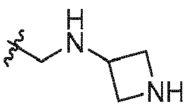, 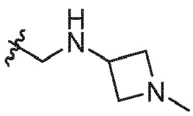, 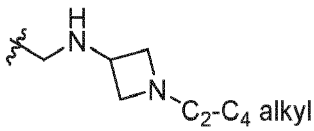,

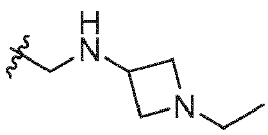, or 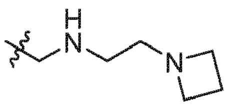.

In some embodiments, one of $R^4$ and $R^{4'}$ is halo, $C_1$-$C_6$ alkyl, or $OR^7$, and the other is

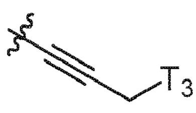,

wherein $T^3$ is 5- to 10-membered heteroaryl or 4- to 12-membered heterocycloalkyl optionally substituted with one or

more of halo, hydroxyl, $C_1$-$C_6$ alkoxyl or $C_1$-$C_6$ alkyl.

In some embodiments, $R^4$ is halo, $C_1$-$C_6$ alkyl, or $OR^7$, and $R^{4'}$ is

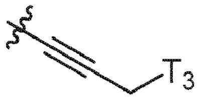
,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

In some embodiments, one of $R^4$ and $R^{4'}$ is $C_1$-$C_6$ alkoxyl and the other is

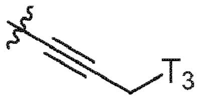
,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

In some embodiments, $R^4$ is $C_1$-$C_6$ alkoxyl, and $R^{4'}$ is

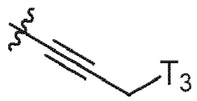
,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

In some embodiments, one of $R^4$ and $R^{4'}$ is $-OCH_3$, and the other is

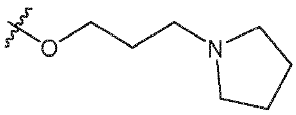
.

In some embodiments, $R^4$ is $-OCH_3$, and $R^{4'}$ is

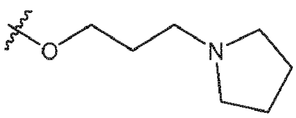
.

For example, in some embodiments, and one of $R^4$ and $R^{4'}$ is $-OCH_3$, and the other is

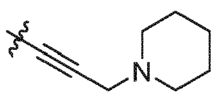
.

In some embodiments, $R^4$ is $-OCH_3$, and $R^{4'}$ is

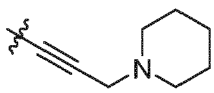
.

In some embodiments, the compounds of Formula (I) include those of any of Formulae (VIIa), (VIIb), (VIIe), (VIId), (VIIe), and (VIIf):

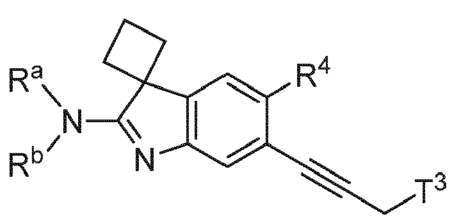 (VIIa), 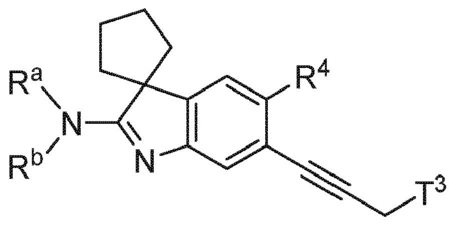 (VIIb),

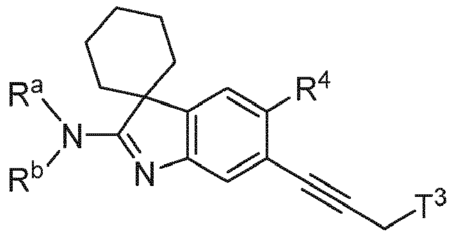 (VIIc), 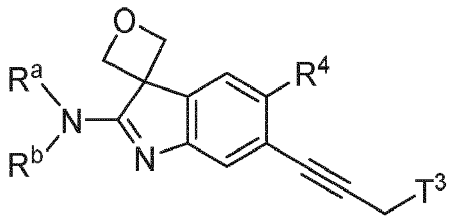 (VIId),

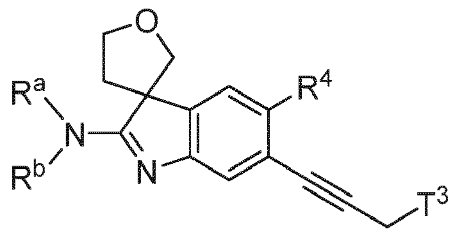 (VIIe), 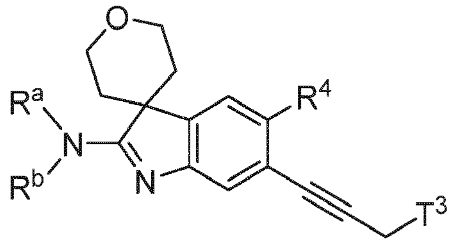 (VIIf),

and tautomers thereof, and pharmaceutically acceptable salts of the compounds or the tautomers, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is halo, $C_1$-$C_6$ alkyl, or $OR^7$;

$T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5-to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

each $R^8$ independently is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo.

In some embodiments, $R^4$ is

In some embodiments, $T^3$ is 5- to 10-membered heteroaryl or 4- to 12-membered heterocycloalkyl optionally

substituted with one or more of halo, hydroxyl, $C_1$-$C_6$ alkoxyl or $C_1$-$C_6$ alkyl.

In some embodiments, the compounds of Formula (I) include those of any of Formulae (VIIIa), (VIIIb), (VIIIc), (VIIId), (VIIIe), and (VIIIf):

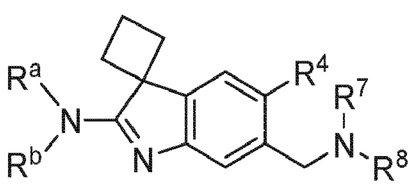 (VIIIa), 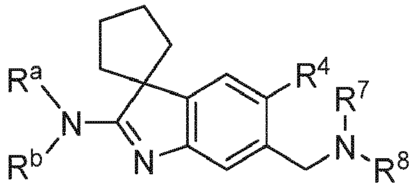 (VIIIb),

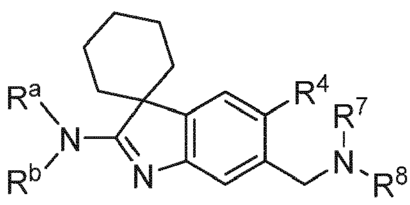 (VIIIc), 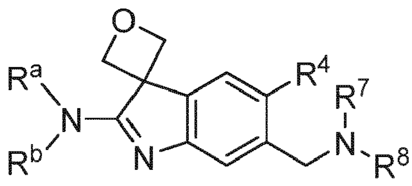 (VIIId),

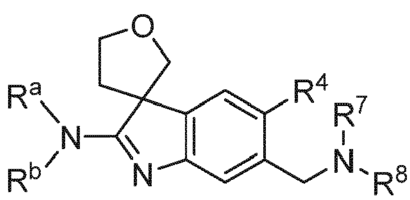 (VIIIe), 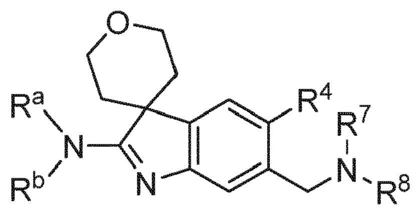 (VIIIf),

and tautomers thereof, and pharmaceutically acceptable salts of the compounds or the tautomers, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is $-Q^3$-$T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

each $R^8$ independently is $-Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5$-$T^5$ is oxo.

In some embodiments, $R^4$ is halo, $C_1$-$C_6$ alkyl, or $OR^7$. In some embodiments, $R^4$ is $C_1$-$C_6$ alkoxyl. In some

embodiments, $R^4$ is

In some embodiments, the compounds of Formula (I) include those of any of Formulae (IXa), (IXb), (IXc), (IXd), (IXe), and (IXf):

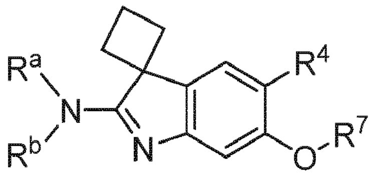 (IXa), 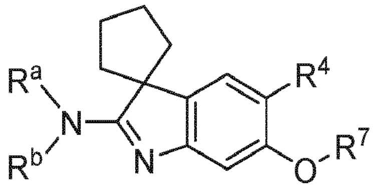 (IXb),

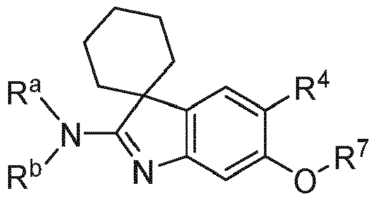 (IXc), 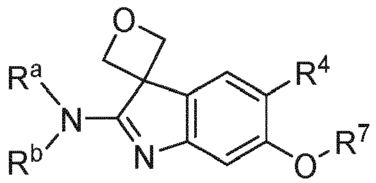 (IXd),

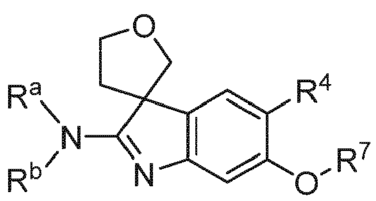 (IXe), 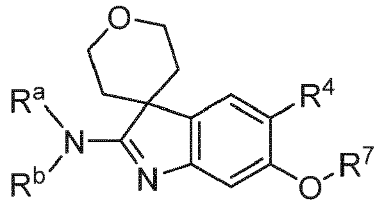 (IXf),

and tautomers thereof, and pharmaceutically acceptable salts of the compounds or the tautomers, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is $-Q^3-T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

each $R^8$ independently is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_2$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo.

In some embodiments, $R^4$ is halo, $C_1$-$C_6$ alkyl, or $OR^7$. In some embodiments, $R^4$ is $C_1$-$C_6$ alkoxyl. In some

embodiments, $R^4$ is

In some embodiments, the compounds of Formula (I) include those of any of Formulae (Xa), (Xb), (Xc), (Xd), (Xe), and (Xf):

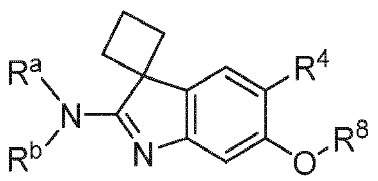 (Xa), 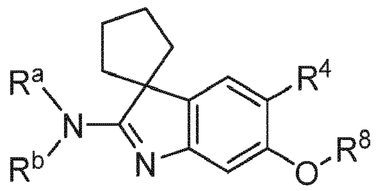 (Xb),

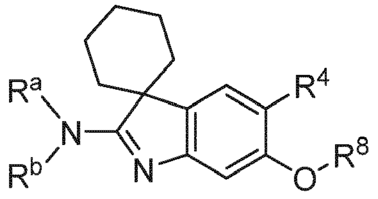 (Xc), 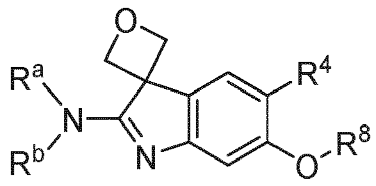 (Xd),

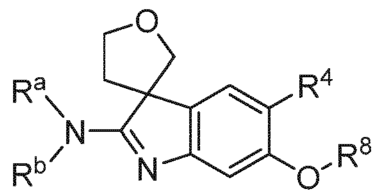 (Xe), 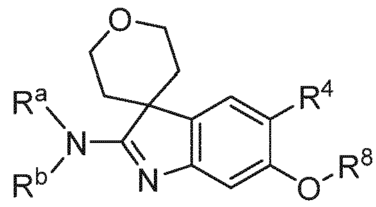 (Xf),

and tautomers thereof, and pharmaceutically acceptable salts of the compounds or the tautomers, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is -$Q^3$-$T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

each $R^8$ independently is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

In some embodiments, $R^4$ is halo, $C_1$-$C_6$ alkyl, or $OR^7$. In some embodiments, $R^4$ is $C_1$-$C_6$ alkoxyl. In some

embodiments, $R^4$ is

For example, in some embodiments, the compound is selected from those in Table 1, tautomers thereof, and pharmaceutically acceptable salts of the compounds and tautomers.

In some embodiments, the compound is selected from the group consisting of Compound Nos. 1-23, 25-36, 38-39, 42-69, 77-78, 81, and 135-137, tautomers thereof, and pharmaceutically acceptable salts of the compounds and tautomers.

In some embodiments, the compound is selected from the group consisting of Compound Nos. 24, 74-75, 82-101, 106-107, 110-134, and 141, tautomers thereof, and pharmaceutically acceptable salts of the compounds and tautomers.

In some embodiments, the compound is selected from the group consisting of Compound Nos. 37, 40-41, 70-73, 76, 79-80, and 138-140, tautomers thereof, and pharmaceutically acceptable salts of the compounds and tautomers.

The present disclosure provides compounds which inhibit a kinase with an enzyme inhibition $IC_{50}$ value of about 100 nM or greater, 1 μM or greater, 10 μM or greater, 100 μM or greater, or 1000 μM or greater.

The present disclosure provides compounds which inhibit a kinase with an enzyme inhibition $IC_{50}$ value of about 1 mM or greater.

The present disclosure provides compounds which inhibit a kinase with an enzyme inhibition $IC_{50}$ value of 1 μM or greater, 2 μM or greater, 5 μM or greater, or 10 μM or greater, wherein the kinase is one or more of the following: Abl, AurA, CHK1, MAP4K, IRAK4, JAK3, EphA2, FGFR3, KDR, Lck, MARK1, MNK2, PKCb2, SIK, and Src.

The present disclosure provides a pharmaceutical composition comprising a compound of any one of the Formulae described herein or a pharmaceutically acceptable salt thereof, and a pharmaceutically acceptable carrier.

The present disclosure provides methods of preventing or treating an EHMT-mediated disease or disorder, *e.g.,* a disease or disorder characterized or caused, at least in part, by aberrant EHMT function, e.g., by a mutated EHMT gene or protein, aberrant expression of EHMT2, or dysregulation of EHMT expression, via inhibition of a methyltransferase enzyme selected from EHMT1 and EHMT2. In some embodiments, the method comprises administering to a subject in need thereof, *e.g.,* a subject having or at risk of developing the EHMT-mediated disease or disorder, a therapeutically effective amount of a compound disclosed herein, e.g., any of Formulae (I)-(Xf). In some embodiments, the EHMT-mediated disease or disorder is an EHMT2-mediated disease or disorder.

The present disclosure provides methods of preventing or treating a blood disorder via inhibition of a methyltransferase enzyme selected from EHMT1 and EHMT2, the method comprising administering to a subject in need thereof a therapeutically effective amount of a compound disclosed herein, e.g., any of Formulae (I)-(Xf). In some embodiments, the blood disorder is an EHMT2-mediated blood disorder.

For example, in some embodiments, the blood disorder is sickle cell anemia or β-thalassemia.

For example, in some embodiments, the blood disorder is a hematological cancer.

For example, in some embodiments, the hematological cancer is acute myeloid leukemia (AML) or chronic lymphocytic leukemia (CLL).

The present disclosure also provides compounds of Formula (I) which are selective inhibitors of EHMT2. For example, in some embodiments, the compounds of Formula (I) inhibit EHMT2 (e.g., human EHMT2) with an enzyme inhibition $IC_{50}$ value of about 1 μM or less, about 500 nM or less, about 200 nM or less, about 100 nM or less, or about 50 nM or less. Accordingly, in some embodiments of the compositions or methods provided herein, compounds of any of Formulae (I)-(Xf) are used or provided that inhibit EHMT2 (e.g., human EHMT2) with an enzyme inhibition $IC_{50}$ value of about 1 μM or less. In some embodiments of the compositions or methods provided herein, compounds of any of Formulae (I)-(Xf) are used or provided that inhibit EHMT2 (e.g., human EHMT2) with an enzyme inhibition $IC_{50}$ value of about 500 nM or less. In some embodiments of the compositions or methods provided herein, compounds of any of Formulae (I)-(Xf) are used or provided that inhibit EHMT2 (e.g., human EHMT2) with an enzyme inhibition $IC_{50}$ value of about 200 nM or less. In some embodiments of the compositions or methods provided herein, compounds of any of Formulae (I)-(Xf) are used or provided that inhibit EHMT2 (e.g., human EHMT2) with an enzyme inhibition $IC_{50}$ value of about 100 nM or less. In some embodiments of the compositions or methods provided herein, compounds of any of Formulae (I)-(Xf) are used or provided that inhibit EHMT2 (e.g., human EHMT2) with an enzyme inhibition $IC_{50}$ value of about 50 nM or less.

Representative compounds of the present disclosure include compounds listed in Table 1 or tautomers and salts thereof.

**Table 1**

| Compound No. | Structure |
|---|---|
| 1 | 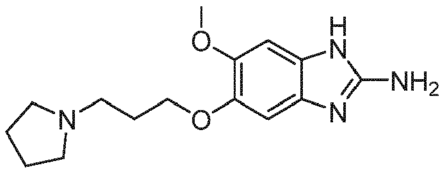 |
| 2 | 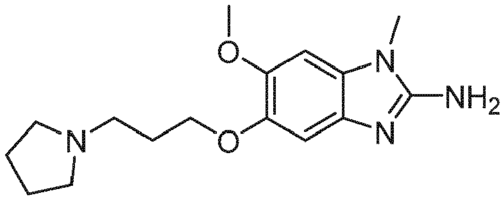 |
| 3 | 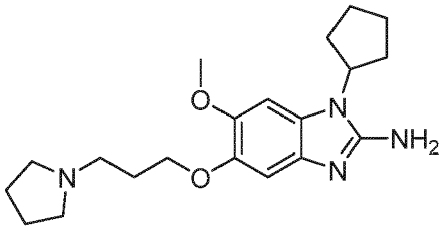 |
| 4 | 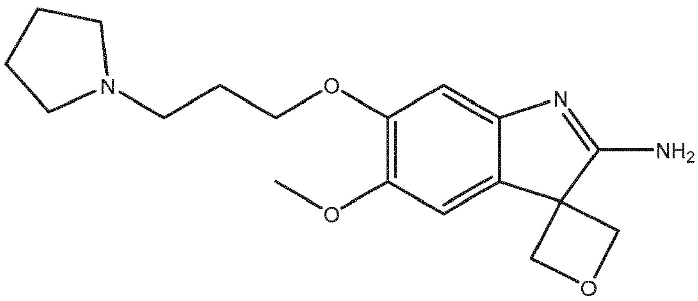 |
| 5 | 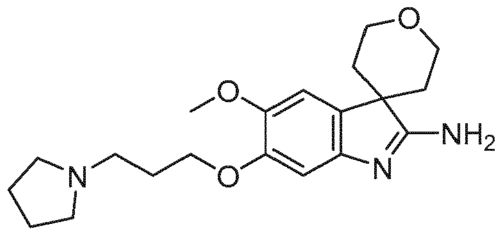 |
| 6 | 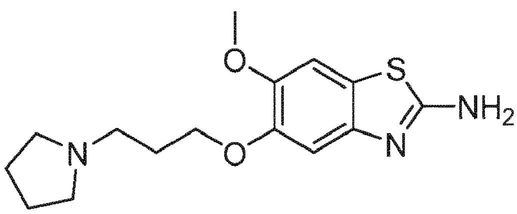 |

(continued)

| Compound No. | Structure |
|---|---|
| 7 | 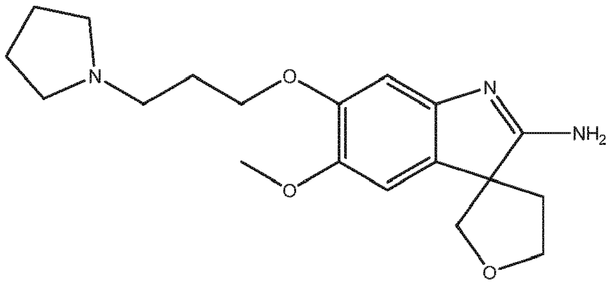 |
| 8 | 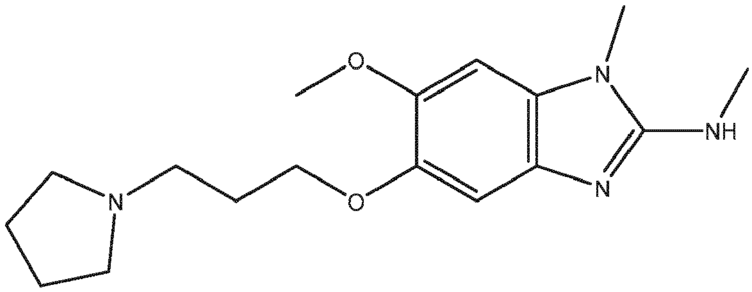 |
| 9 | 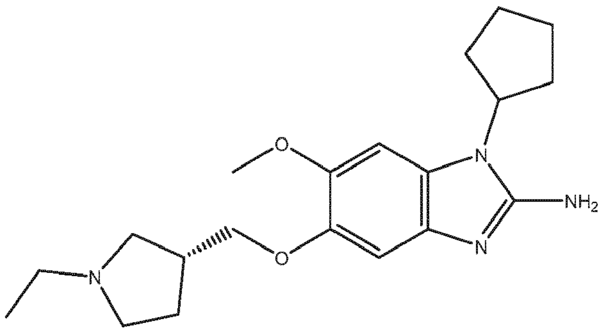 |
| 10 | 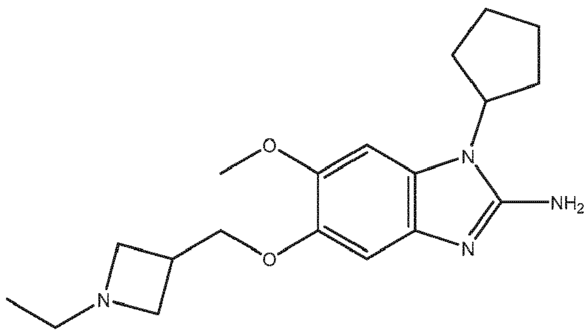 |
| 11 | 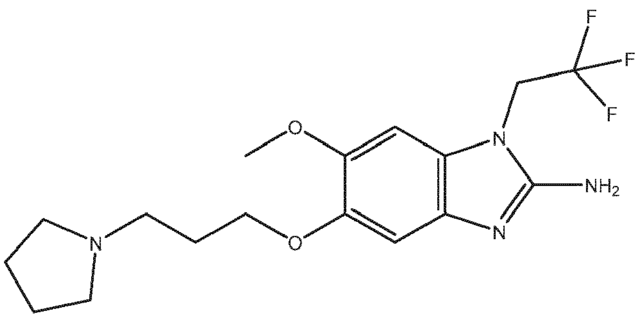 |

(continued)

| Compound No. | Structure |
|---|---|
| 12 | 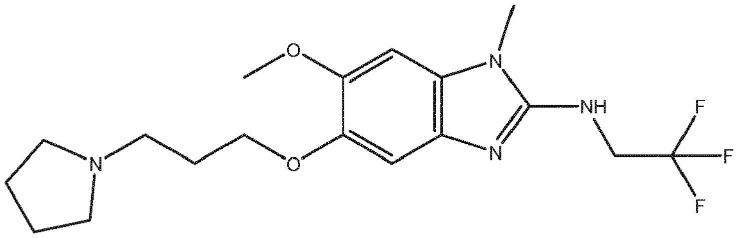 |
| 13 | 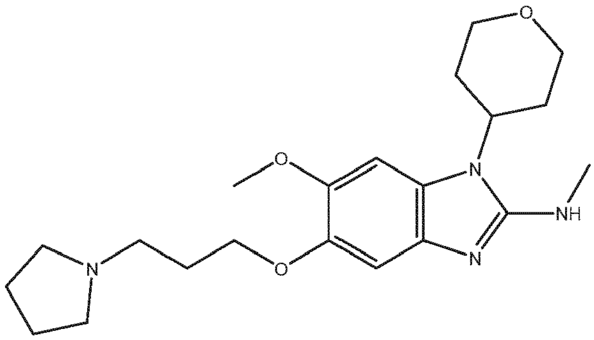 |
| 14 | 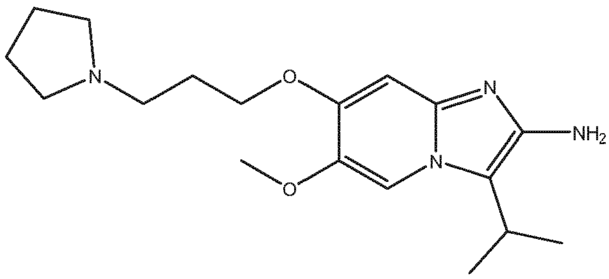 |
| 15 | 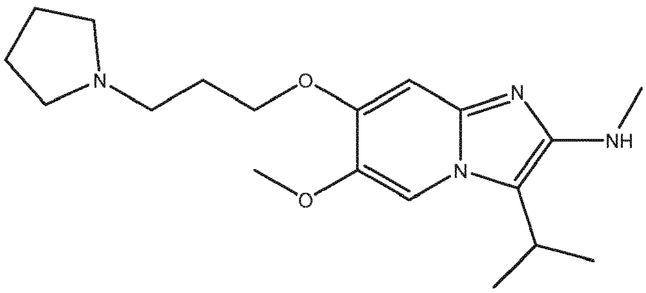 |
| 16 | 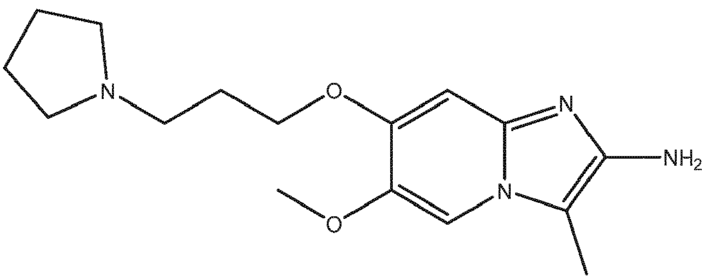 |

(continued)

| Compound No. | Structure |
|---|---|
| 17 | 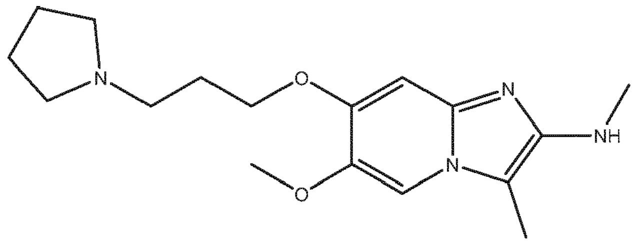 |
| 18 | 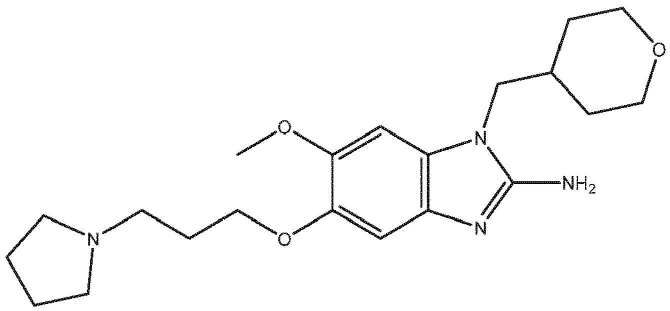 |
| 19 | 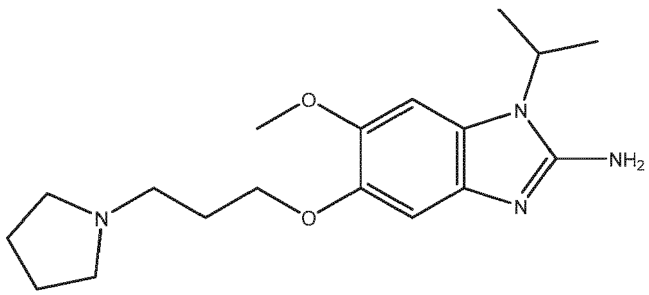 |
| 20 | 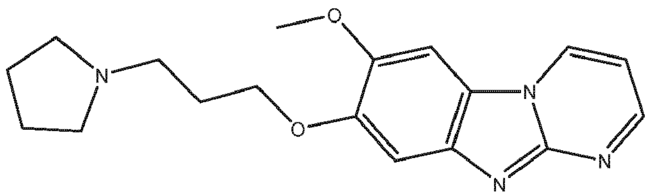 |
| 21 | 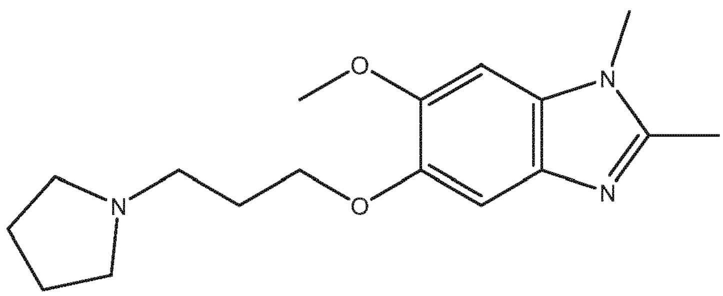 |

(continued)

| Compound No. | Structure |
|---|---|
| 22 | 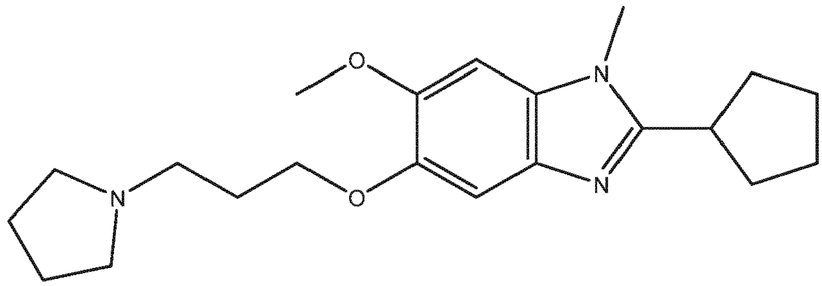 |
| 23 | 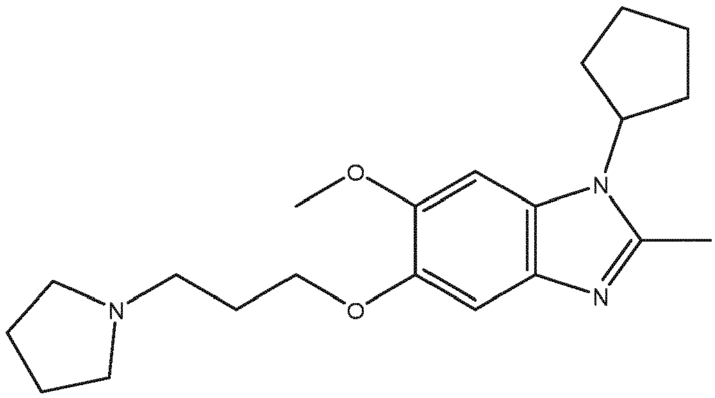 |
| 24 | 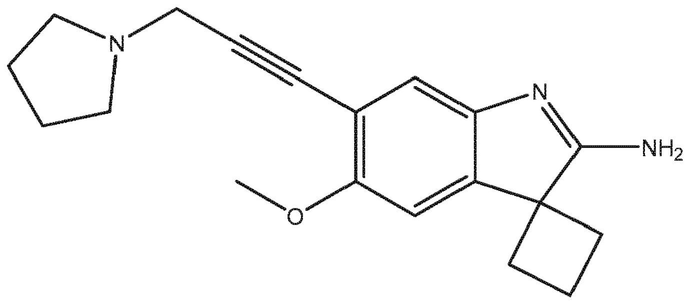 |
| 25 | 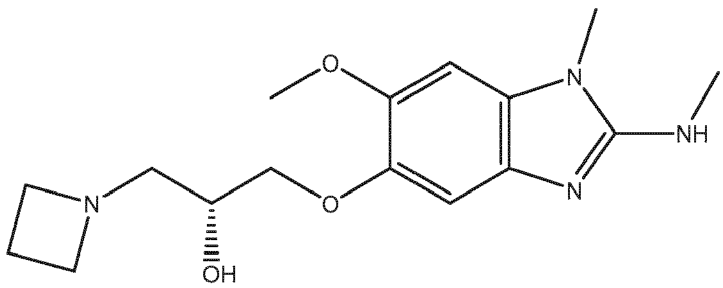 |
| 26 | 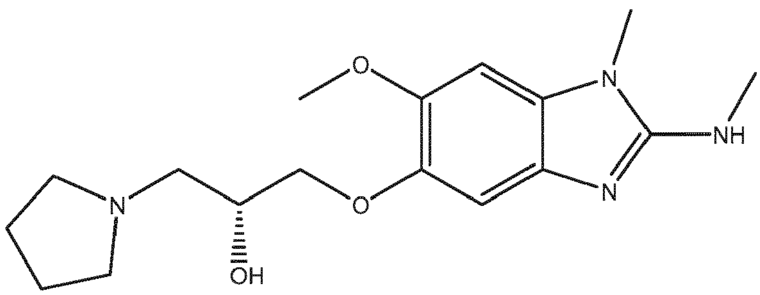 |

(continued)

| Compound No. | Structure |
|---|---|
| 27 | 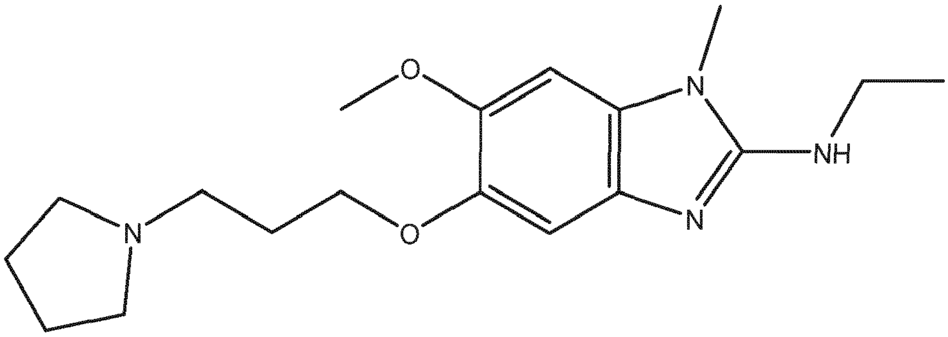 |
| 28 | 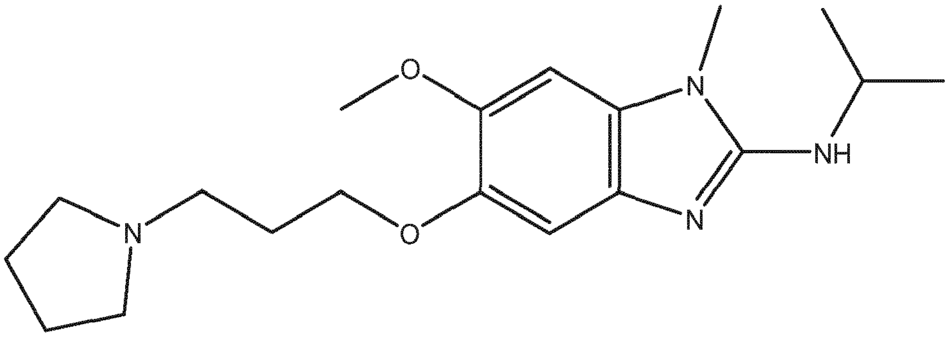 |
| 29 | 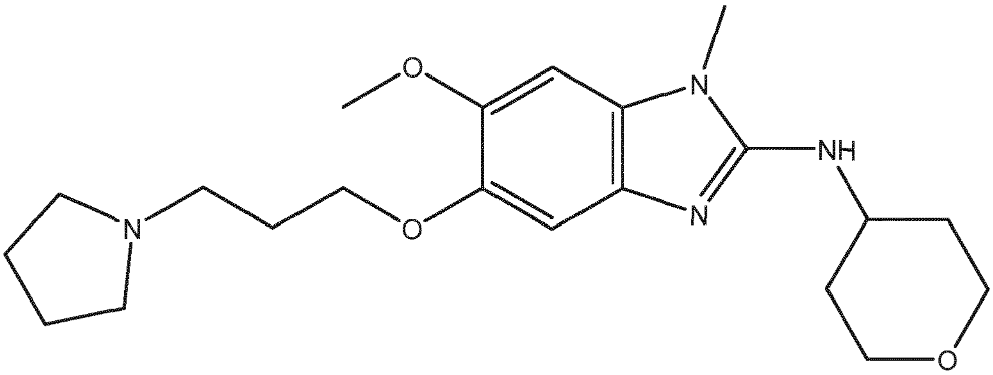 |
| 30 | 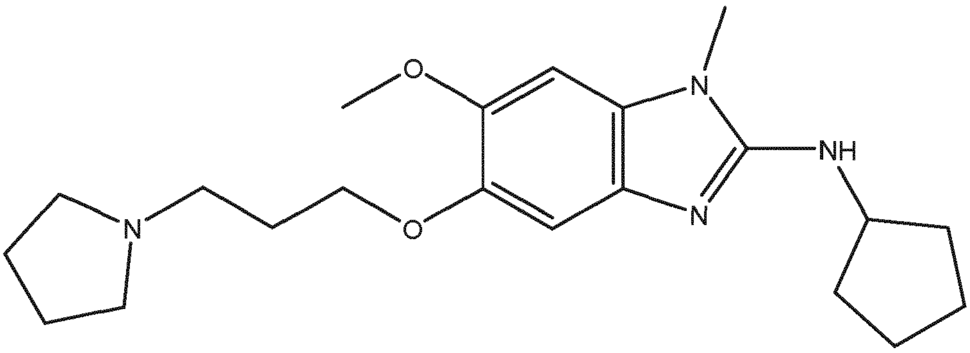 |

(continued)

| Compound No. | Structure |
|---|---|
| 31 | 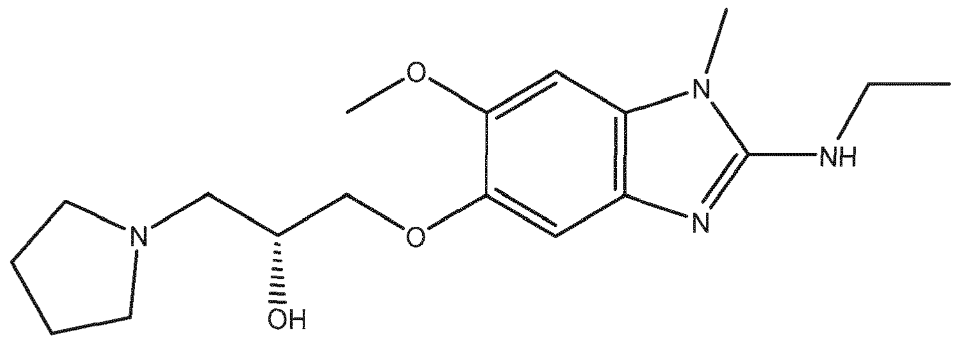 |
| 32 | 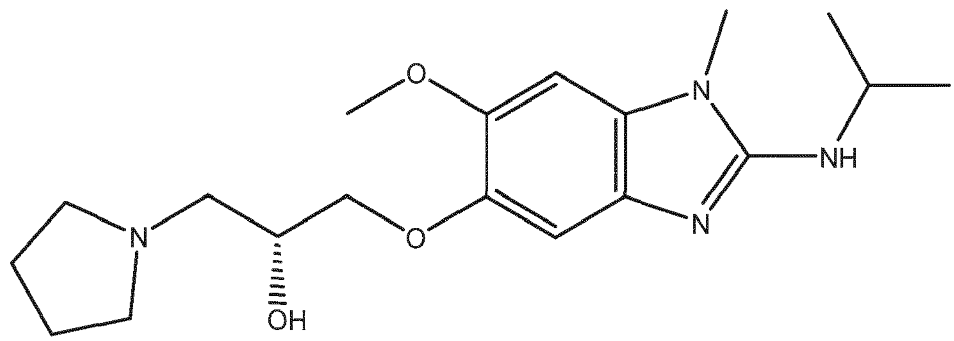 |
| 33 | 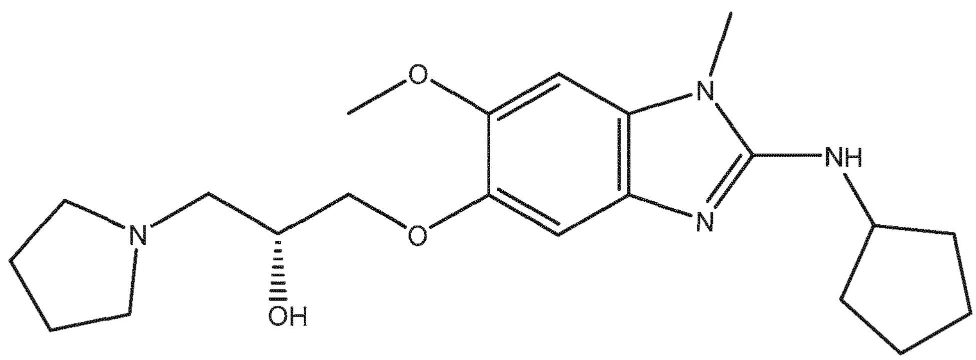 |
| 34 | 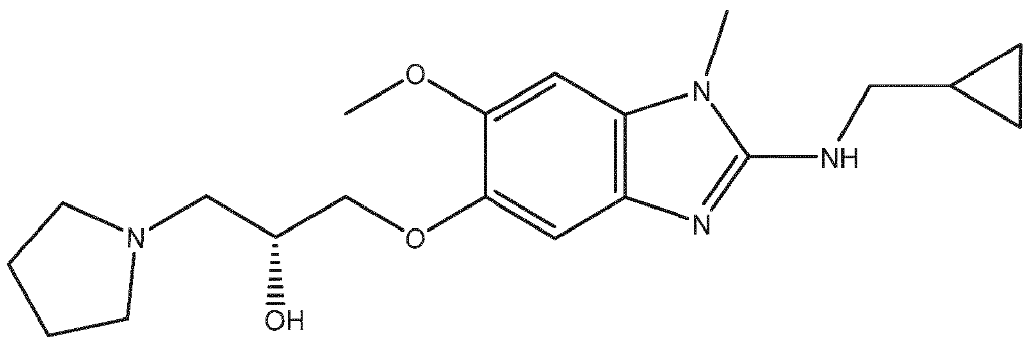 |

(continued)

| Compound No. | Structure |
|---|---|
| 35 | 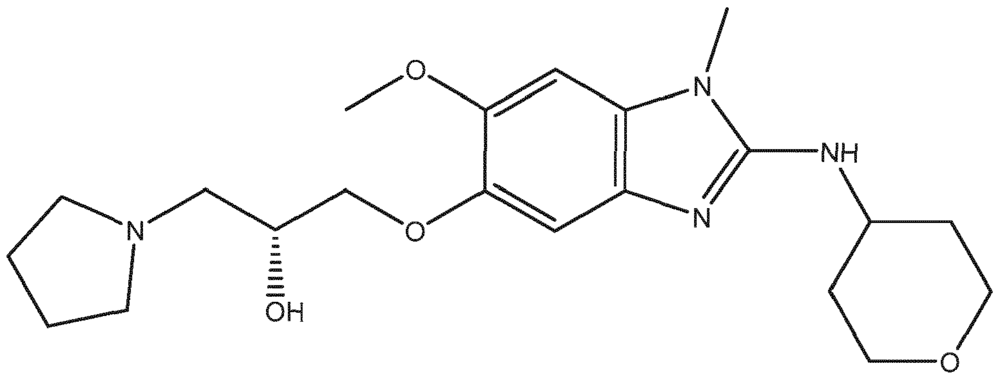 |
| 36 | 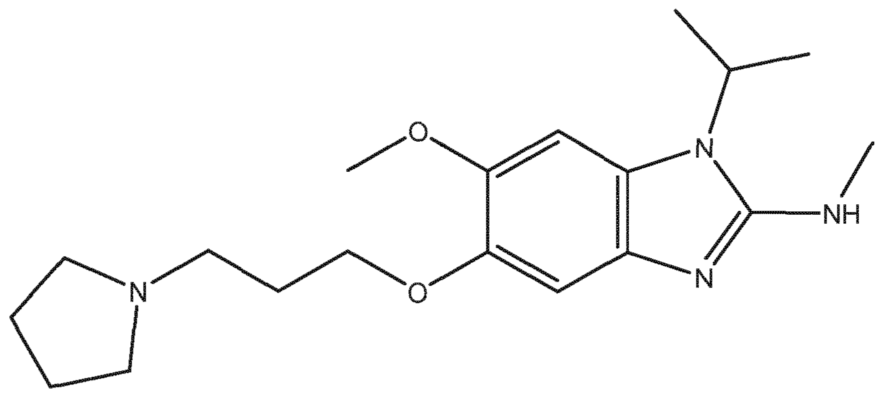 |
| 37 | 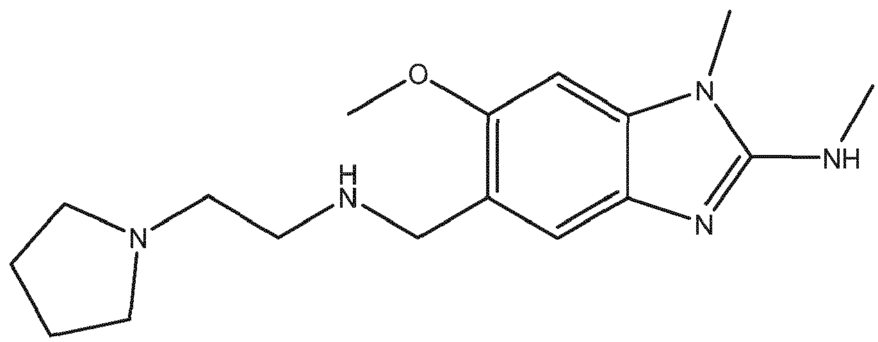 |
| 38 | 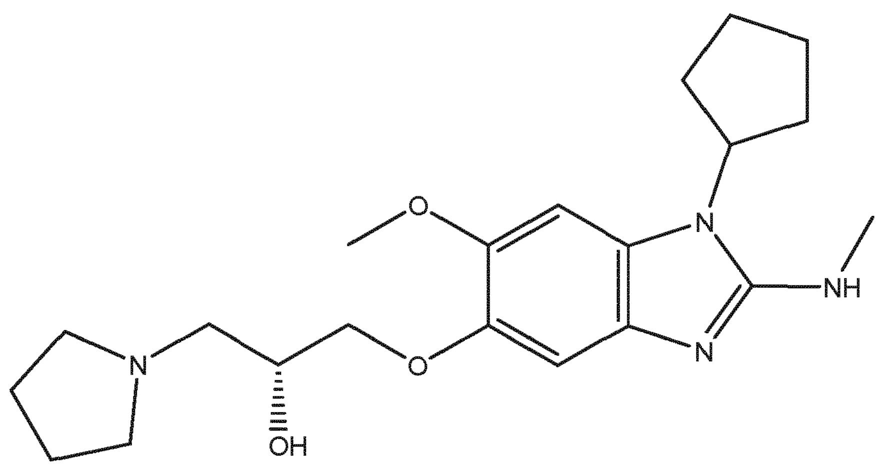 |

(continued)

| Compound No. | Structure |
|---|---|
| 39 | 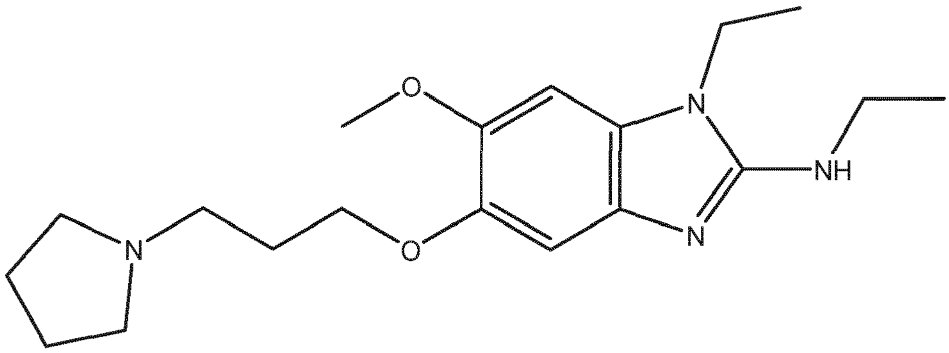 |
| 40 | 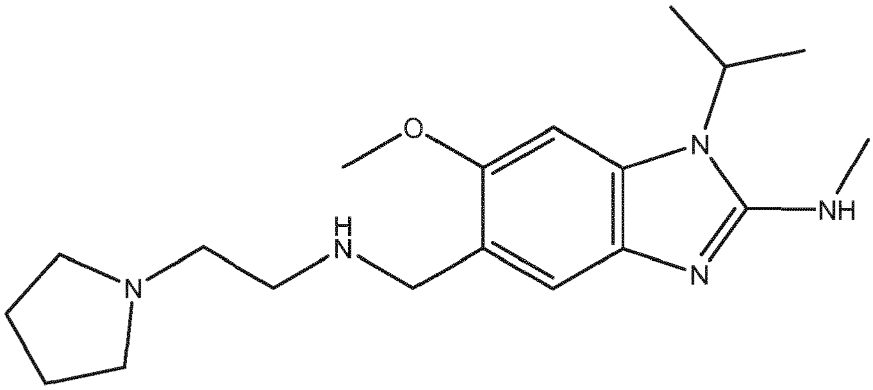 |
| 41 | 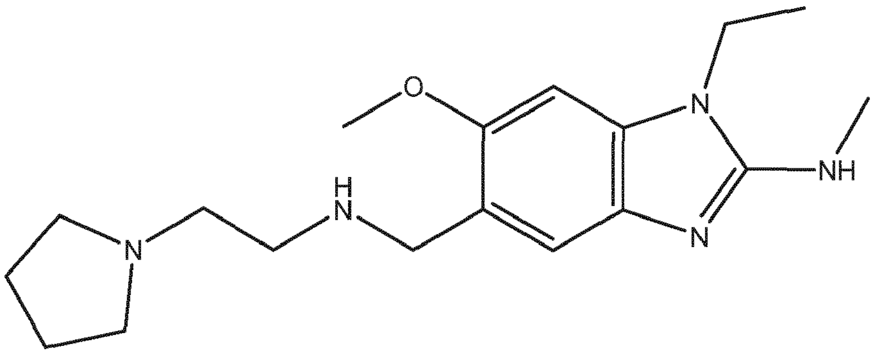 |
| 42 | 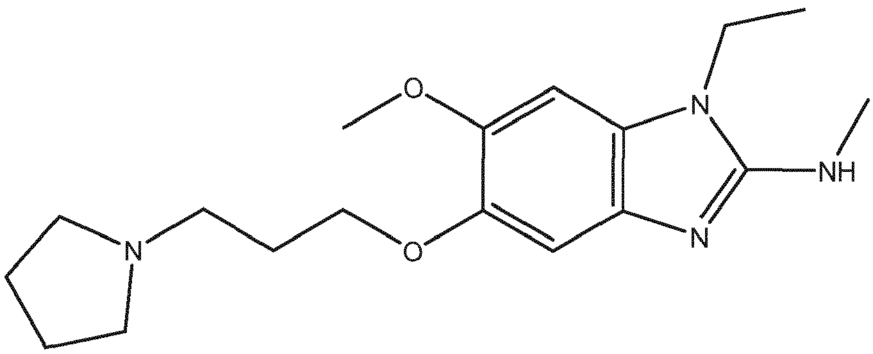 |

(continued)

| Compound No. | Structure |
|---|---|
| 43 | 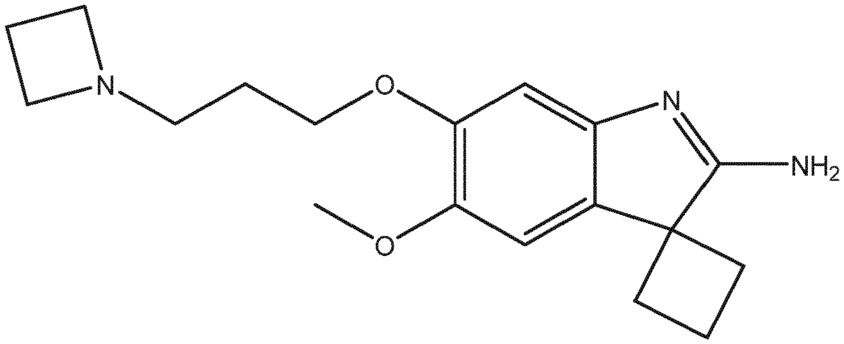 |
| 44 | 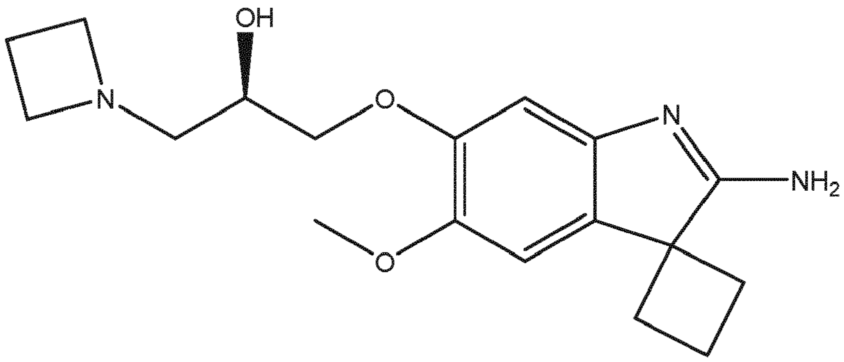 |
| 45 | 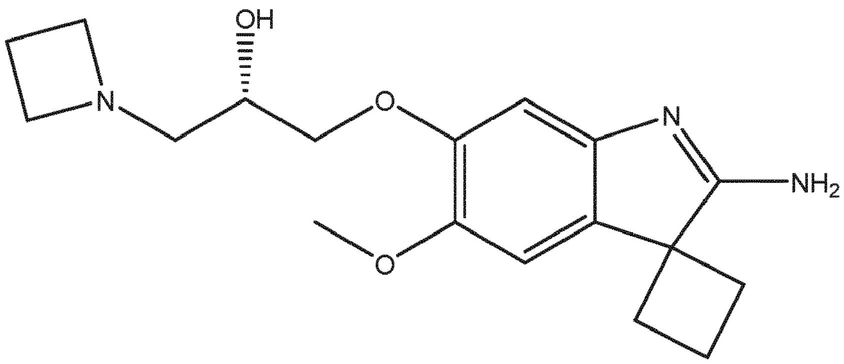 |
| 46 | 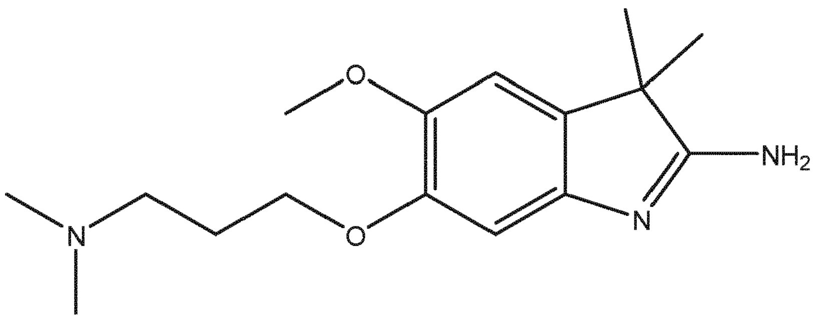 |

(continued)

| Compound No. | Structure |
|---|---|
| 47 | 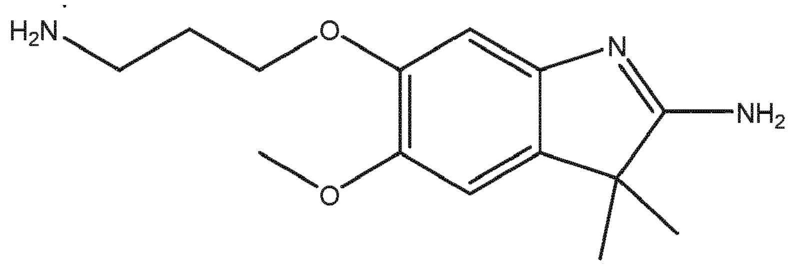 |
| 48 | 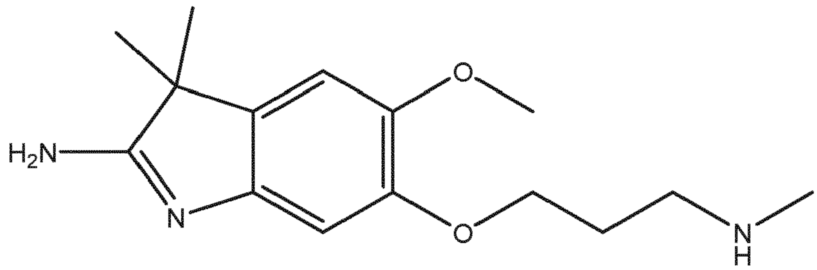 |
| 49 | 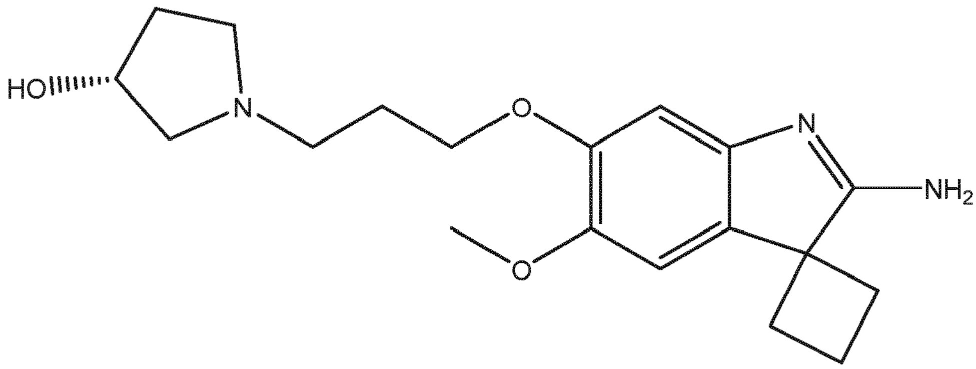 |
| 50 | 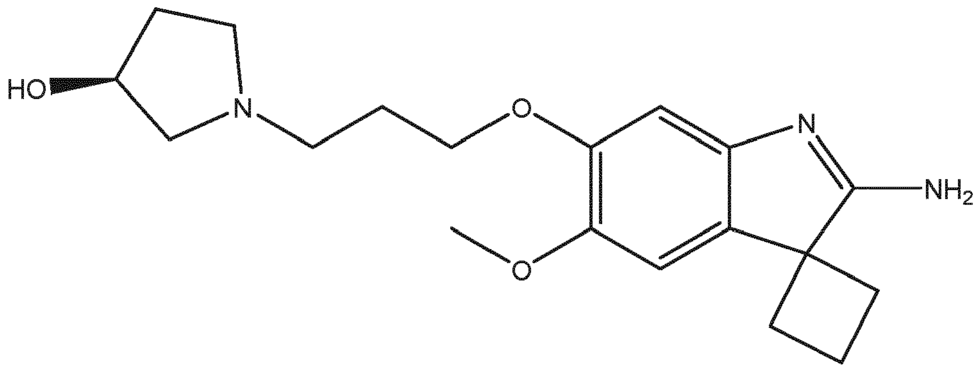 |
| 51 | 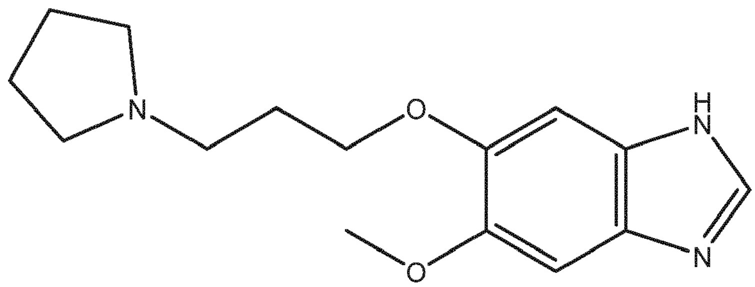 |

(continued)

| Compound No. | Structure |
|---|---|
| 52 | 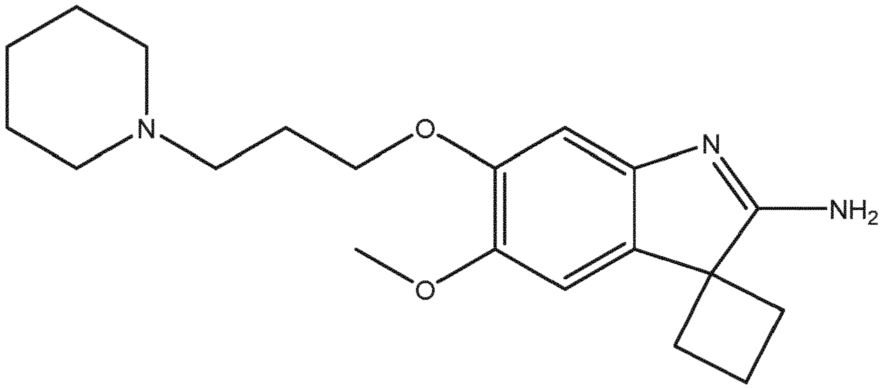 |
| 53 | 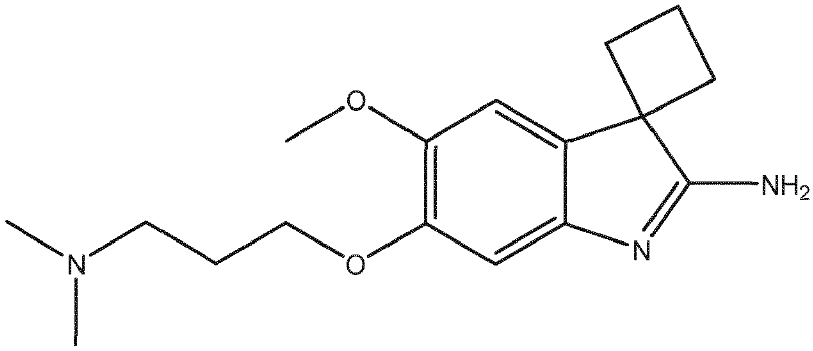 |
| 54 | 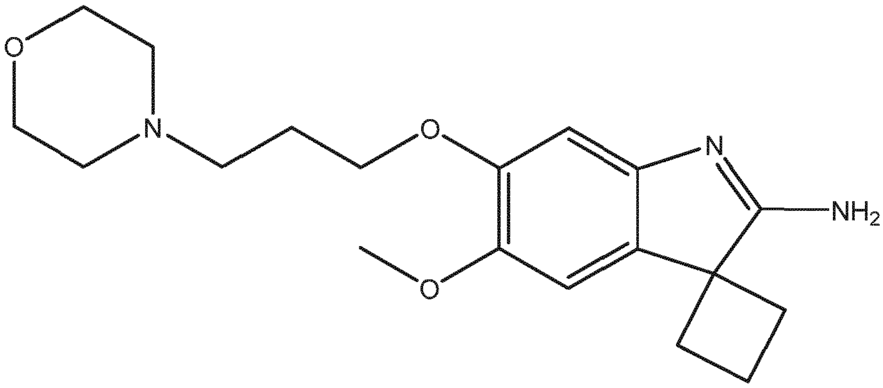 |
| 55 | 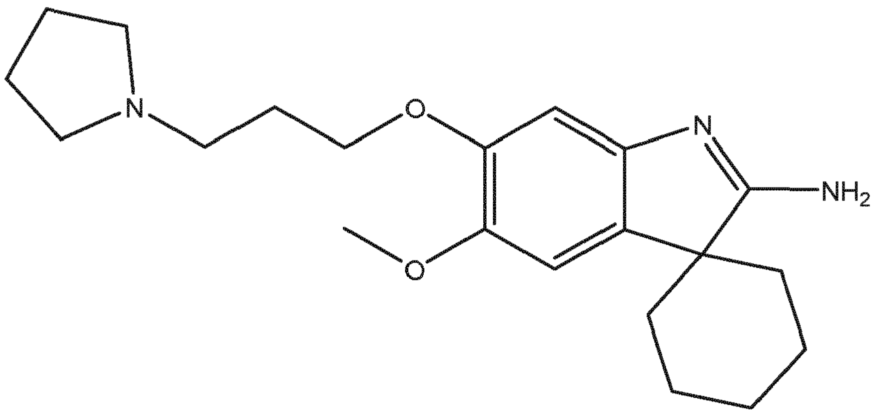 |

(continued)

| Compound No. | Structure |
|---|---|
| 56 | 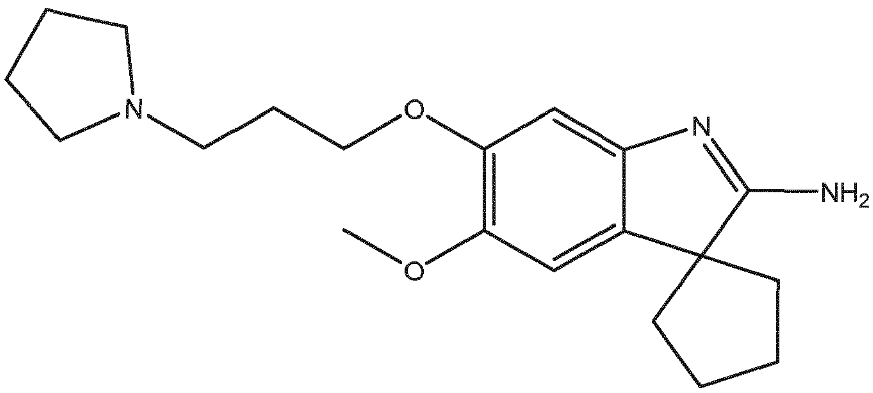 |
| 57 | 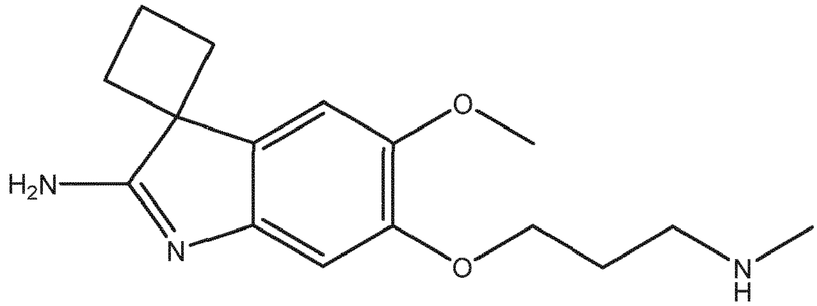 |
| 58 | 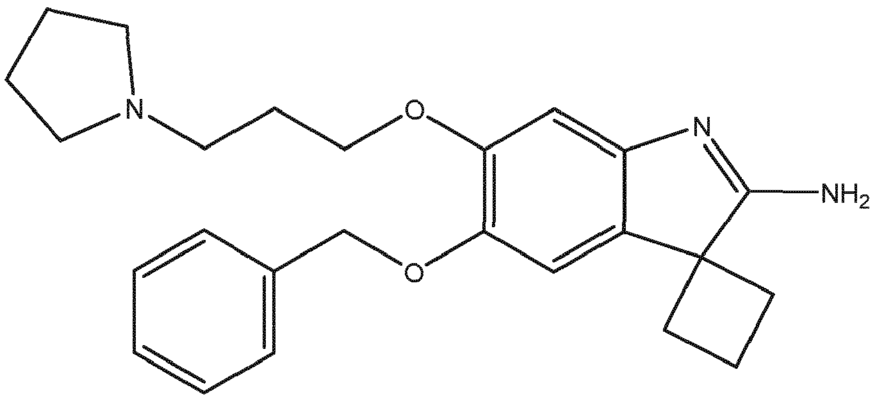 |
| 59 | 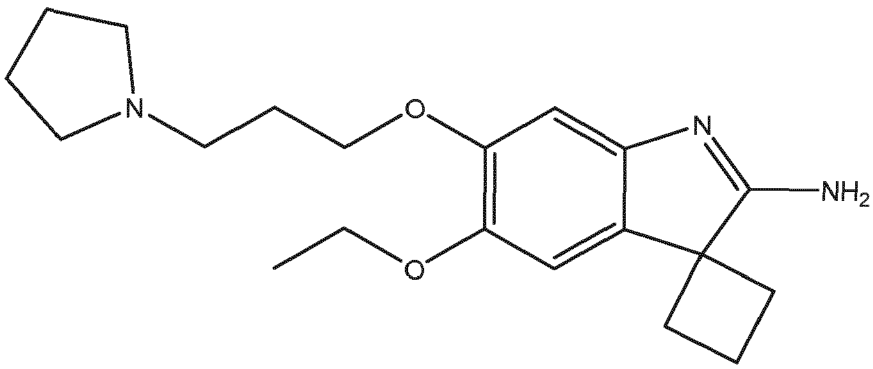 |

(continued)

| Compound No. | Structure |
|---|---|
| 60 | 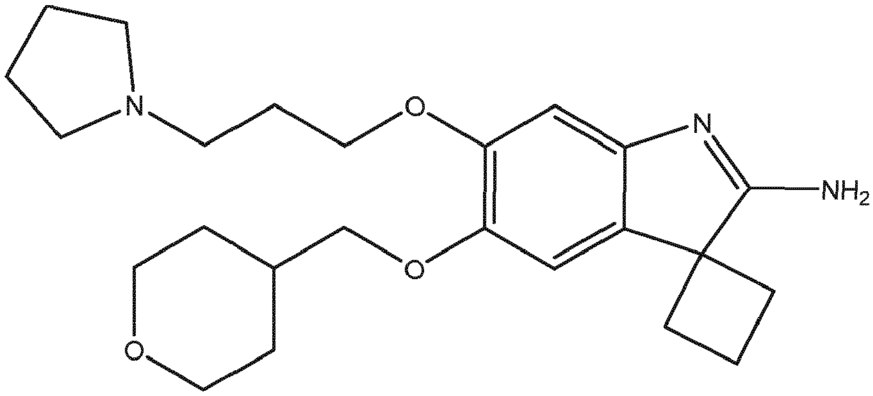 |
| 61 | 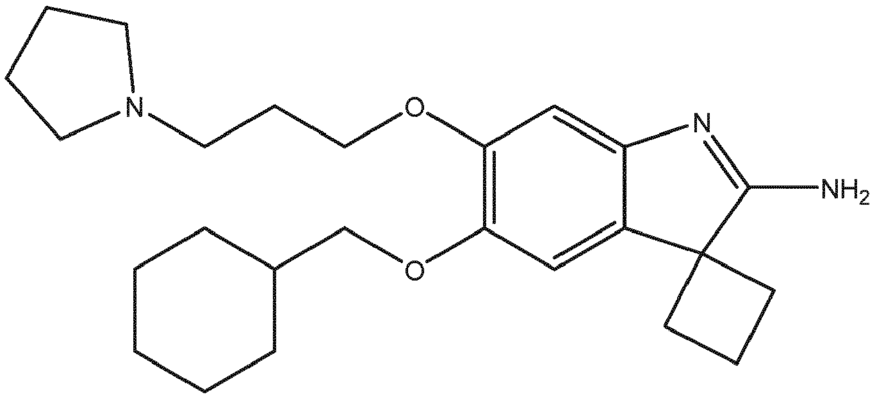 |
| 62 | 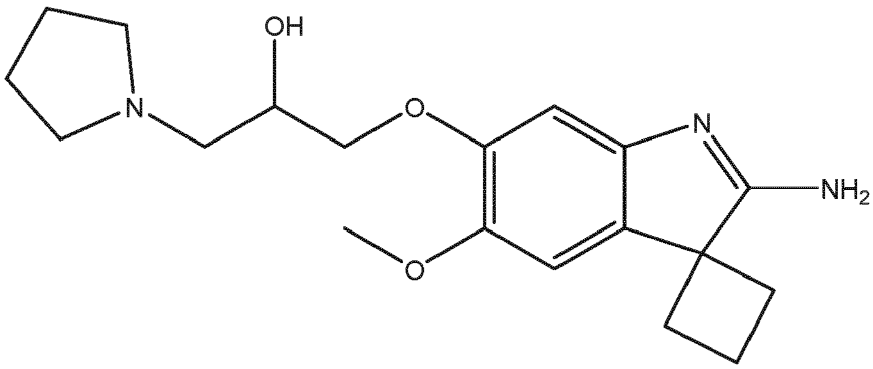 |
| 63 | 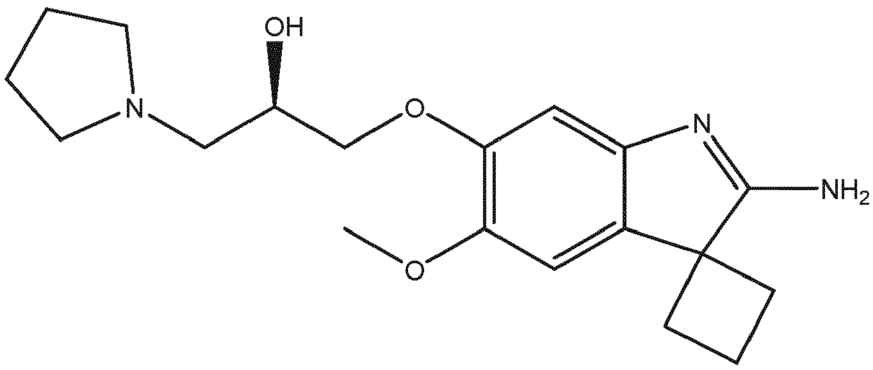 |

(continued)

| Compound No. | Structure |
|---|---|
| 64 | 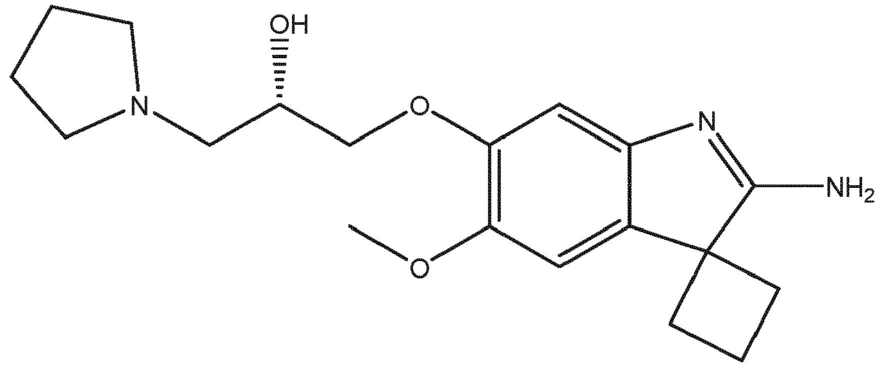 |
| 65 | 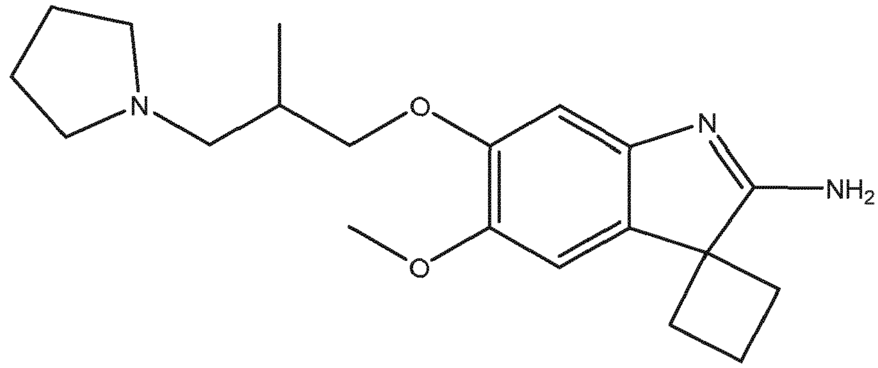 |
| 66 | 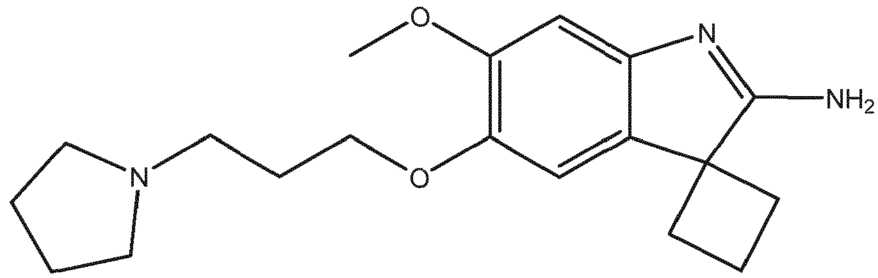 |
| 67 | 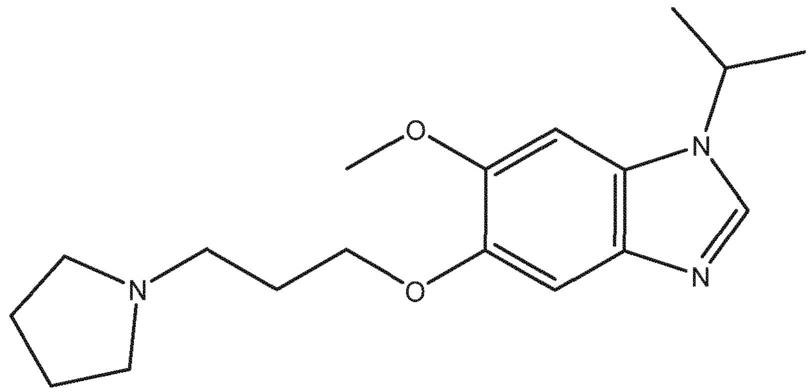 |

(continued)

| Compound No. | Structure |
|---|---|
| 68 | 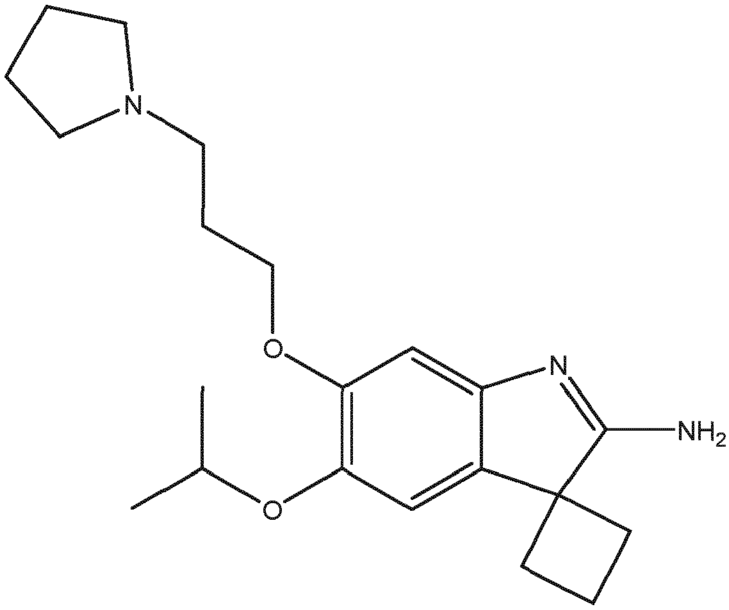 |
| 69 | 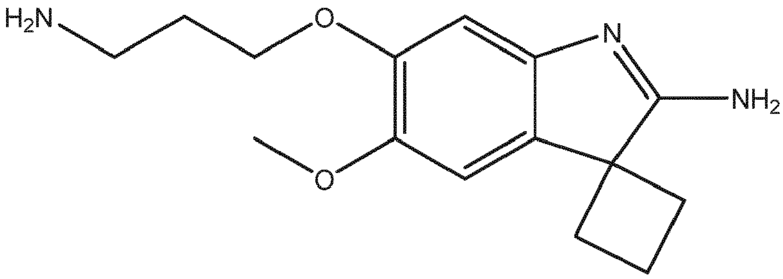 |
| 70 | 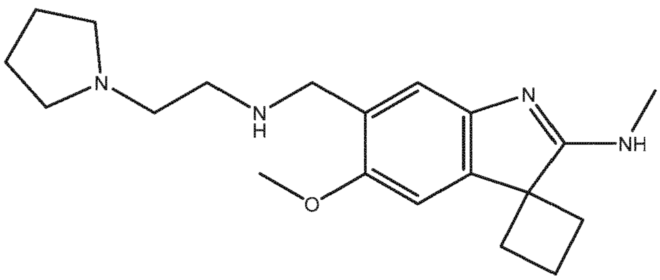 |
| 71 | 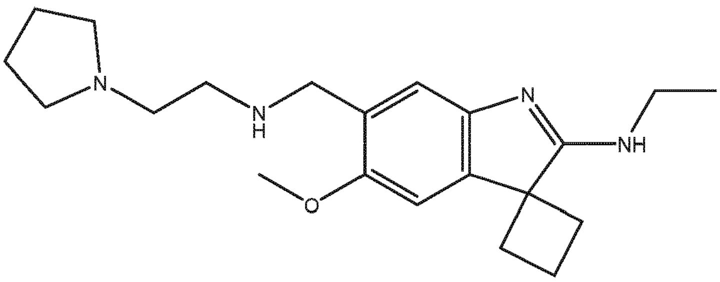 |

(continued)

| Compound No. | Structure |
|---|---|
| 72 | 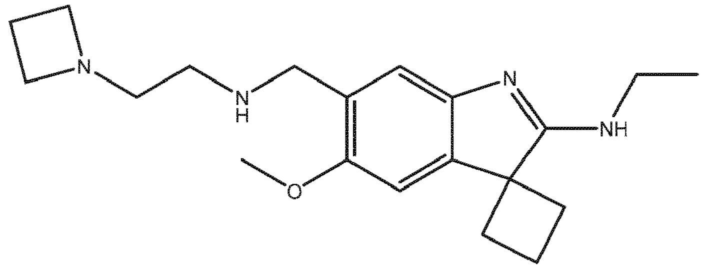 |
| 73 | 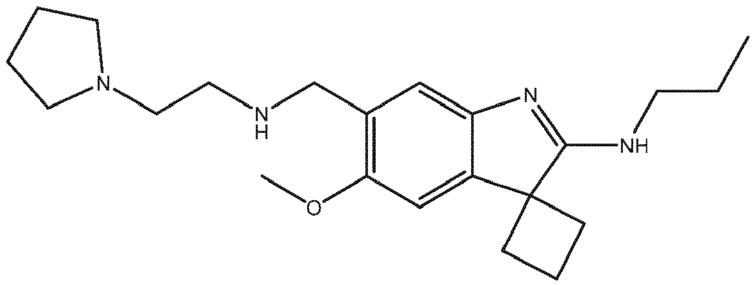 |
| 74 | 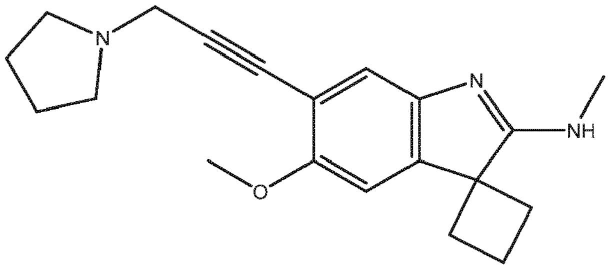 |
| 75 | 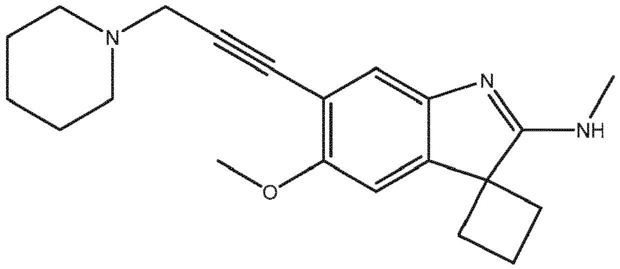 |
| 76 | 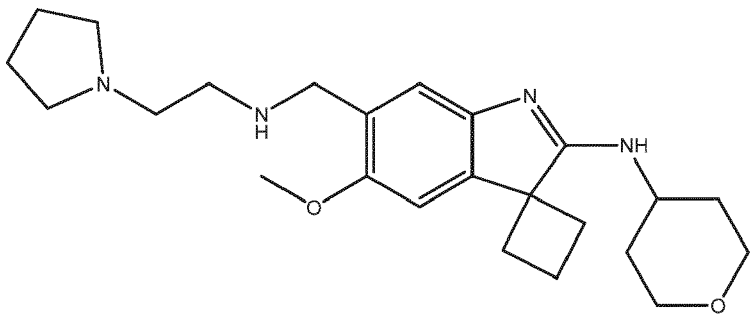 |

(continued)

| Compound No. | Structure |
|---|---|
| 77 | 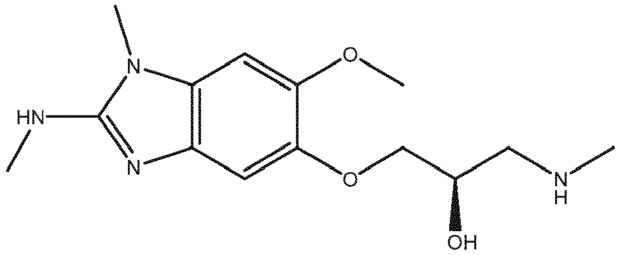 |
| 78 | 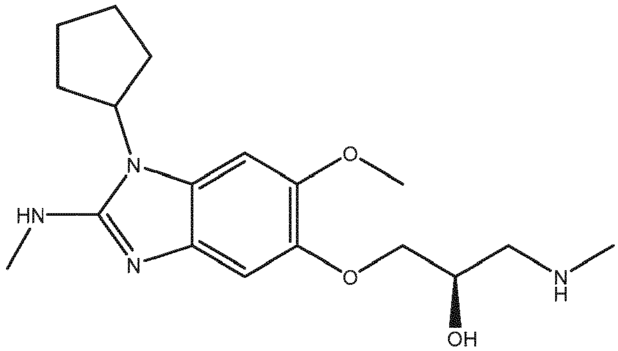 |
| 79 | 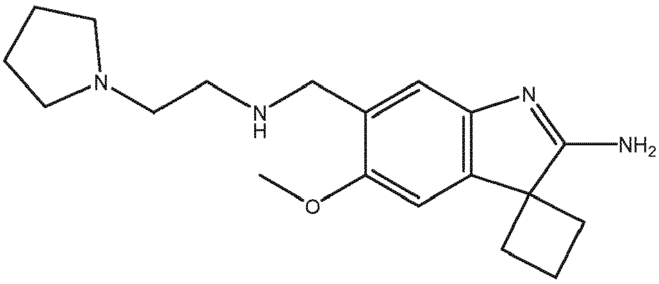 |
| 80 | 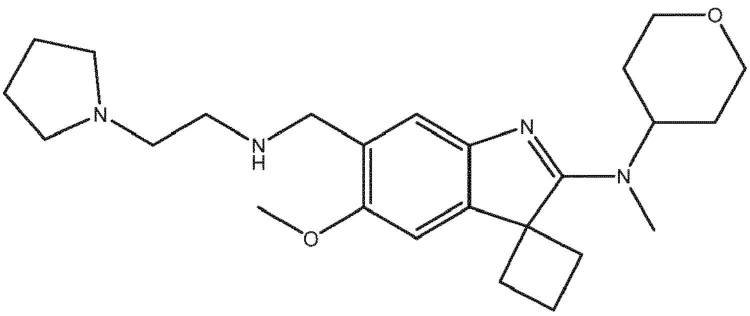 |
| 81 | 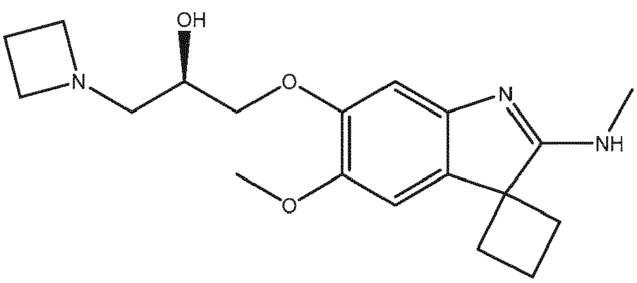 |

(continued)

| Compound No. | Structure |
|---|---|
| 82 | 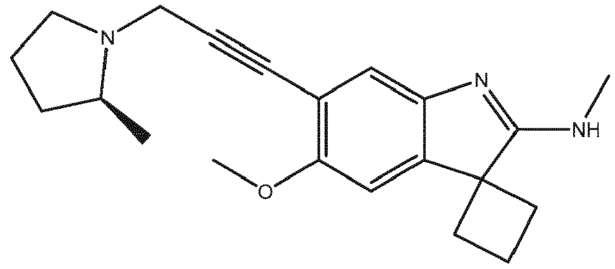 |
| 83 | 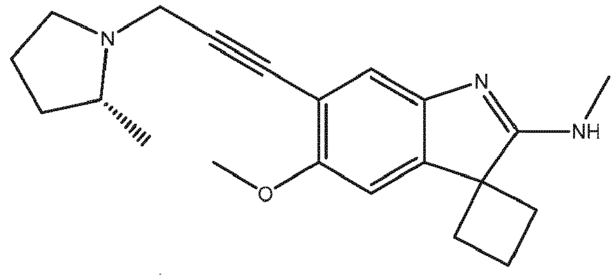 |
| 84 | 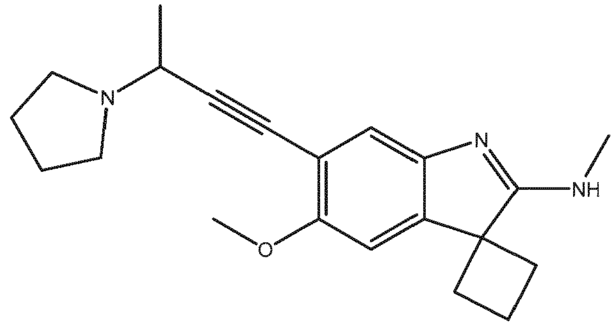 |
| 85 | 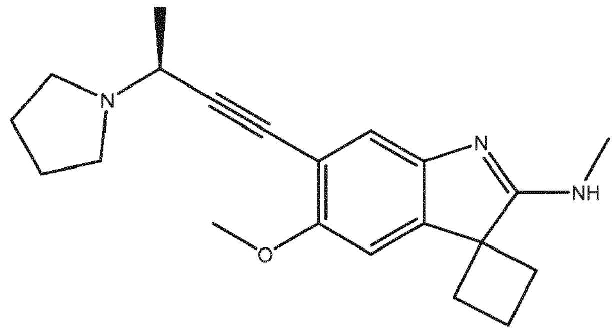 |
| 86 | 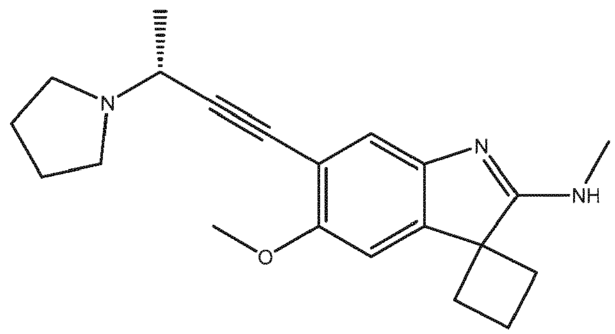 |

(continued)

| Compound No. | Structure |
|---|---|
| 87 | 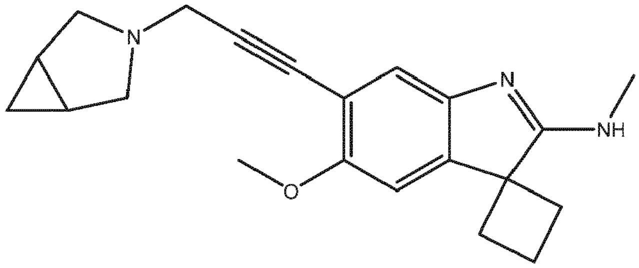 |
| 88 | 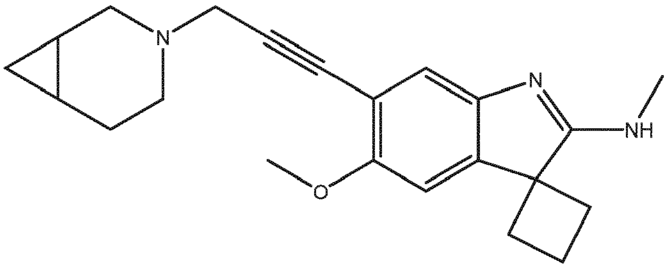 |
| 89 | 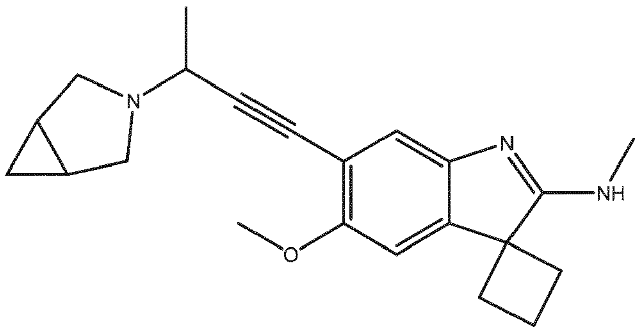 |
| 90 | 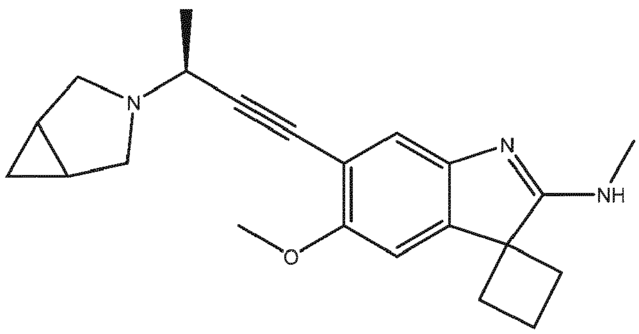 |
| 91 | 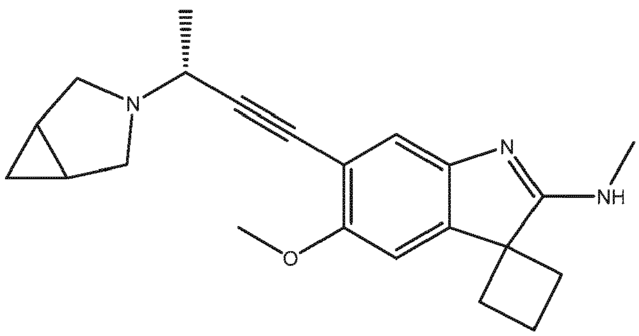 |

(continued)

| Compound No. | Structure |
|---|---|
| 92 | 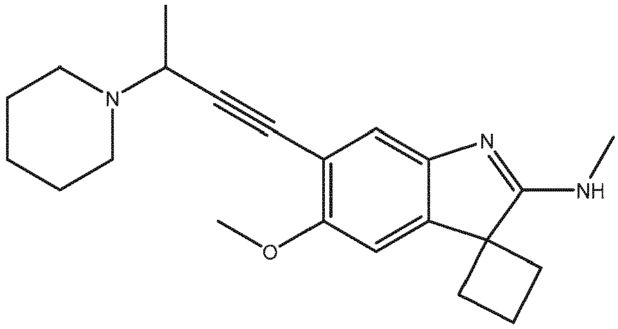 |
| 93 | 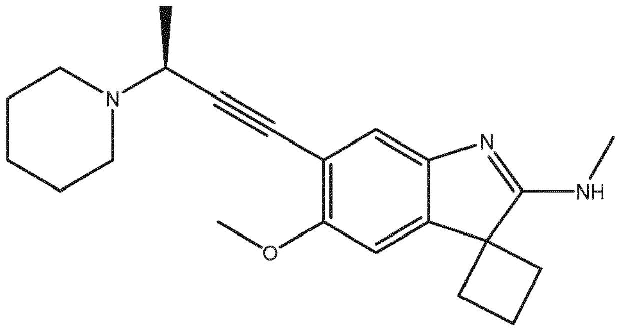 |
| 94 | 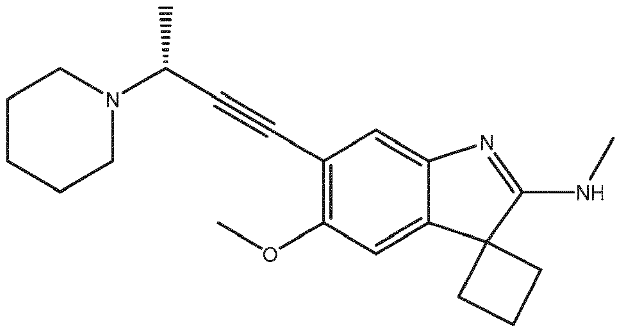 |
| 95 | 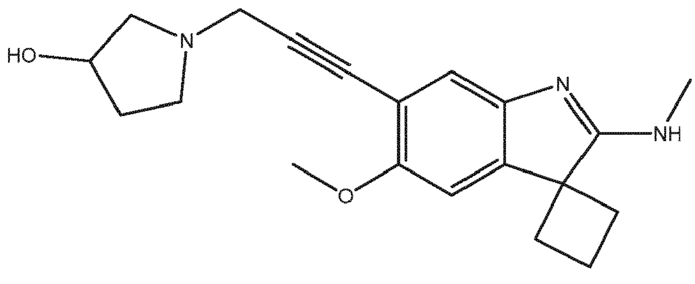 |
| 96 | 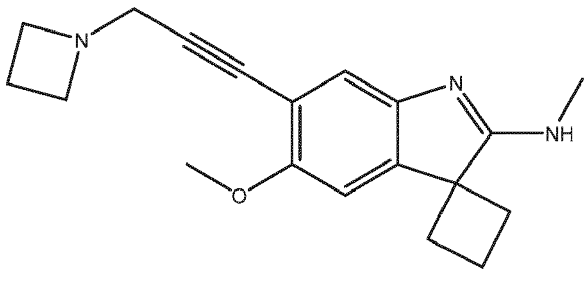 |

(continued)

| Compound No. | Structure |
|---|---|
| 97 | 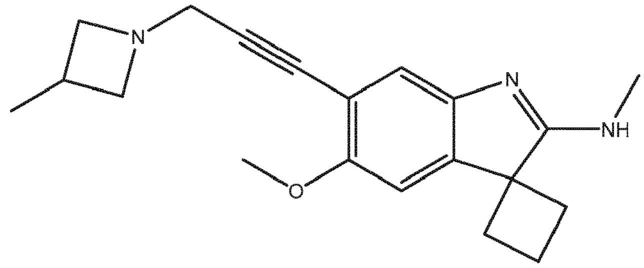 |
| 98 | 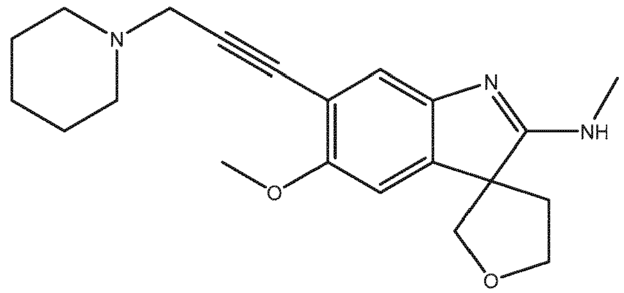 |
| 99 | 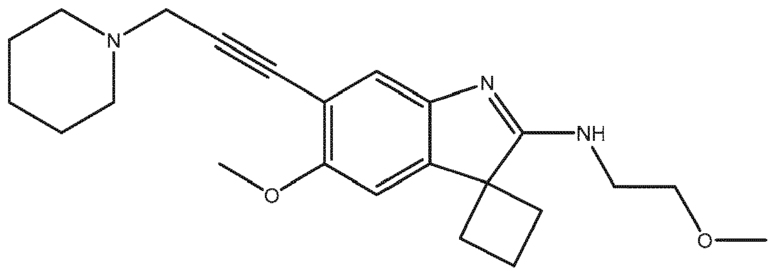 |
| 100 | 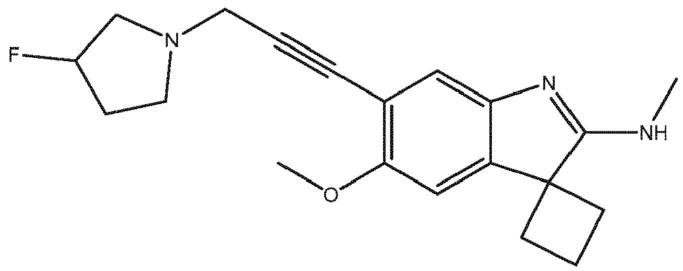 |
| 101 | 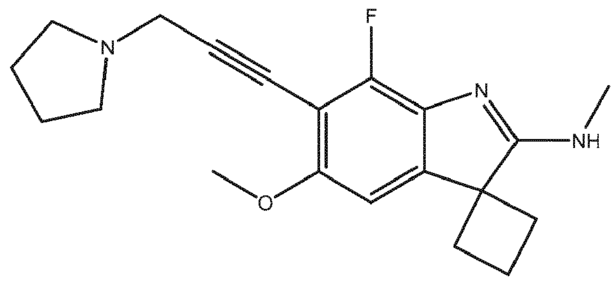 |

(continued)

| Compound No. | Structure |
|---|---|
| 106 | 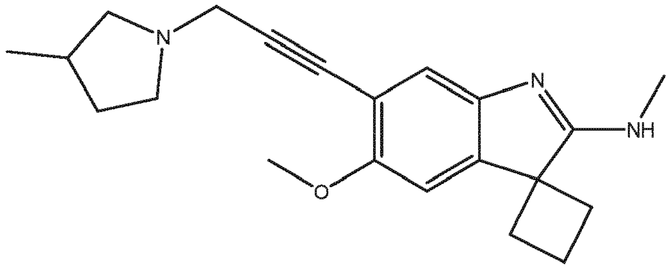 |
| 107 | 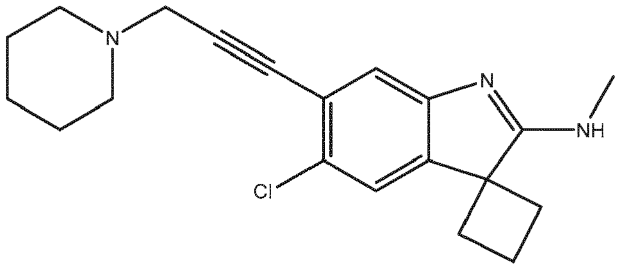 |
| 110 | 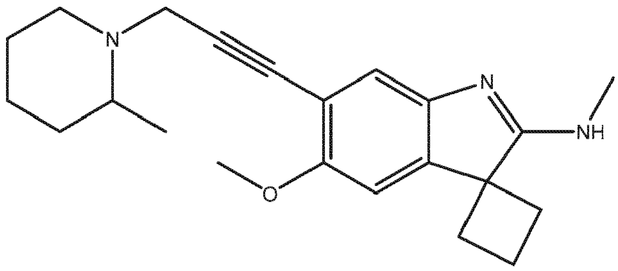 |
| 111 | 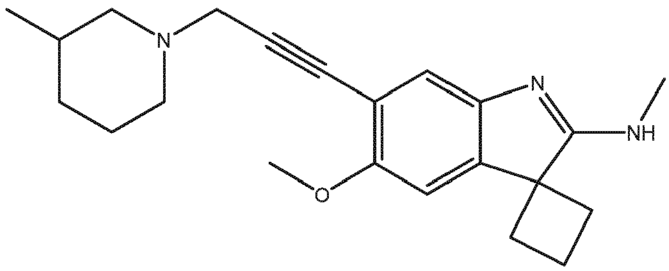 |
| 112 | 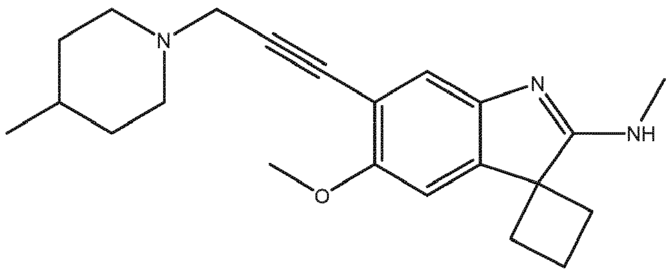 |

(continued)

| Compound No. | Structure |
|---|---|
| 113 | 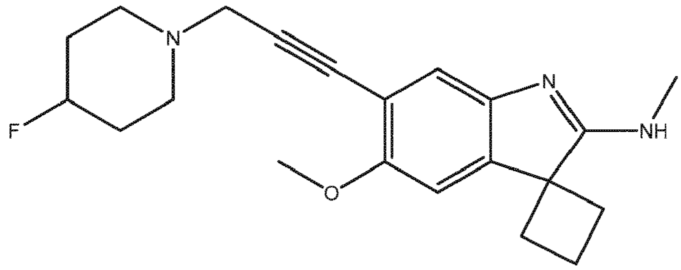 |
| 114 | 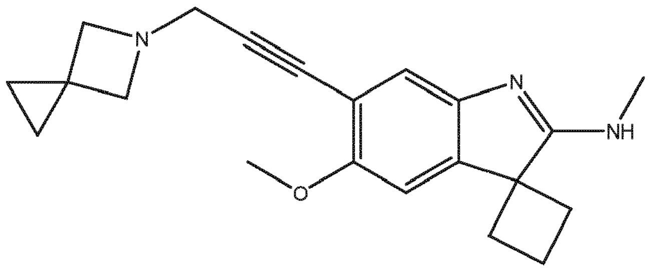 |
| 115 | 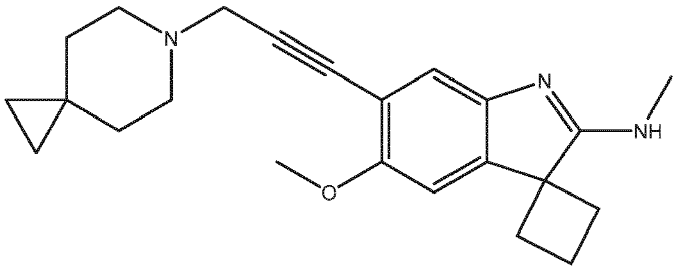 |
| 116 | 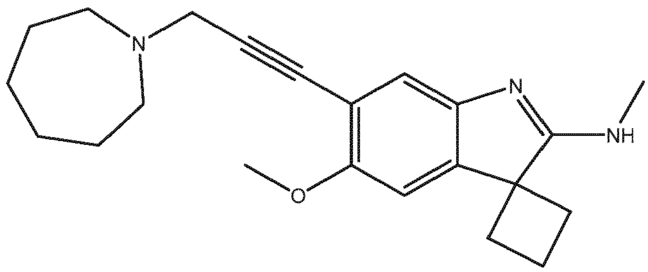 |
| 117 | 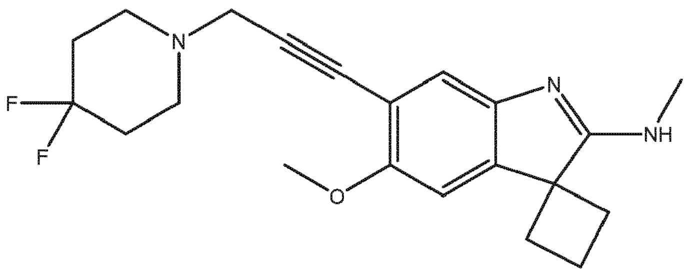 |

(continued)

| Compound No. | Structure |
|---|---|
| 118 | 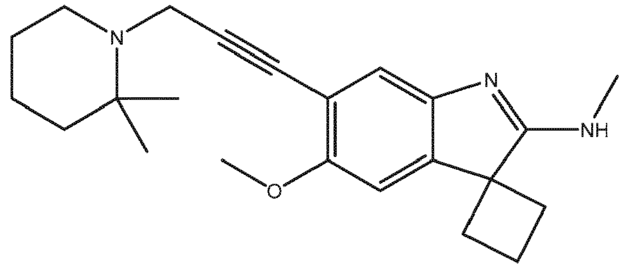 |
| 119 | 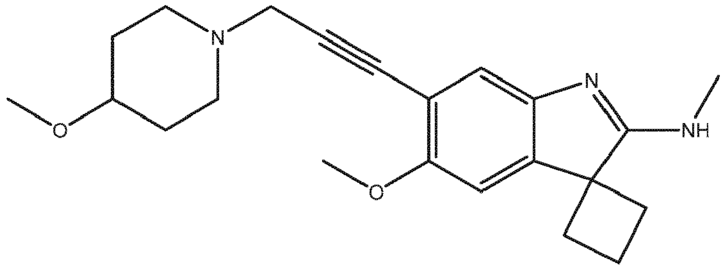 |
| 120 | 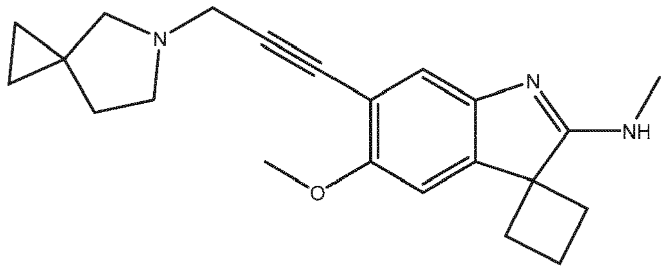 |
| 121 | 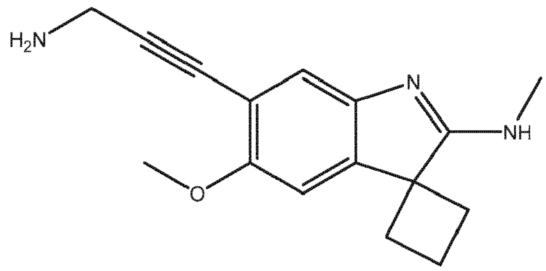 |
| 122 | 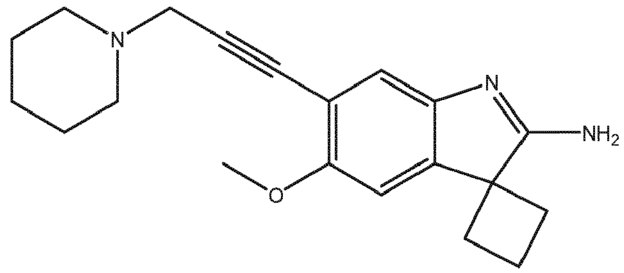 |

(continued)

| Compound No. | Structure |
|---|---|
| 123 | 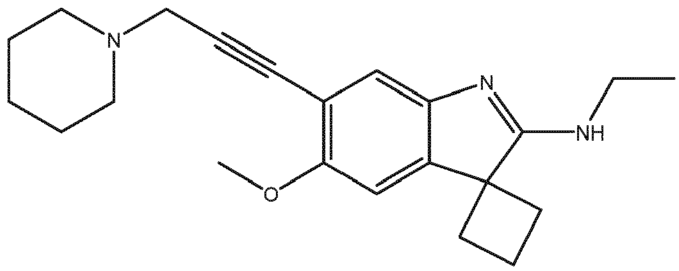 |
| 124 | 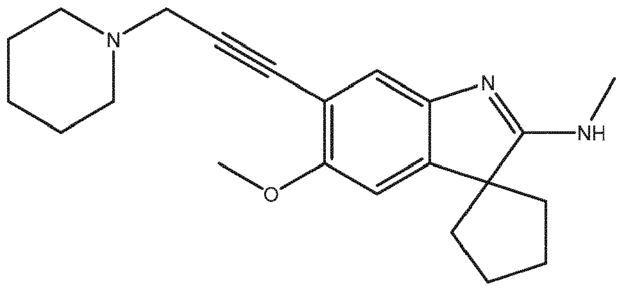 |
| 125 | 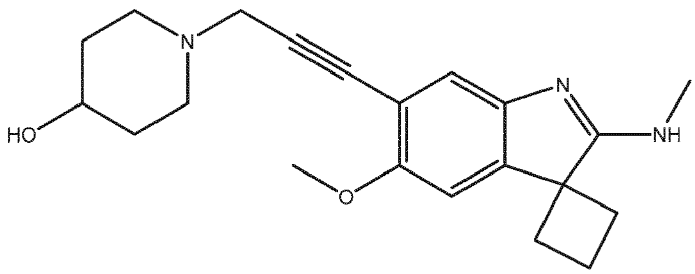 |
| 126 | 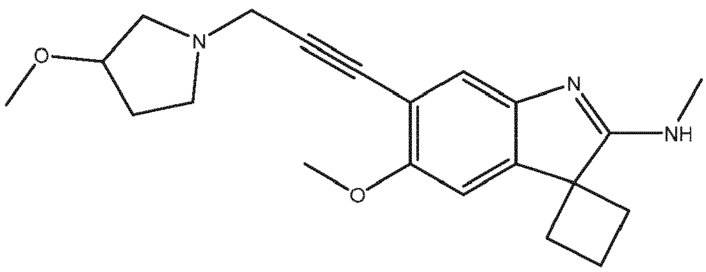 |
| 127 | 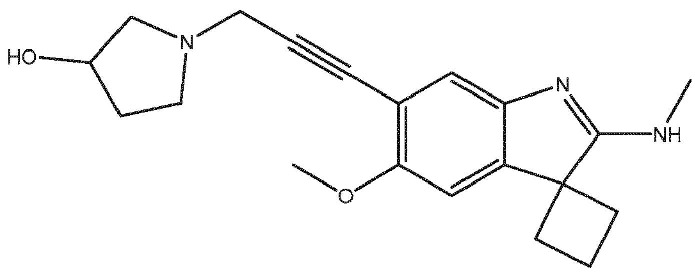 |

(continued)

| Compound No. | Structure |
|---|---|
| 128 | 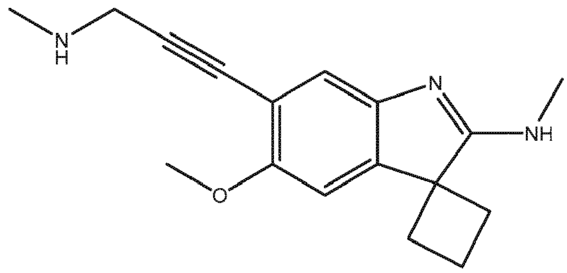 |
| 129 | 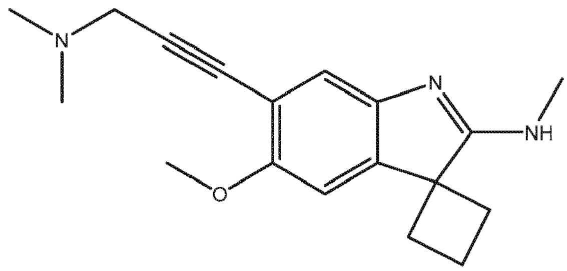 |
| 130 | 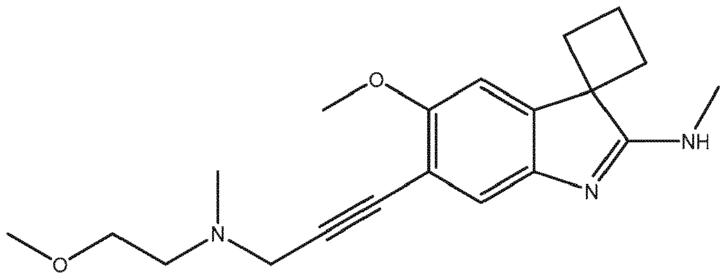 |
| 131 | 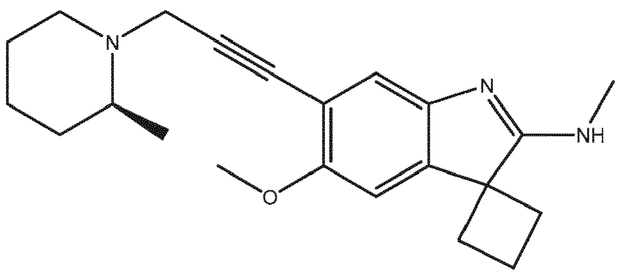 |
| 132 | 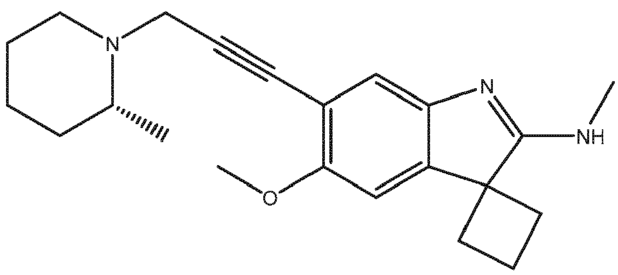 |

(continued)

| Compound No. | Structure |
|---|---|
| 133 | 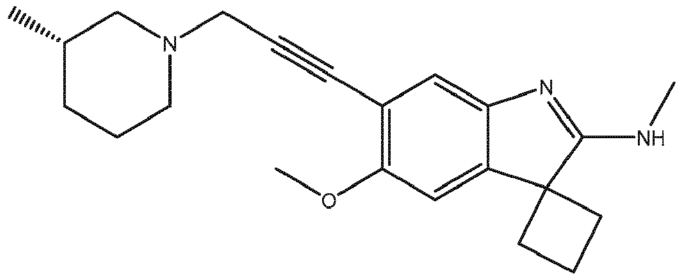 |
| 134 | 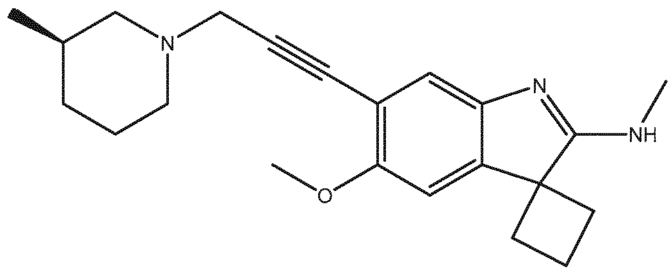 |
| 135 | 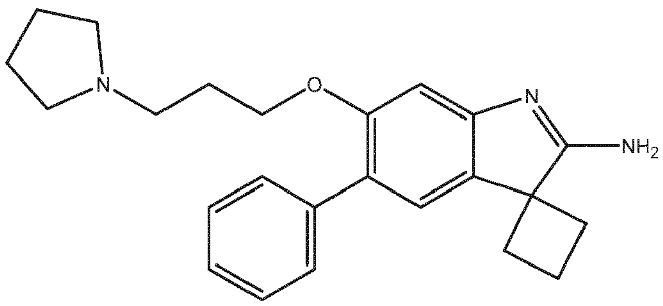 |
| 136 | 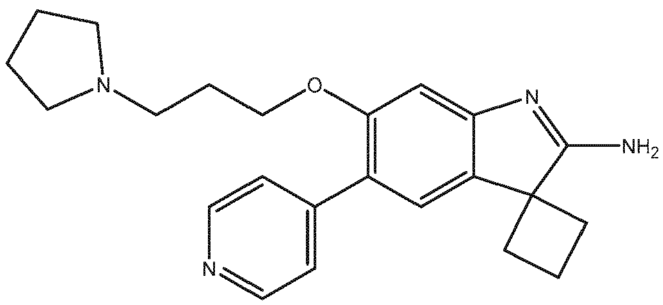 |
| 137 | 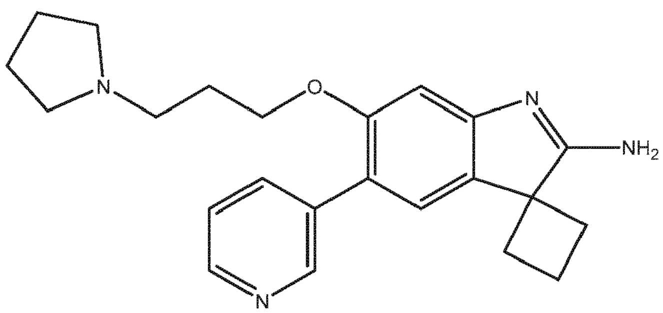 |

(continued)

| Compound No. | Structure |
|---|---|
| 138 | 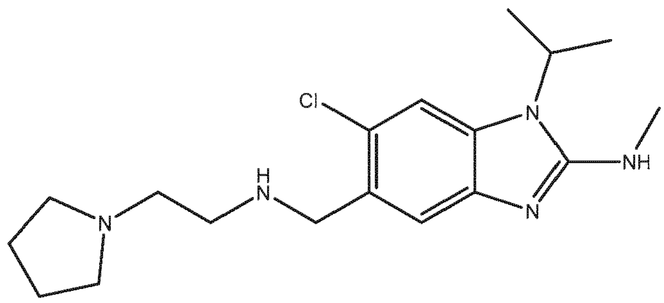 |
| 139 | 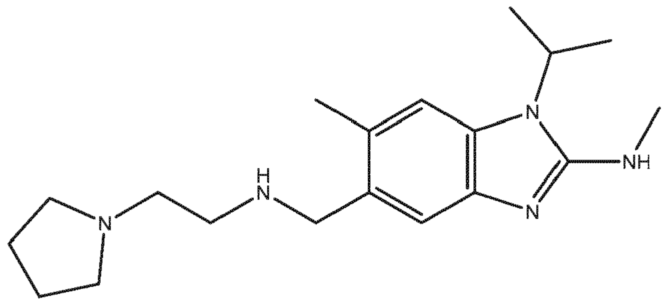 |
| 140 | 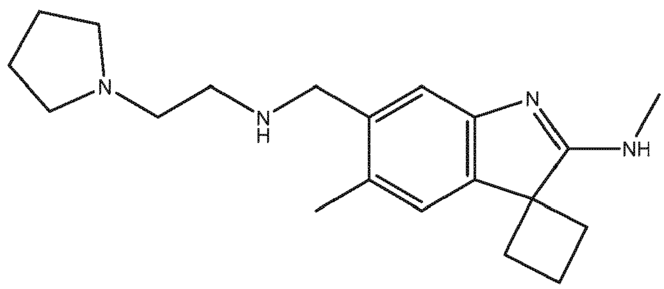 |
| 141 | 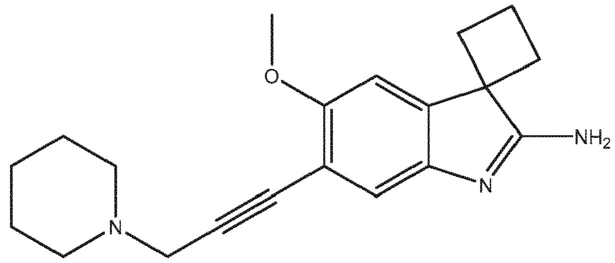 |

As used herein, "alkyl", "$C_1$, $C_2$, $C_3$, $C_4$, $C_5$ or $C_6$ alkyl" or "$C_1$-$C_6$ alkyl" is intended to include $C_1$, $C_2$, $C_3$, $C_4$, $C_5$ or $C_6$ straight chain (linear) saturated aliphatic hydrocarbon groups and $C_3$, $C_4$, $C_5$ or $C_6$ branched saturated aliphatic hydrocarbon groups. For example, $C_1$-$C_6$ alkyl is intended to include $C_1$, $C_2$, $C_3$, $C_4$, $C_5$ and $C_6$ alkyl groups. Examples of alkyl include, moieties having from one to six carbon atoms, such as, but not limited to, methyl, ethyl, n-propyl, i-propyl, n-butyl, s-butyl, t-butyl, n-pentyl, s-pentyl or n-hexyl.

In certain embodiments, a straight chain or branched alkyl has six or fewer carbon atoms (*e.g.,* $C_1$-$C_6$ for straight chain, $C_3$-$C_6$ for branched chain), and in another embodiment, a straight chain or branched alkyl has four or fewer carbon atoms.

As used herein, the term "cycloalkyl" refers to a saturated or unsaturated nonaromatic hydrocarbon mono- or multi-ring (e.g., fused, bridged, or spiro rings) system having 3 to 30 carbon atoms (e.g., $C_3$-$C_{12}$, $C_3$-$C_{10}$, or $C_3$-$C_8$). Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, 1,2,3,4-tetrahydronaphthalenyl, and adamantyl. The term "heterocycloalkyl" refers to a saturated or unsaturated nonaromatic 3-8 membered monocyclic, 7-12 membered bicyclic (fused,

bridged, or spiro rings), or 11-14 membered tricyclic ring system (fused, bridged, or spiro rings) having one or more heteroatoms (such as O, N, S, P, or Se), e.g., 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur, unless specified otherwise. In some embodiments, the one or more heteroatom(s) is selected from the group consisting of nitrogen and oxygen. In some embodiments, the one or more heteroatom(s) is nitrogen. In some embodiments, the one or more heteroatom(s) is oxygen. Examples of heterocycloalkyl groups include, but are not limited to, piperidinyl, piperazinyl, pyrrolidinyl, dioxanyl, tetrahydrofuranyl, isoindolinyl, indolinyl, imidazolidinyl, pyrazolidinyl, oxazolidinyl, isoxazolidinyl, triazolidinyl, oxiranyl, azetidinyl, oxetanyl, thietanyl, 1,2,3,6-tetrahydropyridinyl, tetrahydropyranyl, dihydropyranyl, pyranyl, morpholinyl, tetrahydrothiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 1,4-dioxa-8-azaspiro[4.5]decanyl, 1,4-dioxaspiro[4.5]decanyl, 1-oxaspiro[4.5]decanyl, 1-azaspiro[4.5]decanyl, 3'H-spiro[cyclohexane-1,1'-isobenzofuran]-yl, 7'H-spiro[cyclohexane-1,5'-furo[3,4-b]pyridin]-yl, 3'H-spiro[cyclohexane-1,1'-furo[3,4-c]pyridin]-yl, 3-azabicyclo[3.1.0]hexanyl, 3-azabicyclo[3.1.0]hexan-3-yl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like. In the case of multicyclic nonaromatic rings, only one of the rings needs to be non-aromatic (e.g., 1,2,3,4-tetrahydronaphthalenyl or 2,3-dihydroindole).

The term "optionally substituted alkyl" refers to unsubstituted alkyl or alkyl having designated substituents replacing one or more hydrogen atoms on one or more carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

As used herein, "alkyl linker" or "alkylene linker" is intended to include $C_1$, $C_2$, $C_3$, $C_4$, $C_5$ or $C_6$ straight chain (linear) saturated divalent aliphatic hydrocarbon groups and $C_3$, $C_4$, $C_5$ or $C_6$ branched saturated aliphatic hydrocarbon groups. For example, $C_1$-$C_6$ alkylene linker is intended to include $C_1$, $C_2$, $C_3$, $C_4$, $C_5$ and $C_6$ alkylene linker groups. Examples of alkylene linker include, moieties having from one to six carbon atoms, such as, but not limited to, methyl ($-CH_2-$), ethyl ($-CH_2CH_2-$), n-propyl ($-CH_2CH_2CH_2-$), i-propyl ($-CHCH_3CH_2-$), n-butyl ($-CH_2CH_2CH_2CH_2-$), s-butyl ($-CHCH_3CH_2CH_2-$), i-butyl ($-C(CH_3)_2CH_2-$), n-pentyl ($-CH_2CH_2CH_2CH_2CH_2-$), s-pentyl ($-CHCH_3CH_2CH_2CH_2-$) or n-hexyl ($-CH_2CH_2CH_2CH_2CH_2CH_2-$).

"Alkene linker" or "alkenylene linker" is intended to include $C_2$, $C_3$, $C_4$, $C_5$, and $C_6$ straight chain (linear) unsaturated aliphatic hydrocarbon groups and $C_3$, $C_4$, $C_5$, and $C_6$ branched unsaturated aliphatic hydrocarbon groups that have at least one double bond. For example, $C_2$-$C_6$ alkenylene linker is intended to include $C_2$, $C_3$, $C_4$, $C_5$, and $C_6$ alkenylene linker groups. Examples of alkeylene linker include moieties having from two to six carbon atoms, such as, but not limited to, linear and branched ethenyl, propenyl, butenyl, pentenyl, and hexenyl groups.

"Alkyne linker" or "alkynylene linker" is intended to include $C_2$, $C_3$, $C_4$, $C_5$, and $C_6$ straight chain (linear) unsaturated aliphatic hydrocarbon groups and $C_3$, $C_4$, $C_5$, and $C_6$ branched unsaturated aliphatic hydrocarbon groups that have at least one triple bond. For example, $C_2$-$C_6$ alkynylene linker is intended to include $C_2$, $C_3$, $C_4$, $C_5$, and $C_6$ alkynylene linker groups. Examples of alkynylene linker include moieties having from two to six carbon atoms, such as, but not limited to, linear and branched ethynyl, propynyl, butynyl, pentynyl, and hexynyl groups.

"Alkenyl" includes unsaturated aliphatic groups analogous in length and possible substitution to the alkyls described above, but that contain at least one double bond. For example, the term "alkenyl" includes straight chain alkenyl groups (e.g., ethenyl, propenyl, butenyl, pentenyl, hexenyl, heptenyl, octenyl, nonenyl, decenyl), and branched alkenyl groups.

In certain embodiments, a straight chain or branched alkenyl group has six or fewer carbon atoms in its backbone (e.g., $C_2$-$C_6$ for straight chain, $C_3$-$C_6$ for branched chain). The term "$C_2$-$C_6$" includes alkenyl groups containing two to six carbon atoms. The term "$C_3$-$C_6$" includes alkenyl groups containing three to six carbon atoms.

The term "optionally substituted alkenyl" refers to unsubstituted alkenyl or alkenyl having designated substituents replacing one or more hydrogen atoms on one or more hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato,

sulfamoyl, sulfonamido, nitro, trifluoromethyl, cyano, heterocyclyl, alkylaryl, or an aromatic or heteroaromatic moiety.

"Alkynyl" includes unsaturated aliphatic groups analogous in length and possible substitution to the alkyls described above, but which contain at least one triple bond. For example, "alkynyl" includes straight chain alkynyl groups (e.g., ethynyl, propynyl, butynyl, pentynyl, hexynyl, heptynyl, octynyl, nonynyl, decynyl), and branched alkynyl groups. In certain embodiments, a straight chain or branched alkynyl group has six or fewer carbon atoms in its backbone (e.g., $C_2$-$C_6$ for straight chain, $C_3$-$C_6$ for branched chain). The term "$C_2$-$C_6$" includes alkynyl groups containing two to six carbon atoms. The term "$C_3$-$C_6$" includes alkynyl groups containing three to six carbon atoms. As used herein, "$C_2$-$C_6$ alkenylene linker" or "$C_2$-$C_6$ alkynylene linker" is intended to include $C_2$, $C_3$, $C_4$, $C_5$ or $C_6$ chain (linear or branched) divalent unsaturated aliphatic hydrocarbon groups. For example, $C_2$-$C_6$ alkenylene linker is intended to include $C_2$, $C_3$, $C_4$, $C_5$ and $C_6$ alkenylene linker groups.

The term "optionally substituted alkynyl" refers to unsubstituted alkynyl or alkynyl having designated substituents replacing one or more hydrogen atoms on one or more hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Other optionally substituted moieties (such as optionally substituted cycloalkyl, heterocycloalkyl, aryl, or heteroaryl) include both the unsubstituted moieties and the moieties having one or more of the designated substituents. For example, substituted heterocycloalkyl includes those substituted with one or more alkyl groups, such as, e.g., 2,2,6,6-tetramethyl-piperidinyl and 2,2,6,6-tetramethyl-1,2,3,6-tetrahydropyridinyl.

"Aryl" includes groups with aromaticity, including "conjugated," or multicyclic systems with one or more aromatic rings and do not contain any heteroatom in the ring structure. Examples include phenyl, naphthalenyl, etc.

"Heteroaryl" groups are aryl groups, as defined above, except having from one to four heteroatoms in the ring structure, and may also be referred to as "aryl heterocycles" or "heteroaromatics." As used herein, the term "heteroaryl" is intended to include a stable 5-, 6-, or 7-membered monocyclic or 7-, 8-, 9-, 10-, 11- or 12-membered bicyclic aromatic heterocyclic ring which consists of carbon atoms and one or more heteroatoms, e.g., 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur. The nitrogen atom may be substituted or unsubstituted (*i.e.,* N or NR wherein R is H or other substituents, as defined). The nitrogen and sulfur heteroatoms may optionally be oxidized (*i.e.,* NO and $S(O)_p$, where p = 1 or 2). It is to be noted that total number of S and O atoms in the aromatic heterocycle is not more than 1.

Examples of heteroaryl groups include pyrrole, furan, thiophene, thiazole, isothiazole, imidazole, triazole, tetrazole, pyrazole, oxazole, isoxazole, pyridine, pyrazine, pyridazine, pyrimidine, and the like.

Furthermore, the terms "aryl" and "heteroaryl" include multicyclic aryl and heteroaryl groups, *e.g.,* tricyclic, bicyclic, *e.g.,* naphthalene, benzoxazole, benzodioxazole, benzothiazole, benzoimidazole, benzothiophene, quinoline, isoquinoline, naphthrydine, indole, benzofuran, purine, benzofuran, deazapurine, indolizine.

The cycloalkyl, heterocycloalkyl, aryl, or heteroaryl ring can be substituted at one or more ring positions (e.g., the ring-forming carbon or heteroatom such as N) with such substituents as described above, for example, alkyl, alkenyl, alkynyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl and heteroaryl groups can also be fused or bridged with alicyclic or heterocyclic rings, which are not aromatic so as to form a multi cyclic system (e.g., tetralin, methylenedioxyphenyl such as benzo[d][1,3]dioxole-5-yl).

As used herein, "carbocycle" or "carbocyclic ring" is intended to include any stable monocyclic, bicyclic or tricyclic ring having the specified number of carbons, any of which may be saturated, unsaturated, or aromatic. Carbocycle includes cycloalkyl and aryl. For example, a $C_3$-$C_{14}$ carbocycle is intended to include a monocyclic, bicyclic or tricyclic ring having 3, 4, 5, 6, 7, 8, 9, 10, 11, 12, 13 or 14 carbon atoms. Examples of carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, fluorenyl, phenyl, naphthyl, indanyl, adamantyl and tetrahydronaphthyl. Bridged rings are also included in the definition of carbocycle, including, for example, [3.3.0]bicyclooctane, [4.3.0]bicyclononane, and [4.4.0] bicyclodecane and [2.2.2] bicyclooctane. A bridged ring occurs when one or more

carbon atoms link two non-adjacent carbon atoms. In some embodiments, bridge rings are one or two carbon atoms. It is noted that a bridge always converts a monocyclic ring into a tricyclic ring. When a ring is bridged, the substituents recited for the ring may also be present on the bridge. Fused (e.g., naphthyl, tetrahydronaphthyl) and spiro rings are also included.

As used herein, "heterocycle" or "heterocyclic group" includes any ring structure (saturated, unsaturated, or aromatic) which contains at least one ring heteroatom (e.g., 1-4 heteroatoms selected from N, O and S). Heterocycle includes heterocycloalkyl and heteroaryl. Examples of heterocycles include, but are not limited to, morpholine, pyrrolidine, tetrahydrothiophene, piperidine, piperazine, oxetane, pyran, tetrahydropyran, azetidine, and tetrahydrofuran.

Examples of heterocyclic groups include, but are not limited to, acridinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl (e.g., benzo[d][1,3]dioxole-5-yl), morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2,4-oxadiazol5(4H)-one, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl and xanthenyl.

The term "substituted," as used herein, means that any one or more hydrogen atoms on the designated atom is replaced with a selection from the indicated groups, provided that the designated atom's normal valency is not exceeded, and that the substitution results in a stable compound. When a substituent is oxo or keto (*i.e.,* =O), then 2 hydrogen atoms on the atom are replaced. Keto substituents are not present on aromatic moieties. Ring double bonds, as used herein, are double bonds that are formed between two adjacent ring atoms *(e.g.,* C=C, C=N or N=N). "Stable compound" and "stable structure" are meant to indicate a compound that is sufficiently robust to survive isolation to a useful degree of purity from a reaction mixture, and formulation into an efficacious therapeutic agent.

When a bond to a substituent is shown to cross a bond connecting two atoms in a ring, then such substituent may be bonded to any atom in the ring. When a substituent is listed without indicating the atom via which such substituent is bonded to the rest of the compound of a given formula, then such substituent may be bonded via any atom in such formula. Combinations of substituents and/or variables are permissible, but only if such combinations result in stable compounds.

When any variable (e.g., $R^4$) occurs more than one time in any constituent or formula for a compound, its definition at each occurrence is independent of its definition at every other occurrence. Thus, for example, if a group is shown to be substituted with 0-2 $R^4$ moieties, then the group may optionally be substituted with up to two $R^4$ moieties (e.g., with zero, one, or two $R^4$ moieties), and if more than one $R^4$ moiety is present, $R^4$ at each occurrence is selected independently from the definition of $R^4$ at any other occurrence of $R^4$. Also, combinations of substituents and/or variables are permissible, but only if such combinations result in stable compounds.

The term "hydroxy" or "hydroxyl" includes groups with an -OH or $-O^-$.

As used herein, "halo" or "halogen" refers to fluoro, chloro, bromo and iodo. The term "perhalogenated" generally refers to a moiety wherein all hydrogen atoms are replaced by halogen atoms. The term "haloalkyl" or "haloalkoxyl" refers to an alkyl or alkoxyl substituted with one or more halogen atoms.

The term "carbonyl" includes compounds and moieties which contain a carbon connected with a double bond to an oxygen atom. Examples of moieties containing a carbonyl include, but are not limited to, aldehydes, ketones, carboxylic acids, amides, esters, anhydrides, etc.

The term "carboxyl" refers to -COOH or its $C_1$-$C_6$ alkyl ester.

"Acyl" includes moieties that contain the acyl radical (R-C(O)-) or a carbonyl group. "Substituted acyl" includes acyl groups where one or more of the hydrogen atoms are replaced by, for example, alkyl groups, alkynyl groups, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

"Aroyl" includes moieties with an aryl or heteroaromatic moiety bound to a carbonyl group. Examples of aroyl

groups include phenylcarboxy, naphthyl carboxy, etc.

"Alkoxyalkyl," "alkylaminoalkyl," and "thioalkoxyalkyl" include alkyl groups, as described above, wherein oxygen, nitrogen, or sulfur atoms replace one or more hydrocarbon backbone carbon atoms.

The term "alkoxy" or "alkoxyl" includes substituted and unsubstituted alkyl, alkenyl and alkynyl groups covalently linked to an oxygen atom. Examples of alkoxy groups or alkoxyl radicals include, but are not limited to, methoxy, ethoxy, isopropyloxy, propoxy, butoxy and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy and trichloromethoxy.

The term "ether" or "alkoxy" includes compounds or moieties which contain an oxygen bonded to two carbon atoms or heteroatoms. For example, the term includes "alkoxyalkyl," which refers to an alkyl, alkenyl, or alkynyl group covalently bonded to an oxygen atom which is covalently bonded to an alkyl group.

The term "ester" includes compounds or moieties which contain a carbon or a heteroatom bound to an oxygen atom which is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, etc.

The term "thioalkyl" includes compounds or moieties which contain an alkyl group connected with a sulfur atom. The thioalkyl groups can be substituted with groups such as alkyl, alkenyl, alkynyl, halogen, hydroxyl, alkylcarbonyloxy, arylcarbonyloxy, alkoxycarbonyloxy, aryloxycarbonyloxy, carboxylate, carboxyacid, alkylcarbonyl, arylcarbonyl, alkoxycarbonyl, aminocarbonyl, alkylaminocarbonyl, dialkylaminocarbonyl, alkylthiocarbonyl, alkoxyl, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties.

The term "thiocarbonyl" or "thiocarboxy" includes compounds and moieties which contain a carbon connected with a double bond to a sulfur atom.

The term "thioether" includes moieties which contain a sulfur atom bonded to two carbon atoms or heteroatoms. Examples of thioethers include, but are not limited to alkthioalkyls, alkthioalkenyls, and alkthioalkynyls. The term "alkthioalkyls" include moieties with an alkyl, alkenyl, or alkynyl group bonded to a sulfur atom which is bonded to an alkyl group. Similarly, the term "alkthioalkenyls" refers to moieties wherein an alkyl, alkenyl or alkynyl group is bonded to a sulfur atom which is covalently bonded to an alkenyl group; and alkthioalkynyls" refers to moieties wherein an alkyl, alkenyl or alkynyl group is bonded to a sulfur atom which is covalently bonded to an alkynyl group.

As used herein, "amine" or "amino" refers to $-NH_2$. "Alkylamino" includes groups of compounds wherein the nitrogen of $-NH_2$ is bound to at least one alkyl group. Examples of alkylamino groups include benzylamino, methylamino, ethylamino, phenethylamino, etc. "Dialkylamino" includes groups wherein the nitrogen of $-NH_2$ is bound to two alkyl groups. Examples of dialkylamino groups include, but are not limited to, dimethylamino and diethylamino. "Arylamino" and "diarylamino" include groups wherein the nitrogen is bound to at least one or two aryl groups, respectively. "Aminoaryl" and "aminoaryloxy" refer to aryl and aryloxy substituted with amino. "Alkylarylamino," "alkylaminoaryl" or "arylaminoalkyl" refers to an amino group which is bound to at least one alkyl group and at least one aryl group. "Alkaminoalkyl" refers to an alkyl, alkenyl, or alkynyl group bound to a nitrogen atom which is also bound to an alkyl group. "Acylamino" includes groups wherein nitrogen is bound to an acyl group. Examples of acylamino include, but are not limited to, alkylcarbonylamino, arylcarbonylamino, carbamoyl and ureido groups.

The term "amide" or "aminocarboxy" includes compounds or moieties that contain a nitrogen atom that is bound to the carbon of a carbonyl or a thiocarbonyl group. The term includes "alkaminocarboxy" groups that include alkyl, alkenyl or alkynyl groups bound to an amino group which is bound to the carbon of a carbonyl or thiocarbonyl group. It also includes "arylaminocarboxy" groups that include aryl or heteroaryl moieties bound to an amino group that is bound to the carbon of a carbonyl or thiocarbonyl group. The terms "alkylaminocarboxy", "alkenylaminocarboxy", "alkynylaminocarboxy" and "arylaminocarboxy" include moieties wherein alkyl, alkenyl, alkynyl and aryl moieties, respectively, are bound to a nitrogen atom which is in turn bound to the carbon of a carbonyl group. Amides can be substituted with substituents such as straight chain alkyl, branched alkyl, cycloalkyl, aryl, heteroaryl or heterocycle. Substituents on amide groups may be further substituted.

Compounds of the present disclosure that contain nitrogens can be converted to N-oxides by treatment with an oxidizing agent (e.g., 3-chloroperoxybenzoic acid (*m*CPBA) and/or hydrogen peroxides) to afford other compounds of the present disclosure. Thus, all shown and claimed nitrogen-containing compounds are considered, when allowed

by valency and structure, to include both the compound as shown and its N-oxide derivative (which can be designated as N→O or $N^+$-$O^-$). Furthermore, in other instances, the nitrogens in the compounds of the present disclosure can be converted to N-hydroxy or N-alkoxy compounds. For example, N-hydroxy compounds can be prepared by oxidation of the parent amine by an oxidizing agent such as m-CPBA. All shown and claimed nitrogen-containing compounds are also considered, when allowed by valency and structure, to cover both the compound as shown and its N-hydroxy *(i.e.,* N-OH) and N-alkoxy (*i.e.,* N-OR, wherein R is substituted or unsubstituted $C_1$-$C_6$ alkyl, $C_1$-$C_6$ alkenyl, $C_1$-$C_6$ alkynyl, 3-14-membered carbocycle or 3-14-membered heterocycle) derivatives.

In the present specification, the structural formula of the compound represents a certain isomer for convenience in some cases, but the present disclosure includes all isomers, such as geometrical isomers, optical isomers based on an asymmetrical carbon, stereoisomers, tautomers, and the like, it being understood that not all isomers may have the same level of activity. In addition, a crystal polymorphism may be present for the compounds represented by the formula. It is noted that any crystal form, crystal form mixture, or anhydride or hydrate thereof is included in the scope of the present disclosure.

"Isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

A carbon atom bonded to four nonidentical substituents is termed a "chiral center."

"Chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the Sequence Rule of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

"Geometric isomer" means the diastereomers that owe their existence to hindered rotation about double bonds or a cycloalkyl linker (e.g., 1,3-cylcobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds of the present disclosure may be depicted as different chiral isomers or geometric isomers. It should also be understood that when compounds have chiral isomeric or geometric isomeric forms, all isomeric forms are intended to be included in the scope of the present disclosure, and the naming of the compounds does not exclude any isomeric forms, it being understood that not all isomers may have the same level of activity.

Furthermore, the structures and other compounds discussed in this disclosure include all atropic isomers thereof, it being understood that not all atropic isomers may have the same level of activity. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent and pH. The concept of tautomers that are interconvertable by tautomerizations is called tautomerism.

Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

Common tautomeric pairs are: ketone-enol, amide-nitrile, lactam-lactim, amide-imidic acid tautomerism in heterocyclic rings *(e.g.,* in nucleobases such as guanine, thymine and cytosine), imine-enamine and enamine-enamine. Examples of lactam-lactim tautomerism are as shown below.

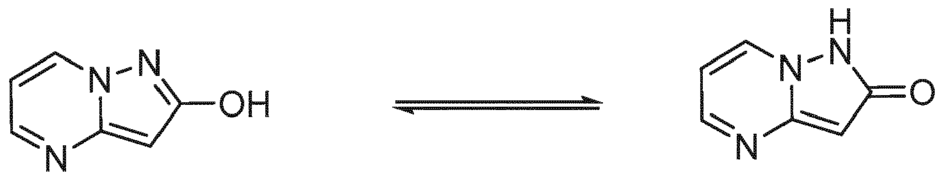

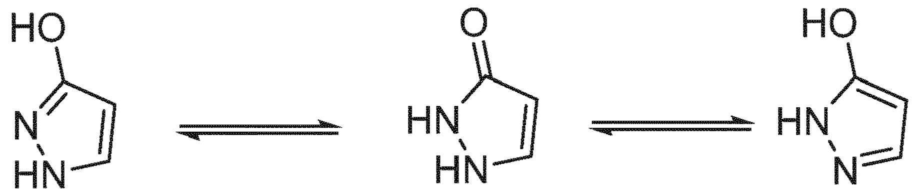

It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form. It will be understood that certain tautomers may have a higher level of activity than others.

The term "crystal polymorphs", "polymorphs" or "crystal forms" means crystal structures in which a compound (or a salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

The compounds of any Formula described herein include the compounds themselves, as well as their salts, and their solvates, if applicable. A salt, for example, can be formed between an anion and a positively charged group (e.g., amino) on a substituted benzene compound. Suitable anions include chloride, bromide, iodide, sulfate, bisulfate, sulfamate, nitrate, phosphate, citrate, methanesulfonate, trifluoroacetate, glutamate, glucuronate, glutarate, malate, maleate, succinate, fumarate, tartrate, tosylate, salicylate, lactate, naphthalenesulfonate, and acetate (e.g., trifluoroacetate). The term "pharmaceutically acceptable anion" refers to an anion suitable for forming a pharmaceutically acceptable salt. Likewise, a salt can also be formed between a cation and a negatively charged group (e.g., carboxylate) on a substituted benzene compound. Suitable cations include sodium ion, potassium ion, magnesium ion, calcium ion, and an ammonium cation such as tetramethylammonium ion. The substituted benzene compounds also include those salts containing quaternary nitrogen atoms.

Additionally, the compounds of the present disclosure, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvate" means solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as $H_2O$.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative" refers to compounds that have a common core structure, and are substituted with various groups as described herein. For example, all of the compounds represented by Formula (I) are substituted fused bi- or tri- heterocyclic compounds, and have Formula (I) as a common core.

The term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include, but are not limited to, acyl sulfonimides, tetrazoles, sulfonates and phosphonates. See, *e.g.,* Patani and LaVoie, Chem. Rev. 96, 3147-3176, 1996.

The present disclosure is intended to include all isotopes of atoms occurring in the present compounds. Isotopes include those atoms having the same atomic number but different mass numbers. By way of general example and without

limitation, isotopes of hydrogen include tritium and deuterium, and isotopes of carbon include C-13 and C-14.

The present disclosure provides methods for the synthesis of the compounds of any of the Formulae described herein. The present disclosure also provides detailed methods for the synthesis of various disclosed compounds of the present disclosure according to the following schemes as well as those shown in the Examples.

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

The synthetic processes of the disclosure can tolerate a wide variety of functional groups, therefore various substituted starting materials can be used. The processes generally provide the desired final compound at or near the end of the overall process, although it may be desirable in certain instances to further convert the compound to a pharmaceutically acceptable salt thereof.

Compounds of the present disclosure can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March's Advanced Organic Chemistry: Reactions, Mechanisms, and Structure, 5th edition, John Wiley & Sons: New York, 2001; Greene, T.W., Wuts, P.G. M., Protective Groups in Organic Synthesis, 3rd edition, John Wiley & Sons: New York, 1999; R. Larock, Comprehensive Organic Transformations, VCH Publishers (1989); L. Fieser and M. Fieser, Fieser and Fieser's Reagents for Organic Synthesis, John Wiley and Sons (1994); and L. Paquette, ed., Encyclopedia ofReagents for Organic Synthesis, John Wiley and Sons (1995), incorporated by reference herein, are useful and recognized reference textbooks of organic synthesis known to those in the art. The following descriptions of synthetic methods are designed to illustrate, but not to limit, general procedures for the preparation of compounds of the present disclosure.

Compounds of the present disclosure can be conveniently prepared by a variety of methods familiar to those skilled in the art. The compounds of this disclosure having any of the Formulae described herein may be prepared according to the procedures illustrated in Schemes 1-5 below, from commercially available starting materials or starting materials which can be prepared using literature procedures. Certain variables (such as $R^{1'}$ and $R^8$) in Schemes 1-5 are as defined in any Formula described herein, unless otherwise specified.

One of ordinary skill in the art will note that, during the reaction sequences and synthetic schemes described herein, the order of certain steps may be changed, such as the introduction and removal of protecting groups.

One of ordinary skill in the art will recognize that certain groups may require protection from the reaction conditions via the use of protecting groups. Protecting groups may also be used to differentiate similar functional groups in molecules. A list of protecting groups and how to introduce and remove these groups can be found in Greene, T.W., Wuts, P.G. M., Protective Groups in Organic Synthesis, 3rd edition, John Wiley & Sons: New York, 1999.

Preferred protecting groups include, but are not limited to:

For a hydroxyl moiety: TBS, benzyl, THP, Ac;
For carboxylic acids: benzyl ester, methyl ester, ethyl ester, allyl ester;
For amines: Cbz, BOC, DMB;
For diols: Ac (x2) TBS (x2), or when taken together acetonides;
For thiols: Ac;
For benzimidazoles: SEM, benzyl, PMB, DMB;
For aldehydes: di-alkyl acetals such as dimethoxy acetal or diethyl acetyl.

In the reaction schemes described herein, multiple stereoisomers may be produced. When no particular stereoisomer is indicated, it is understood to mean all possible stereoisomers that could be produced from the reaction. A person of ordinary skill in the art will recognize that the reactions can be optimized to give one isomer preferentially, or new schemes may be devised to produce a single isomer. If mixtures are produced, techniques such as preparative thin layer chromatography, preparative HPLC, preparative chiral HPLC, or preparative SFC may be used to separate the isomers.

The following abbreviations are used throughout the specification and are defined below:

| | |
|---|---|
| ACN | acetonitrile |
| Ac | acetyl |

| | |
|---|---|
| AcOH | acetic acid |
| AlCl3 | aluminum chloride |
| BINAP | (2,2'-bis(diphenylphosphino)-1,1'-binaphthyl) |
| t-BuOK | potassium t-butoxide |
| tBuONa or t-BuONa | sodium t-butoxide |
| br | broad |
| BOC | tert-butoxy carbonyl |
| Cbz | benzyloxy carbonyl |
| $CDCl_3CHCl_3$ | chloroform |
| $CH_2Cl_2$ | dichloromethane |
| $CH_3CN$ | acetonitrile |
| $CsCO_3$ | cesium carbonate |
| $CH_3NO_3$ | nitromethane |
| d | doublet |
| dd | doublet of doublets |
| dq | doublet of quartets |
| DCE | 1,2 dichloroethane |
| DCM | dichloromethane |
| $\Delta$ | heat |
| $\delta$ | chemical shift |
| DIEA | N,N-diisopropylethylamine (Hunig's base) |
| DMB | 2,4 dimethoxy benzyl |
| DMF | N,N-Dimethylformamide |
| DMSO | Dimethyl sulfoxide |
| DMSO-*d6* | deuterated dimethyl sulfoxide |
| EA or EtOAc | Ethyl acetate |
| ES | electrospray |
| $Et_3N$ | triethylamine |
| equiv | equivalents |
| g | grams |
| h | hours |
| $H_2O$ | water |
| HCl | hydrogen chloride or hydrochloric acid |
| HPLC | High performance liquid chromatography |
| Hz | Hertz |
| IPA | isopropyl alcohol |
| i-PrOH | isopropyl alcohol |
| J | NMR coupling constant |
| $K_2CO_3$ | potassium carbonate |
| HI | potassium iodide |
| KCN | potassium cyanide |
| LCMS or LC-MS | Liquid chromatography mass spectrum |
| M | molar |
| m | multiplet |
| mg | milligram |
| MHz | megahertz |
| mL | milliliter |
| mm | millimeter |
| mmol | millimole |
| mol | mole |
| [M+1] | molecular ion plus one mass unit |
| m/z | mass/charge ratio |
| m-CPBA | meta-chloroperbenzoic acid |
| MeCN | Acetonitrile |
| MeOH | methanol |
| MeI | Methyl iodide |
| min | minutes |
| $\mu$m | micron |

| | |
|---|---|
| MsCl | Mesyl chloride |
| MW | microwave irradiation |
| N | normal |
| $Na_2SO_4$ | sodium sulfate |
| $NH_3$ | ammonia |
| $NaBH(AcO)_3$ | sodium triacetoxyborohydride |
| NaI | sodium iodide |
| $Na_2SO_4$ | sodium sulfate |
| $NH_4Cl$ | ammonium chloride |
| $NH_4HCO_3$ | ammonium bicarbonate |
| nm | nanometer |
| NMP | N-methylpyrrolidinone |
| NMR | Nuclear Magnetic Resonance |
| $Pd(OAc)_2$ | palladium (II) acetate |
| Pd/C | Palladium on carbon |
| $Pd_2(dba)_3$ | Tris(dibenzylideneacetone)dipalladium(0) |
| PMB | para methoxybenzyl |
| ppm | parts per million |
| $POCl_3$ | phosphoryl chloride |
| prep-HPLC | preparative High Performance Liquid Chromatography |
| PTSA | para-toluenesulfonic acid |
| p-TsOH | para-toluenesulfonic acid |
| RT | retention time |
| rt | room temperature |
| s | singlet |
| t | triplet |
| t-BuXPhos | 2-Di-*tert*-butylphosphino-2', 4', 6'-triisopropylbiphenyl |
| TEA | Triethylamine |
| TFA | trifluoroacetic acid |
| TfO | triflate |
| THP | tetrahydropyran |
| TsOH | tosic acid |
| UV | ultraviolet |

Scheme 1 shows the synthesis of 4-(4-(benzyloxy)-3-methoxyphenyl)spiroxy-4-carbonitrile intermediates **C1** following a general route. 1-bromo-2-(2-bromoethoxy)ethane or like reagent is combined in an organic solvent *(e.g.* DMF) with benzonitrile **A1** and base *(e.g.* NaH). The resulting spirotetrahydropyran or related analog **C1** can be used in further elaboration such as nitration, nitro reduction and intramolecular cyclization

## Scheme 2

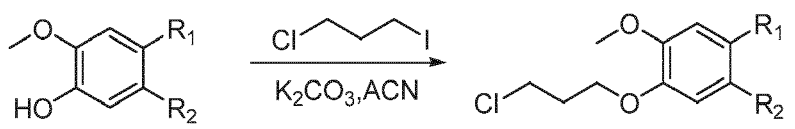


**A2** **B2**

Scheme 2 shows alkylation of a 2-methoxyphenol derivative A2 following a general route. 1-chloro-1-iodopropane is combined in organic solvent (e.g. ACN) with the phenol reactant **A2** under heating to afford the resulting 1-(3-chloropropoxy)-2-methoxybenzene derivative **B2.**

## Scheme 3

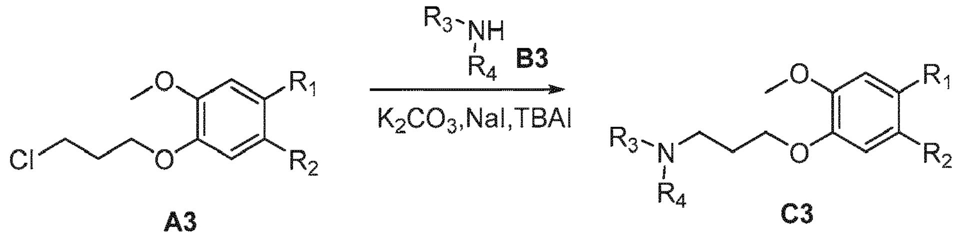


A3 C3

Scheme 3 shows reaction of a basic amine to an aliphatic halide following a general route. A 1-(3-chloropropoxy)-2-methoxybenzene derivative **A3** is combined with an amine (*e.g.* pyrrolidine) in an organic solvent *(e.g.* ACN) and a base (*e.g.* $K_2CO_3$) in the presence of NaI and TBAI to facilitate conversion to afford a 1-(3-(2-methoxyphenoxy)propyl)pyrrolidine derivative **C3** or related compound.

## Scheme 4

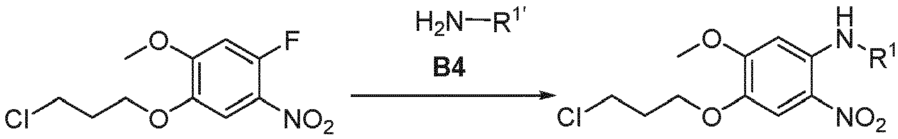


A4 C4

Scheme 4 depicts $S_NAr$ chemistry whereby an arylflouride derivative A4 is reactive with a substituted amine following a general route. An aryl fluoride A4 is combined with a basic amine (*e.g.* methylamine) in an organic solvent *(e.g.* ACN) and base (*e.g.* $K_2CO_3$) under reflux heating to afford aniline derivative C4.

## Scheme 5

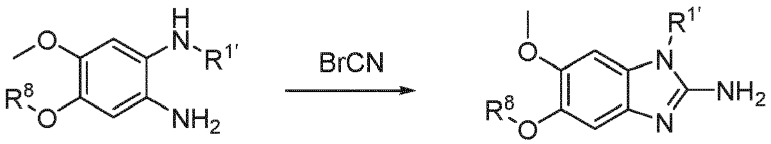


A5 B5

Scheme 5 depicts ring closure of a diaminoaryl compound to an aminobenzimidazole following a general route. A diaminobenzene derivative **A5** is treated with cyanic bromide in an organic solvent (e.g. ACN/H2O) under mild heating to afford cyclized aminobenzimidazoles of type **B5.**

A person of ordinary skill in the art will recognize that in the above schemes the order of many of the steps are interchangeable.

## Scheme 6

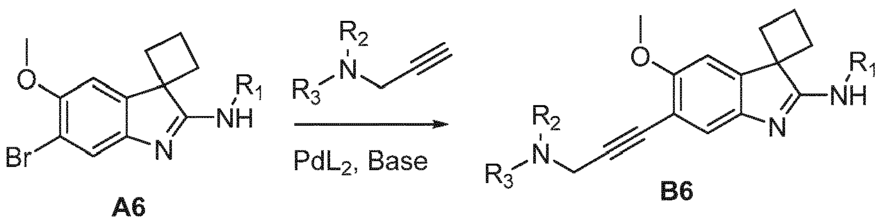


A6 B6

Scheme 6 depicts addition of an acetylene moiety to a halogenated aromatic following a general route. An aryl bromide **A6** is treated with a modified propargylamine in the presence of a palladium catalyst and inorganic base in a polar solvent to afford the aryl acetylene derivatives of type **B6.**

Compounds of the present disclosure inhibit the histone methyltransferase activity of G9a, also known as

KMT1C (lysine methyltransferase 1C) or EHMT2 (euchromatic histone methyltransferase 2), or a mutant thereof and, accordingly, in one aspect of the disclosure, certain compounds disclosed herein are candidates for treating, or preventing certain conditions, diseases, and disorders in which EHMT2 plays a role. The present disclosure provides methods for treating conditions and diseases the course of which can be influenced by modulating the methylation status of histones or other proteins, wherein said methylation status is mediated at least in part by the activity of EHMT2. Modulation of the methylation status of histones can in turn influence the level of expression of target genes activated by methylation, and/or target genes suppressed by methylation. The method includes administering to a subject in need of such treatment, a therapeutically effective amount of a compound of the present disclosure, or a pharmaceutically acceptable salt, polymorph, solvate, or stereoisomer thereof.

Unless otherwise stated, any description of a method of treatment includes use of the compounds to provide such treatment or prophylaxis as is described herein, as well as use of the compounds to prepare a medicament to treat or prevent such condition. The treatment includes treatment of human or non-human animals including rodents and other disease models.

In some aspects, this disclosure relates to methods of modulating the activity of EHMT2, which catalyzes the dimethylation of lysine 9 on histone H3 (H3K9). In some embodiments, the methods relate to modulating the activity of EHMT2, which catalyzes the dimethylation of lysine 9 on histone H3 (H3K9) in a subject in need thereof. In some embodiments, the method comprises the step of administering to a subject in need thereof a therapeutically effective amount of a compound described herein, wherein the compound(s) inhibits histone methyltransferase activity of EHMT2, thereby modulating the EHMT2 activity in the subject. In some embodiments, the subject has an EHMT2-mediated disease or disorder. In some embodiments, the subject has an EHMT2-mediated blood disorder. In some embodiments, the subject has an imprinting disorder (e.g., an EHMT2-mediated imprinting disorder). In some embodiments, the subject has an EHMT2-mediated cancer, e.g., a cancer expressing a mutant EHMT2. For example, in some embodiments, the EHMT2-mediated cancer is selected from the group consisting of leukemia, prostate carcinoma, hepatocellular carcinoma, and lung cancer.

For example, in some embodiments, the compounds disclosed herein can be used for treating cancer. For example, in some embodiments, the cancer is a hematological cancer.

For example, in some embodiments, the cancer is selected from the group consisting of brain and central nervous system (CNS) cancer, head and neck cancer, kidney cancer, ovarian cancer, pancreatic cancer, leukemia, lung cancer, lymphoma, myeloma, sarcoma, breast cancer, and prostate cancer. Preferably, a subject in need thereof is one who had, is having or is predisposed to developing brain and CNS cancer, kidney cancer, ovarian cancer, pancreatic cancer, leukemia, lymphoma, myeloma, and/or sarcoma. Exemplary brain and central CNS cancer includes medulloblastoma, oligodendroglioma, atypical teratoid/rhabdoid tumor, choroid plexus carcinoma, choroid plexus papilloma, ependymoma, glioblastoma, meningioma, neuroglial tumor, oligoastrocytoma, oligodendroglioma, and pineoblastoma. Exemplary ovarian cancer includes ovarian clear cell adenocarcinoma, ovarian endomethrioid adenocarcinoma, and ovarian serous adenocarcinoma. Exemplary pancreatic cancer includes pancreatic ductal adenocarcinoma and pancreatic endocrine tumor. Exemplary sarcoma includes chondrosarcoma, clear cell sarcoma of soft tissue, ewing sarcoma, gastrointestinal stromal tumor, osteosarcoma, rhabdomyosarcoma, and not otherwise specified (NOS) sarcoma. Alternatively, cancers to be treated by the compounds of the present invention are non NHL cancers.

For example, in some embodiments, the cancer is selected from the group consisting of acute myeloid leukemia (AML) or chronic lymphocytic leukemia (CLL), medulloblastoma, oligodendroglioma, ovarian clear cell adenocarcinoma, ovarian endomethrioid adenocarcinoma, ovarian serous adenocarcinoma, pancreatic ductal adenocarcinoma, pancreatic endocrine tumor, malignant rhabdoid tumor, astrocytoma, atypical teratoid/rhabdoid tumor, choroid plexus carcinoma, choroid plexus papilloma, ependymoma, glioblastoma, meningioma, neuroglial tumor, oligoastrocytoma, oligodendroglioma, pineoblastoma, carcinosarcoma, chordoma, extragonadal germ cell tumor, extrarenal rhabdoid tumor, schwannoma, skin squamous cell carcinoma, chondrosarcoma, clear cell sarcoma of soft tissue, ewing sarcoma, gastrointestinal stromal tumor, osteosarcoma, rhabdomyosarcoma, and not otherwise specified (NOS) sarcoma. Preferably, the cancer is acute myeloid leukemia (AML), chronic lymphocytic leukemia (CLL), medulloblastoma, ovarian clear cell adenocarcinoma, ovarian endomethrioid adenocarcinoma, pancreatic ductal adenocarcinoma, malignant rhabdoid tumor, atypical teratoid/rhabdoid tumor, choroid plexus carcinoma, choroid plexus papilloma, glioblastoma, meningioma, pineoblastoma, carcinosarcoma, extrarenal rhabdoid tumor, schwannoma, skin squamous cell carcinoma, chondrosarcoma, ewing sarcoma, epithelioid sarcoma, renal medullary carcinoma, diffuse large B-cell lymphoma, follicular lymphoma and/or NOS sarcoma.

For example, in some embodiments, the EHMT2-mediated disorder is a hematological disorder.

In some embodiments, the imprinting disorder is Prader-Willi syndrome (PWS), transient neonatal diabetes mellitus (TNDM), Silver-Russell syndrome (SRS), Albright hereditary osteodystrophy (AHO), pseudohypoparathyroidism (PHP), Birk-Barel mental retardation, Beckwith-Wiedemann syndrome (BWS), Temple syndrome (UPD(14)mat), Kagami-Ogata syndrome (UPD(14)pat), Angelman syndrome (AS), precocious puberty, Schaaf-Yang syndrome (SHFYNG), sporadic pseudohypoparathyroidism Ib, or maternal uniparental disomy of chromosome 20 syndrome (upd(20)mat).

The compound(s) of the present disclosure inhibit the histone methyltransferase activity of EHMT2 or a mutant thereof and, accordingly, the present disclosure also provides methods for treating conditions and diseases the course of which can be influenced by modulating the methylation status of histones or other proteins, wherein said methylation status is mediated at least in part by the activity of EHMT2. In one aspect of the disclosure, certain compounds disclosed herein are candidates for treating, or preventing certain conditions, diseases, and disorders. Modulation of the methylation status of histones can in turn influence the level of expression of target genes activated by methylation, and/or target genes suppressed by methylation. The method includes administering to a subject in need of such treatment, a therapeutically effective amount of a compound of the present disclosure.

As used herein, a "subject" is interchangeable with a "subject in need thereof", both of which refer to a subject having a disorder in which EHMT2-mediated protein methylation plays a part, or a subject having an increased risk of developing such disorder relative to the population at large. A "subject" includes a mammal. The mammal can be *e.g.,* a human or appropriate non-human mammal, such as primate, mouse, rat, dog, cat, cow, horse, goat, camel, sheep or a pig. The subject can also be a bird or fowl. In some embodiments, the mammal is a human. A subject in need thereof can be one who has been previously diagnosed or identified as having cancer or a precancerous condition. A subject in need thereof can also be one who has (e.g., is suffering from) cancer or a precancerous condition. Alternatively, a subject in need thereof can be one who has an increased risk of developing such disorder relative to the population at large (*i.e.,* a subject who is predisposed to developing such disorder relative to the population at large). A subject in need thereof can have a precancerous condition. A subject in need thereof can have refractory or resistant cancer (i.e., cancer that doesn't respond or hasn't yet responded to treatment). The subject may be resistant at start of treatment or may become resistant during treatment. In some embodiments, the subject in need thereof has cancer recurrence following remission on most recent therapy. In some embodiments, the subject in need thereof received and failed all known effective therapies for cancer treatment. In some embodiments, the subject in need thereof received at least one prior therapy. In a preferred embodiment, the subject has cancer or a cancerous condition. For example, the cancer is leukemia, prostate carcinoma, hepatocellular carcinoma, and lung cancer.

As used herein, "candidate compound" refers to a compound of the present disclosure, or a pharmaceutically acceptable salt, polymorph or solvate thereof, that has been or will be tested in one or more *in vitro* or *in vivo* biological assays, in order to determine if that compound is likely to elicit a desired biological or medical response in a cell, tissue, system, animal or human that is being sought by a researcher or clinician. A candidate compound is a compound of the present disclosure, or a pharmaceutically acceptable salt, polymorph or solvate thereof. The biological or medical response can be the treatment of cancer. The biological or medical response can be treatment or prevention of a cell proliferative disorder. The biological response or effect can also include a change in cell proliferation or growth that occurs *in vitro* or in an animal model, as well as other biological changes that are observable *in vitro. In vitro* or *in vivo* biological assays can include, but are not limited to, enzymatic activity assays, electrophoretic mobility shift assays, reporter gene assays, *in vitro* cell viability assays, and the assays described herein.

For example, an *in vitro* biological assay that can be used includes the steps of (1) mixing a histone substrate (e.g., an isolated histone sample or an isolated histone peptide representative of human histone H3 residues 1-15) with recombinant EHMT2 enzymes; (2) adding a compound of the disclosure to this mixture; (3) adding non-radioactive and $^3$H-labeled S-Adenosyl methionine (SAM) to start the reaction; (4) adding excessive amount of non-radioactive SAM to stop the reaction; (4) washing off the free non-incorporated $^3$H-SAM; and (5) detecting the quantity of $^3$H-labeled histone substrate by any methods known in the art *(e.g.,* by a PerkinElmer TopCount platereader).

For example, an *in vitro* study that can be used includes the steps of (1) treating cancer cells *(e.g.,* breast cancer cells) with a compound of this disclosure; (2) incubating the cells for a set period of time; (3) fixing the cells; (4) treating the cells with primary antibodies that bind to dimethylated histone substrates; (5) treating the cells with a secondary antibody (*e.g.* an antibody conjugated to an infrared dye); (6) detecting the quantity of bound antibody by any methods known in the art *(e.g.,* by a Licor Odyssey Infrared Scanner).

As used herein, "treating" or "treat" describes the management and care of a patient for the purpose of combating a disease, condition, or disorder and includes the administration of a compound of the present disclosure, or a pharmaceutically acceptable salt, polymorph or solvate thereof, to alleviate the symptoms or complications of a disease, condition or disorder, or to eliminate the disease, condition or disorder. The term "treat" can also include treatment of a cell *in vitro* or an animal model.

A compound of the present disclosure, or a pharmaceutically acceptable salt, polymorph or solvate thereof, can or may also be used to prevent a relevant disease, condition or disorder, or used to identify suitable candidates for such purposes. As used herein, "preventing," "prevent," or "protecting against" describes reducing or eliminating the onset of the symptoms or complications of such disease, condition or disorder.

One skilled in the art may refer to general reference texts for detailed descriptions of known techniques discussed herein or equivalent techniques. These texts include Ausubel et al., Current Protocols in Molecular Biology, John Wiley and Sons, Inc. (2005); Sambrook et al., Molecular Cloning, A Laboratory Manual (3rd edition), Cold Spring Harbor Press, Cold Spring Harbor, New York (2000); Coligan et al., Current Protocols in Immunology, John Wiley & Sons, N.Y.; Enna

et al., Current Protocols in Pharmacology, John Wiley & Sons, N.Y.; Fingl et al., The Pharmacological Basis of Therapeutics (1975), Remington's Pharmaceutical Sciences, Mack Publishing Co., Easton, PA, 18th edition (1990). These texts can, of course, also be referred to in making or using an aspect of the disclosure.

As used herein, "combination therapy" or "co-therapy" includes the administration of a compound of the present disclosure, or a pharmaceutically acceptable salt, polymorph or solvate thereof, and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents.

The present disclosure also provides pharmaceutical compositions comprising a compound of any of the Formulae described herein in combination with at least one pharmaceutically acceptable excipient or carrier.

A "pharmaceutical composition" is a formulation containing the compounds of the present disclosure in a form suitable for administration to a subject. In some embodiments, the pharmaceutical composition is in bulk or in unit dosage form. The unit dosage form is any of a variety of forms, including, for example, a capsule, an IV bag, a tablet, a single pump on an aerosol inhaler or a vial. The quantity of active ingredient (e.g., a formulation of the disclosed compound or salt, hydrate, solvate or isomer thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this disclosure include powders, sprays, ointments, pastes, creams, lotions, gels, solutions, patches and inhalants. In some embodiments, the active compound is mixed under sterile conditions with a pharmaceutically acceptable carrier, and with any preservatives, buffers, or propellants that are required.

As used herein, the phrase "pharmaceutically acceptable" refers to those compounds, anions, cations, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

A pharmaceutical composition of the disclosure is formulated to be compatible with its intended route of administration. Examples of routes of administration include parenteral, e.g., intravenous, intradermal, subcutaneous, oral (e.g., inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

A compound or pharmaceutical composition of the disclosure can be administered to a subject in many of the well-known methods currently used for chemotherapeutic treatment. For example, for treatment of cancers, a compound of the disclosure may be injected directly into tumors, injected into the blood stream or body cavities or taken orally or applied through the skin with patches. The dose chosen should be sufficient to constitute effective treatment but not so high as to cause unacceptable side effects. The state of the disease condition (e.g., cancer, precancer, and the like) and the health of the patient should preferably be closely monitored during and for a reasonable period after treatment.

The term "therapeutically effective amount", as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or condition to be treated is cancer. In another aspect, the disease or condition to be treated is a cell proliferative disorder.

For any compound, the therapeutically effective amount can be estimated initially either in cell culture assays, e.g., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may

be determined by standard pharmaceutical procedures in cell cultures or experimental animals, e.g., $ED_{50}$ (the dose therapeutically effective in 50% of the population) and $LD_{50}$ (the dose lethal to 50% of the population). The dose ratio between toxic and therapeutic effects is the therapeutic index, and it can be expressed as the ratio, $LD_{50}/ED_{50}$. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

The pharmaceutical compositions containing active compounds of the present disclosure may be manufactured in a manner that is generally known, e.g., by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol and sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The active compounds can be prepared with pharmaceutically acceptable carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved.

In therapeutic applications, the dosages of the pharmaceutical compositions used in accordance with the disclosure vary depending on the agent, the age, weight, and clinical condition of the recipient patient, and the experience and judgment of the clinician or practitioner administering the therapy, among other factors affecting the selected dosage. Generally, the dose should be sufficient to result in slowing, and preferably regressing, the growth of the tumors and also preferably causing complete regression of the cancer. Dosages can range from about 0.01 mg/kg per day to about 5000 mg/kg per day. In preferred aspects, dosages can range from about 1 mg/kg per day to about 1000 mg/kg per day. In an aspect, the dose will be in the range of about 0.1 mg/day to about 50 g/day; about 0.1 mg/day to about 25 g/day; about 0.1 mg/day to about 10 g/day; about 0.1 mg to about 3 g/day; or about 0.1 mg to about 1 g/day, in single, divided, or continuous doses (which dose may be adjusted for the patient's weight in kg, body surface area in $m^2$, and age in years). An effective amount of a pharmaceutical agent is that which provides an objectively identifiable improvement as noted by the clinician or other qualified observer. For example, regression of a tumor in a patient may be measured with reference to the diameter of a tumor. Decrease in the diameter of a tumor indicates regression. Regression is also indicated by failure of tumors to reoccur after treatment has stopped. As used herein, the term "dosage effective manner" refers to amount of an active compound to produce the desired biological effect in a subject or cell.

The pharmaceutical compositions can be included in a container, pack, or dispenser together with instructions for administration.

The compounds of the present disclosure are capable of further forming salts. All of these forms are also contemplated within the scope of the claimed disclosure.

As used herein, "pharmaceutically acceptable salts" refer to derivatives of the compounds of the present disclosure wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like. In the salt form, it is understood that the ratio of the compound to the cation or anion of the salt can be 1:1, or any ration other than 1:1, e.g., 3:1, 2:1, 1:2, or 1:3.

It should be understood that all references to pharmaceutically acceptable salts include solvent addition forms (solvates) or crystal forms (polymorphs) as defined herein, of the same salt.

The compounds of the present disclosure can also be prepared as esters, for example, pharmaceutically acceptable esters. For example, a carboxylic acid function group in a compound can be converted to its corresponding ester, *e.g.,* a methyl, ethyl or other ester. Also, an alcohol group in a compound can be converted to its corresponding ester, e.g., acetate, propionate or other ester.

The compounds, or pharmaceutically acceptable salts thereof, are administered orally, nasally, transdermally,

pulmonary, inhalationally, buccally, sublingually, intraperitoneally, subcutaneously, intramuscularly, intravenously, rectally, intrapleurally, intrathecally and parenterally. In some embodiments, the compound is administered orally. One skilled in the art will recognize the advantages of certain routes of administration.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Techniques for formulation and administration of the disclosed compounds of the disclosure can be found in Remington: the Science and Practice of Pharmacy, 19th edition, Mack Publishing Co., Easton, PA (1995). In an embodiment, the compounds described herein, and the pharmaceutically acceptable salts thereof, are used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein.

All percentages and ratios used herein, unless otherwise indicated, are by weight. Other features and advantages of the present disclosure are apparent from the different examples. The provided examples illustrate different components and methodology useful in practicing the present disclosure. The examples do not limit the claimed disclosure. Based on the present disclosure the skilled artisan can identify and employ other components and methodology useful for practicing the present disclosure.

In the synthetic schemes described herein, compounds may be drawn with one particular configuration for simplicity. Such particular configurations are not to be construed as limiting the disclosure to one or another isomer, tautomer, regioisomer or stereoisomer, nor does it exclude mixtures of isomers, tautomers, regioisomers or stereoisomers; however, it will be understood that a given isomer, tautomer, regioisomer or stereoisomer may have a higher level of activity than another isomer, tautomer, regioisomer or stereoisomer.

Compounds designed, selected and/or optimized by methods described above, once produced, can be characterized using a variety of assays known to those skilled in the art to determine whether the compounds have biological activity. For example, the molecules can be characterized by conventional assays, including but not limited to those assays described below, to determine whether they have a predicted activity, binding activity and/or binding specificity.

Furthermore, high-throughput screening can be used to speed up analysis using such assays. As a result, it can be possible to rapidly screen the molecules described herein for activity, using techniques known in the art. General methodologies for performing high-throughput screening are described, for example, in Devlin (1998) High Throughput Screening, Marcel Dekker; and U.S. Patent No. 5,763,263. High-throughput assays can use one or more different assay techniques including, but not limited to, those described below.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

**Example 1: Synthesis of Compound 1**

**Synthesis of 6-methoxy-5-(3-(pyrrolidin-1-yl)propoxy)-1H-benzo[d]imidazol-2-amine:**

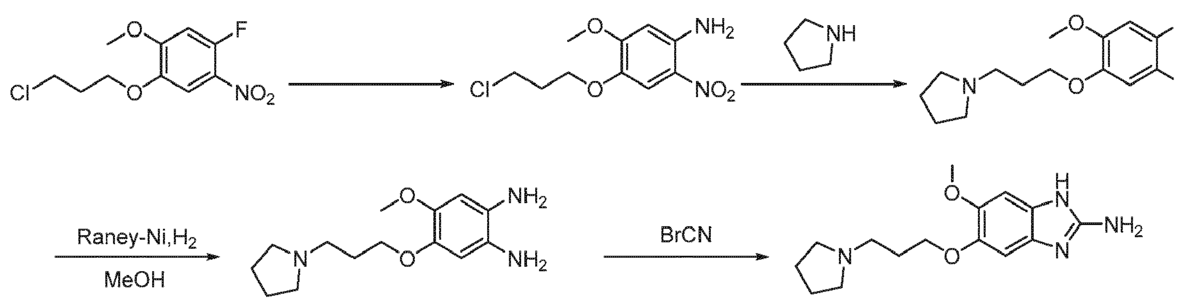

**Step 1:** Synthesis of 4-(3-chloropropoxy)-5-methoxy-2-nitroaniline:

Into a 20-mL round-bottom flask was placed 1-(3-chloropropoxy)-4-fluoro-2-methoxy-5-nitrobenzene (800 mg, 3.03 mmol, 1 equiv), $NH_3$/ methanol (10 mL). The resulting solution was stirred for 12 h at 60 °C in an oil bath. The crude product was purified by Flash-Prep-HPLC A 1:1. This resulted in 170 mg (21%) of the title compound as a light yellow solid.

Analytical Data: LC-MS: (ES, *m*/*z*) = 261 [M+1], RT = 1.29 min.

**Step 2:** Synthesis of 5-methoxy-2-nitro-4-[3-(pyrrolidin-1-yl)propoxy]aniline:

Into a 50-mL round-bottom flask was placed 4-(3-chloropropoxy)-5-methoxy-2-nitroaniline (160 mg, 0.61 mmol, 1 equiv), pyrrolidine (131 mg, 1.84 mmol, 3.00 equiv), NaI (92 mg, 1 equiv), potassium carbonate (255 mg, 1.85 mmol, 3.00 equiv), ACN (10 mL). The resulting solution was stirred for 12 h at 80 °C in an oil bath. The solids were filtered out. The crude product was purified by Flash-Prep-HPLC. This resulted in 180 mg (99%) of the title compound as a light yellow solid.

Analytical Data: LC-MS: (ES, m/z): 296 [M+1], RT = 0.59 min. $^1$H-NMR: (DMSO-$d_6$, *ppm*): $\delta$ 7.47 (s, 2H), 7.35 (s, 1H), 6.52 (s, 1H), 4.00 - 3.86 (m, 2H), 3.82 (s, 3H), 2.55 - 2.51 (m, 2H), 2.48 - 2.12 (m, 4H), 2.01-1.85 (m, 2H), 1.78 - 1.61 (m, 4H).

**Step 3:** Synthesis of 4-methoxy-5-[3-(pyrrolidin-1-yl)propoxy]benzene-1,2-diamine:

Into a 50-mL round-bottom flask was placed 5-methoxy-2-nitro-4-[3-(pyrrolidin-1-yl)propoxy]aniline (120 mg, 0.41 mmol, 1 equiv), Rancy Ni (100 mg), methanol (8 mL), hydrogen. The resulting solution was stirred for 1 h at 25 °C. The resulting mixture was concentrated under vacuum. This resulted in 100 mg (93%) of the title compound as a solid.

Analytical Data: LC-MS: (ES, *m*/*z*): 266 [M+1], RT = 0.30 min.

**Step 4:** Synthesis of 6-methoxy-5-(3-(pyrrolidin-1-yl)propoxy)-1H-benzo[d]imidazol-2-amine:

Into a 50-mL round-bottom flask was placed 4-methoxy-5-[3-(pyrrolidin-1-yl)propoxy]benzene-1,2-diamine (100 mg, 0.38 mmol, 1 equiv), ACN (5 mL), water(5 mL), cyanic bromide (100 mg, 0.94 mmol, 2.51 equiv). The resulting solution was stirred for 12 h at 50 °C in an oil bath. This resulted in 31.0 mg (20%) of the title compound as a solid.

**Example 2: Synthesis of Compound 2**

**Synthesis of 6-methoxy-1-methyl-5-(3-(pyrrolidin-1-yl)propoxy)-1H-benzo [d] imidazol-2-amine:**

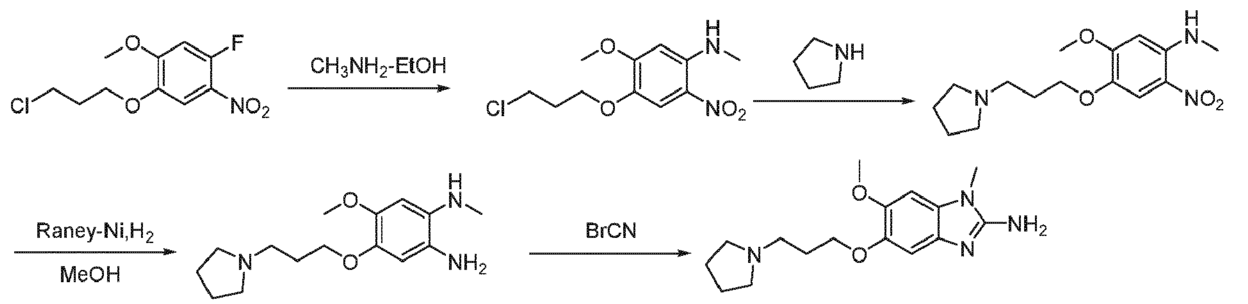


Synthesis of 6-methoxy-1-methyl-5-(3-(pyrrolidin-1-yl)propoxy)-1H-benzo[d]imidazol-2-amine:

6-methoxy-1-methyl-5-(3-(pyrrolidin-1-yl)propoxy)-1H-benzo[d]imidazol-2-amine was synthesized as for 6-methoxy-5-(3-(pyrrolidin-1-yl)propoxy)-1H-benzo[d]imidazol-2-amine using methylamine in place of ammonia in step 1.

**Example 3: Synthesis of Compound 3**

**Synthesis of 1-cyclopentyl-6-methoxy-5-(3-(pyrrolidin-1-yl)propoxy)-1H-benzo [d] imidazol-2-amine:**

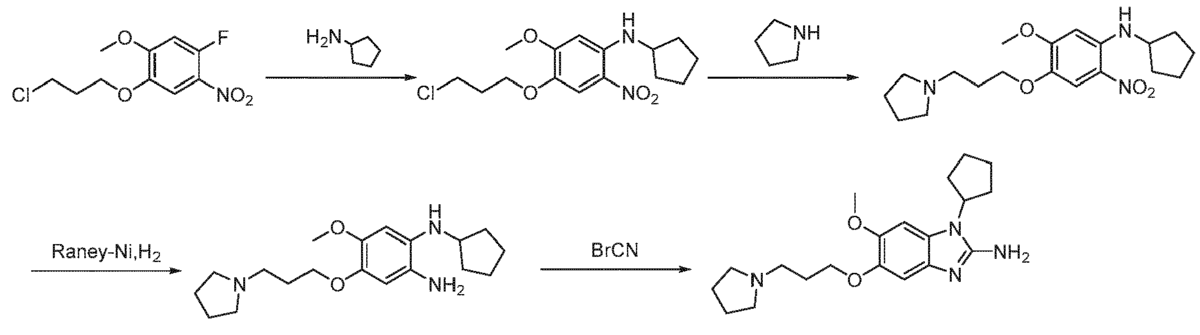


Synthesis of 1-cyclopentyl-6-methoxy-5-(3-(pyrrolidin-1-yl)propoxy)-1H-benzo[d]imidazol-2-amine:

1-cyclopentyl-6-methoxy-5-(3-(pyrrolidin-1-yl)propoxy)-1H-benzo[d]imidazol-2-amine was synthesized as for 6-methoxy-5-(3-(pyrrolidin-1-yl)propoxy)-1H-benzo[d]imidazol-2-amine using cyclopentylamine in place of ammonia in step 1.

**Example 4: Synthesis of Compound 5**

**Synthesis of 5-methoxy-6-(3-(pyrrolidin-1-yl)propoxy)-2',3',5',6'-tetrahydrospiro[indole-3,4'-pyran]-2-amine:**

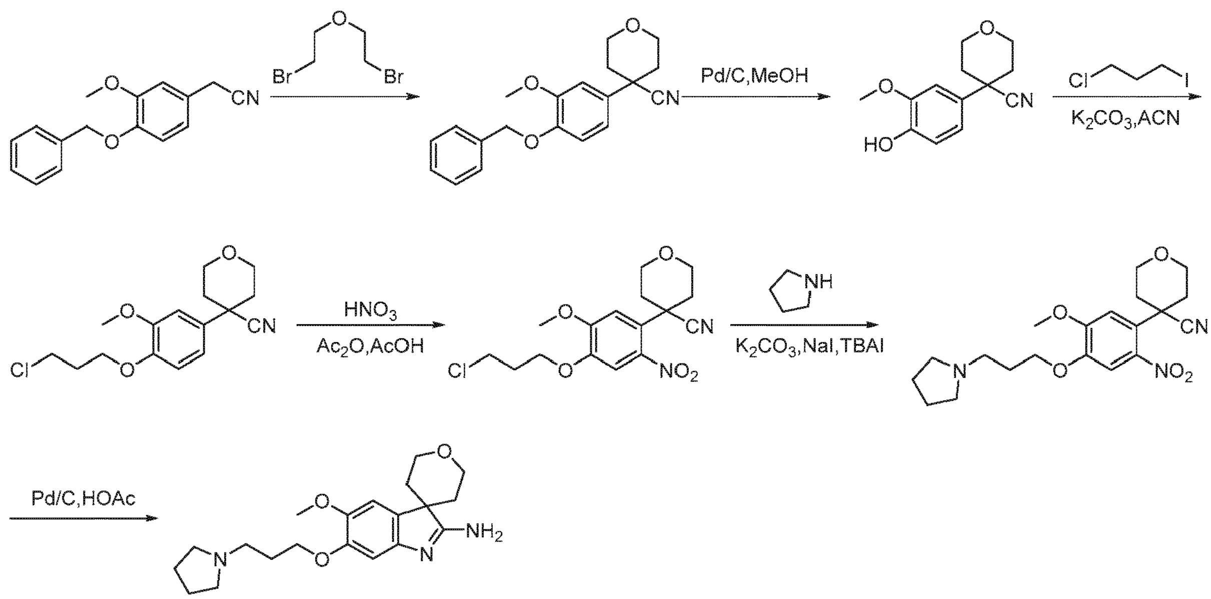


**Step 1:** Synthesis of 4-[4-(benzyloxy)-3-methoxyphenyl]oxane-4-carbonitrile:

Into a 50-mL 3-necked round-bottom flask purged and maintained with an inert atmosphere of nitrogen, was placed 2-[4-(benzyloxy)-3-methoxyphenyl]acetonitrile (2.5 g, 9.87 mmol, 1.00 equiv), N,N-dimethylformamide (15 mL). This was followed by the addition of sodium hydride (988 mg, 41.17 mmol, 2.50 equiv), in portions at 0 °C. To this was added 1-bromo-2-(2-bromoethoxy)ethane (2.98 mg, 0.01 mmol, 1.30 equiv) dropwise with stirring at 0 °C. The resulting solution was stirred for 3 h at room temperature in a water/ice bath. The reaction was then quenched by the addition of 20 mL of water. The resulting solution was extracted with 3x40 mL of ethyl acetate and the organic layers combined. The resulting mixture was washed with 3x15 mL of brine. The solid was dried in an oven under reduced pressure. The residue was applied onto a silica gel column with ethyl acetate/petroleum ether (15/85). The collected fractions were combined and concentrated under vacuum. This resulted in 2.5 g (78%) of the title compound as a light yellow solid.

Analytical Data: LC-MS: (ES, *m/z*): RT = 1.344 min, LCMS 33: *m/z* = 324.15 [M+1]. $^1$H-NMR: (300 MHz, Methanol-d4) δ 7.51 - 7.33 (m, 5H), 7.13 (s, 1H), 7.05 (d, *J* = 1.4 Hz, 2H), 5.14 (s, 2H), 4.13 - 4.01 (m, 2H), 3.98 - 3.76 (m, 5H), 2.23 - 2.01 (m, 4H).

**Step 2:** Synthesis of 4-(4-hydroxy-3-methoxyphenyl)oxane-4-carbonitrile:

Into a 100-mL round-bottom flask was placed 4-[4-(benzyloxy)-3-methoxyphenyl]oxane-4-carbonitrile (1 g, 3.09 mmol, 1.00 equiv), methanol (30 mL), Palladium carbon (300 mg). The resulting solution was stirred for 2 h at room temperature. The solids were filtered out. The resulting mixture was concentrated under vacuum. This resulted in 660 mg (91%) of the title compound as an off-white solid.

Analytical Data: LC-MS: (ES, m/z): RT = 1.128 min, m/z = 234.11 [M+1]. 1H-NMR: (300 MHz, Chloroform-d) δ 7.01 - 6.89 (m, 3H), 5.71 (s, 1H), 4.19 - 4.03 (m, 2H), 3.97 - 3.86 (m, 5H), 2.16 - 1.99 (m, 4H).

**Step 3:** Synthesis of 4-[4-(3-chloropropoxy)-3-methoxyphenyl]oxane-4-carbonitrile:

Into a 100-mL round-bottom flask was placed 4-(4-hydroxy-3-methoxyphenyl)oxane-4-carbonitrile (660 mg, 2.83 mmol, 1.00 equiv), potassium carbonate (1.17 g, 8.47 mmol, 2.99 equiv), ACN (20 mL), 1-chloro-3-iodopropane (1.15 g, 5.63 mmol, 1.99 equiv). The resulting solution was stirred for 3 h at 90 °C. This resulted in 1 g crude of the title compound as a light yellow solid.

Analytical Data: LC-MS: (ES, *m*/*z*): RT = 0.959, *m*/*z* = 310.11 [M+1].

**Step 4:** Synthesis of 4-[5-methoxy-2-nitro-4-[3-(pyrrolidin-1-yl)propoxy]phenyl]oxane-4-carbonitrile:

Into a 100-mL round-bottom flask was placed 4-[4-(3-chloropropoxy)-5-methoxy-2-nitrophenyl]oxane-4-carbonitrile (500 mg, 1.41 mmol, 1.00 equiv), TBAI (52 mg, 0.14 mmol, 0.10 equiv), NaI (212 mg), potassium carbonate (585 mg, 4.23 mmol, 3.00 equiv), ACN (30 mL), pyrrolidine (201 mg, 2.83 mmol, 2.01 equiv). The resulting solution was stirred for 3 h at 90 °C. The solids were filtered out. The resulting mixture was concentrated under vacuum. The crude product was purified by Flash-Prep-HPLC. This resulted in 450 mg (82%) of the title compound as a light yellow solid.

Analytical Data: LC-MS: (ES, *m*/*z):* RT = 0.646, *m*/*z* = 390.40 [M+1].

**Step 5:** Synthesis of 5-methoxy-6-(3-(pyrrolidin-1-yl)propoxy)-2',3',5',6'-tetrahydrospiro[indole-3,4'-pyran]-2-amine:

Into a 100-mL round-bottom flask was placed 4-[5-methoxy-2-nitro-4-[3-(pyrrolidin-1-yl)propoxy]phenyl]oxane-4-carbonitrile (450 mg, 1.16 mmol, 1.00 equiv), AcOH (10 mL), Palladium carbon (100 mg). The resulting solution was stirred for 3 h at 80 °C. The solids were filtered out. The crude product was purified by Prep-HPLC. This resulted in 39.8 mg (9.58%) of the title compound as an off-white solid.

**Example 6: Synthesis of Compound 6**

**6-methoxy-5-[3-(pyrrolidin-1-yl)propoxyl-1,3-benzothiazol-2-amine:**

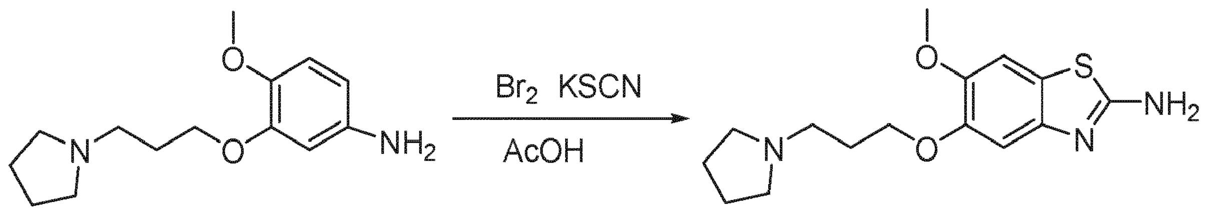


**Step 1**: Synthesis of 6-methoxy-5-[3-(pyrrolidin-1-yl)propoxy]-1,3-benzothiazol-2-amine:

Into a 50-mL round-bottom flask was placed AcOH (5 mL), 4-methoxy-3-[3-(pyrrolidin-1-yl)propoxy]aniline (200 mg, 0.80 mmol, 1.00 equiv), KSCN (116 mg, 1.20 mmol, 1.50 equiv).The resulting solution was stirred for 0.5 h at 20 °C. This was followed by the addition of a solution of $Br_2$ (166 mg, 1.04 mmol, 1.30 equiv) in AcOH (1 mL) dropwise with stirring. The resulting solution was allowed to react, with stirring, for an additional 20 h at 14 °C. The resulting mixture was concentrated under vacuum. The resulting solution was diluted with 5 mL of $H_2O$. The pH value of the solution was adjusted to 10 with ammonia. The resulting solution was extracted with 3×10 mL of dichloromethane and the organic layers combined and concentrated under vacuum. The crude product (250 mg) was purified by Prep-HPLC. 126.9 mg product was obtained. This resulted in 126.9 mg (52%) of the title compound as a solid.

**Example 7: Synthesis of Compound** 7

**5'-methoxy-6'-(3-(pyrrolidin-1-yl)propoxy)-4,5-dihydro-2H-spiro[furan-3,3'-indol]**-2'-amine:

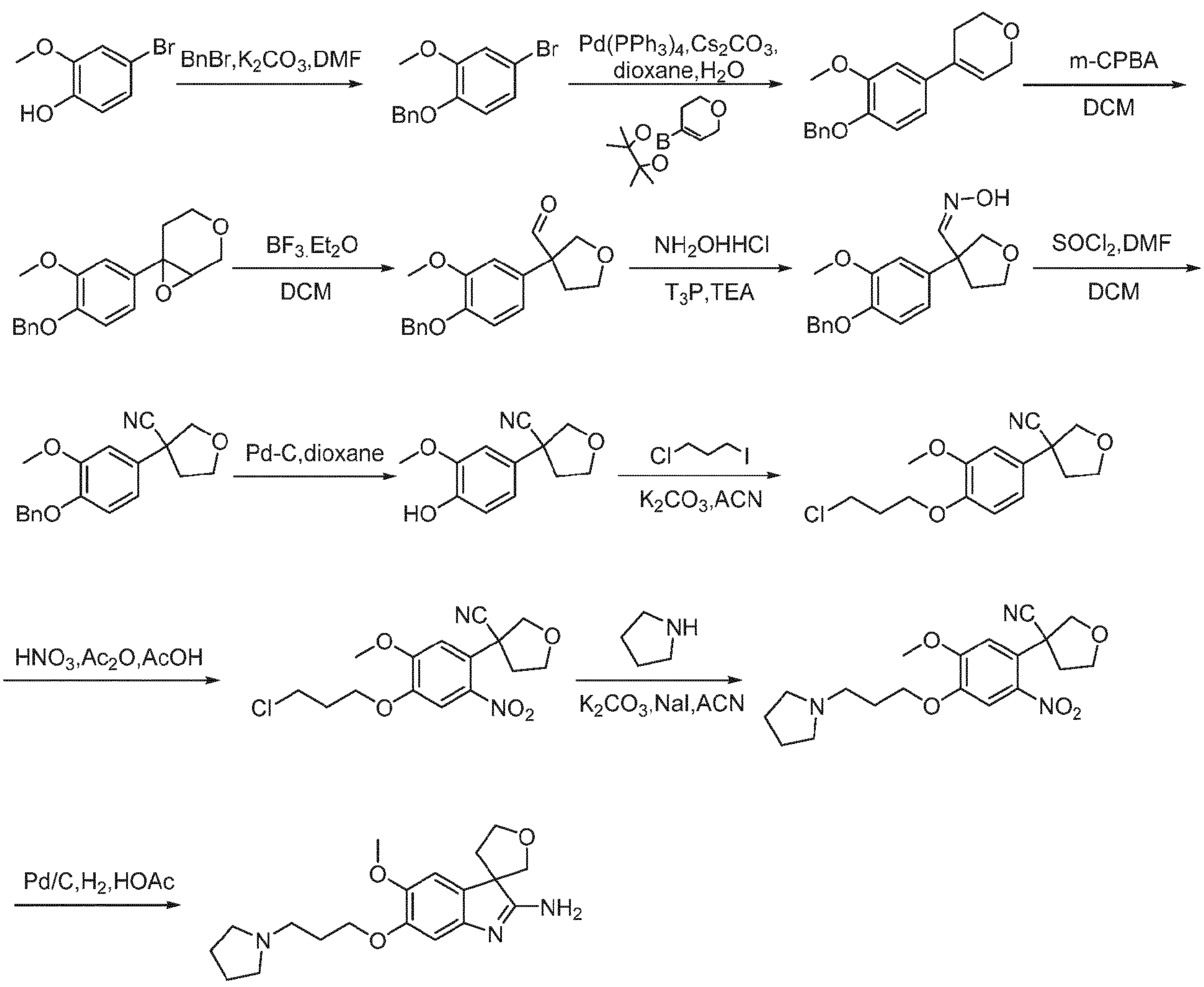


**Step** 1: Synthesis of 1-(benzyloxy)-4-bromo-2-methoxybenzene

Into a 250-mL round-bottom flask was placed 4-bromo-2-methoxyphenol (7.5 g, 36.94 mmol, 1.00 equiv), potassium carbonate (15 g, 108.53 mmol, 2.94 equiv), N,N-dimethylformamide (75 mL), (bromomethyl)benzene (6.5 g, 38.00 mmol, 1.03 equiv). The resulting solution was stirred for 12 h at 80 °C. The resulting solution was diluted with $H_2O$, extracted with ethyl acetate, and the organic layers combined. The resulting mixture was washed with water and brine. The solid was dried in an oven under reduced pressure. This resulted in 10.429 g (96%) of the title compound as a light red solid.

Analytical Data: $^1H$ NMR (300 MHz, Methanol-$d_4$) δ 7.48 - 7.28 (m, 5H), 7.11 (d, $J$ = 2.3 Hz, 1H), 7.01 (dd, $J$ = 8.6, 2.3 Hz, 1H), 6.91 (d, $J$ = 8.6 Hz, 1H), 5.09 (s, 2H), 3.85 (s, 3H).

**Step 2:** Synthesis of 4-[4-(benzyloxy)-3-methoxyphenyl]-3,6-dihydro-2H-pyran

Into a 250-mL round-bottom flask was placed 1-(benzyloxy)-4-bromo-2-methoxybenzene (6 g, 20.47 mmol, 1.00 equiv), 2-(3,6-dihydro-2H-pyran-4-yl)-4,4,5,5-tetramethyl-1,3,2-dioxaborolane (5.187 g, 24.69 mmol, 1.21 equiv), $Pd(PPh_3)_4$ (237 mg, 0.21 mmol, 0.01 equiv), $Cs_2CO_3$ (20.05 g, 61.54 mmol, 3.01 equiv), dioxane (90 mL), water(30 mL). The resulting solution was stirred for 3 h at 90 °C. The solids were filtered out. The resulting mixture was concentrated under vacuum. The residue was applied onto a silica gel column with ethyl acetate/petroleum ether (9.1/90.9). This

resulted in 5 g (82%) of the title compound as an off-white solid.

Analytical Data: LC-MS: (ES, *m/z):* RT=1.305 min, m/z=395 [M+1]. $^1$H NMR (400 MHz, Methanol-$d_4$) δ 7.48 - 7.44 (m, 2H), 7.41 - 7.28 (m, 3H), 7.08 - 7.05 (m, 1H), 6.96 - 6.94 (m, 2H), 6.12 - 6.09 (m, 1H), 5.11 (s, 2H), 4.30 (q, *J* = 2.8 Hz, 2H), 3.92 (t, *J* = 5.5 Hz, 2H), 3.88 (s, 3H).

**Step 3:** Synthesis of 6-[4-(benzyloxy)-3-methoxyphenyl]-3,7-dioxabicyclo[4.1.0]heptane:

Into a 250-mL round-bottom flask was placed 4-[4-(benzyloxy)-3-methoxyphenyl]-3,6-dihydro-2H-pyran (1.3 g, 4.39 mmol, 1.00 equiv), m-CPBA (894 mg, 5.18 mmol, 1.18 equiv), dichloromethane (80 mL). The resulting solution was stirred for 1 h at room temperature. The reaction was then quenched by the addition of aqueous sodium bicarbonate. The resulting solution was extracted with ethyl acetate and the organic layers combined. The resulting mixture was washed with water and brine. The mixture was dried over anhydrous sodium sulfate and concentrated under vacuum. This resulted in 2.0 g (crude) of the title compound as a yellow oil.

Analytical Data: LC-MS: (ES, *m/z*): RT=2.478 min, m/z=313 [M+H].

**Step 4:** Synthesis of 3-[4-(benzyloxy)-3-methoxyphenyl]oxolane-3-carbaldehyde

Into a 250-mL round-bottom flask was placed 6-[4-(benzyloxy)-3-methoxyphenyl]-3,7-dioxabicyclo[4.1.0]heptane (2 g, 6.40 mmol, 1.00 equiv), $BF_3.Et_2O$ (1 mL), dichloromethane (50 mL). The resulting solution was stirred for 1 h at room temperature. The resulting solution was diluted with $H_2O$. The resulting solution was extracted with ethyl acetate, the organic layers combined and dried over anhydrous sodium sulfate, and concentrated under vacuum. This resulted in 1.4 g (70%) of the title compound as a yellow oil.

**Step 5:** Synthesis of (Z)-N-([3-[4-(benzyloxy)-3-methoxyphenyl]oxolan-3-yl]methylidene)hydroxylamine

Into a 100-mL round-bottom flask was placed 3-[4-(benzyloxy)-3-methoxyphenyl]oxolane-3-carbaldehyde (1.4 g, 4.48 mmol, 1.00 equiv), hydroxylamine hydrochloride (475 mg, 6.84 mmol, 1.53 equiv), TEA (1.39 g, 13.74 mmol, 3.06 equiv), N,N-dimethylformamide (35 mL), T3P (2.92 g). The resulting solution was stirred overnight at 110 °C. The resulting solution was diluted with $H_2O$. The resulting solution was extracted with ethyl acetate, the organic layers combined and dried over anhydrous sodium sulfate and concentrated under vacuum. This resulted in 1.3 g (89%) of the title compound as a yellow oil.

**Step 6:** Synthesis of 3-[4-(benzyloxy)-3-methoxyphenyl]oxolane-3-carbonitrile

Into a 100-mL round-bottom flask was placed (Z)-N-([3-[4-(benzyloxy)-3-methoxyphenyl]oxolan-3-yl]methylidene)hydroxylamine (1.3 g, 3.97 mmol, 1.00 equiv), thionyl chloride (4.73 g), N,N-dimethylformamide (3.19 g, 43.64 mmol, 10.99 equiv), dichloromethane (35 mL). The resulting solution was stirred for 1 h at room temperature. The resulting solution was diluted with $H_2O$. The resulting solution was extracted with ethyl acetate and the organic layers combined. The resulting mixture was washed with water and brine. The mixture was dried over anhydrous sodium sulfate and concentrated under vacuum. The crude product was purified by Flash-Prep-HPLC with the following conditions (IntelFlash-1): Column, silica gel; mobile phase, methanol/$H_2O$=0/100 increasing to methanol/$H_2O$=70/30 within 30 min; Detector, UV 254 nm. This resulted in 887 mg (72%) of the title compound as a light yellow solid.

**Step 7:** Synthesis of 3-(4-hydroxy-3-methoxyphenyl)oxolane-3-carbonitrile

Into a 250-mL round-bottom flask was placed 3-[4-(benzyloxy)-3-methoxyphenyl]oxolane-3-carbonitrile (887 mg, 2.87 mmol, 1.00 equiv), and palladium on carbon (600 mg), dioxane (120 mL). The resulting solution was stirred for 1 h at 80 °C. The solids were filtered out. The resulting mixture was concentrated under vacuum. This resulted in 237 mg (38%) of the title compound as an off-white solid.

Analytical Data: LC-MS: (ES, *m/z):* RT=0.475 min, LCMS 27, m/z=218 [M+1].

**Step 8:** Synthesis of 3-[4-(3-chloropropoxy)-3-methoxyphenyl]oxolane-3-carbonitrile

Into a 50-mL round-bottom flask was placed 3-(4-hydroxy-3-methoxyphenyl)oxolane-3-carbonitrile (165 mg, 0.75 mmol, 1.00 equiv), 1-chloro-3-iodopropane (306 mg, 1.50 mmol, 1.99 equiv.), potassium carbonate (310 mg, 2.24 mmol, 2.98 equiv), ACN (15 mL). The resulting solution was stirred for 3 h at 78 °C. The resulting solution was diluted with $H_2O$. The resulting solution was extracted with ethyl acetate and the organic layers combined. The resulting mixture was washed with water and brine. The mixture was dried over anhydrous sodium sulfate and concentrated under vacuum.

This resulted in 237 mg (106%) of the title compound as a light red oil.

Analytical Data: LC-MS: (ES, *m*/*z*): RT=1.189 min, m/z=341 [M+1].

**Step 8:** Synthesis of 3-[4-(3-chloropropoxy)-5-methoxy-2-nitrophenyl]oxolane-3-carbonitrile:

Into a 50-mL round-bottom flask was placed 3-[4-(3-chloropropoxy)-3-methoxyphenyl]oxolane-3-carbonitrile (160 mg, 0.54 mmol, 1.00 equiv), AcOH (10 mL), acetic anhydride (10 mL), $HNO_3$ (0.4 mL). The resulting solution was stirred for 2 h at 0 °C. The resulting solution was diluted with $H_2O$. The resulting solution was extracted with ethyl acetate and the organic layers combined. The resulting mixture was washed with water and brine. The mixture was dried over anhydrous sodium sulfate and concentrated under vacuum. This resulted in 146 mg (79%) of the title compound as a red oil.

Analytical Data: LC-MS: (ES, *m*/*z*): RT=1.189 min, m/z=341 [M+1].

**Step 9:** Synthesis of 3-[5-methoxy-2-nitro-4-[3-(pyrrolidin-1-yl)propoxy]phenyl]oxolane-3-carbonitrile:

Into a 25-mL round-bottom flask was placed 3-[4-(3-chloropropoxy)-5-methoxy-2-nitrophenyl]oxolane-3-carbonitrile (146 mg, 0.43 mmol, 1.00 equiv), NaI (64 mg), potassium carbonate (59 mg, 0.43 mmol, 1.00 equiv), pyrrolidine (91 mg, 1.28 mmol, 2.99 equiv), and ACN (15 mL). The resulting solution was stirred for 3 h at 78 °C. The solids were filtered out. The resulting mixture was concentrated under vacuum. The resulting solution was extracted with ethyl acetate and the organic layers combined. The resulting mixture was washed with water and brine. The mixture was dried over anhydrous sodium sulfate. This resulted in 160 mg (99%) of the title compound as a red oil.

Analytical Data: LC-MS: (ES, *m*/*z):* RT=0.964 min, m/z=376 [M+1].

**Step 10:** Synthesis of 5'-methoxy-6'-(3-(pyrrolidin-1-yl)propoxy)-4,5-dihydro-2H-spiro[furan-3,3'-indol]-2'-amine

Into a 50-mL round-bottom flask was placed 3-[5-methoxy-2-nitro-4-[3-(pyrrolidin-1-yl)propoxy]phenyl]oxolane-3-carbonitrile (160 mg, 0.43 mmol, 1.00 equiv), Pd-C (100 mg), AcOH (10 mL). The resulting solution was stirred for 1 h at 80 °C. The crude product was purified by Prep-HPLC with the following conditions (2#-AnalyseHPLC-SHIMADZU(HPLC-10)): Column, XSelect CSH Prep C18 OBD Column,, 19x250 mm, 5 μm; mobile phase, Water (0.05%TFA) and ACN (3.0% ACN up to 15.0% in 12 min); Detector, UV 254/220 nm. This resulted in 59.8 mg (31%) of the title compound as the trifluoroacetic acid salt.

**Example 8: Synthesis of Compound 8:**

Synthesis of **6-methoxy-N,1-dimethyl-5-(3-(pyrrolidin-1-yl)propoxy)-1H-benzo [d] imidazol-2-amine:**

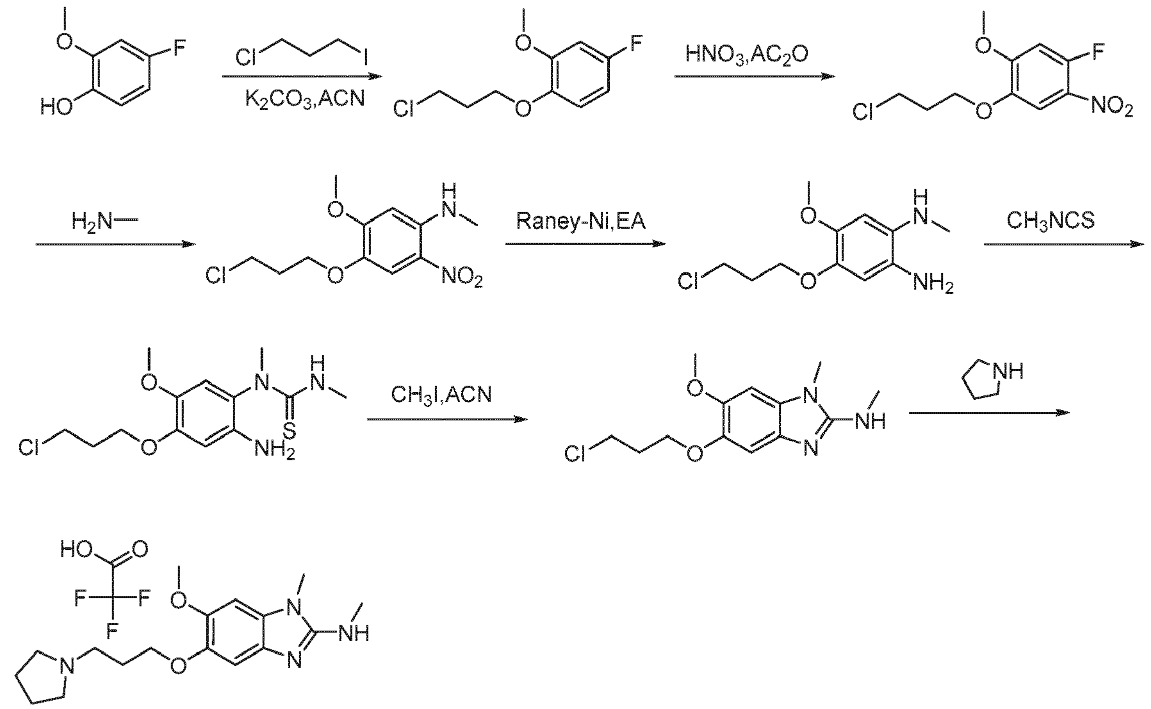


**Step 1:** Synthesis of 1-(3-chloropropoxy)-4-fluoro-2-methoxybenzene:

Into a 100-mL round-bottom flask was placed ACN (100 mL), 4-fluoro-2-methoxyphenol (5 g, 35.18 mmol, 1.00 equiv.), 1-chloro-3-iodopropane (14.4 g, 70.44 mmol, 2.00 equiv), and potassium carbonate (14.6 g, 105.64 mmol, 3.00 equiv). The resulting solution was stirred for 4 h at 80 °C. The solids were filtered out. The resulting mixture was concentrated under vacuum. This resulted in 7.7 g (100%) of the title compound as a yellow oil.

Analytical Data: LC-MS: (ES, *m/z):* RT = 1.365 min, *m/z* = 219 [M+1].

**Step 2:** Synthesis of 1-(3-chloropropoxy)-4-fluoro-2-methoxy-5-nitrobenzene:

Into a 500-mL round-bottom flask was placed acetic anhydride (14.4 g), 1-(3-chloropropoxy)-4-fluoro-2-methoxybenzene (7.7 g, 35.22 mmol, 1.00 equiv), $HNO_3$ (8.9 g). This was followed by addition of $HNO_3$ at 0 °C. The resulting solution was stirred for 12 h at 20 °C, then diluted with 100 mL of $H_2O$. The resulting solution was extracted with 4x60 mL of ethyl acetate and the organic layers combined. The resulting mixture was washed with 4x40 mL of sodium bicarbonate. The mixture was dried over anhydrous sodium sulfate and concentrated under vacuum. This resulted in 9.5 g (102%) of the title compound as a yellow solid.

Analytical Data: LC-MS: (ES, *m/z*): RT = 1.343 min, *m/z* = 264 [M+1].

**Step 3:** Synthesis of 4-(3-chloropropoxy)-5-methoxy-N-methyl-2-nitroaniline

Into a 250-mL round-bottom flask was placed 1-(3-chloropropoxy)-4-fluoro-2-methoxy-5-nitrobenzene (4 g, 15.17 mmol, 1.00 equiv), $CH_3NH_2 \cdot THF$ (100 mL). The resulting solution was stirred for 12 h at 20 °C. The resulting mixture was concentrated under vacuum. The residue was applied onto a silica gel column with $H_2O$/ACN (1:1). This resulted in 2.1 g (50%) of the title compound as a yellow solid.

Analytical Data: LC-MS: (ES, *m/z*): RT = 1.320 min, *m/z* = 275 [M+1].

**Step 4:** Synthesis of 4-(3-chloropropoxy)-5-methoxy-1-N-methylbenzene-1,2-diamine

Into a 250-mL round-bottom flask was placed ethyl acetate (10 mL), 4-(3-chloropropoxy)-5-methoxy-N-methyl-2-nitroaniline (250 mg, 0.91 mmol, 1.00 equiv), Raney-Ni (100 mg).The flask was purged and maintained with $H_2$. The resulting solution was stirred for 1 h at 20 °C. The solids were filtered out, and the resulting mixture was concentrated under vacuum. This resulted in 196 mg (88%) of the title compound as an oil.

Analytical Data: LC-MS: (ES, *m/z*): RT = 0.979 min, *m/z* = 245 [M+1].

**Step 5:** Synthesis of 1-[2-amino-4-(3-chloropropoxy)-5-methoxyphenyl]-1,3-dimethylthiourea:

Into a 100-mL round-bottom flask was placed tetrahydrofuran (10 mL), 4-(3-chloropropoxy)-5-methoxy-1-N-methylbenzene-1,2-diamine (196 mg, 0.80 mmol, 1.00 equiv), isothiocyanatomethane (70 mg, 0.96 mmol, 1.20 equiv). The resulting solution was stirred for 20 h at 20 °C. The flask was purged and maintained with $N_2$.The resulting mixture was concentrated under vacuum. This resulted in 216 mg (85%) of the title compound as a solid.

Analytical Data: LC-MS: (ES, *m/z*): RT = 1.173 min, *m/z* = 318 [M+1].

**Step 6:** Synthesis of 5-(3-chloropropoxy)-6-methoxy-N,1-dimethyl-1H-1,3-benzodiazol-2-amine

Into a 100-mL round-bottom flask was placed ACN (20 mL), 1-[2-amino-4-(3-chloropropoxy)-5-methoxyphenyl]-1,3-dimethylthiourea (216 mg, 0.68 mmol, 1.00 equiv). The solution was cooled below 0 °C and $CH_3I$ (115 mg, 0.81 mmol, 1.19 equiv) was added. The resulting solution was stirred for 12 h at 20 °C, then concentrated under vacuum. This resulted in 270 mg (140%) of the title compound as an oil.

Analytical Data: LC-MS: (ES, *m/z*): RT = 1.062 min, *m/z* = 284 [M+1].

**Step 6:** Synthesis of 6-methoxy-N,1-dimethyl-5-[3-(pyrrolidin-1-yl)propoxy]-1H-1,3-benzodiazol-2-amine

Into a 100-mL round-bottom flask was placed ACN (20 mL), 5-(3-chloropropoxy)-6-methoxy-N,1-dimethyl-1H-1,3-benzodiazol-2-amine (270 mg, 0.95 mmol, 1.00 equiv), pyrrolidine (203 mg, 2.85 mmol, 3.00 equiv), potassium carbonate (143 mg, 1.03 mmol, 1.09 equiv), NaI (395 mg). The resulting solution was stirred for 12 h at 80 °C. The resulting mixture was concentrated under vacuum. The crude product (100 mg) was purified by Prep-HPLC with the following conditions (2#-AnalyseHPLC-SHIMADZU(HPLC-10)): Column, XBridge Prep OBD C18 Column, 25×100 mm 5 μm; mobile phase, Water (0.08%TFA) and ACN (3.0% ACN up to 8.0% in 10 min); Detector, 10 μm. 42.6 mg product was obtained. This resulted in 42.6 mg (10%) of the title compound as the trifluoroacetic acid salt as a solid.

**Example 9: Synthesis of Compound 9:**

**Synthesis of (R)-1-cyclopentyl-5-((1-ethylpyrrolidin-3-yl)methoxy)-6-methoxy-1H-benzo [d] imidazole-2-amine:**

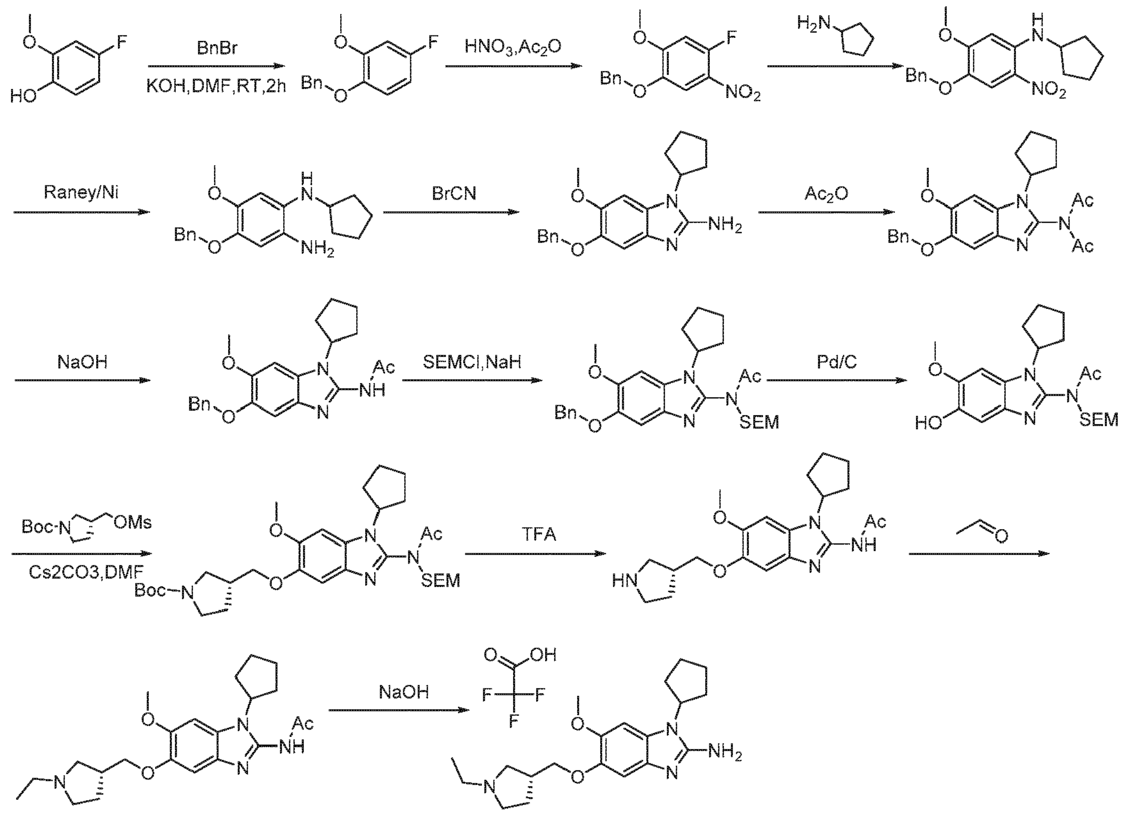

**Step 1:** Synthesis of 1-(benzyloxy)-4-fluoro-2-methoxybenzene:

Into a 100-mL round-bottom flask was placed 4-fluoro-2-methoxyphenol (3 g, 21.11 mmol, 1.00 equiv), followed by BnBr (4.3 g, 25.14 mmol, 1.19 equiv), N,N-dimethylformamide (3 mL), water (1 mL), potassium hydroxide (3.5 g, 62.38 mmol, 2.96 equiv). The resulting solution was stirred for 2 h at 0 °C. The resulting solution was extracted with dichloromethane and the organic layers combined and concentrated under vacuum. This resulted in 5 g (98%) of the title compound as an off-white solid.

**Step 2:** Synthesis of 1-(benzyloxy)-4-fluoro-2-methoxy-5-nitrobenzene

Into a 500-mL round-bottom flask was placed 1-(benzyloxy)-4-fluoro-2-methoxybenzene (5 g, 21.53 mmol, 1.00 equiv), $Ac_2O$ (40 mL), $HNO_3$ (12.2 mL). The resulting solution was stirred overnight at room temperature. The resulting mixture was concentrated under vacuum. The residue was applied onto a silica gel column with ethyl acetate/petroleum ether (76/24). This resulted in 5.6 g (94%) of the title compound as a light yellow solid.

Analytical Data: $^1H$ NMR (300 MHz, Chloroform-d) δ 7.68 (d, *J* = 7.2 Hz, 1H), 7.47 - 7.40 (m, 5H), 6.77 (d, *J* = 12.4 Hz, 1H), 5.16 (d, *J* = 13.2 Hz, 2H), 3.99 (s, 3H).

**Step 3:** Synthesis of 4-(benzyloxy)-N-cyclopentyl-5-methoxy-2-nitroaniline:

Into a 100-mL round-bottom flask was placed 1-(benzyloxy)-4-fluoro-2-methoxy-5-nitrobenzene (5.6 g, 20.20 mmol, 1.00 equiv), $Cs_2CO_3$ (20 g, 61.38 mmol, 3.04 equiv), ACN (15 mL), and cyclopentanamine (2.1 g, 24.66 mmol, 1.22 equiv). The resulting solution was stirred for 4 h at 50 °C. The solids were filtered out. The resulting solution was extracted with dichloromethane and the organic layers combined and concentrated under vacuum. This resulted in 6.8 g (98%) of the title compound as a yellow solid.

Analytical Data: LC-MS: (ES, m/z): RT=1.198 min, m/z=343[M+1]. $^1H$ NMR (300 MHz, Chloroform-d) δ 8.55 (d, *J* = 6.2 Hz, 1H), 7.69 (s, 1H), 7.51 - 7.26 (m, 5H), 6.21 (s, 1H), 5.08 (s, 2H), 3.95 (s, 3H), 2.21 -1.62 (m, 7H).

**Step 4:** Synthesis of 4-(benzyloxy)-1-N-cyclopentyl-5-methoxybenzene-1,2-diamine:

Into a 250-mL round-bottom flask was placed 4-(benzyloxy)-N-cyclopentyl-5-methoxy-2-nitroaniline (4 g, 11.68 mmol, 1.00 equiv), Raney-Ni (2 g), methanol (20 mL), hydrogen. The resulting solution was stirred for 5 h at room temperature. The solids were filtered out. The resulting mixture was concentrated under vacuum. This resulted in 4 g (98%) of the title compound as a brown liquid.

Analytical Data: LC-MS: (ES, *m/z):* RT=0.677 min, m/z=313 [M+1].

**Step 5:** Synthesis of 5-(benzyloxy)-1-cyclopentyl-6-methoxy-1H-1,3-benzodiazol-2-amine:

Into a 250-mL round-bottom flask was placed 4-(benzyloxy)-1-N-cyclopentyl-5-methoxybenzene-1,2-diamine (4 g, 12.80 mmol, 1.00 equiv), BrCN (2.7 g), ACN (2 mL), and water (15 mL). The resulting solution was stirred for 2 h at 65 °C. The resulting mixture was concentrated under vacuum. The crude product was purified by Flash-Prep-HPLC with the following conditions (IntelFlash-1): Column, C18 silica gel; mobile phase, $H_2O$/ACN=100/0 increasing to $H_2O$/ACN=65/35 within 30 min; Detector, UV 254 nm. This resulted in 3.2 g (74%) of the title compound as a brown solid.

Analytical Data: LC-MS: (ES, *m/z*): RT=0.870 min, m/z=338 [M+1]. $^1H$ NMR (300 MHz, Methanol-$d_4$) δ 7.53 - 7.20 (m, 5H), 7.06 (d, *J* = 8.9 Hz, 2H), 5.18 (d, *J* = 21.6 Hz, 2H), 3.96 (d, *J* = 18.5 Hz, 3H), 2.34 - 1.76 (m, 7H).

**Step 6:** Synthesis of N-acetyl-N-[5-(benzyloxy)-1-cyclopentyl-6-methoxy-1H-1,3-benzodiazol-2-yl]acetamide:

Into a 250-mL round-bottom flask was placed 5-(benzyloxy)-1-cyclopentyl-6-methoxy-1H-1,3-benzodiazol-2-amine (3.2 g, 9.48 mmol, 1.00 equiv), TEA (14 mL), tetrahydrofuran (10 mL), 4-dimethylaminopyridine (115 mg, 0.94 mmol, 0.10 equiv), acetic anhydride (40 mL). The resulting solution was stirred overnight at room temperature. The resulting mixture was concentrated under vacuum. The crude product was purified by Flash-Prep-HPLC with the following conditions (IntelFlash-1): Column, C18 silica gel; mobile phase, $H_2O$/ACN=100/0 increasing to $H_2O$/ACN=47/53 within 42 min; Detector, UV 254 nm. This resulted in 3.8 g (95%) of as an off-white solid.

Analytical Data: LC-MS: (ES, *m/z*): RT=1.334 min, m/z=422 [M+1].

**Step 7:** Synthesis of N-(5-(benzyloxy)-1-cyclopentyl-6-methoxy-1H-benzo[d]imidazol-2-yl)acetamide:

Into a 100-mL round-bottom flask was placed N-acetyl-N-[5-(benzyloxy)-1-cyclopentyl-6-methoxy-1H-1,3-benzodiazol-2-yl]acetamide (50 mg, 0.12 mmol, 1.00 equiv), methanol (2 mL), sodium hydroxide (14.3 mg, 0.36 mmol, 3.01 equiv). The resulting solution was stirred for 1 h at room temperature.

Analytical Data: LC-MS: (ES, *m/z*): RT=0.980 min, m/z=380 [M+1].

**Step 8:** Synthesis of N-[5-(benzyloxy)-1-cyclopentyl-6-methoxy-1H-1,3-benzodiazol-2-yl]-N-[[2-(trimethylsilyl)ethoxy]methyl]acetamide:

Into a 100-mL round-bottom flask was placed N-[5-(benzyloxy)-1-cyclopentyl-6-methoxy-1H-1,3-benzodiazol-2-yl]acetamide (1.71 g, 4.51 mmol, 1.00 equiv), sodium hydride (902 mg, 37.58 mmol, 8.34 equiv), and tetrahydrofuran (10 mL), SEMCl (899 mg). The resulting solution was stirred for 1 h at 0 °C. The resulting solution was allowed to react, with stirring, for an additional 2 h at room temperature. The resulting solution was extracted with dichloromethane and the organic layers combined and concentrated under vacuum. The crude product was purified by Flash-Prep-HPLC with the following conditions (IntelFlash-1): Column, C18 silica gel; mobile phase, $H_2O$/ACN=100/0 increasing to $H_2O$/ACN=55/45 within 37 min; Detector, UV 254 nm.This resulted in 1.96 g (85%) of the title compound as a light yellow solid.

Analytical Data: LC-MS: (ES, *m/z):* RT=1.181 min, m/z=510 [M+1].

**Step 9:** Synthesis of N-(1-cyclopentyl-5-hydroxy-6-methoxy-1H-1,3-benzodiazol-2-yl)-N-[[2-(trimethylsilyl)ethoxy]methyl]acetamide:

Into a 100-mL round-bottom flask was placed N-[5-(benzyloxy)-1-cyclopentyl-6-methoxy-1H-1,3-benzodiazol-2-yl]-N-[[2-(trimethylsilyl)ethoxy]methyl]acetamide (1.1 g, 2.16 mmol, 1.00 equiv), Palladium on carbon (1 g), methanol (10 mL), and hydrogen gas (1 L). The resulting solution was stirred for 0.5 h at room temperature. The solids were filtered out, and the resulting mixture was concentrated under vacuum. This resulted in 710 mg (78%) of the title compound as a light yellow solid.

Analytical Data: LC-MS: (ES, *m/z):* RT=1.101 min, m/z=420 [M+1].

**Step 10:** Synthesis of tert-butyl (3R)-3-([[1-cyclopentyl-6-methoxy-2-(N-[[2-(trimethylsilyl)ethoxy]methyl]acetamido)-1H-1,3-benzodiazol-5-yl]oxy]methyl)pyrrolidine-1-carboxylate:

Into a 10-mL vial was placed N-(1-cyclopentyl-5-hydroxy-6-methoxy-1H-1,3-benzodiazol-2-yl)-N-[[2-(trimethylsilyl)ethoxy]methyl]acetamide (600 mg, 1.43 mmol, 1.00 equiv), $Cs_2CO_3$ (1.4 g, 4.30 mmol, 3.00 equiv), N,N-dimethylformamide (3 mL), and tert-butyl (3R)-3-[(methanesulfonyloxy)methyl]pyrrolidine-1-carboxylate (479 mg, 1.71 mmol, 1.20 equiv). The resulting solution was stirred for 2 h at 80 °C. The solids were filtered out, and the crude product was purified by Flash-Prep-HPLC with the following conditions (IntelFlash-1): Column, C18 silica gel; mobile phase, $H_2O$/ACN=100/0 increasing to $H_2O$/ACN=73/27 within 32 min; Detector, UV 254 nm. This resulted in 671 mg (78%) of the title compound as a brown oil.

Analytical Data: LC-MS: (ES, m/z): RT=1.024 min, m/z=603 [M+1].

**Step 11.** Synthesis of N-[1-cyclopentyl-6-methoxy-5-[(3R)-pyrrolidin-3-ylmethoxy]-1H-1,3-benzodiazol-2-yl]acetamide:

Into a 100-mL round-bottom flask was placed tert-butyl (3R)-3-([[1-cyclopentyl-6-methoxy-2-(N-[[2-(trimethylsilyl)ethoxy]methyl]acetamido)-1H-1,3-benzodiazol-5-yl]oxy]methyl)pyrrolidine-1-carboxylate (671 mg, 1.11 mmol, 1.00 equiv), and trifluoroacetic acid (10 mL). The resulting solution was stirred overnight at room temperature. The resulting mixture was concentrated under vacuum. This resulted in 415 mg (100%) of the title compound as a light yellow oil.

Analytical Data: LC-MS: (ES, *m/z):* RT=0.572 min, m/z=373 [M+1].

**Step 12:** Synthesis of N-(1-cyclopentyl-5-[[(3R)-1-ethylpyrrolidin-3-yl]methoxy]-6-methoxy-1H-1,3-benzodiazol-2-yl)acetamide:

Into a 100-mL round-bottom flask was placed N-[1-cyclopentyl-6-methoxy-5-[(3R)-pyrrolidin-3-ylmethoxy]-1H-1,3-benzodiazol-2-yl]acetamide (100 mg, 0.27 mmol, 1.00 equiv), $NaBH_3CN$ (85 mg, 1.35 mmol, 5.04 equiv), methanol (5 mL), $CH_3CHO$ (1 mL). The resulting solution was stirred for 2 h at 0 °C. The resulting solution was extracted with dichloromethane and the organic layers combined and concentrated under vacuum. This resulted in 96 mg (89%) of the title compound as a brown oil.

Analytical Data: LC-MS: (ES, *m/z*): RT=0.578 min, m/z=401 [M+1].

**Step 13:** Synthesis of 1-cyclopentyl-5-[[(3R)-1-ethylpyrrolidin-3-yl]methoxy]-6-methoxy-1H-1,3-benzodiazol-2-amine:

Into a 100-mL round-bottom flask was placed N-(1-cyclopentyl-5-[[(3R)-1-ethylpyrrolidin-3-yl]methoxy]-6-methoxy-1H-1,3-benzodiazol-2-yl)acetamide (120 mg, 0.30 mmol, 1.00 equiv), sodiumol (100 mg, 2.50 mmol, 8.34 equiv), ethanol (2 mL), and water(2 mL). The resulting solution was stirred for 5 h at 80 °C. Column: X Select C18, 19x150 mm, 5 μm; mobile phase, Mobile Phase A:Water/0.05% TFA, Mobile Phase B: ACN;Detector, 254. This resulted in 52.4 mg (37%) of the title compound as the trifluoroacetic acid salt as light brown oil.

**Example 10: Synthesis of Compound 10:**

**Synthesis of 1-cyclopentyl-5-((1-ethylazetidin-3-yl)methoxy)-6-methoxy-1H-benzo [d] imidazol-2-amine:**

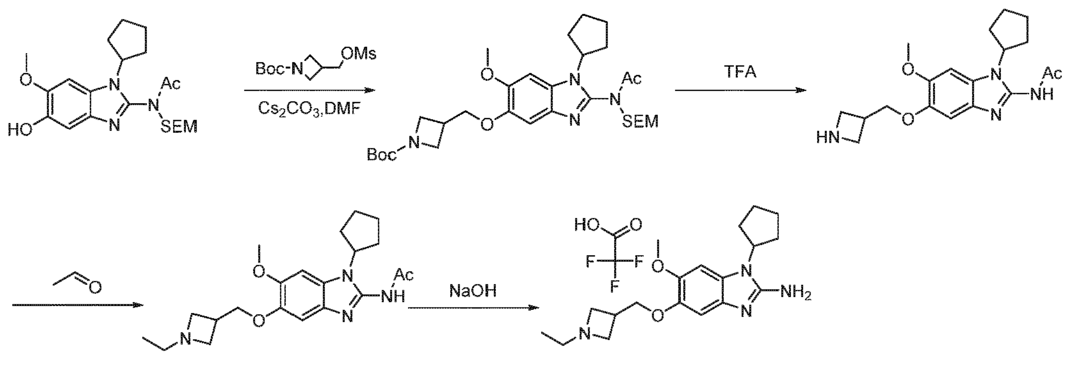


**Step 1:** Synthesis of : tert-butyl 3-([[1-cyclopentyl-6-methoxy-2-(N-[[2-(trimethylsilyl)ethoxy]methyl]acetamido)-1H-1,3-benzodiazol-5-yl]oxy]methyl)azetidine-1-carboxylate:

Into a 10-mL vial was placed N-(1-cyclopentyl-5-hydroxy-6-methoxy-1H-1,3-benzodiazol-2-yl)-N-[[2-(trimethylsilyl)ethoxy]methyl]acetamide (600 mg, 1.43 mmol, 1.00 equiv), $Cs_2CO_3$ (1.4 g, 4.30 mmol, 3.00 equiv), tert-butyl 3-[(methanesulfonyloxy)methyl]azetidine-1-carboxylate (455 mg, 1.71 mmol, 1.20 equiv), N,N-dimethylformamide (2 mL). The resulting solution was stirred for 2 h at 80 °C. The solids were filtered out. The crude product was purified by Flash-Prep-HPLC with the following conditions (IntelFlash-1): Column, C18 silica gel; mobile phase, $H_2O$/ACN=100/0 increasing to $H_2O$/ACN=63/37 within 35 min; Detector, UV 254 nm.This resulted in 596 mg (71%) of the title compound as a brown oil.

Analytical Data: LC-MS: (ES, *m/z):* RT=0.980 min, m/z=589 [M+1].

**Step 2:** Synthesis of N-[5-(azetidin-3-ylmethoxy)-1-cyclopentyl-6-methoxy-1H-1,3-benzodiazol-2-yl]acetamide:

Into a 100-mL round-bottom flask was placed tert-butyl 3-([[1-cyclopentyl-6-methoxy-2-(N-[[2-(trimethylsilyl)ethoxy]methyl]acetamido)-1H-1,3-benzodiazol-5-yl]oxy]methyl)azetidine-1-carboxylate (596 mg, 1.01 mmol, 1.00 equiv), and trifluoroacetic acid (5 mL). The resulting solution was stirred overnight at room temperature. The resulting mixture was concentrated under vacuum. This resulted in 360 mg (99%) of the title compound as a light yellow oil.

Analytical Data: LC-MS: (ES, *m/z*): RT=0.554 min, m/z=359 [M+1].

**Step 3:** Synthesis of N-[1-cyclopentyl-5-[(1-ethylazetidin-3-yl)methoxy]-6-methoxy-1H-1,3-benzodiazol-2-yl]acetamide:

Into a 100-mL round-bottom flask was placed N-[5-(azetidin-3-ylmethoxy)-1-cyclopentyl-6-methoxy-1H-1,3-benzodiazol-2-yl]acetamide (100 mg, 0.28 mmol, 1.00 equiv), $NaBH_3CN$ (92 mg, 1.46 mmol, 5.25 equiv), methanol (5 mL), and $CH_3CHO$ (1 mL). The resulting solution was stirred for 2 h at 0 °C. The resulting solution was extracted with dichloromethane and the organic layers combined and concentrated under vacuum. This resulted in 100 mg (93%) of the title compound as light yellow oil.

Analytical Data: LC-MS: (ES, *m/z*): RT=0.567 min, m/z=387 [M+1].

Step 4: Synthesis of 1-cyclopentyl-5-[(1-ethylazetidin-3-yl)methoxy]-6-methoxy-1H-1,3-benzodiazol-2-amine

Into a 100-mL round-bottom flask was placed N-[1-cyclopentyl-5-[(1-ethylazetidin-3-yl)methoxy]-6-methoxy-1H-1,3-benzodiazol-2-yl]acetamide (120 mg, 0.31 mmol, 1.00 equiv), sodiumol (100 mg, 2.50 mmol, 8.05 equiv), ethanol (2 mL), and water (2 mL). The resulting solution was stirred for 5 h at 80 °C. The crude product was purified by Prep-HPLC with the following conditions: Column, Column: X Select C18, 19*150 mm, 5 μm;mobile phase, Mobile Phase A:Water/0.05% TFA, Mobile Phase B: ACN;; Detector, 254. This resulted in 71.6 mg (50%) of the title compound as the trifluoroacetic acid salt as a light brown oil.

Example 11: **Synthesis of Compound 11:**

**Synthesis of 6-methoxy-5-(3-(pyrrolidin-1-yl)propoxy)-1-(2,2,2-trifluoroethyl)-1H-benzo [d] imidazole-2-amine:**

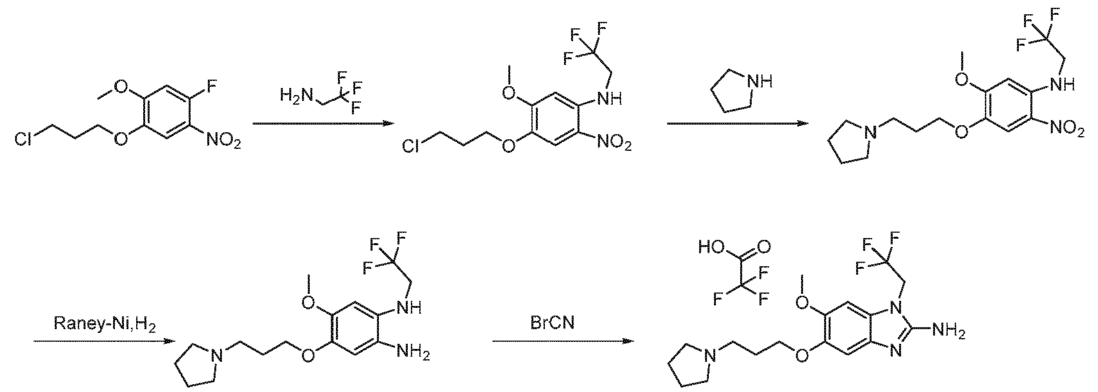


Step 1: Synthesis of 4-(3-chloropropoxy)-5-methoxy-2-nitro-N-(2,2,2-trifluoroethyl)aniline:

Into a 20-mL round-bottom flask was placed 1-(3-chloropropoxy)-4-fluoro-2-methoxy-5-nitrobenzene (1.2 g, 4.55 mmol, 1.00 equiv), 2,2,2-trifluoroethan-1-amine (9 mL), DMSO (3 mL). The resulting solution was stirred for 4 h at 80 °C in an oil bath. The resulting mixture was concentrated under vacuum. The crude product was purified by Flash-Prep-HPLC with the following conditions (IntelFlash-1): Column, silica gel; mobile phase, methanol/$H_2O$=1/1; Detector, UV 254 nm. This resulted in 500 mg (32%) of the title compound as a yellow solid.

Analytical Data: LC-MS: (ES, *m/z*)*:* RT= 1.39 min, m/z = 343 [M+1].

**Step 2:** Synthesis of 5-methoxy-2-nitro-4-[3-(pyrrolidin-1-yl)propoxy]-N-(2,2,2-trifluoroethyl)aniline:

Into a 100-mL round-bottom flask was placed 4-(3-chloropropoxy)-5-methoxy-2-nitro-N-(2,2,2-trifluoroethyl)aniline (500 mg, 1.46 mmol, 1.00 equiv), pyrrolidine (311 mg, 4.37 mmol, 3.00 equiv), NaI (219 mg, 1.00 equiv), potassium carbonate (605 mg, 4.38 mmol, 3.00 equiv), and ACN (10 mL). The resulting solution was stirred for 12 h at 80 °C in an oil bath. The solids were filtered out. The crude product was purified by Flash-Prep-HPLC with the following conditions (IntelFlash-1): Column, silica gel; mobile phase, methanol/H2O=1/1; Detector, UV 254 nm. This resulted in 350 mg (64%) of the title compound as a yellow solid.

Analytical Data: LC-MS: (ES, *m/z*)*:* RT= 1.02 min, m/z = 378 [M+1].

**Step 3:** Synthesis of 5-methoxy-4-[3-(pyrrolidin-1-yl)propoxy]-1-N-(2,2,2-trifluoroethyl)benzene-1,2-diamine:

Into a 100-mL round-bottom flask was placed 5-methoxy-2-nitro-4-[3-(pyrrolidin-1-yl)propoxy]-N-(2,2,2-trifluoroethyl)aniline (300 mg, 0.79 mmol, 1.00 equiv), Rancy Ni (500 mg), methanol (10 mL), and hydrogen. The resulting solution was stirred for 1.5 h at 25 °C. The solids were filtered out. The resulting mixture was concentrated under vacuum. This resulted in 200 mg (72%) of the title compound as a colorless oil.

Analytical Data: LC-MS: (ES, *m/z*)*:* RT= 0.82 min, m/z = 320 [M+1].

**Step 4:** Synthesis of 6-methoxy-5-[3-(pyrrolidin-1-yl)propoxy]-1-(2,2,2-trifluoroethyl)-1H-1,3-benzodiazol-2-amine:

Into a 50-mL round-bottom flask was placed 5-methoxy-4-[3-(pyrrolidin-1-yl)propoxy]-1-N-(2,2,2-trifluoroethyl)benzene-1,2-diamine (150 mg, 0.43 mmol, 1.00 equiv), carbononitridic bromide (500 mg, 4.72 mmol, 10.93 equiv), ACN (3 mL), and water (9 mL). The resulting solution was stirred for 2 h at 50 °C in an oil bath. The resulting mixture was concentrated under vacuum. The crude product was purified by Flash-Prep-HPLC with the following conditions (IntelFlash-1): Column, silica gel; mobile phase, methanol/$H_2O$=1/1; Detector, UV 254 nm. This resulted in 10.3 mg (5%) of the title compound as the trifluoroacetic acid salt as a brown oil.

**Example 12: Synthesis of Compound 12**

**Synthesis of 6-methoxy-1-methyl-5-(3-(pyrrolidin-1-yl)propoxy)-N-(2,2,2-trifluoroethyl)-1H-benzo [d] imidazol-2-amine:**

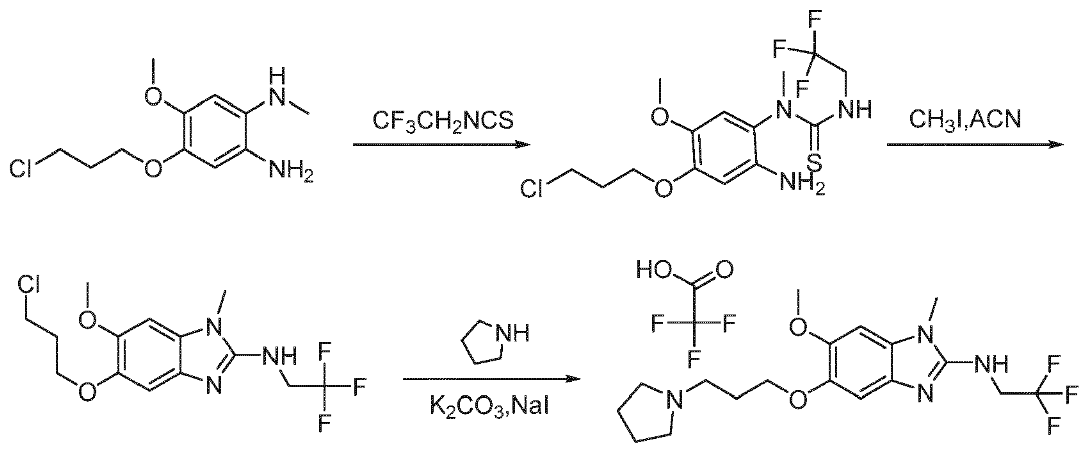


**Step 1:** Synthesis of 1-[2-amino-4-(3-chloropropoxy)-5-methoxyphenyl]-1-methyl-3-(2,2,2-trifluoroethyl)thiourea:

Into a 100-mL round-bottom flask was placed tetrahydrofuran (10 mL), and 4-(3-chloropropoxy)-5-methoxy-1-N-methylbenzene-1,2-diamine (309 mg, 1.26 mmol, 1.00 equiv), 1,1,1-trifluoro-2-isothiocyanatoethane (213 mg, 1.51 mmol, 1.20 equiv).The flask was purged and maintained with $N_2$. The resulting solution was stirred for 12 h at 80 °C. The resulting mixture was concentrated under vacuum. This resulted in 495 mg (102%) of the title compound as an oil.

Analytical Data: LC-MS: (ES, *m*/*z*)*:* RT = 1.360min, *m*/*z* = 386 [M+1].

**Step 2:** Synthesis of 5-(3-chloropropoxy)-6-methoxy-1-methyl-N-(2,2,2-trifluoroethyl)-1H-1,3-benzodiazol-2-amine:

Into a 100-mL round-bottom flask was placed ACN (30 mL), 1-[2-amino-4-(3-chloropropoxy)-5-methoxyphenyl]-1-methyl-3-(2,2,2-trifluoroethyl)thiourea (495 mg, 1.28 mmol, 1.00 equiv), and iodomethane (218 mg, 1.54 mmol, 1.20 equiv). The resulting solution was stirred for 12 h at 20 °C. The resulting mixture was concentrated under vacuum. This resulted in 485 mg (107%) of the title compound as an oil.

Analytical Data: LC-MS: (ES, *m*/*z*): RT = 1.155min, *m*/*z* = 352 [M+1].

**Step 3:** Synthesis of 6-methoxy-1-methyl-5-[3-(pyrrolidin-1-yl)propoxy]-N-(2,2,2-trifluoroethyl)-1H-1,3-benzodiazol-2-amine:

Into a 100-mL round-bottom flask was placed ACN (30 mL), 5-(3-chloropropoxy)-6-methoxy-1-methyl-N-(2,2,2-trifluoroethyl)-1H-1,3-benzodiazol-2-amine (485 mg, 1.38 mmol, 1.00 equiv), pyrrolidine (294 mg, 4.13 mmol, 3.00 equiv), potassium carbonate (572 mg, 4.14 mmol, 3.00 equiv), and NaI (207 mg). The resulting solution was stirred for 12 h at 80 °C. The resulting mixture was concentrated under vacuum. The crude product (200 mg) was purified by Prep-HPLC with the following conditions (2#-AnalyseHPLC-SHIMADZU (HPLC-10)): Column, XBridge Prep OBD C18 Column, 25×100mm 5 μm; mobile phase, Water (0.08% TFA) and ACN (3.0% ACN up to 8.0% in 10 min); Detector, 10 μm. This resulted in 62 mg (9%) of the title compound as the trifluoroacetic acid salt as a yellow solid.

**Example 13: Synthesis of Compound 13:**

**Synthesis of 6-methoxy-N-methyl-5-(3-(pyrrolidin-1-yl)propoxy)-1-(tetrahydro-2H-pyran-4-yl)-1H-benzo[d]imidazol-2-amine:**

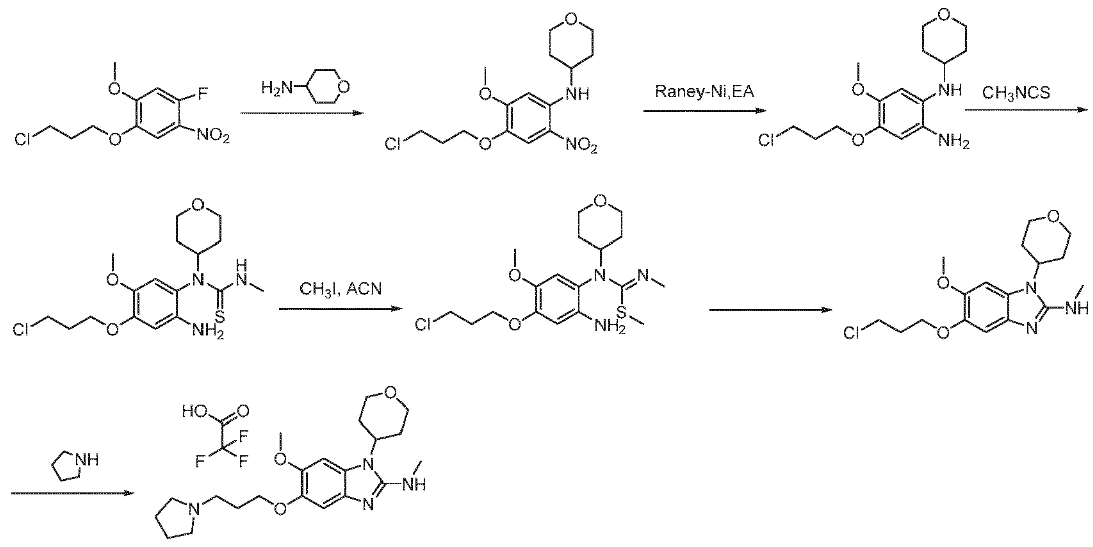


**Step 1:** Synthesis of N-[4-(3-chloropropoxy)-5-methoxy-2-nitrophenyl]oxan-4-amine:

Into a 20-mL vial, was placed 1-(3-chloropropoxy)-4-fluoro-2-methoxy-5-nitrobenzene (1 g, 3.79 mmol, 1.00 equiv), oxan-4-amine (461 mg, 4.56 mmol, 1.20 equiv), and DMSO (10 mL). The resulting solution was stirred for 6 h at 80 °C in an oil bath. The resulting solution was extracted with ethyl acetate and the organic layers combined. The crude product was purified by Flash-Prep-HPLC with the following conditions (IntelFlash-1): Column, silica gel; mobile phase, PE:EA=1/1; Detector, UV 254 nm. This resulted in 400 mg (31%) of as a yellow solid.

Analytical Data: LC-MS: (ES, *m/z*)*:* RT= 1.06 min, m/z = 345 [M+1].

**Step 2:** Synthesis of 4-(3-chloropropoxy)-5-methoxy-N1-(tetrahydro-2H-pyran-4-yl)benzene-1,2-diamine:

Into a 50-mL round-bottom flask was placed N-[4-(3-chloropropoxy)-5-methoxy-2-nitrophenyl]oxan-4-amine (200 mg, 0.58 mmol, 1.00 equiv), Raney-Ni (200 mg), and ethyl acetate (10 mL). The resulting solution was stirred for 1 h at 25 °C. The resulting mixture was concentrated under vacuum.

Analytical Data: LC-MS: (ES, *m/z*)*:* RT= 0.96 min, m/z = 315 [M+1].

**Step 3:** Synthesis of 1-[2-amino-4-(3-chloropropoxy)-5-methoxyphenyl]-3-methyl-1-(oxan-4-yl)thiourea:

Into a 50-mL round-bottom flask was placed 4-(3-chloropropoxy)-5-methoxy-1-N-(oxan-4-yl)benzene-1,2-diamine (150 mg, 0.48 mmol, 1.00 equiv), isothiocyanatomethane (42 mg, 0.57 mmol, 1.20 equiv), and tetrahydrofuran (10 mL). The resulting solution was stirred for 12 h at 25 °C. The resulting mixture was concentrated under vacuum. This resulted in 160 mg (87%) of the title compound as brown oil.

Analytical Data: LC-MS: (ES, *m/z):* RT= 1.04 min, m/z = 388 [M+1].

**Step 4:** Synthesis of methyl (Z)-N-(2-amino-4-(3-chloropropoxy)-5-methoxyphenyl)-N'-methyl-N-(tetrahydro-2H-pyran-4-yl)carbamimidothioate:

Into a 50-mL round-bottom flask was placed 1-[2-amino-4-(3-chloropropoxy)-5-methoxyphenyl]-3-methyl-1-(oxan-4-yl)thiourea (160 mg, 0.41 mmol, 1.00 equiv), iodomethane (71 mg, 0.50 mmol, 1.20 equiv), and ACN (10 mL). The resulting solution was stirred for 12 h at 25 °C. The resulting mixture was concentrated under vacuum. This resulted in 150 mg (90%) of the title compound as a light yellow oil.

Analytical Data: LC-MS: (ES, m/z): RT= 1.01 min, m/z = 402 [M+1].

**Step 5:** Synthesis of 5-(3-chloropropoxy)-6-methoxy-N-methyl-1-(oxan-4-yl)-1H-1,3-benzodiazol-2-amine:

Into a 50-mL round-bottom flask was placed (Z)-N-[2-amino-4-(3-chloropropoxy)-5-methoxyphenyl]-N_ethyl-N-(oxan-4-yl)(methylsulfanyl)methanimidamide (150 mg, 0.37 mmol, 1.00 equiv), and methanol (10 mL). The resulting solution was stirred for 48 h at 45 °C in an oil bath. The resulting mixture was concentrated under vacuum. This resulted in 120 mg (91%) of the title compound as a light yellow oil.

Analytical Data: LC-MS: (ES, *m/z*): RT= 0.78 min, m/z = 354 [M+1].

**Step 6:** Synthesis of 6-methoxy-N-methyl-1-(oxan-4-yl)-5-[3-(pyrrolidin-1-yl)propoxy]-1H-1,3-benzodiazol-2-amine:

Into a 50-mL round-bottom flask was placed 5-(3-chloropropoxy)-6-methoxy-N-methyl-1-(oxan-4-yl)-1H-1,3-benzodiazol-2-amine (120 mg, 0.34 mmol, 1.00 equiv), pyrrolidine (72 mg, 1.01 mmol, 1.00 equiv), NaI (51 mg, 1.00 equiv), potassium carbonate (140.7 mg, 1.02 mmol, 3.00 equiv), and ACN (10 mL). The resulting solution was stirred for 12 h at 80 °C in an oil bath. The resulting solution was extracted with ethyl acetate and the organic layers combined. The crude product was purified by Flash-Prep-HPLC with the following conditions (IntelFlash-1): Column, silica gel; mobile phase, methanol:$H_2O$=1/1; Detector, UV 254 nm. This resulted in 114.5 mg (67%) of the title compound as the trifluoroacetic acid salt as a brown solid.

**Example 14: Synthesis of Compound 21:**

**Synthesis of 6-methoxy-1,2-dimethyl-5-(3-(pyrrolidin-1-yl)propoxy)-1H-benzo [d] imidazole:**

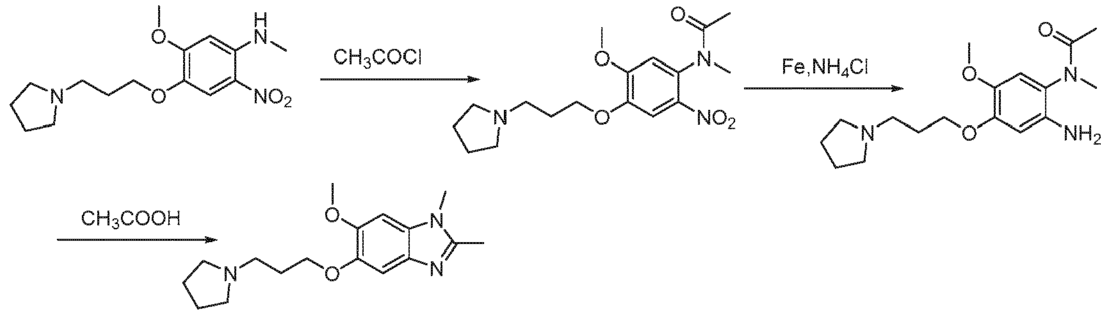


**Step 1:** Synthesis of N-[5-methoxy-2-nitro-4-[3-(pyrrolidin-1-yl)propoxy]phenyl]-N-methylacetamide:

Into a 100-mL round-bottom flask was placed toluene (10 mL), 5-methoxy-N-methyl-2-nitro-4-[3-(pyrrolidin-1-yl)propoxy]aniline (300 mg, 0.97 mmol, 1.00 equiv), acetyl chloride (77 mg, 0.98 mmol, 1.01 equiv), and DIEA (188 mg, 1.45 mmol, 1.50 equiv). The resulting solution was stirred for 2 h at 80 °C. The resulting mixture was concentrated under vacuum, then diluted with 30 mL of ethyl acetate. The resulting mixture was washed with 3x30 mL of $H_2O$. The mixture was dried over anhydrous sodium sulfate and concentrated under vacuum. This resulted in 830 mg (244%) of the title compound as an oil.

Analytical Data: LC-MS: (ES, *m/z*)*:* RT = 0.581 min, *m/z* = 352 [M+1].

**Step 2:** Synthesis of N-[2-amino-5-methoxy-4-[3-(pyrrolidin-1-yl)propoxy]phenyl]-N-methylacetamide:

Into a 100-mL round-bottom flask was placed EtOH (10 mL), N-[5-methoxy-2-nitro-4-[3-(pyrrolidin-1-yl)propoxy]phenyl]-N-methylacetamide (500 mg, 1.42 mmol, 1.00 equiv), Fe (300 mg), $NH_4Cl$ (226 mg, 4.23 mmol, 2.97 equiv), and water (10 mL). The resulting solution was stirred for 12 h at 90 °C. The solids were filtered out, and the resulting mixture was concentrated under vacuum. This resulted in 400 mg (87%) of the title compound as an oil.

Analytical Data: LC-MS: (ES, *m/z*): RT = 0.514 min, *m/z* = 322 [M+1].

**Step 3:** Synthesis of 6-methoxy-1,2-dimethyl-5-[3-(pyrrolidin-1-yl)propoxy]-1H-1,3-benzodiazole:

Into a 100-mL round-bottom flask was placed acetic acid (10 mL), and N-[2-amino-5-methoxy-4-[3-(pyrrolidin-1-yl)propoxy]phenyl]-N-methylacetamide (400 mg, 1.24 mmol, 1.00 equiv). The resulting solution was stirred for 12 h at

90 °C. The resulting mixture was concentrated under vacuum. The crude product (100 mg) was purified by Prep-HPLC with the following conditions (2#-AnalyseHPLC-SHIMADZU(HPLC-10)): Column, XBridge BEH130 Prep C18 OBD Column, 19x150mm 5 μm 13nm; mobile phase, Water(10MMOL/L NH4HCO3) and ACN (27.0% ACN up to 33.0% in 10 min); Detector, UV 254/220 nm. This resulted in 51.1 mg (14%) of the title compound as an off-white solid.

**Example 15: Synthesis of Compound 22:**

**Synthesis of 2-cyclopentyl-6-methoxy-1-methyl-5-(3-(pyrrolidin-1-yl)propoxy)-1H-benzo [d] imidazole:**

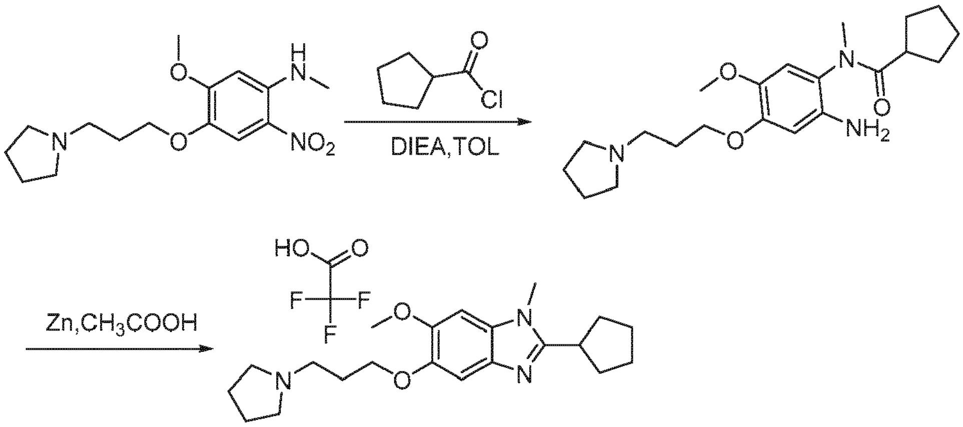


**Step 1:** Synthesis of N-[5-methoxy-2-nitro-4-[3-(pyrrolidin-1-yl)propoxy]phenyl]-N-methylcyclopentanecarboxamide:

Into a 100-mL round-bottom flask was placed toluene (30 mL), cyclopentanecarbonyl chloride (200 mg, 1.51 mmol, 1.00 equiv), 5-methoxy-N-methyl-2-nitro-4-[3-(pyrrolidin-1-yl)propoxy]aniline (1 g, 3.23 mmol, 2.14 equiv), and DIEA (1 g, 7.74 mmol, 5.13 equiv). The resulting solution was stirred for 12 h at 80 °C. The resulting mixture was concentrated under vacuum. The resulting solution was diluted with 20 mL of ethyl acetate. The resulting mixture was washed with 3x30 mL of $H_2O$. The resulting mixture was concentrated under vacuum. This resulted in 1.4 g (229%) of the title compound as a yellow oil.

Analytical Data: LC-MS: (ES, *m/z*): RT = 0.699 min, *m/z* = 406 [M+1].

**Step 2:** Synthesis of 2-cyclopentyl-6-methoxy-1-methyl-5-[3-(pyrrolidin-1-yl)propoxy]-1H-1,3-benzodiazole:

Into a 250-mL round-bottom flask was placed acetic acid (50 mg, 0.83 mmol, 0.68 equiv), N-[5-methoxy-2-nitro-4-[3-(pyrrolidin-1-yl)propoxy]phenyl]-N-methylcyclopentanecarboxamide (500 mg, 1.23 mmol, 1.00 equiv), and Zn (100 mg). The resulting solution was stirred for 12 h at 90 °C. The resulting mixture was concentrated under vacuum. The solids were filtered out. The crude product (200 mg) was purified by Prep-HPLC with the following conditions (2#-AnalyseHPLC-SHIMADZU(HPLC-10)): Column, XSelect CSH Prep C18 OBD Column,, 19x250mm,5 μm; mobile phase, water (0.05%TFA) and ACN (5.0% ACN up to 30.0% in 7 min); Detector, UV 254/220nm. This resulted in 121.1 mg (21%) of the title compound as the trifluoroacetic acid salt as an off-white oil.

**Example 16: Synthesis of Compound 23:**

**Synthesis of 1-cyclopentyl-6-methoxy-2-methyl-5-(3-(pyrrolidin-1-yl)propoxy)-1H-benzo [d]imidazole:**

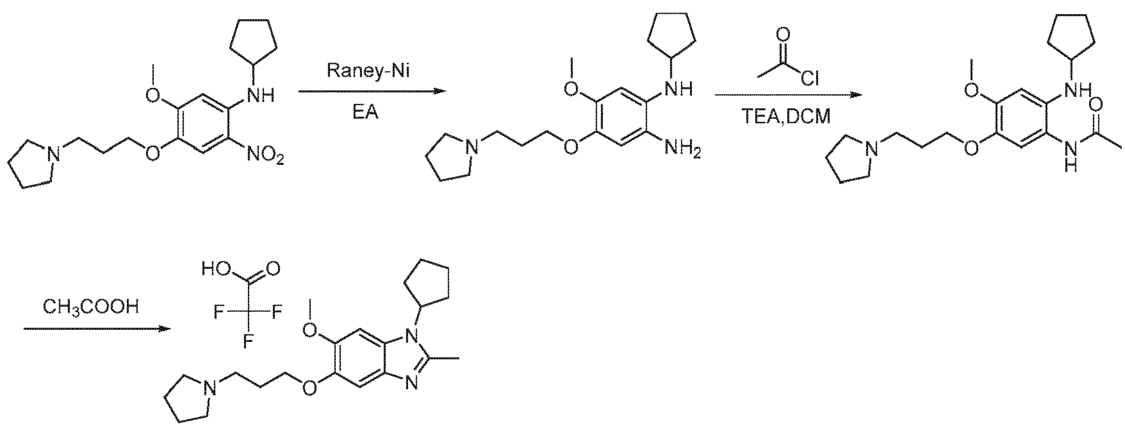


**Step 1:** Synthesis of 1-N-cyclopentyl-5-methoxy-4-[3-(pyrrolidin-1-yl)propoxy]benzene-1,2-diamine:

Into a 100-mL round-bottom flask was placed methanol (30 mL), N-cyclopentyl-5-methoxy-2-nitro-4-[3-(pyrrolidin-1-yl)propoxy]aniline (240 mg, 0.66 mmol, 1.00 equiv), and Raney-Ni (30 mg).The flask was purged and maintained with $H_2$. The resulting solution was stirred for 1 h at 20 °C. The solids were filtered out. The resulting mixture was concentrated under vacuum. This resulted in 160 mg (73%) of the title compound as an oil.

Analytical Data: LC-MS: (ES, *m/z*): RT = 0.542 min, *m/z* = 334 [M+1].

**Step 2:** Synthesis of N-[2-(cyclopentylamino)-4-methoxy-5-[3-(pyrrolidin-1-yl)propoxy]phenyl]acetamide:

Into a 100-mL round-bottom flask was placed dichloromethane (10 mL), 1-N-cyclopentyl-5-methoxy-4-[3-(pyrrolidin-1-yl)propoxy]benzene-1,2-diamine (165 mg, 0.49 mmol, 1.00 equiv), $CH_3COCl$ (43 mg, 0.55 mmol, 1.11 equiv), and TEA (150 mg, 1.48 mmol, 3.00 equiv). The resulting solution was stirred for 12 h at 90 °C. The resulting mixture was concentrated under vacuum. This resulted in 150 mg (81%) of the title compound as an oil.

Analytical Data: LC-MS: (ES, *m/z*): RT = 0.959 min, *m/z* = 376 [M+1].

**Step 3:** Synthesis of 1-cyclopentyl-6-methoxy-2-methyl-5-[3-(pyrrolidin-1-yl)propoxy]-1H-1,3-benzodiazole:

Into a 50-mL round-bottom flask was placed N-[2-(cyclopentylamino)-4-methoxy-5-[3-(pyrrolidin-1-yl)propoxy]phenyl]acetamide (140 mg, 0.37 mmol, 1.00 equiv), and acetic acid (10 mL). The resulting solution was stirred for 12 h at 90 °C. The resulting mixture was concentrated under vacuum. The crude product (70 mg) was purified by Prep-HPLC with the following conditions (2#-AnalyseHPLC-SHIMADZU(HPLC-10)): Column, XSelect CSH Prep C18 OBD Column,, 19x250mm,5 μm; mobile phase, Water (0.05%TFA) and ACN (5.0% ACN up to 26.0% in 7 min); Detector, UV 254/220nm. 21.9 mg product was obtained. This resulted in 21.9 mg (12%) of the title compound as the trifluoroacetic acid salt as a gray solid.

**Example 17: Synthesis of Compound 24:**

**Synthesis of 5'-methoxy-6'-(3-(pyrrolidin-1-yl)prop-1-yn-1-yl)spiro[cyclobutane-1,3'-indol]-2'-amine:**

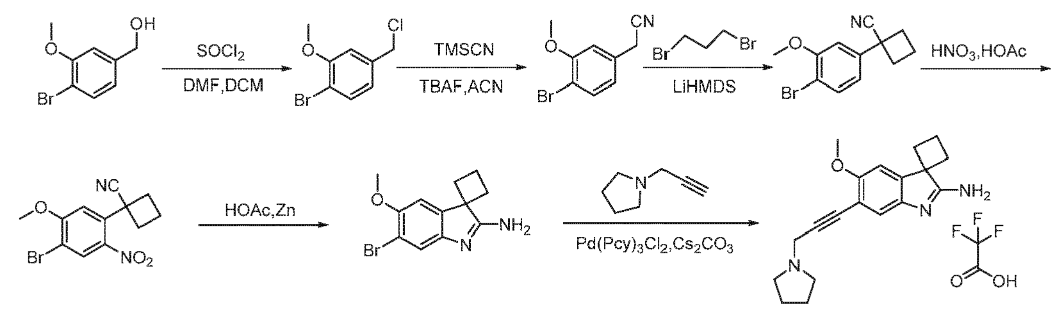

**Step 1:** Synthesis of 1-bromo-4-(chloromethyl)-2-methoxybenzene:

Into a 100-mL 3-necked round-bottom flask was placed (4-bromo-3-methoxyphenyl)methanol (1 g, 4.61 mmol, 1.00 equiv), dichloromethane (30 mL), thionyl chloride (1.64 g, 13.90 mmol, 3.00 equiv), and N,N-dimethylformamide (0.2 mL). The resulting solution was stirred for 12 h at 20 °C. The resulting mixture was washed with 3×10 mL of $H_2O$. The mixture was dried over anhydrous sodium sulfate and concentrated under vacuum. This resulted in 1 g (92%) of the title compound as a red crude oil.

Analytical Data: $^1$H NMR (300 MHz, DMSO-$d_6$) δ 7.57 (d, *J*= 8.1 Hz, 1H), 7.20 (d, *J* = 2.0 Hz, 1H), 6.97 (d, *J* = 8.1, 2.0 Hz, 1H), 4.74 (s, 2H), 3.85 (s, 3H).

**Step 2:** Synthesis of 2-(4-bromo-3-methoxyphenyl)acetonitrile:

Into a 100-mL round-bottom flask was placed 1-bromo-4-(chloromethyl)-2-methoxybenzene (1 g, 4.25 mmol, 1.00 equiv), ACN (30 mL), TMSCN (1.26 g, 12.73 mmol, 3.00 equiv), TBAF (2.22 g, 8.49 mmol, 2.00 equiv). The resulting solution was stirred for 5 h at 50 °C in an oil bath. The crude product was purified by Flash-Prep-HPLC with the following conditions (IntelFlash-1): Column, C18 silica gel; mobile phase,$H_2O$:ACN = 50%; Detector, UV 254 nm. This resulted in 850 mg (89%) of the title compound as a colorless oil.

Analytical Data: $^1$H NMR (300 MHz, DMSO-$d_6$) δ 7.59 (d, *J* = 8.1 Hz, 1H), 7.10 (d, *J* = 2.0 Hz, 1H), 6.94 - 6.84 (m, 1H), 4.02 (t, *J*= 0.7 Hz, 2H), 3.85 (s, 3H).

**Step 3:** Synthesis of 1-(4-bromo-3-methoxyphenyl)cyclobutane-1-carbonitrile:

Into a 100-mL 3-necked round-bottom flask was placed 2-(4-bromo-3-methoxyphenyl)acetonitrile (610 mg, 2.70 mmol, 1.00 equiv), and tetrahydrofuran (20 mL). This was followed by the addition of LiHDMS (8.1 mL, 3.00 equiv) dropwise with stirring at 0 °C, 1mol/L. To this was added 1,3-dibromopropane (657 mg, 3.25 mmol, 1.20 equiv) dropwise with stirring at 0 °C. The resulting solution was stirred for 4 h at 0 °C in an ice/salt bath. The reaction was then quenched by the addition of 10 mL of water. The resulting solution was extracted with 3x50 mL of ethyl acetate and the organic layers combined. The residue was applied onto a silica gel column with ethyl acetate/petroleum ether (5%). This resulted in 320 mg (45%) of the title compound as a colorless oil.

Analytical Data: $^1$H NMR (300 MHz, Methanol-$d_4$) δ 7.58 (d, *J* = 8.2 Hz, 1H), 7.06 (d, *J* = 2.2 Hz, 1H), 6.97 - 6.95 (m, 1H), 3.93 (s, 3H), 2.87 - 2.60 (m, 4H), 2.52 - 2.30 (m, 1H), 2.27 - 2.00 (m, 1H).

**Step 4:** Synthesis of 1-(4-bromo-5-methoxy-2-nitrophenyl)cyclobutane-1-carbonitrile:

Into a 50-mL round-bottom flask was placed 1-(4-bromo-3-methoxyphenyl)cyclobutane-1-carbonitrile (320 mg, 1.20 mmol, 1.00 equiv), acetyl acetate (3 mL), acetic acid (3 mL), HNO3 (351 mg, 5.57 mmol, 3.00 equiv). The resulting solution was stirred for 12 h at 60 °C in an oil bath. The crude product was purified by Flash-Prep-HPLC with the following conditions (IntelFlash-1): Column,C18 silica gel; mobile phase,$H_2O$:ACN = 55%; Detector, UV 254 nm. This resulted in 160 mg (43%) of the title compound as a light yellow solid.

Analytical Data: $^1$H NMR (300 MHz, DMSO-$d_6$) δ 8.35 (s, 1H), 7.14 (s, 1H), 4.07 (s, 3H), 2.91 - 2.56 (m, 4H), 2.43 - 2.17 (m, 1H), 1.94 - 1.88 (m, 1H).

**Step 5:** Synthesis of 6'-bromo-5'-methoxyspiro[cyclobutane-1,3'-indole]-2'-amine:

Into a 50-mL round-bottom flask was placed 1-(4-bromo-5-methoxy-2-nitrophenyl)cyclobutane-1-carbonitrile (160 mg, 0.51 mmol, 1.00 equiv), acetic acid (5 mL), and Zn (167.7 mg, 2.58 mmol, 5.00 equiv). The resulting solution was stirred for 12 h at 80 °C in an oil bath. The solids were filtered out. The crude product was purified by Flash-Prep-HPLC with the following conditions (IntelFlash-1): Column, silica gel C18; mobile phase, $H_2O$: ACN = 20% ; Detector, UV 254 nm. This resulted in 150 mg (104%) of the title compound as a brown solid.

Analytical Data: LC-MS: (ES, *m/z):* RT = 0.961 min, m/z = 281 [M+1].

**Step 6:** Synthesis of 5'-methoxy-6'-(3-(pyrrolidin-1-yl)prop-1-yn-1-yl)spiro[cyclobutane-1,3'-indol]-2'-amine:

Into a 50-mL round-bottom flask purged and maintained with an inert atmosphere of nitrogen, was placed 6'-bromo-5'-methoxyspiro[cyclobutane-1,3'-indole]-2'-amine (140 mg, 0.50 mmol, 1.00 equiv), 1-(prop-2-yn-1-yl)pyrrolidine hydrochloride (87 mg, 0.60 mmol, 1.10 equiv), $Pd(PCy)_3Cl_2$ (73.8 mg, 0.10 mmol, 0.20 equiv), $Cs_2CO_3$ (489 mg, 1.50 mmol, 3.00 equiv), and DMSO (5 mL). The resulting solution was stirred for 12 h at 100 °C in an oil bath. The solids were filtered out. The crude product was purified by Prep-HPLC with the following conditions (2#-AnalyseHPLC-SHIMADZU (HPLC-10)): Column, XBridge Prep C18 OBD Column, 19x150mm 5 μm; mobile phase, Water(10 mmol/L $NH_4HCO_3$)and ACN (20.0% ACN up to 35.0% in 10 min); Detector, UV 254/220nm. This resulted in 23.1 mg (15%) of the title compound as the trifluoroacetic acid salt as an off-white solid.

**Example 18: Synthesis of Compound 75:**

**Synthesis of 5'-methoxy-6'-(3-(pyrrolidin-1-yl)prop-1-yn-1-yl)spiro[cyclobutane-1,3'-indol]-2'-amine:**

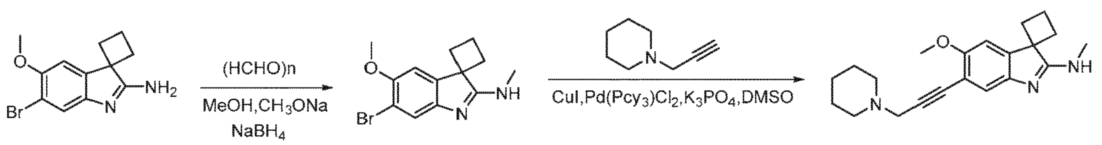


**Step 1:** Synthesis of 6'-bromo-5'-methoxy-N-methylspiro[cyclobutane-1,3'-indol]-2'-amine

Into a 50-mL round-bottom flask, was placed a solution of 6-bromo-5-methoxy-5-dihydrospiro[cyclobutane-1,3-indole]-2-amine (200 mg, 0.71 mmol, 1.00 equiv) in methanol (10 mL), sodium methylate/MeOH (643 mg, 5.00 equiv), (HCHO)n (107 mg, 5.00 equiv) and stirred for 5 h at 70 °C. Then $NaBH_4$ (271 mg, 7.16 mmol, 10.00 equiv.) was added. The resulting solution was stirred for 2 days at 70 °C. The resulting mixture was concentrated under vacuum. The residue was applied onto a silica gel column with ACN/$H_2O$ (0.05% $NH_4HCO_3$) (1/1). This resulted in 115 mg (55%) of the target compound as a yellow solid.

Analytical Data: LC-MS: (ES, *m*/*z*): RT = 0.964 min; *m*/*z* = 295 [M+1].

**Step 2:** Synthesis of 5'-methoxy-N-methyl-6'-(3-(piperidin-1-yl)prop-1-yn-1-yl)spiro[cyclobutane-1,3'-indol]-2'-amine

Into a 20-mL round-bottom flask, was placed 6-Bromo-5-Methoxy-N-methylspiro[cyclobutane-1,3-Indole]-2-amine (100 mg, 0.34 mmol, 1.00 equiv), 1-(prop-2-yn-1-yl)piperidine (50 mg, 0.41 mmol, 1.20 equiv.), CuI (10 mg, 0.05 mmol, 0.15 equiv), Pd($PCy_3$)$Cl_2$ (50 mg), $K_3PO_4$ (330 mg, 1.55 mmol, 4.59 equiv.), DMSO (8 mL). The resulting solution was stirred for 1 overnight at 100 °C. The crude product was purified by Prep-HPLC with the following conditions: Kinetex EVO C18 Column, 21.2 × 150.5 um; mobile phase, water (10 mM NH4HCO3)and ACN (10.0% ACN up to 27.0% in 7 min); Detector, UV 254/220nm. This resulted in 36.9 mg (24%) of the title compounds as the trifluoroacetic acid as an off-white solid.

Other compounds were synthesized in the similar manner and the characterization data are listed in Table 2 below.

**Table 2**

| Cpd No. | Data |
|---|---|
| 1 | LC-MS: (ES, m/z): RT = 0.88 min; m/z = 291.10 [M+1].$^1$H-NMR: (Methanol-*d*$_4$, *ppm):* δ 7.06 (s, 2H), 4.18 (t, *J* = 5.5 Hz, 2H), 3.91 (s, 3H), 3.88 - 3.75 (m, 2H), 3.59 - 3.48 (m, 2H), 3.37-3.06 (m, 2H), 2.46 - 2.05 (m, 4H), 2.30 - 2.05 (m, 2H). |
| 2 | LC-MS: (ES, *m/z):* RT = 0.88 min; m/z = 305.20 [M+1].$^1$H NMR (300 MHz, Methanol-*d*$_4$) δ 7.21 (s, 1H), 7.09 (s, 1H), 4.19 (t, *J* = 5.6 Hz, 2H), 3.96 (s, 3H), 3.82 - 3.80 (m, 2H), 3.67 (s, 3H), 3.49 (t, *J* = 7.2 Hz, 2H), 3.17 - 3.15 (m, 2H), 2.25-2.01 (m, 6H). |
| 3 | LC-MS: (ES, *m*/*z*): RT = 1.08 min; m/z = 359 [M+1].$^1$H-NMR: (Methanol-*d*$_4$, *ppm)*: δ 7.12 (s, 1H), 7.06 (s, 1H), 4.86 (s, 1H), 4.21 (t, *J* = 5.5 Hz, 2H), 3.95 (s, 3H), 3.91 - 3.78 (m, 2H), 3.50 (t, *J* = 7.2 Hz, 2H), 3.25 - 3.10 (m, 2H), 2.45 - 2.03 (m, 12H), 1.97 - 1.78 (m, 2H). |
| 5 | LC-MS: (ES, *m*/*z*): RT = 0.880 min; m/z = 360.3 [M+1].$^1$H-NMR: ($CDCl_3$, *ppm)*: $^1$H NMR (300 MHz, Methanol-d4) δ 7.40 (s, 1H), 6.78 (s, 1H), 4.33 - 3.94 (m, 6H), 3.85 (s, 3H), 2.88 - 2.62 (m, 6H), 2.29 - 1.98 (m, 4H), 1.95 - 1.77 (m, 4H), 1.51 - 1.26 (m, 2H). |
| 6 | LC-MS: (ES, *m/z):* RT = 1.34 min, *m*/*z* = 308 [M+1].$^1$H-NMR (300 MHz, Methanol-*d*$_4$) δ 7.23 (s, 1H), 7.03 (s, 1H), 4.09 (t, *J* = 6.1 Hz, 2H), 3.85 (s, 3H), 2.85-2.75 (m, 3H), 2.70-2.60(m, 4H), 2.10-2.00 (m, 2H), 1.93 - 1.82 (m, 4H). |

(continued)

| Cpd No. | Data |
|---|---|
| 7 | LC-MS: (ES, *m/z)*: RT=0.901 min, *m/z* =346.2 [M+1]. $^1$H NMR (400 MHz, Methanol-$d_4$) δ 7.19 (s, 1H), 6.93 (s, 1H), 4.38 - 4.23 (m, 2H), 4.19 (t, *J* = 5.6 Hz, 2H), 4.12 (d, *J* = 9.2 Hz, 1H), 3.99 (d, *J* = 9.2 Hz, 1H), 3.90 (s, 3H), 3.85 - 3.77 (m, 2H), 3.48 (t, *J* = 7.1 Hz, 2H), 3.21 - 3.12 (m, 2H), 2.59 - 2.47 (m, 2H), 2.34 - 2.26 (m, 2H), 2.26 - 2.16 (m, 2H), 2.14 - 2.02 (m, 3H). |
| 8 | LC-MS: (ES, *m/z)*: RT = 0.826 min, *m/z* = 319 [M+1].$^1$H NMR (300 MHz, Methanol-$d_4$) δ 7.22 (s, 1H), 7.09 (s, 1H), 4.20 (t, *J*= 5.5 Hz, 2H), 4.02 - 3.93 (s, 3H),3.89 - 3.76 (m, 2H), 3.64 (s, 3H), 3.50 (t, *J* = 7.1 Hz, 2H), 3.21-3.06 (m, 5H), 2.37-2.01 (m, 6H). |
| 9 | LC-MS: (ES, *m/z):* RT=1.031 min, *m/z* = 359 [M+1].$^1$H NMR (300 MHz, Methanol-$d_4$) δ 7.12 (d, *J* = 3.9 Hz, 1H), 7.04 (d, *J* = 1.3 Hz, 1H), 4.82 (q, *J* = 8.7 Hz, 1H), 4.20 - 3.64 (m, 7H), 3.44 - 2.85 (m, 5H), 2.51 - 1.75 (m, 10H), 1.38 (t, *J* = 7.3 Hz, 3H). |
| 10 | LC-MS: (ES, *m/z)*: RT=1.021 min, *m/z* =345 [M+1].$^1$H NMR (300 MHz, Methanol-$d_4$) δ 7.17 (d, *J*= 20.9 Hz, 1H), 7.06 (s, 1H), 4.94 - 4.76 (m, 1H), 4.53 - 4.32 (m, 2H), 4.26 - 3.90 (m, 7H), 3.52 - 3.32 (m, 2H), 3.30 - 3.19 (m, 1H), 2.33 - 1.99 (m, 6H), 1.96 - 1.76 (m, 2H), 1.32 - 1.16 (m, 3H). |
| 11 | LC-MS: (ES, *m/z):* RT= 1.05 min, *m/z* = 373 [M+1].$^1$H-NMR: (Methanol-$d_4$, ppm): δ 7.33 (s, 1H), 7.11 (s, 1H), 5.23 - 5.04 (m, 2H), 4.21 (t, *J* = 5.5 Hz, 2H), 3.94 (s, 3H), 3.89 - 3.76 (m, 2H), 3.49 (t, *J* = 7.1 Hz, 2H), 3.24 - 3.09 (m, 2H), 2.37 - 2.00 (m, 6H). |
| 12 | LC-MS: (ES, *m/z*): RT = 2.30 min, *m/z* = 387 [M+1].$^1$H NMR (400 MHz, Methanol-$d_4$) δ 7.28 (s, 1H), 7.11 (s, 1H), 4.32 - 4.18 (m, 4H), 3.98 (s, 3H), 3.85 - 3.75 (m, 2H), 3.74 (s, 3H), 3.50 (t, *J*= 7.2 Hz, 2H), 3.23 - 3.12 (m, 2H), 2.35 - 1.98(m, 6H). |
| 13 | LC-MS: (ES, *m/z*): RT= 1.04 min, *m/z* = 389 [M+1].$^1$H -NMR: (Methanol-$d_4$, ppm): δ 7.26 (s, 1H), 7.11 (s, 1H), 4.67 - 4.53 (m, 1H), 4.22 - 4.15 (m, 4H), 3.98 (s, 3H), 3.89 - 3.76 (m, 2H), 3.70 - 3.44 (m, 4H), 3.21 - 3.12(m, 5H), 2.50 -2.42 (m, 2H), 2.3 8 - 2.01 (m, 6H), 1.96 - 1.83 (m, 2H). |
| 18 | LC-MS: (ES, m/z): RT= 0.97 min, m/z = 389 [M+1].$^1$H-NMR: (Methanol-d4, ppm): δ 7.25 (s, 1H), 7.09 (s, 1H), 4.19 (t, J = 5.5 Hz, 2H), 4.10 - 3.90 (m, 7H), 3.89 - 3.80 (m, 2H), 3.55 - 3.28 (m, 4H), 3.24 - 3.09 (m, 2H), 2.37 - 2.02 (m, 7H), 1.60- 1.42 (m, 4H). |
| 19 | LC-MS: (ES, m/z): RT= 0.99 min, m/z = 333 [M+1].$^1$H-NMR: (Methanol-d4, ppm): δ 7.23 (s, 1H), 7.10 (s, 1H), 4.84 - 4.72 (m, 1H), 4.19 (t, J = 5.5 Hz, 2H), 3.95 (s, 3H), 3.89 - 3.80 (m, 2H), 3.49 (t, J = 7.1 Hz, 2H), 3.24 - 3.09 (m, 2H), 2.37 - 2.00 (m, 6H), 1.67 (d, J = 6.9 Hz, 6H). |
| 21 | LC-MS: (ES, *m/z*): RT = 1.764 min, *m/z* = 304 [M+1].$^1$H NMR (400 MHz, Methanol-$d_4$) δ 7.09 (d, *J* = 27.2 Hz, 2H), 4.09 (t, *J* = 6.2 Hz, 2H), 3.92 (s, 3H), 3.75 (s, 3H), 2.81 - 2.73 (m, 2H), 2.71 - 2.62 (m, 4H), 2.55 (s, 3H), 2.13 - 2.01 (m, 2H), 1.92- 1.80 (m, 4H). |
| 22 | LC-MS: (ES, *m/z):* RT = 1.036 min, *m/z* = 358 [M+1].$^1$H NMR (300 MHz, Methanol-$d_4$) δ 7.46 (s, 1H), 7.25 (s, 1H), 4.26 (t, *J* = 5.5 Hz, 2H), 4.02 (d, *J* = 2.4 Hz, 6H), 3.90 - 3.65 (m, 3H), 3.50 (t, *J* = 7.2 Hz, 2H), 3.27 - 3.08 (m 2H), 2.46 - 2.16 (m, 6H), 2.15 - 1.79 (m, 8H). |
| 23 | LC-MS: (ES, *m/z*): RT =1.655 min, *m/z* = 358[M+1]. $^1$H -NMR: $^1$H NMR (400 MHz, Methanol-$d_4$) δ 7.28 (d, *J*= 19.2 Hz, 2H), 5.31 - 5.12 (m, 1H), 4.26 (t, *J* = 5.6 Hz, 2H), 4.01 (s, 3H), 3.83 - 3.75 (m, 2H), 3.50 (t, *J* = 7.2 Hz, 2H), 3.22 - 3.11 (m, 2H), 2.87 (s, 3H), 2.41 - 2.03 (m, 12H), 1.98 - 1.79 (m, 2H). |
| 24 | LC-MS: (ES, *m/z*): RT = 0.917 min, *m/z* = 310.2 [M+1].$^1$H NMR (300 MHz, Methanol-$d_4$) δ 7.53 (s, 1H), 7.17 (s, 1H), 4.40 (s, 2H), 4.00 (s, 3H), 3.98 - 3.54 (m, 2H), 3.32 - 3.30 (m, 2H), 2.89 - 2.75 (m, 2H), 2.72 - 2.56 (m, 3H), 2.41 - 2.25 (m, 1H), 2.25 - 2.05(m, 4H). |
| 25 | LC-MS: (ES, m/z): RT= 1.26 min, m/z = 321 [M+1].$^1$H-NMR: (Methanol-d4, ppm): δ 7.22 (s, 1H), 7.12 (s, 1H), 4.37 - 4.14 (m, 5H), 4.12 - 3.94 (m, 5H), 3.64 (s, 3H), 3.52 (d, J = 3.3 Hz, 1H), 3.41 - 3.24 (m, 1H), 3.14 (s, 3H), 2.60 - 2.54 (m, 1H), 2.56 - 2.41 (m, 1H). |
| 26 | LC-MS: (ES, m/z): RT= 0.85 min, m/z = 335 [M+1].$^1$H-NMR: (Methanol-d4, ppm):δ 7.21 (s, 1H), 7.13 (s, 1H), 4.45 -4.28 (m, 1H), 4.15 - 3.99 (m, 2H), 3.96 (s, 3H), 3.77 (td, J = 8.8, 7.0, 3.4 Hz, 2H), 3.64 (s, 3H), 3.53 - 3.39 (m, 2H), 3.18 - 3.02 (m, 5H), 2.25 - 2.03 (m, 4H). |

(continued)

| Cpd No. | Data |
|---|---|
| 27 | LC-MS: (ES, m/z): RT=0.952 min, m/z =333.2 [M+1].1H NMR (400 MHz, Methanol-d4) δ 7.22 (s, 1H), 7.08 (s, 1H), 4.20 (t, J = 5.5 Hz, 2H), 3.96 (s, 3H), 3.86 - 3.79 (m, 2H), 3.66 (s, 3H), 3.55 - 3.47 (m, 5H), 3.21 - 3.12 (m, 2H), 2.33 - 2.19 (m, 4H), 2.14 - 2.04 (m, 2H), 1.40 (t, J = 7.2 Hz, 3H). |
| 28 | LC-MS: (ES, m/z): RT=1.670 min, m/z =347.2 [M+1].1H NMR (400 MHz, Methanol-d4) δ 7.21 (d, J = 2.0 Hz, 1H), 7.08 (s, 1H), 4.20 (t, J = 5.5 Hz, 2H), 3.96 (s, 3H), 3.94 - 3.90 (m, 1H), 3.86 - 3.79 (m, 2H), 3.66 (s, 3H), 3.50 (t, J = 7.1 Hz, 2H), 3.22 - 3.11 (m, 2H), 2.34 - 2.17 (m, 4H), 2.14 - 2.05 (m, 2H), 1.41 (d, J = 6.4 Hz, 6H). |
| 29 | LC-MS: (ES, m/z): RT=0.972 min, m/z =389.2 [M+1].1H NMR (400 MHz, Methanol-d4) δ 7.23 (s, 1H), 7.10 (s, 1H), 4.20 (t, J = 5.5 Hz, 2H), 4.11 - 4.02 (m, 2H), 3.96 (s, 3H), 3.86 - 3.77 (m, 3H), 3.68 (s, 3H), 3.61 - 3.46 (m, 4H), 3.22 - 3.12 (m, 2H), 2.33 - 2.16 (m, 4H), 2.15 - 2.00 (m, 4H), 1.85 - 1.73 (m, 2H). |
| 30 | LC-MS: (ES, m/z): RT=1.079 min, m/z =373.2 [M+1].1H NMR (300 MHz, Methanol-d4) δ 7.21 (s, 1H), 7.08 (d, J = 1.5 Hz, 1H), 4.20 (t, J = 5.5 Hz, 2H), 4.11 - 4.00 (m, 1H), 3.96 (s, 3H), 3.87 - 3.78 (m, 2H), 3.67 (s, 3H), 3.50 (t, J = 7.1 Hz, 2H), 3.23 - 3.10 (m, 2H), 2.34-2.03 (m, 8H), 1.87 (m, 2H), 1.81 - 1.65 (m, 4H). |
| 31 | LC-MS: (ES, m/z): RT=0.919 min, m/z=349 [M+1].1H NMR (300 MHz, Methanol-d4) δ 6.98 (s, 1H), 6.87 (s, 1H), 4.19 - 4.05 (m, 1H), 4.05 - 3.85 (m, 5H), 3.52 - 3.37 (m, 5H), 2.87 - 2.75 (m, 1H), 2.72 - 2.58 (m, 5H), 1.90 - 1.77 (m, 4H), 1.29 (t, J = 7.2 Hz, 3H). |
| 32 | LC-MS: (ES, m/z): RT=0.960 min, m/z=363 [M+1].1H NMR (300 MHz, Methanol-d4) δ 6.98 (s, 1H), 6.87 (s, 1H), 4.20 - 3.85 (m, 7H), 3.48 (s, 3H), 2.84 (dd, J = 12.7, 4.0 Hz, 1H), 2.75 - 2.60 (m, 5H), 1.83 (p, J = 3.3 Hz, 4H), 1.30 (d, J = 6.5 Hz, 6H). |
| 33 | LC-MS: (ES, m/z): RT=1.039 min, m/z=389 [M+1].1H NMR (300 MHz, Methanol-d4) δ 6.99 (s, 1H), 6.87 (s, 1H), 4.26 - 4.05 (m, 2H), 4.05 - 3.85 (m, 5H), 3.49 (s, 3H), 2.83 (dd, J = 12.7, 4.0 Hz, 1H), 2.74 - 2.60 (m, 5H), 2.17 - 2.02 (m, 2H), 1.90 - 1.51 (m, 10H). |
| 34 | LC-MS: (ES, m/z): RT=0.995 min, m/z=375 [M+1].1H NMR (300 MHz, Methanol-d4) δ 6.98 (s, 1H), 6.88 (s, 1H), 4.19 - 4.05 (m, 1H), 4.05 - 3.86 (m, 5H), 3.51 (s, 3H), 3.26 (d, J = 6.9 Hz, 2H), 2.84 (dd, J = 12.7, 4.1 Hz, 1H), 2.77 - 2.61 (m, 5H), 1.89 - 1.75 (m, 4H), 1.27 - 1.13 (m, 1H), 0.61 - 0.48 (m, 2H), 0.36 - 0.25 (m, 2H). |
| 35 | LC-MS: (ES, m/z): RT=0.933 min, m/z=405 [M+1].1H NMR (300 MHz, Methanol-d4) δ 6.98 (s, 1H), 6.89 (s, 1H), 4.20 - 3.82 (m, 9H), 3.64 - 3.47 (m, 5H), 2.84 (dd, J = 12.7, 4.1 Hz, 1H), 2.75 - 2.61 (m, 5H), 2.11 - 1.99 (m, 2H), 1.89- 1.77 (m, 4H), 1.74- 1.54 (m, 2H). |
| 36 | LC-MS: (ES, m/z): RT = 0.964 min, m/z = 347 [M+1].1H NMR (400 MHz, Methanol-d4) δ 7.23 (s, 1H), 7.10 (s, 1H), 4.74 - 4.62 (m, 1H), 4.20 (t, J = 5.5 Hz, 2H), 3.96 (s, 3H), 3.87 - 3.77 (m, 2H), 3.50 (t, J = 7.2 Hz, 2H), 3.22 - 3.14 (m, 2H), 3.13 (s, 3H), 2.36 - 2.00 (m, 6H), 1.66 (d, J = 6.9 Hz, 6H). |
| 37 | LC-MS: (ES, m/z): RT = 4.695 min; m/z = 318 [M+1].1H NMR (300 MHz, Methanol-d4) δ 7.54 (s, 1H), 7.28 (s, 1H), 4.42 (s, 2H), 4.04 (s, 3H), 3.76 - 3.39 (m, 11H), 3.17 (s, 3H), 2.14 (q, J = 7.0 Hz, 4H). |
| 39 | LC-MS: (ES, m/z): RT=0.649 min, m/z =347.3 [M+1].1H NMR (400 MHz, Methanol-d4) δ 7.23 (s, 1H), 7.09 (s, 1H), 4.25 - 4.14 (m, 4H), 3.96 (s, 3H), 3.87 - 3.78 (m, 2H), 3.56 - 3.46 (m, 4H), 3.23 - 3.12 (m, 2H), 2.34 - 2.17 (m, 4H), 2.14 - 2.04 (m, 2H), 1.39 (t, 6H). |
| 40 | LC-MS: (ES, *m/z*): RT = 1.402 min; m/z = 346 [M+1].1H NMR (300 MHz, Methanol-d4) δ 7.65 (s, 1H), 7.29 (s, 1H), 4.76 - 4.73(m, 1H), 4.44 (s, 2H), 4.06 (s, 3H), 3.80 (s, 2H), 3.69 (d, J = 6.0 Hz, 2H), 3.67 - 3.56 (m, 2H), 3.16 (s, 5H), 2.22 (s, 2H), 2.14 - 2.06 (m, 2H), 1.69 (d, J = 3.3 Hz, 6H). |
| 41 | LC-MS: (ES, m/z): RT = 0.804 min; m/z = 332 [M+1].1H NMR (300 MHz, Methanol-d4) δ 7.53 (s, 1H), 7.32 (s, 1H), 4.41 (s, 2H), 4.22 - 4.18 (m, 2H), 4.04 (s, 3H), 3.77 - 3.53 (m, 5H), 3.46 (s, 3H), 3.16 (s, 3H), 2.15 (d, J = 6.0 Hz, 4H), 1.41-1.38 (m, 3H). |
| 42 | LC-MS: (ES, m/z): RT = 0.965 min; m/z = 333 [M+1].1H NMR (300 MHz, Methanol-d4) δ 7.23 (s, 1H), 7.10 (s, 1H), 4.26 - 4.10 (m, 4H), 3.96 (s, 3H), 3.89 - 3.76 (m, 2H), 3.50 (t, J = 7.2 Hz, 2H), 3.29 - 3.01 (m, 5H), 2.47 - 2.23 (m,4H), 2.22 - 2.02 (m, 2H), 1.39 (t, J = 7.2 Hz, 3H). |

(continued)

| Cpd No. | Data |
|---|---|
| 75 | LC-MS: (ES, *m/z):* RT = 0.935 min, m/z = 338.0 [M+1].1H NMR (400 MHz, Methanol-d4) δ 7.55 (s, 1H), 7.20 (s, 1H), 4.32 (s, 2H), 4.01 (s, 3H), 3.74 (d, J = 12.2 Hz, 2H), 3.21 (s, 3H), 3.13 (t, J = 12.5 Hz, 2H), 2.84 - 2.76 (m, 2H), 2.71 - 2.56 (m, 3H), 2.40 - 2.29 (m, 1H), 2.05 (d, J = 14.6 Hz, 2H), 1.96 - 1.75 (m, 3H), 1.65 - 1.50 (m, 1H). |

**Example 19: HPLC Methods for Compound Purification**

Compounds and intermediates purified by HPLC used the methods described below:

**Method A. Column: IntelFlash-1, C18 silica gel; Detector, UV 254 nm**

A. Mobile phase, $H_2O$/CAN
A MeOH. Mobile phase, methanol
A Grad. (IntelFlash-1): Mobile phase, $H_2O$/ACN=100/0 increasing to $H_2O$/ACN=30/70 within 30 min.
A 1:1. Mobile phase, ACN/H2O=1/1
A DCM/MeOH. Mobile phase, DCM/MeOH
A EA/PE. Mobile phase, EA/PE

**Method B. Column, XBridge Prep C18 OBD Column, 30x100mm,5 μm; Detector, UV 254 nm**

B HCl. Mobile phase, Water (0.05% HCl) and ACN (Gradient)
B TFA. Mobile phase, Water (0.05% TFA) and ACN (Gradient)
B NH4HCO3. Mobile Phase, Water with 10 mmol $NH_4HCO_3$ and ACN (Gradient)

**Method B. Column, XBridge Prep C18 OBD Column, 30x100mm,5 μm; Detector, UV 254 nm**

C HCl. Mobile phase, Water (0.05% HCl) and ACN (Gradient)
C TFA. Mobile phase, Water (0.1% TFA) and CAN (Gradient)
C NH3. Mobile phase, Water (0.05% NH3-H2O) and ACN (Gradient)
C NH4HCO3. Mobile Phase, Water with 10 mmol NH4HCO3 and ACN (Gradient)

**Method D. Column, XSelect CSH Prep C18 OBD Column, 19x250 mm, 5 um; Detector, uv 254 nm**

DHCl. Mobile phase, Water (0.05% HCl) and ACN (Gradient)
D TFA. Mobile phase, Water (0.06% TFA) and ACN (Gradient); Detector 254 nm.
D NH3. Mobile phase, Water (0.05% $NH_3$-$H_2O$) and ACN (20.0% ACN up to 60.0% in 7 min); Detector, UV 254 nm
D NH4HCO3. Mobile Phase, Water with 10 mmol $NH_4HCO_3$ and CAN (Gradient)

**Method E. Column: X Select C18, 19×150 mm, 5 um; Mobile Phase A: Water/0.05% HCl, Mobile Phase B: ACN; Detector 254 nm.**
**Method F. Column: X Bridge RP, 19×150 mm, 5 um; Detector 254 nm.**

F HCl. Mobile phase Water (0.05% HCl) and ACN (Gradient)
F TFA. Mobile phase Water (0.05% TFA) and ACN (Gradient)
F NH3. Mobile phase, Water (0.05% $NH_3$-$H_2O$) and ACN (20.0% ACN up to 60.0% in 7 min); Detector, UV 254 nm

**Method G. Column: GeminisoNX C18 AXAI Packed, 21.2×150 mm 5 um; Detector, UV 254 nm.**

G HCl Mobile phase, Water (0.05% HCl) and ACN (3.0% ACN up to 10.0% in 10 min)
G NH4HCO3. Mobile Phase, Water with 10 mmol $NH_4HCO_3$ and ACN (Gradient)

**Method H. Column: Sunfire Prep C18 OBD Column, 10 um, 19x250 mm; Mobile phase, Water (0.05% HCl) and methanol (3.0% methanol-up to 20.0% in 8 min); Detector, UV 254 nm.**
**Method I. Chiral IC. Column: Chiralpak IC, 2x25 cm, 5 um; Mobile phase, Hex0.1%DEA- and IPA- (hold 25.0%**

**IPA- in 21 min); Detector, UV 220/254 nm.**

**Method J. Chiral ID. Column: Chiralpak ID-2, 2x25 cm, 5 um; Mobile phase, Hex(0.1%DEA)- and ethanol- (hold 50.0% ethanol- in 14 min); Detector, UV 220/254 nm**

**Method K. Chiral IB4. Column: Chiralpak IB4.6x250, 5 um HPLC Chiral-A(IB)001IB00CE-LA026; Mobile phase, Hex (0.1%DEA):EtOH=50:50; Detector, 254 nm**

**Method L. Chiral IF. Column: CHIRALPAK IF, 2x25 cm, 5 um; Mobile phase, Hex(0.2%DEA)- and IPA- (hold 30.0% IPA- in 22 min); Detector, UV 220/254 nm**

**Example 20: Bioactivity Assays**

MATERIALS AND EQUIPMENT:

Recombinant purified human EHMT2 913-1193 (55 μM) synthesized by Viva was used for all experiments. Biotinylated histone peptides were synthesized by Biopeptide and HPLC-purified to > 95% purity. Streptavidin Flashplates and seals were purchased from PerkinElmer and 384 Well V-bottom Polypropylene Plates were from Greiner. $^3$H-labeled *S*-adenosylmethionine ($^3$H-SAM) was obtained from American Radiolabeled Chemicals with a specific activity of 80 Ci/mmol. Unlabeled SAM and *S*-adenosylhomocysteine (SAH) were obtained from American Radiolabeled Chemicals and Sigma-Aldrich respectively. Flashplates were washed in a Biotek ELx-405 with 0.1% Tween. 384-well Flashplates and 96-well filter binding plates were read on a TopCount microplate reader (PerkinElmer). Compound serial dilutions were performed on a Freedom EVO (Tecan) and spotted into assay plates using a Thermo Scientific Matrix PlateMate (Thermo Scientific). Reagent cocktails were added by Multidrop Combi (Thermo Scientific).

MDA-MB-231 cell line was purchased from ATCC (Manassas, VA, USA). RPMI/Glutamax medium, Penicillin-Streptomycin, Heat Inactivated Fetal Bovine Serum, and D-PBS were purchased from Life Technologies (Grand Island, NY, USA). Odyssey blocking buffer, 800CW goat anti-mouse IgG (H+L) antibody, and Licor Odyssey Infrared Scanner were purchased from Licor Biosciences, Lincoln, NE, USA. H3K9me2 mouse monoclonal antibody (Cat #1220) was purchased from Abcam (Cambridge, MA, USA). 16% Paraformaldehyde was purchased from Electron Microscopy Sciences, Hatfield, PA, USA).MDA-MB-231 cells were maintained in complete growth medium (RPMI supplemented with 10% v/v heat inactivated fetal bovine serum) and cultured at 37 °C under 5% $CO_2$. UNC0638 was purchased from Sigma-Aldrich (St. Louis, MO, USA).

Various *In vitro* or *in vivo* biological assays are may be suitable for detecting the effect of the compounds of the present disclosure. These *in vitro* or *in vivo* biological assays can include, but are not limited to, enzymatic activity assays, electrophoretic mobility shift assays, reporter gene assays, *in vitro* cell viability assays, and the assays described herein.

**General Procedure for EHMT2 Enzyme Assay on Histone Peptide Substrate.** 10-point curves of test compounds were made on a Freedom EVO (Tecan) using serial 3-fold dilutions in DMSO, beginning at 2.5 mM (final top concentration of compound was 50 μM and the DMSO was 2%). A 1 μL aliquot of the inhibitor dilution series was spotted in a polypropylene 384-well V-bottom plate (Greiner) using a Thermo Scientific Matrix PlateMate (Thermo Scientific). The 100% inhibition control consisted of 1 mM final concentration of the product inhibitor S-adenosylhomocysteine (SAH, Sigma-Aldrich). Compounds were incubated for 30 minutes with 40 μL per well of 0.031 nM EHMT2 (recombinant purified human EHMT2 913-1193, Viva) in IX assay buffer (20 mM Bicine [pH 7.5], 0.002% Tween 20, 0.005% Bovine Skin Gelatin and 1 mM TCEP). 10 μL per well of substrate mix comprising assay buffer, $^3$H-SAM ($^3$H-labeled *S*-adenosylmethionine, American Radiolabeled Chemicals, specific activity of 80 Ci/mmol), unlabeled SAM (American Radiolabeled Chemicals), and peptide representing histone H3 residues 1-15 containing C-terminal biotin (appended to a C-terminal amide-capped lysine, synthesized by Biopeptide and HPLC-purified to greater than 95% purity) were added to initiate the reaction (both substrates were present in the final reaction mixture at their respective $K_m$ values, an assay format referred to as "balanced conditions"). Reactions were incubated for 60 minutes at room temperature and quenched with 10 μL per well of 400 μM unlabeled SAM, then transferred to a 384-well streptavidin Flashplate (PerkinElmer) and washed in a Biotek ELx-405 well washer with 0.1% Tween after 60 minutes. 384-well Flashplates were read on a TopCount microplate reader (PerkinElmer).

**General Procedure for MDA-MB-231 HEK9me2 in-cell Western Assay.** Compound (100 nL) was added directly to 384-well cell plate. MDA-MB-231 cells (ATCC) were seeded in assay medium (RPMI/Glutamax supplemented with 10% v/v heat inactivated fetal bovine serum and 1% Penicillin/Streptomycin, Life Technologies) at a concentration of 3,000 cells per well to a Poly-D-Lysine coated 384-well cell culture plate with 50 μL per well. Plates were incubated at 37°C, 5% $CO_2$ for 48 hours (BD Biosciences 356697). Plates were incubated at room temperature for 30 minutes and then incubated at 37°C, 5% $CO_2$ for additional 48 hours. After the incubation, 50 μL per well of 8% paraformaldehyde (Electron Microscopy Sciences) in PBS was added to the plates and incubated at room temperature for 20 minutes. Plates were transferred to a Biotek 406 plate washer and washed 2 times with 100 μL per well of wash buffer (IX PBS containing 0.3% Triton X-100 (v/v)). Next, 60 μL per well of Odyssey blocking buffer (Licor Biosciences) was added to

each plate and incubated for 1 hour at room temperature. Blocking buffer was removed and 20 μL of monoclonal primary antibody α-H3K9me2 (Abcam) diluted 1:800 in Odyssey buffer with 0.1% Tween 20 (v/v) were added and plates were incubated overnight (16 hours) at 4 °C. Plates were washed 5 times with 100 μL per well of wash buffer. Next 20 μL per well of secondary antibody was added (1:500 800CW donkey anti-mouse IgG (H+L) antibody (Licor Biosciences), 1:1000 DRAQ5 (Cell Signaling Technology) in Odyssey buffer with 0.1% Tween 20 (v/v)) and incubated for 1 hour at room temperature. The plates were washed 5 times with 100 μL per well wash buffer then 2 times with 100 μL per well of water. Plates were allowed to dry at room temperature then imaged on a Licor Odyssey Infrared Scanner (Licor Biosciences) which measured integrated intensity at 700 nm and 800 nm wavelengths. Both 700 and 800 channels were scanned.

**% Inhibition Calculation.** First, the ratio for each well was determined by:

$$\left(\frac{H3K9me2\ 800nm\ value}{DRAQ5\ 700nm\ value}\right).$$

Each plate included fourteen control wells of DMSO only treatment (Minimum Inhibition) as well as fourteen control wells (background wells) for maximum inhibition treated with control compound UNC0638 (Background wells).

The average of the ratio values for each well was calculated and used to determine the percent inhibition for each test well in the plate. Control compound was serially diluted three-fold in DMSO for a total of 10 test concentrations beginning at 1 μM. Percent inhibition was calculated as:

$$\text{Percent Inhibition} = 100-\left(\left(\frac{\text{(Individual Test Sample Ratio) - (Background Avg Ratio)}}{\text{(Minimum Inhibition Ratio) - (Background Average Ratio)}}\right) * 100\right)$$

$IC_{50}$ curves were generated using triplicate wells per concentration of compound. The $IC_{50}$ is the concentration of compound at which measured methylation is inhibited by 50% as interpolated from the dose response curves. $IC_{50}$ values were calculated using a non-linear regression (variable slope-four parameter fit model) with by the following formula:

$$\%\ inhibition = Bottom + \left(\frac{Top - Bottom}{(1 + (IC_{50}/[I])^n)}\right)$$

, where *Top* is fixed at 100% and *Bottom* is fixed to 0%, [I] = concentration of inhibitor, $IC_{50}$ = half maximal inhibitory concentration , and *n* = Hill Slope.

The $IC_{50}$ values are listed in Table 3 below ("A" means $IC_{50}$ <100 nM; "B" means $IC_{50}$ ranging between 100 nM and 1 μM; "C" means $IC_{50}$ ranging between >1 μM and 10 μM; "D" means $IC_{50}$ > 10 μM; "ND" means not determined).

**Table 3**

| Compound No. | EHMT2 PEP ($IC_{50}$ μM) | EHMT1 PEP ($IC_{50}$ μM) | EHMT2 ICW ($IC_{50}$ μM) |
|---|---|---|---|
| 1 | A | A | C |
| 2 | A | A | B |
| 3 | A | A | B |
| 5 | A | A | B |
| 6 | C | C | D |
| 7 | A | A | B |
| 8 | A | A | A |
| 9 | A | A | B |
| 10 | B | A | B |
| 11 | B | B | C |
| 12 | C | C | C |

(continued)

| Compound No. | EHMT2 PEP ($IC_{50}$ μM) | EHMT1 PEP ($IC_{50}$ μM) | EHMT2 ICW ($IC_{50}$ μM) |
|---|---|---|---|
| 13 | A | A | B |
| 18 | B | B | C |
| 19 | A | A | B |
| 21 | C | C | D |
| 22 | C | C | D |
| 23 | B | C | C |
| 24 | A | A | A |
| 25 | B | A | B |
| 26 | B | A | B |
| 27 | B | A | B |
| 28 | B | B | C |
| 29 | C | C | C |
| 30 | B | B | C |
| 31 | B | A | B |
| 32 | B | B | C |
| 33 | B | B | C |
| 34 | B | A | B |
| 35 | C | C | D |
| 36 | A | A | A |
| 37 | A | A | B |
| 38 | A | A | B |
| 39 | A | A | B |
| 40 | B | A | C |
| 41 | A | A | B |
| 42 | A | A | A |
| 43 | A | A | ND |
| 46 | B | B | ND |
| 47 | C | C | ND |
| 48 | B | B | ND |
| 49 | A | A | ND |
| 50 | A | A | ND |
| 51 | C | C | ND |
| 52 | A | B | ND |
| 53 | A | A | ND |
| 54 | B | B | ND |
| 55 | A | A | ND |
| 56 | A | A | ND |
| 57 | C | C | ND |

(continued)

| Compound No. | EHMT2 PEP ($IC_{50}$ $\mu$M) | EHMT1 PEP ($IC_{50}$ $\mu$M) | EHMT2 ICW ($IC_{50}$ $\mu$M) |
|---|---|---|---|
| 58 | C | C | ND |
| 59 | A | A | ND |
| 60 | C | C | ND |
| 61 | D | D | ND |
| 62 | A | A | ND |
| 65 | B | B | ND |
| 66 | C | C | ND |
| 67 | C | D | ND |
| 68 | C | C | ND |
| 69 | C | C | ND |
| 70 | A | A | B |
| 71 | A | A | C |
| 72 | A | A | C |
| 73 | A | A | B |
| 74 | A | A | A |
| 75 | A | A | A |
| 76 | C | C | C |
| 84 | A | A | B |
| 85 | A | A | A |
| 86 | B | B | B |
| 87 | A | A | ND |
| 89 | A | A | ND |
| 92 | A | A | B |
| 93 | A | A | ND |
| 94 | B | B | ND |
| 95 | B | A | ND |
| 99 | A | A | B |
| 100 | B | B | C |
| 135 | D | C | ND |
| 136 | D | C | ND |
| 137 | D | D | ND |
| 138 | C | B | C |
| 139 | C | B | C |

The invention can be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of equivalency of the claims are intended to be embraced therein.

The following are clauses describing embodiments of the invention. They are not claims.

1. A compound of Formula (I):

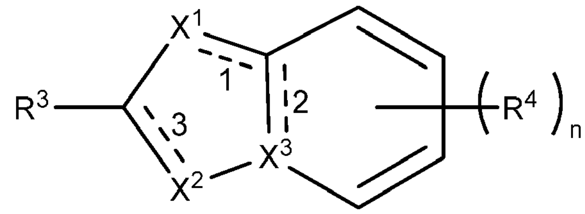

(I),

or a tautomer thereof, or a pharmaceutically acceptable salt of the compound or the tautomer, wherein

$X^1$ is O, S, $CR^1R^{11}$, or $NR^{1'}$ when $\overset{1}{\text{-----}}$ is a single bond, or $X^1$ is N when $\overset{1}{\text{-----}}$ is a double bond;

$X^2$ is N or $CR^2$ when $\overset{3}{\text{-----}}$ is a double bond, or $X^2$ is $NR^{2'}$ when $\overset{3}{\text{-----}}$ is a single bond;

$X^3$ is N or C; when $X^3$ is N, $\overset{1}{\text{-----}}$ is a double bond and $\overset{2}{\text{-----}}$ is a single bond, and when $X^3$ is C, $\overset{1}{\text{-----}}$ is a single bond and $\overset{2}{\text{-----}}$ is a double bond;

each of $R^1$, $R^2$ and $R^{11}$, independently, is $-Q^1-T^1$, in which each $Q^1$ independently is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and each $T^1$ independently is H, halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$, $-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, - $C(O)R^6$, $-SO_2R^5$, $-SO_2N(R^5)_2$, $-NR^5C(O)R^6$, amino, mono- or di- alkylamino, or $C_1$-$C_6$ alkoxyl; or

$R^1$ and $R^{11}$ together with the carbon atom to which they are attached form a $C_3$-$C_{12}$ cycloalkyl or 4- to 12-membered heterocycloalkyl containing 1-4 heteroatoms selected from N, O, and S, wherein the $C_3$-$C_{12}$ cycloalkyl or 4- to 12-membered heterocycloalkyl is optionally substituted with one or more of halo, $C_1$-$C_6$ alkyl, hydroxyl, oxo, amino, mono- or di- alkylamino, or $C_1$-$C_6$ alkoxyl;

each of $R^{1'}$ and $R^{2'}$, independently, is $-Q^2-T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S2}$ is optionally substituted with one or more of halo, $C_1$-$C_6$ alkyl, hydroxyl, oxo, $-C(O)R^6$, $-SO_2R^5$, $-SO_2N(R^5)_2$, $-NR^5C(O)R^6$, amino, mono- or dialkylamino, or $C_1$-$C_6$ alkoxyl;

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, C3-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or;

$R^3$ and one of $R^{1'}$, $R^{2'}$ , $R^1$, $R^2$ and $R^{11}$, together with the atoms to which they are attached, form a 5- or 6-membered heteroaryl that is optionally substituted with one or more of halo, $C_1$-$C_3$ alkyl, hydroxyl or $C_1$-$C_3$ alkoxyl; or

$R^3$ is oxo and $\overset{3}{\text{-----}}$ is a single bond;

each $R^4$ independently is $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di-alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl, or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted

with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl;

$R^8$ is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo; and

n is 1, 2, 3, or 4, and wherein the compound is not

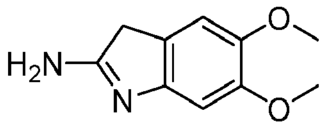,
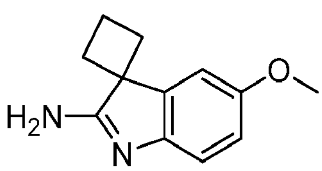,
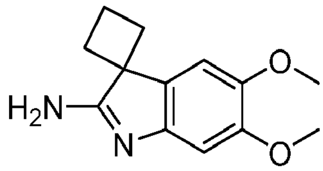,

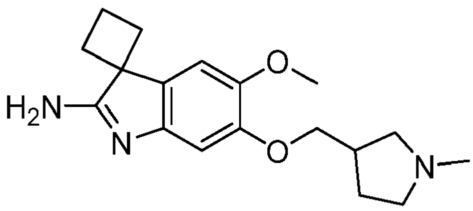,
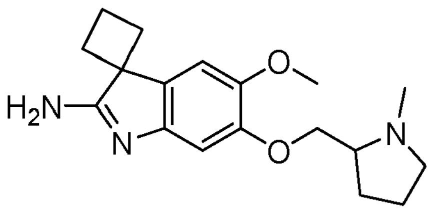,

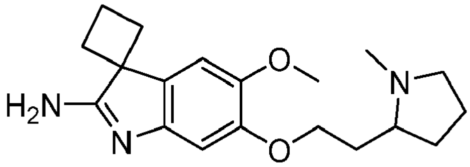,
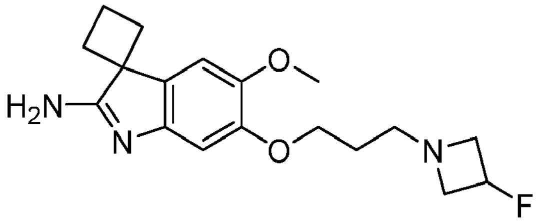,

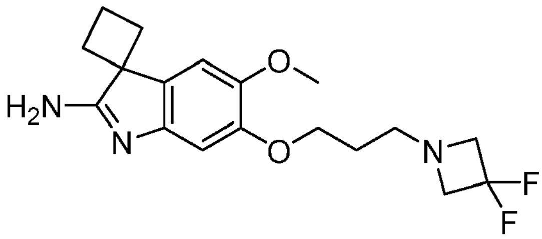,
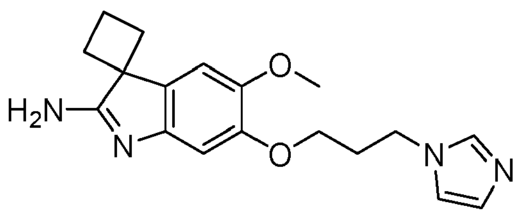,

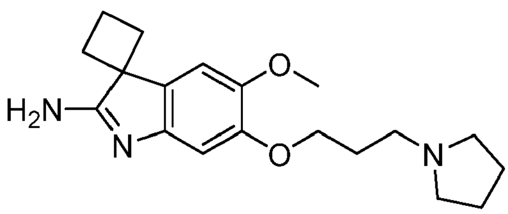,
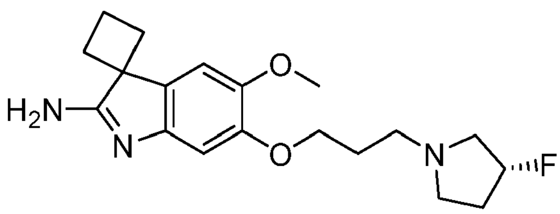,

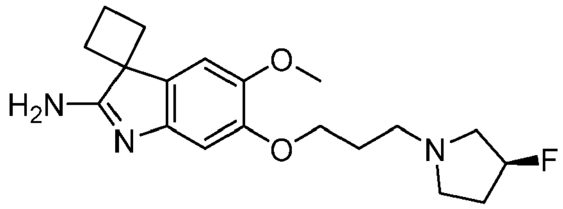

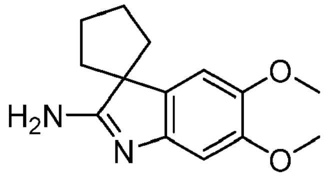

,

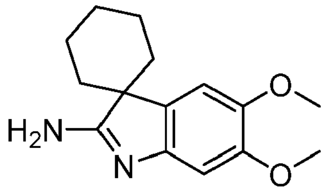

,

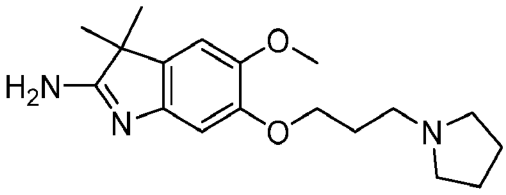

, 

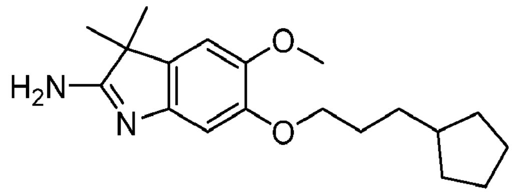

, or .

2. The compound of clause 1, wherein when n is 2, $X^1$ is $CR^1R^{11}$, $X^2$ is N , $X^3$ is C, $R^3$ is $NH_2$, and at least one $R^4$ is $OR^7$, then one of (1)-(4) below applies:

(1) at least one of $R^1$ and $R^{11}$ is $-Q^1-T^1$, in which $Q^1$ is a $C_1$-$C_6$ alkylene linker optionally substituted with one or more of halo, cyano, hydroxyl, or $C_1$-$C_6$ alkoxyl, and $T^1$ is cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$, $-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, $-SO_2N(R^5)_2$, - $NR^5C(O)R^6$, amino, mono- or di- alkylamino, or $C_1$-$C_6$ alkoxyl; or
(2) at least one of $R^1$ and $R^{11}$ is $-Q^1-T^1$, in which $Q^1$ is a $C_2$-$C_6$ alkenylene or $C_2$-$C_6$ alkynylene linker optionally substituted with one or more of halo, cyano, hydroxyl, or $C_1$-$C_6$ alkoxyl, and $T^1$ is H, halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$, $-NR^5C(O)OR^6$, $OR^5$, or $R^{S1}$, in which $R^{S1}$ is $C_3$-$C_{12}$ cycloalkyl, phenyl, 4-to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, $-SO_2N(R^5)_2$, $-NR^5C(O)R^6$, amino, mono- or di- alkylamino, or $C_1$-$C_6$ alkoxyl; or
(3) at least one of $R^1$ and $R^{11}$ is $-Q^1-T^1$, in which $Q^1$ is a bond, and $T^1$ is halo, cyano, $NR^5R^6$, $C(O)NR^5R^6$, $-OC(O)NR^5R^6$, $C(O)OR^5$, $-OC(O)R^5$, $C(O)R^5$, $-NR^5C(O)R^6$, $-NR^5C(O)OR^6$, $OR^5$, or $R^{S1}$, in which $R^{S1}$ is $C_3$-$C_{12}$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, $-C(O)R^6$, $-SO_2R^5$, - $SO_2N(R^5)_2$, $-NR^5C(O)R^6$, amino, mono- or di- alkylamino, or $C_1$-$C_6$ alkoxyl; or
(4) $R^1$ and $R^{11}$ together with the carbon atom to which they are attached form a $C_7$-$C_{12}$ cycloalkyl or 4- to 12-membered heterocycloalkyl containing 1-4 heteroatoms selected from N, O, and S, wherein the $C_7$-$C_{12}$ cycloalkyl or 4- to 12-membered heterocycloalkyl is optionally substituted with one or more of halo, $C_1$-$C_6$ alkyl, hydroxyl, oxo, amino, mono- or di- alkylamino, or $C_1$-$C_6$ alkoxyl.

3. The compound of clause 1, wherein at least one of $X^2$ and $X^3$ is N.

4. The compound of any one of the preceding clauses, wherein at least two of $X^1$, $X^2$, and $X^3$ comprise N.

5. The compound of any one of the preceding clauses, wherein at least one of $\overset{1}{\text{-----}}$ , $\overset{2}{\text{-----}}$ and $\overset{3}{\text{-----}}$ is a double bond.

6. The compound of any one of the preceding clauses, wherein $\overset{3}{\text{-----}}$ is a double bond.

7. The compound of any one of the preceding clauses, wherein $\overset{3}{\text{-----}}$ is a single bond.

8. The compound of any one of the preceding clauses, wherein $X^2$ is $NR^{2'}$ and $R^3$ is oxo.

9. The compound of any one of the preceding clauses, wherein $X^2$ is N and $X^3$ is C.

10. The compound of any one of the preceding clauses, wherein $X^2$ is $CR^2$ and $X^3$ is N.

11. The compound of any one of the preceding clauses, wherein $X^1$ is S.

12. The compound of any one of the preceding clauses, wherein $X^1$ is $NR^{1'}$.

13. The compound of any one of the preceding clauses, wherein $X^1$ is $CR^1R^{11}$.

14. The compound of any one of the preceding clauses, wherein $R^1$ and $R^{11}$ together with the carbon atom to which they are attached form a 4- to 7-membered heterocycloalkyl containing 1-4 heteroatoms selected from N, O, and S, wherein the 4- to 7-membered heterocycloalkyl is optionally substituted with one or more of halo, $C_1$-$C_6$ alkyl, hydroxyl, oxo, amino, mono- or dialkylamino, or $C_1$-$C_6$ alkoxyl.

15. The compound of any one of the preceding clauses, wherein n is 1 or 2.

16. The compound of any one of the preceding clauses, being of Formula (IIa), (IIb), (IIc), (IId) or (IIe):

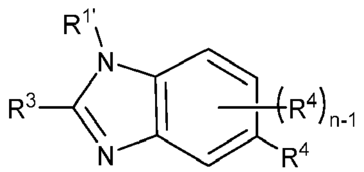
(IIa),
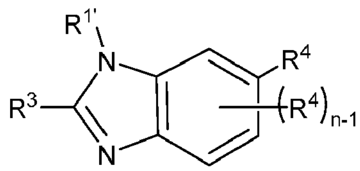
(IIb),
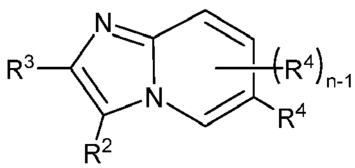
(IIc),
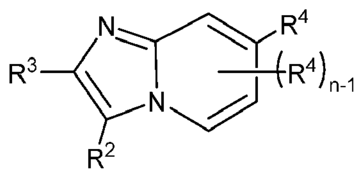
(IId), or
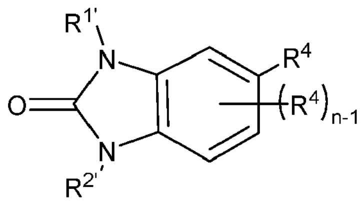
(IIe),

or a tautomer thereof, or a pharmaceutically acceptable salt of the compound or the tautomer.

17. The compound of any one of the preceding clauses, being of Formula (IIIa), (IIIb), (IIIc), (IIId), (IIIe) or (IIIf):

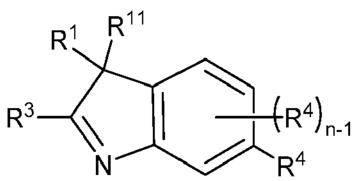
(IIIa),
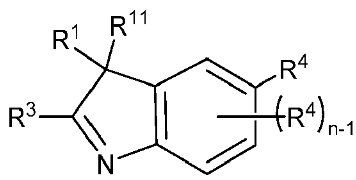
(IIIb),
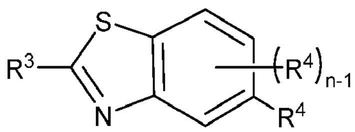
(IIIc),
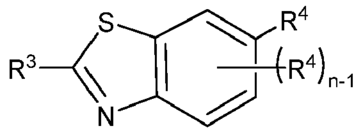
(IIId),

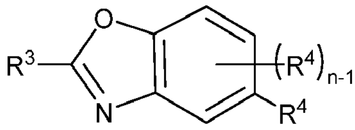 (IIIe), or 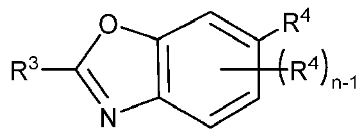 (IIIf),

or a tautomer thereof, or a pharmaceutically acceptable salt of the compound or the tautomer.

18. The compound of any one of the preceding clauses, being of Formula (IVa) or (IVb):

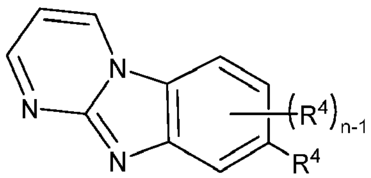 (IVa), or 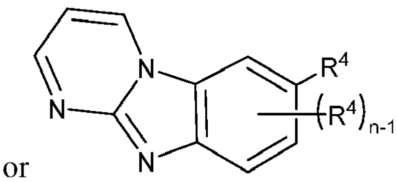 (IVb),

or a tautomer thereof, or a pharmaceutically acceptable salt of the compound or the tautomer.

19. The compound of any one of the preceding clauses, being of Formula (IIf), (IIg), (IIh), (IIIi), (IIIj), (IIIk), or (IIIl):

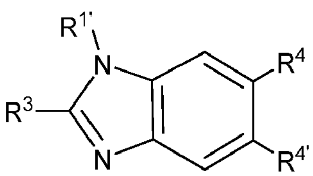 (IIf), 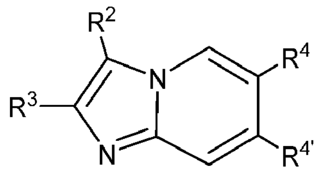 (IIg), 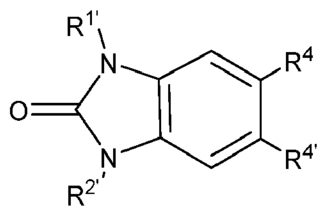 (IIh),

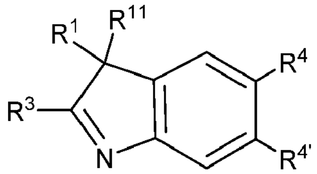 (IIIi), 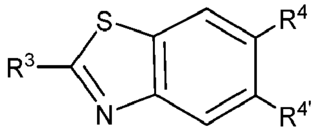 (IIIj),

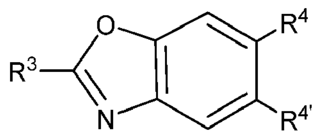 (IIIk), or 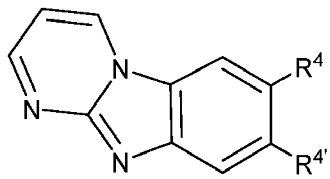 (IIIl),

or a tautomer thereof, or a pharmaceutically acceptable salt of the compound or the tautomer, wherein

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, C3-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S;

each of $R^4$ and $R^{4'}$ independently is $-Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$,

$NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl;

$R^8$ is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo.

20. The compound of any one of the preceding clauses, wherein n is 2.

21. The compound of any one of the preceding clauses, wherein $R^2$ is $-Q^1-T^1$, in which $Q^1$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^1$ is H, halo, cyano, or $R^{S1}$, in which $R^{S1}$ is $C_3$-$C_8$ cycloalkyl, phenyl, 4- to 12-membered heterocycloalkyl containing 1-4 heteroatoms selected from N, O, and S, or a 5- or 6-membered heteroaryl and $R^{S1}$ is optionally substituted with one or more of halo, $C_1$-$C_6$ alkyl, hydroxyl, oxo, amino, mono- or di- alkylamino, or $C_1$-$C_6$ alkoxyl.

22. The compound of any one of the preceding clauses, wherein $R^2$ is $C_1$-$C_6$ alkyl optionally substituted with one or more of halo, cyano, hydroxyl, or $C_1$-$C_6$ alkoxyl.

23. The compound of any one of the preceding clauses, wherein $R^2$ is unsubstituted $C_1$-$C_6$ alkyl.

24. The compound of any one of the preceding clauses, wherein $R^{1'}$ is $-Q^2-T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_8$ cycloalkyl, phenyl, 4- to 12-membered heterocycloalkyl containing 1-4 heteroatoms selected from N, O, and S, or a 5- or 6-membered heteroaryl and $R^{S2}$ is optionally substituted with one or more of halo, $C_1$-$C_6$ alkyl, hydroxyl, oxo, amino, mono- or di- alkylamino, or $C_1$-$C_6$ alkoxyl.

25. The compound of any one of the preceding clauses, wherein $R^{2'}$ is $-Q^2-T^2$, in which $Q^2$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^2$ is H, halo, cyano, or $R^{S2}$, in which $R^{S2}$ is $C_3$-$C_8$ cycloalkyl, phenyl, 4- to 12-membered heterocycloalkyl containing 1-4 heteroatoms selected from N, O, and S, or a 5- or 6-membered heteroaryl and $R^{S2}$ is optionally substituted with one or more of halo, $C_1$-$C_6$ alkyl, hydroxyl, oxo, amino, mono- or di- alkylamino, or $C_1$-$C_6$ alkoxyl.

26. The compound of any one of the preceding clauses, wherein each $Q^2$ independently is a bond or $C_1$-$C_6$ alkylene, $C_2$-$C_6$ alkenylene, or $C_2$-$C_6$ alkynylene linker optionally substituted with one or more of halo and each $T^2$ independently is H, halo, $C_3$-$C_8$ cycloalkyl, or a 4- to 7-membered heterocycloalkyl.

27. The compound of any one of the preceding clauses, wherein $R^{2'}$ is H or $C_1$-$C_6$ alkyl.

28. The compound of any one of the preceding clauses, being of Formula (Va), (Vb), (Vc), (Vd), (Ve), or (Vf):.

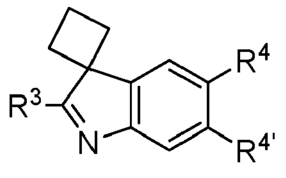 (Va), 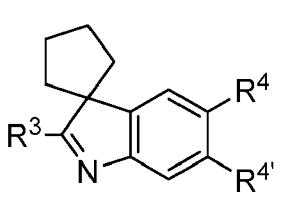 (Vb), 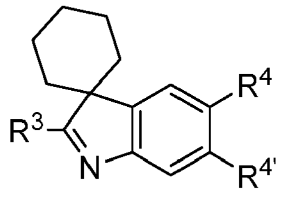 (Vc),

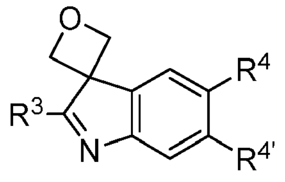 (Vd), 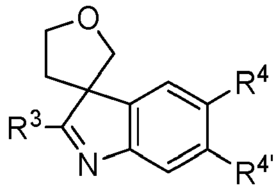 (Ve), 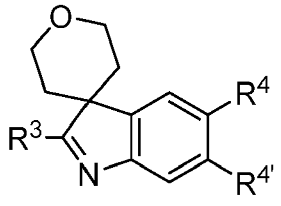 (Vf),

or a tautomer thereof, or a pharmaceutically acceptable salt of the compound or the tautomer, wherein

$R^3$ is H, $NR^aR^b$, $OR^a$, or $R^{S4}$, in which $R^{S4}$ is $C_1$-$C_6$ alkyl, $C_2$-$C_6$ alkenyl, $C_2$-$C_6$ alkynyl, $C_3$-$C_{12}$ cycloalkyl, phenyl, 5- or 6-membered heteroaryl, or 4- to 12-membered heterocycloalkyl containing 1-4 heteroatoms selected from N, O, and S, wherein each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4- to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di- alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S;

each of $R^4$ and $R^{4'}$ independently is $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and $R^8$ is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo.

29. The compound of any one of the preceding clauses, wherein when $R^3$ is $-NH_2$, then $R^4$ is not $-OCH_3$.

30. The compound of any one of the preceding clauses, wherein when $R^3$ is $-NH_2$, and $R^4$ is not $-OCH_3$, then $R^{4'}$ is not $OR^8$.

31. The compound of any one of the preceding clauses, wherein $R^3$ is $NR^aR^b$ or $OR^a$, wherein each of $R^a$ and $R^b$ independently is H or $C_1$-$C_6$ alkyl optionally substituted with one or more of halo, hydroxyl, amino, mono- or di-alkylamino, or $C_1$-$C_6$ alkoxyl.

32. The compound of any one of the preceding clauses, wherein $R^3$ is $NR^aR^b$ or $OR^a$, wherein each of $R^a$ and $R^b$ independently is H or $C_1$-$C_6$ alkyl optionally substituted with one or more of halo, hydroxyl, amino, mono- or di-alkylamino, $C_1$-$C_6$ alkoxyl, $C_3$-$C_{12}$ cycloalkyl, or 4- to 12-membered heterocycloalkyl containing 1-4 heteroatoms selected from N, O, and S.

33. The compound of any one of the preceding clauses, wherein $R^3$ is H, $C_1$-$C_6$ alkyl, $C_3$-$C_{12}$ cycloalkyl, or 4- to 12-membered heterocycloalkyl.

34. The compound of any one of the preceding clauses, wherein $R^3$ is $NH_2$.

35. The compound of any one of the preceding clauses, wherein $R^3$ is $NR^aR^b$, in which one of $R^a$ and $R^b$ is H and the other is $C_1$-$C_6$ alkyl optionally substituted with one or more of halo.

36. The compound of any one of the preceding clauses, wherein $R^3$ is $OR^a$, in which $R^a$ is H or $C_1$-$C_6$ alkyl.

37. The compound of any one of the preceding clauses, wherein $R^3$ is

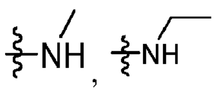 ,

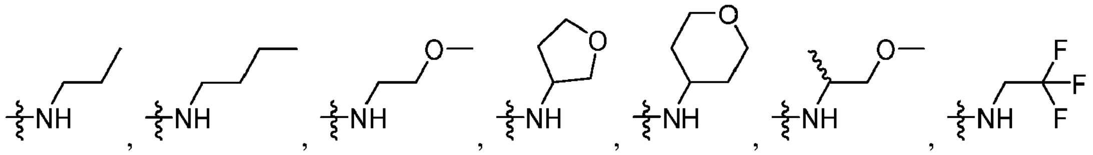 ,

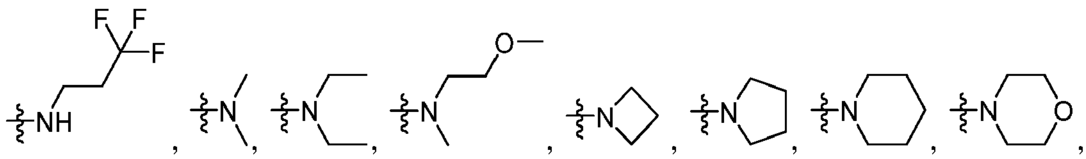 ,

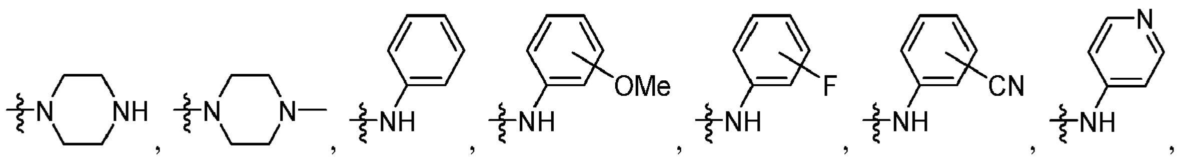 ,

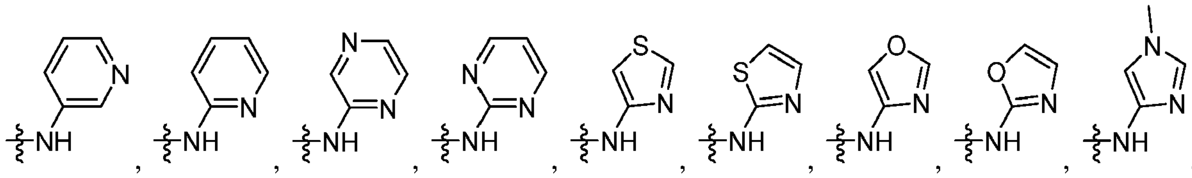 ,

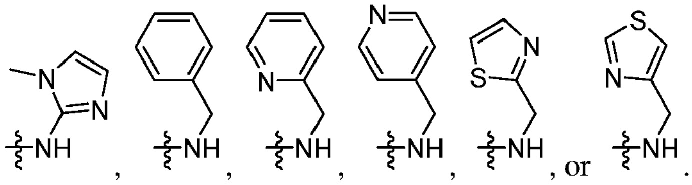 .

38. The compound of any one of the preceding clauses, being of Formula (VIa), (VIb), (VIc), (VId), (VIe), or (VIf):

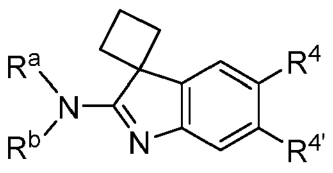 (VIa), 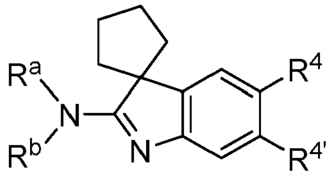 (VIb),

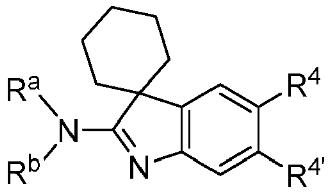 (VIc), 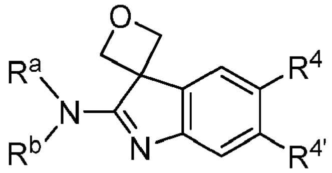 (VId),

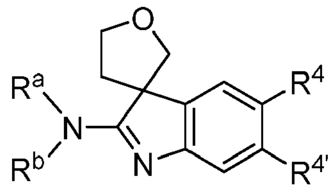 (VIe), 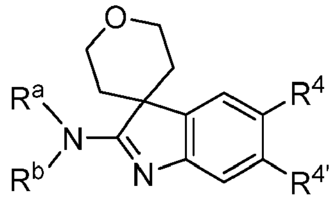 (VIf),

or a tautomer thereof, or a pharmaceutically acceptable salt of the compound or the tautomer, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

each of $R^4$ and $R^{4'}$ independently is -$Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and $R^8$ is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

39. The compound of any one of the preceding clauses, wherein at least one of $R^a$ and $R^b$ is $R^{S5}$.

40. The compound of any one of the preceding clauses, wherein when both of $R^a$ and $R^b$ are H, then $R^4$ is not -$OCH_3$.

41. The compound of any one of the preceding clauses, wherein when both of $R^a$ and $R^b$ are H, and $R^4$ is -$OCH_3$,

then $R^{4'}$ is not $OR^8$.

42. The compound of any one of the preceding clauses, wherein each of $R^4$ and $R^{4'}$ is independently $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, $OR^7$, $OR^8$, $NR^7R^8$, $C_6$-$C_{10}$ aryl, 5- to 10-membered heteroaryl, $C_3$-$C_{12}$ cycloalkyl, or 4- to 12-membered heterocycloalkyl.

43. The compound of any one of the preceding clauses, wherein $R^4$ is $-Q^3-T^3$, in which $Q^3$ is a bond or $C_1$-$C_6$ alkylene linker, and $T^3$ is H, halo, $OR^7$, $C_6$-$C_{10}$ aryl, or 5- to 10-membered heteroaryl.

44. The compound of any one of the preceding clauses, wherein $R^{4'}$ is $-Q^3-T^3$, in which $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, $OR^7$, $OR^8$, $NR^7R^8$, $C_3$-$C_{12}$ cycloalkyl, or 4- to 12-membered heterocycloalkyl.

45. The compound of any one of the preceding clauses, wherein at least one of $R^4$ and $R^{4'}$ is halo, $C_1$-$C_6$ alkyl, or $OR^7$.

46. The compound of any one of the preceding clauses, wherein $R^4$ is halo, $C_1$-$C_6$ alkyl, or $OR^7$.

47. The compound of any one of the preceding clauses, wherein at least one of $R^4$ and $R^{4'}$ is $C_1$-$C_6$ alkyl.

48. The compound of any one of the preceding clauses, wherein $R^4$ is $C_1$-$C_6$ alkyl.

49. The compound of any one of the preceding clauses, wherein at least one of $R^4$ and $R^{4'}$ is $CH_3$.

50. The compound of any one of the preceding clauses, wherein $R^4$ is $CH_3$.

51. The compound of any one of the preceding clauses, wherein at least one of $R^4$ and $R^{4'}$ is halo.

52. The compound of any one of the preceding clauses, wherein $R^4$ is halo.

53. The compound of any one of the preceding clauses, wherein at least one of $R^4$ and $R^{4'}$ is F or Cl.

54. The compound of any one of the preceding clauses, wherein $R^4$ is F or Cl.

55. The compound of any one of the preceding clauses, wherein at least one of $R^4$ and $R^{4'}$ is $C_6$-$C_{10}$ aryl.

56. The compound of any one of the preceding clauses, wherein $R^4$ is $C_6$-$C_{10}$ aryl.

57. The compound of any one of the preceding clauses, wherein at least one of $R^4$ and $R^{4'}$ is

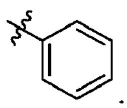

58. The compound of any one of the preceding clauses, wherein at least one of $R^4$ and $R^{4'}$ is

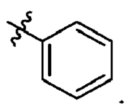

59. The compound of any one of the preceding clauses, wherein at least one of $R^4$ and $R^{4'}$ is 5-to 10-membered heteroaryl.

60. The compound of any one of the preceding clauses, wherein $R^4$ is 5- to 10-membered heteroaryl.

61. The compound of any one of the preceding clauses, wherein at least one of $R^4$ and $R^{4'}$ is

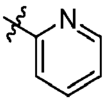, 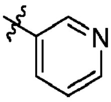, or 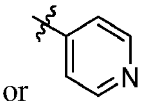.

62. The compound of any one of the preceding clauses, wherein $R^4$ is

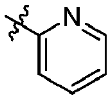, 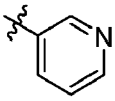,

or

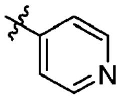.

63. The compound of any one of the preceding clauses, wherein at least one of $R^4$ and $R^{4'}$ is

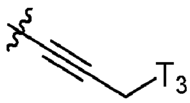,

wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$.

64. The compound of any one of the preceding clauses, wherein $R^{4'}$ is

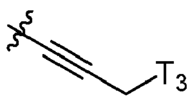,

wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5-to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$.

65. The compound of any one of the preceding clauses, wherein at least one of $R^4$ and $R^{4'}$ is

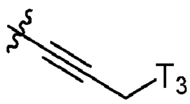,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

66. The compound of any one of the preceding clauses, wherein $R^{4'}$ is

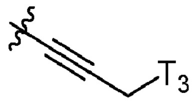,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl.

67. The compound of any one of the preceding clauses, wherein at least one of $R^4$ and $R^{4'}$ is

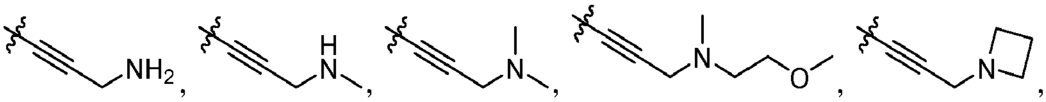,

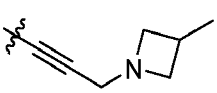, 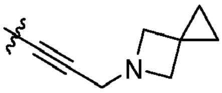, 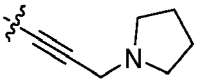, 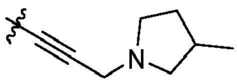, 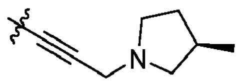,

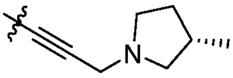, 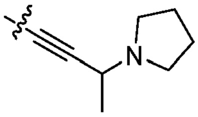, 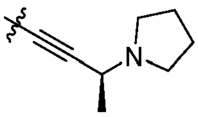, 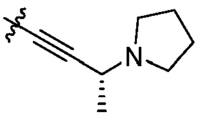, 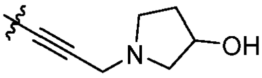,

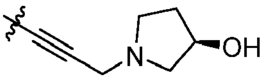, 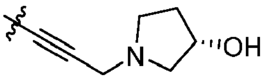, 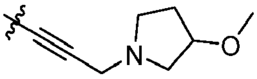, 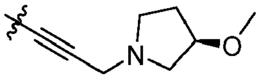,

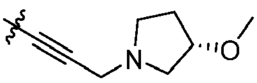, 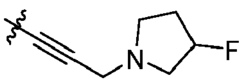, 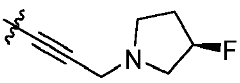, 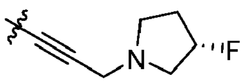,

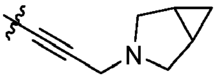, 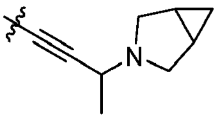, 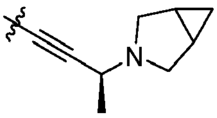, 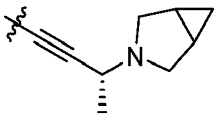, 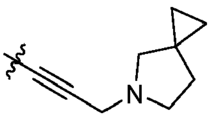,

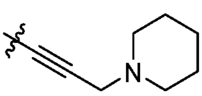, 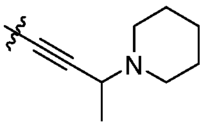, 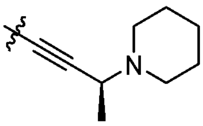, 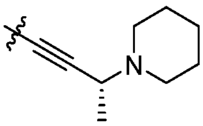, 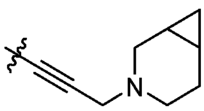,

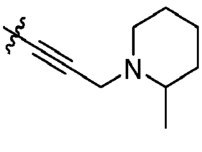, 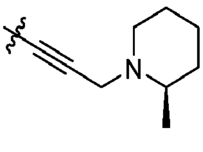, 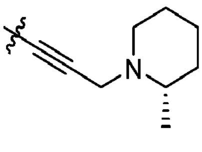, 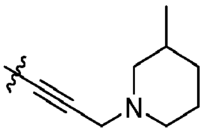, 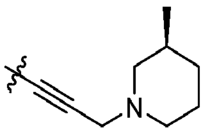,

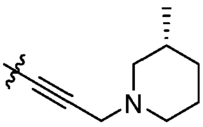, 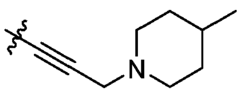, 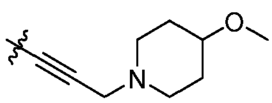, 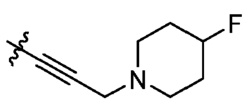,

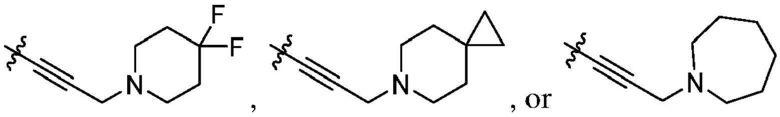, , or .

68. The compound of any one of the preceding clauses, wherein $R^{4'}$ is

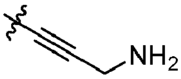,

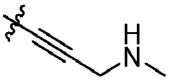, 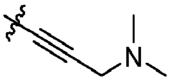, 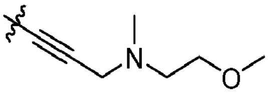, 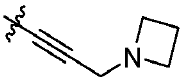, 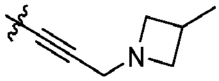,

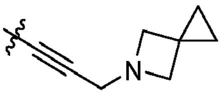, 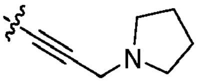, 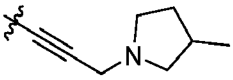, 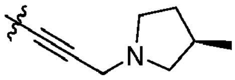, 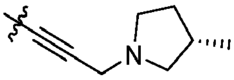,

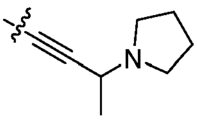, 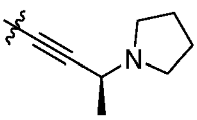, 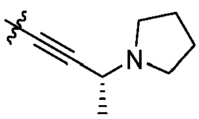, 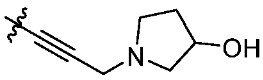, 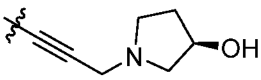,

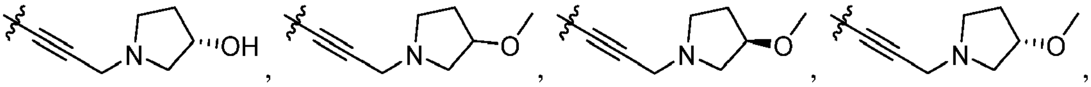, , , ,

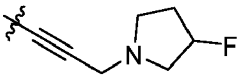, 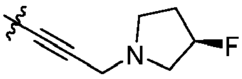, 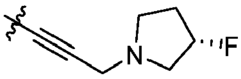, 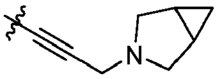, 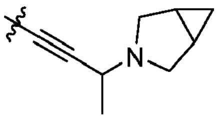,

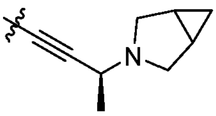, 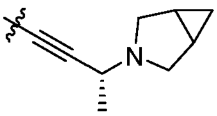, 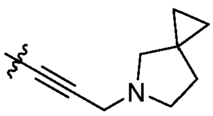, 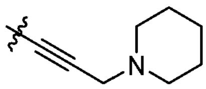, 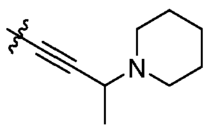,

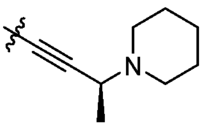, 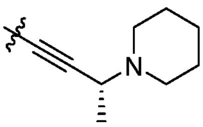, 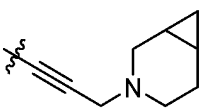, 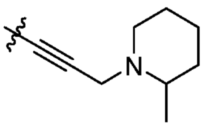, 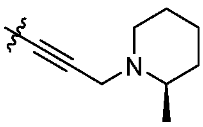,

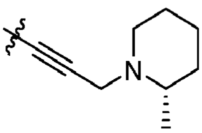, 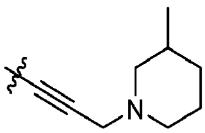, 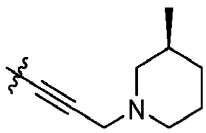, 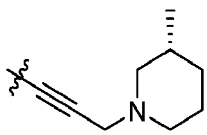, 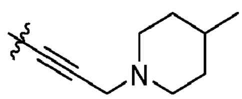,

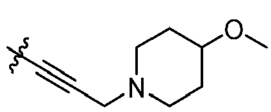, 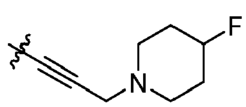, 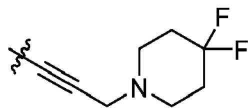, 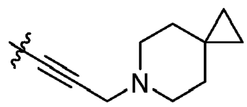,

or

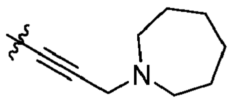.

69. The compound of any one of the preceding clauses, wherein at least one of $R^4$ and $R^{4'}$ is $OR^7$.

70. The compound of any one of the preceding clauses, wherein $R^4$ is $OR^7$.

71. The compound of any one of the preceding clauses, wherein $R^{4'}$ is $OR^7$.

72. The compound of any one of the preceding clauses, wherein at least one of $R^4$ and $R^{4'}$ is $OR^8$.

73. The compound of any one of the preceding clauses, wherein $R^{4'}$ is $OR^8$.

74. The compound of any one of the preceding clauses, wherein at least one of $R^4$ and $R^{4'}$ is $-CH_2-OR_8$.

75. The compound of any one of the preceding clauses, wherein $R^{4'}$ is $-CH_2-OR_8$.

76. The compound of any one of the preceding clauses, wherein at least one of $R^4$ and $R^{4'}$ is $-CH_2-NR_7R_8$.

77. The compound of any one of the preceding clauses, wherein $R^{4'}$ is $-CH_2-NR_7R_8$.

78. The compound of any one of the preceding clauses, wherein $R^7$ is H or $C_1$-$C_6$ alkyl optionally substituted with one or more of hydroxyl, amino or mono- or di- alkylamino.

79. The compound of any one of the preceding clauses, wherein $R^8$ is $-Q^4-T^4$, in which $Q^4$ is a $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is $C_3$-$C_{12}$ cycloalkyl, $C_6$-$C_{10}$ aryl, or 4- to 12-membered heterocycloalkyl containing 1-4 heteroatoms selected from N, O and S which is optionally substituted with one or more $-Q^5-T^5$.

80. The compound of any one of the preceding clauses, wherein each $Q^5$ independently is a bond or $C_1$-$C_3$ alkylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_8$ cycloalkyl, or 4- to 7-membered heterocycloalkyl containing 1-4 heteroatoms selected from N, O, and S.

81. The compound of any one of the preceding clauses, wherein at least one of $R^4$ and $R^{4'}$ is $C_1$-$C_6$ alkoxyl.

82. The compound of any one of the preceding clauses, wherein $R^4$ is $C_1$-$C_6$ alkoxyl.

83. The compound of any one of the preceding clauses, wherein at least one of $R^4$ and $R^{4'}$ is $-OCH_3$, $-OCH_2CH_3$, or $-OCH(CH_3)_2$.

84. The compound of any one of the preceding clauses, wherein $R^4$ is $-OCH_3$, $-OCH_2CH_3$, or $-OCH(CH_3)_2$.

85. The compound of any one of the preceding clauses, wherein at least one of $R^4$ and $R^{4'}$ is $-OCH_3$.

86. The compound of any one of the preceding clauses, wherein $R^4$ is $-OCH_3$.

87. The compound of any one of the preceding clauses, wherein at least one of $R^4$ and $R^{4'}$ is

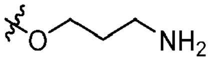, 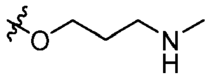, 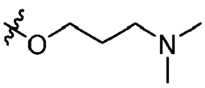, 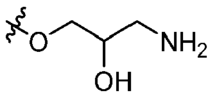, 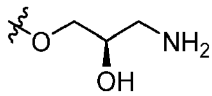,

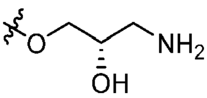, 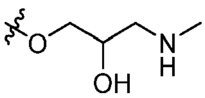, 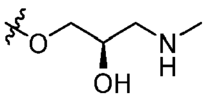, 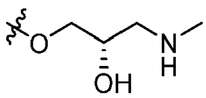, 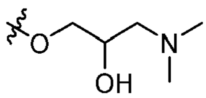,

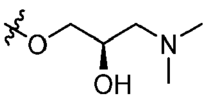, or 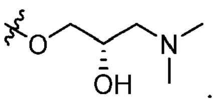.

88. The compound of any one of the preceding clauses, wherein $R^{4'}$ is

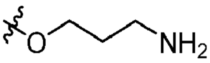,

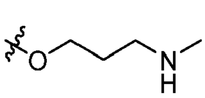, 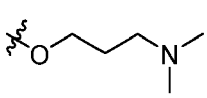, 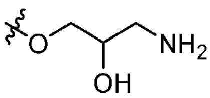, 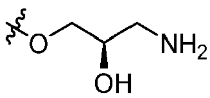, 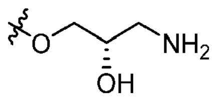,

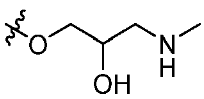, 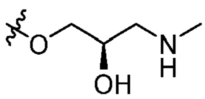, 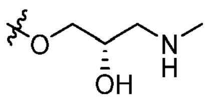, 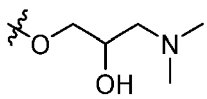, 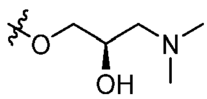,

or

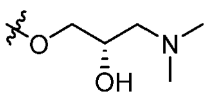.

89. The compound of any one of the preceding clauses, wherein at least one of $R^4$ and $R^{4'}$ is

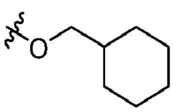, 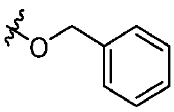, or 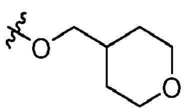.

90. The compound of any one of the preceding clauses, wherein $R^{4'}$ is

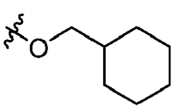,

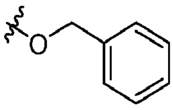, or 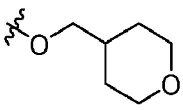.

91. The compound of any one of the preceding clauses, wherein at least one of $R^4$ and $R^{4'}$ is

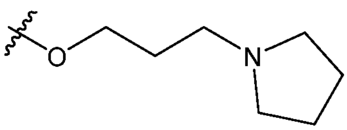 , 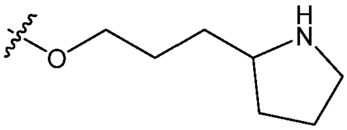 , 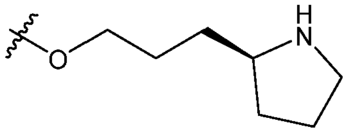 ,

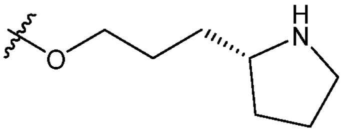 , 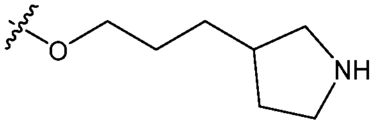 , 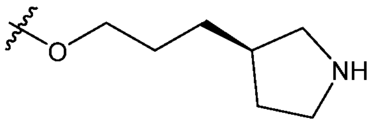 ,

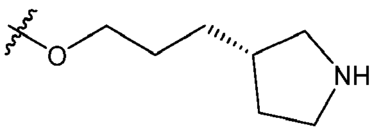 , 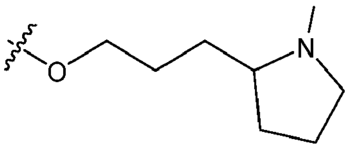 , 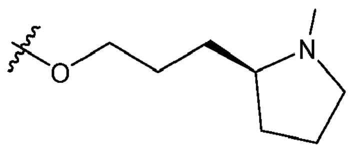 ,

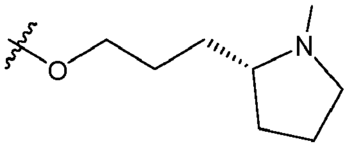 , 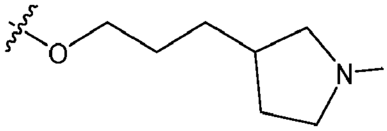 , 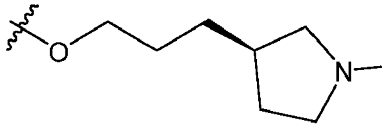 ,

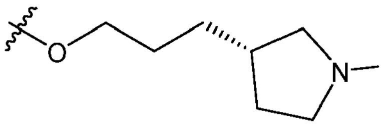 , 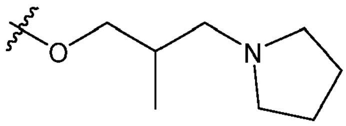 , 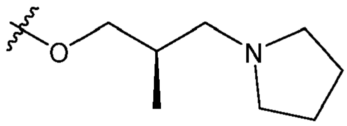 ,

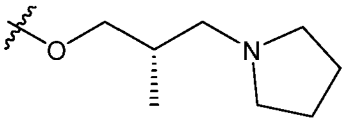 , 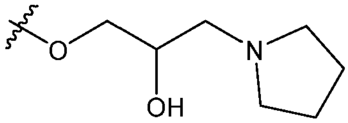 , 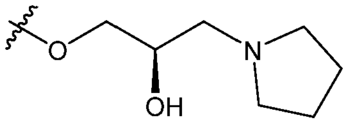 ,

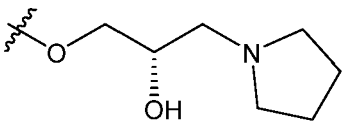 , 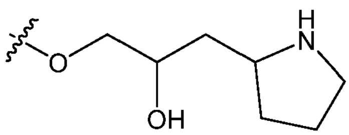 , 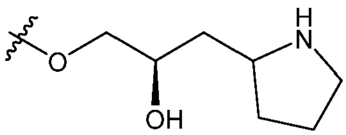 ,

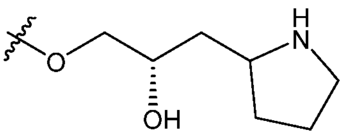 , 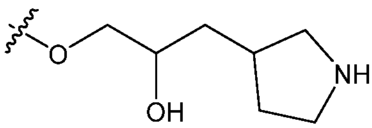 , 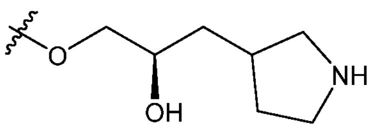 ,

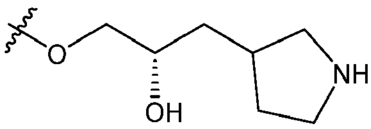 , 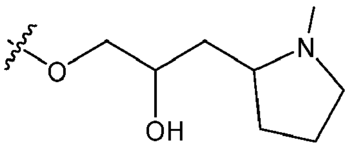 , 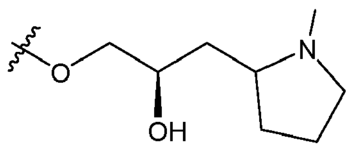 ,

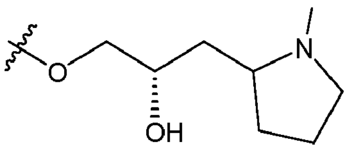, 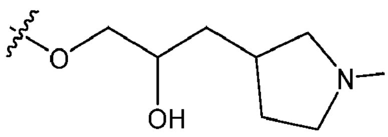, 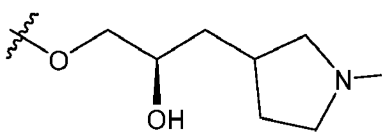,

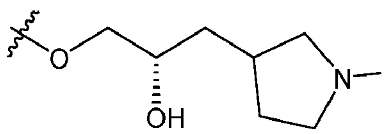, 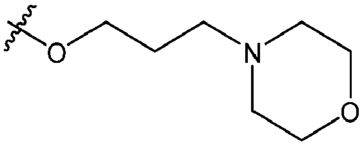, 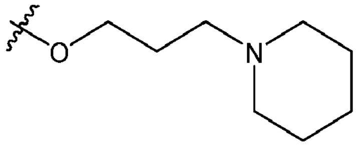,

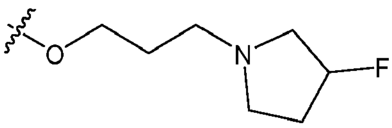, 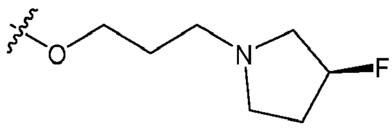, 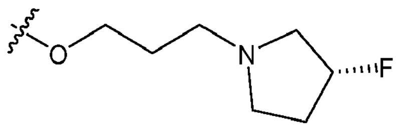,

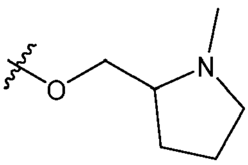, 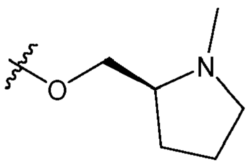, 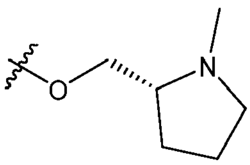, 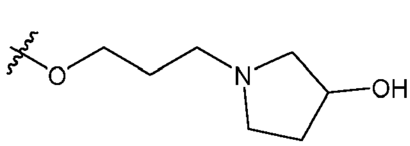,

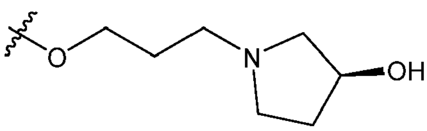, 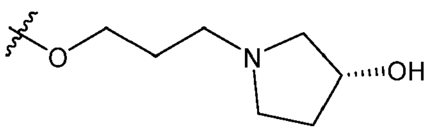, 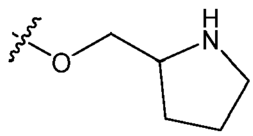,

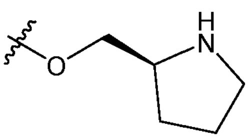, 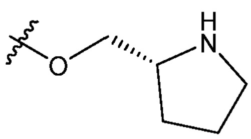, 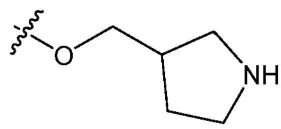, 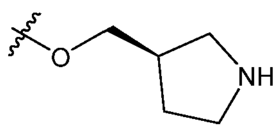,

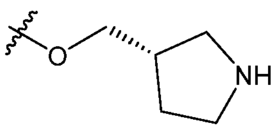, 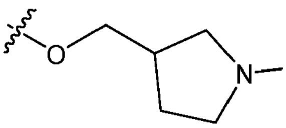, 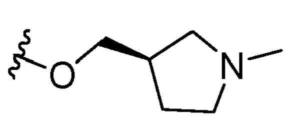, 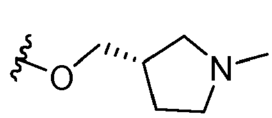,

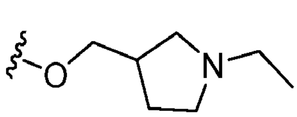, 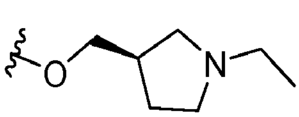, 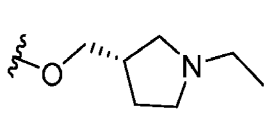, 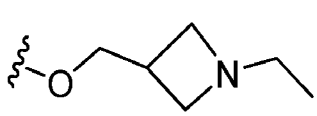

, 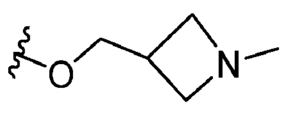, 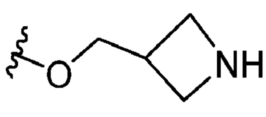, 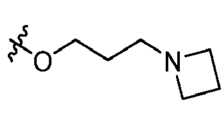, 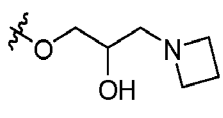,

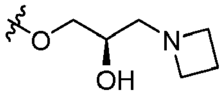, or 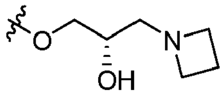.

92. The compound of any one of the preceding clauses, wherein $R^{4'}$ is

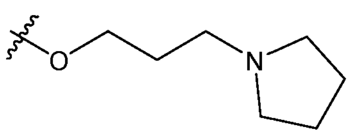 , 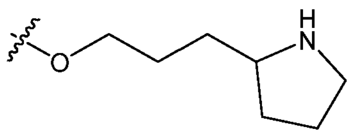 , 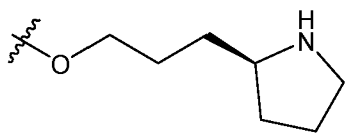 ,

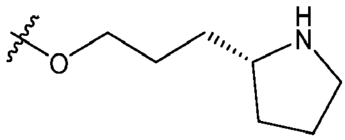 , 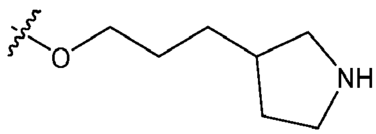 , 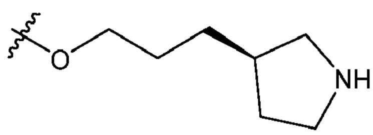 ,

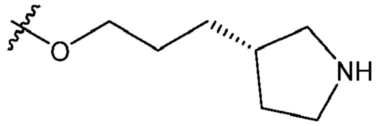 , 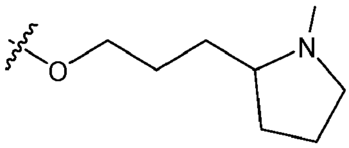 , 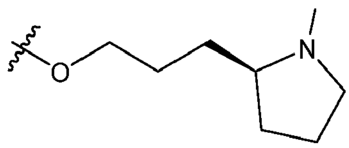 ,

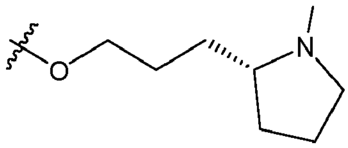 , 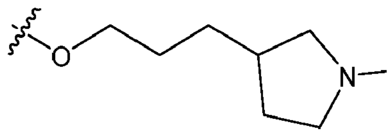 , 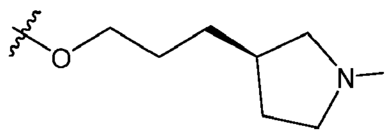 ,

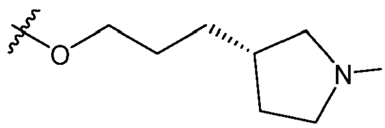 , 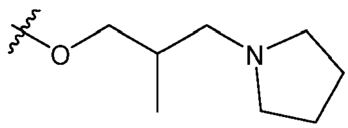 , 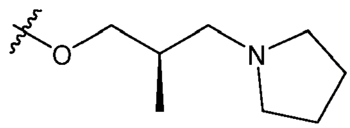 ,

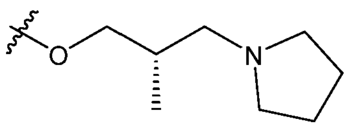 , 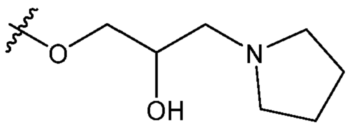 , 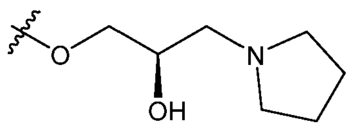 ,

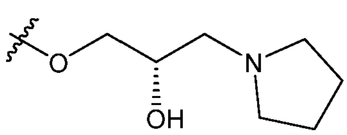 , 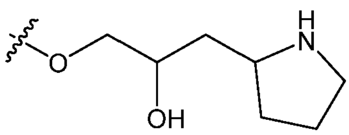 , 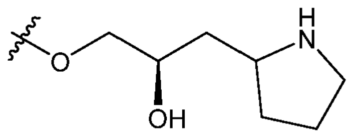 ,

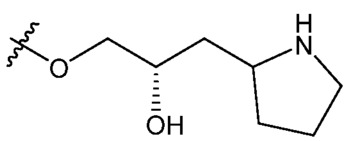 , 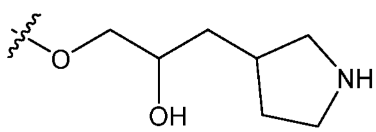 , 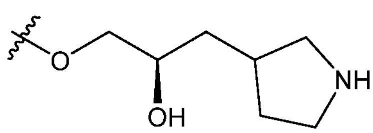 ,

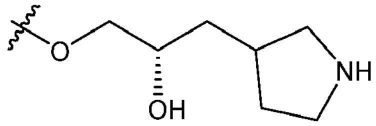 , 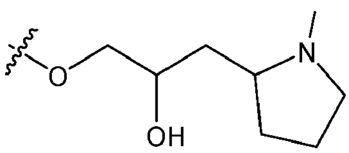 , 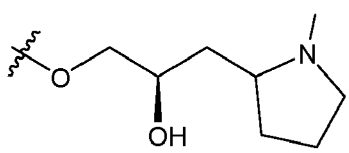 ,

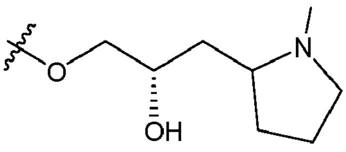, 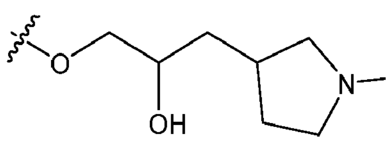, 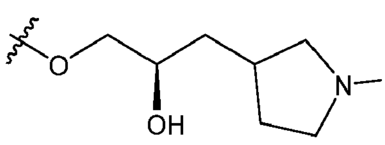,

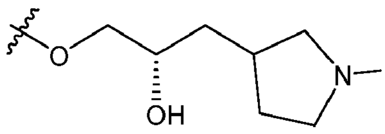, 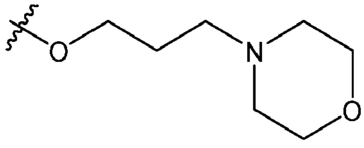, 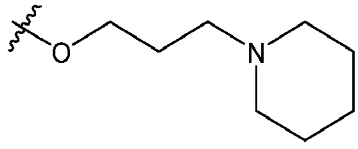,

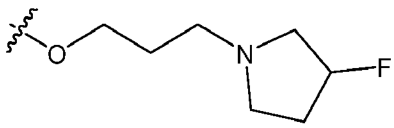, 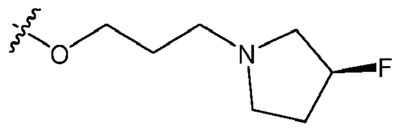, 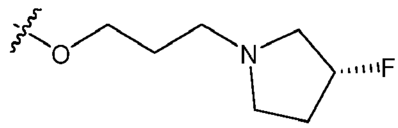,

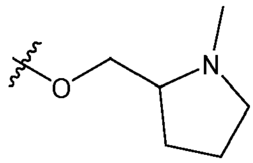, 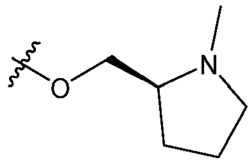, 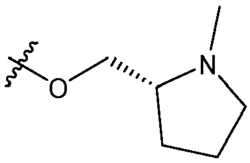, 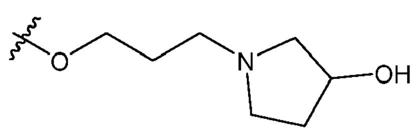,

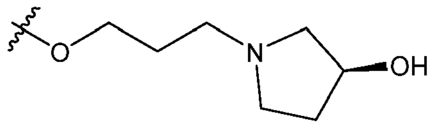, 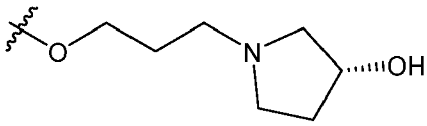, 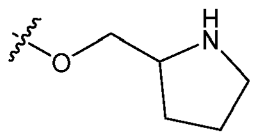,

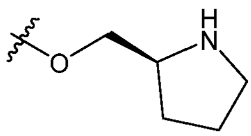, 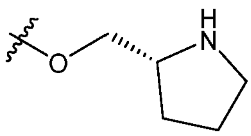, 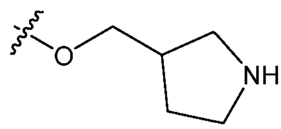, 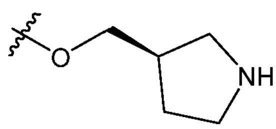,

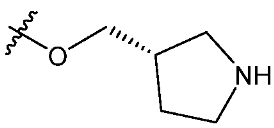, 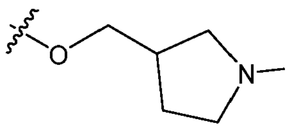, 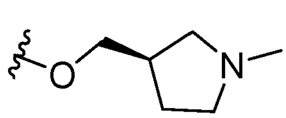, 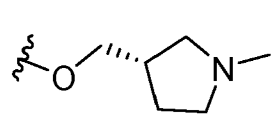,

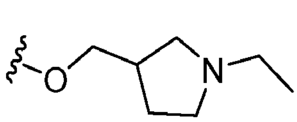, 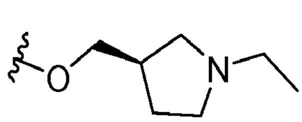, 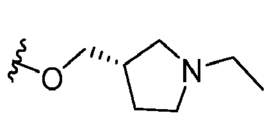, 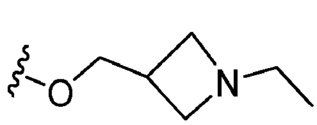

, 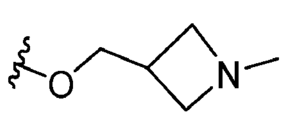, 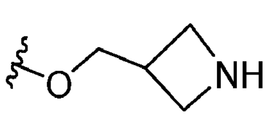, 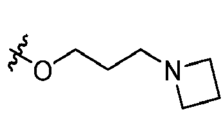, 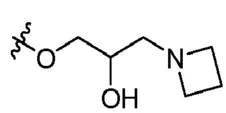,

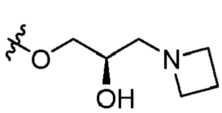, or 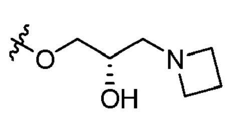.

93. The compound of any one of the preceding clauses, wherein at least one of $R^4$ and $R^{4'}$ is

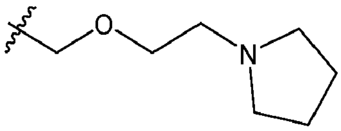.

94. The compound of any one of the preceding clauses, wherein $R^{4'}$ is

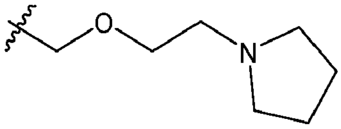.

95. The compound of any one of the preceding clauses, wherein at least one of $R^4$ and $R^{4'}$ is

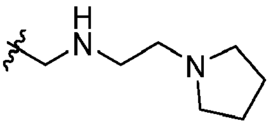, 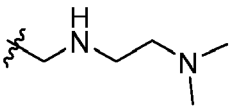, 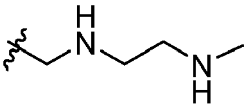, 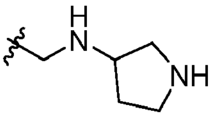,

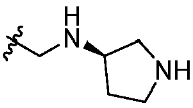, 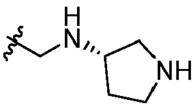, 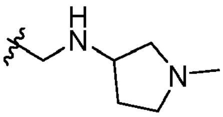, 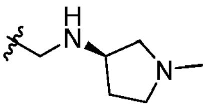, 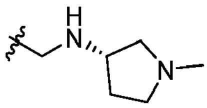,

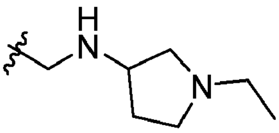, 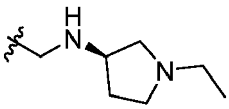, 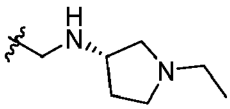, 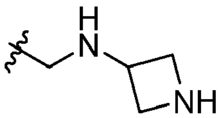,

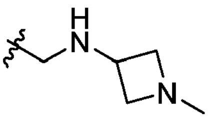, 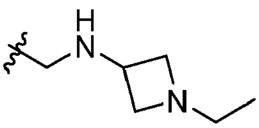, or 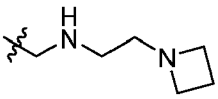.

96. The compound of any one of the preceding clauses, wherein $R^{4'}$ is

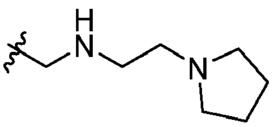,

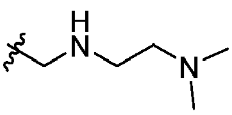, 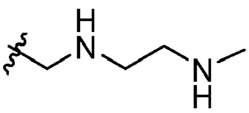, 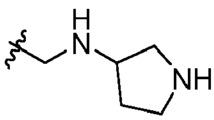, 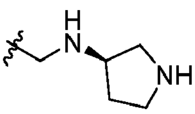, 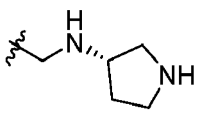,

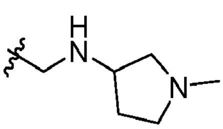, 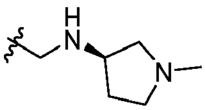, 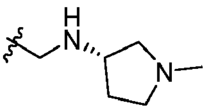, 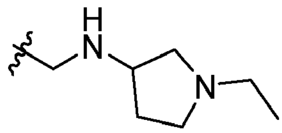, 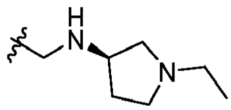,

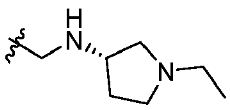, 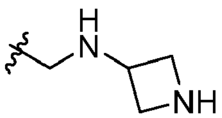, 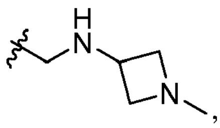, 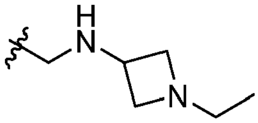, or

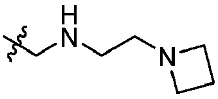.

97. The compound of any one of the preceding clauses, wherein n is 2, one of $R^4$ and $R^{4'}$ is - $OCH_3$, and the other is

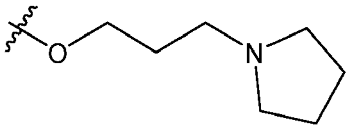.

98. The compound of any one of the preceding clauses, wherein n is 2, $R^4$ is $-OCH_3$, and $R^{4'}$ is

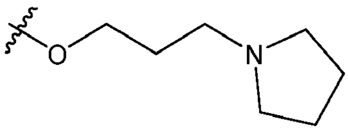.

99. The compound of any one of the preceding clauses, being of Formula (VIIa), (VIIb), (VIIc), (VIId), (Vile), or (VIIf):

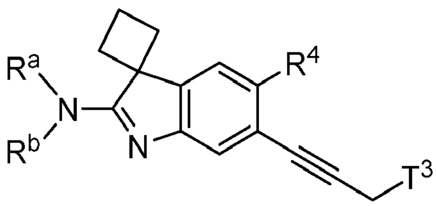 (VIIa), 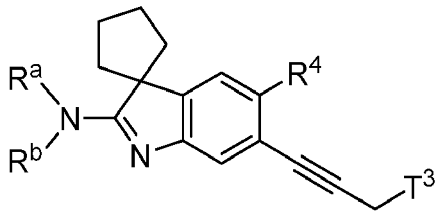 (VIIb),

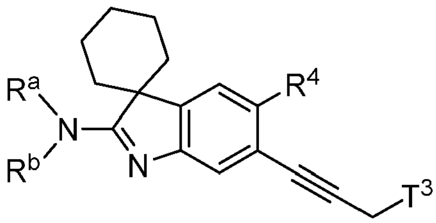 (VIIc), 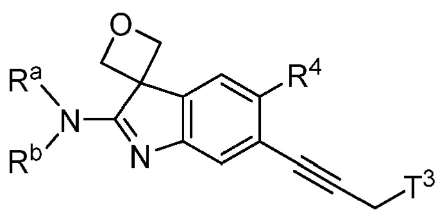 (VIId),

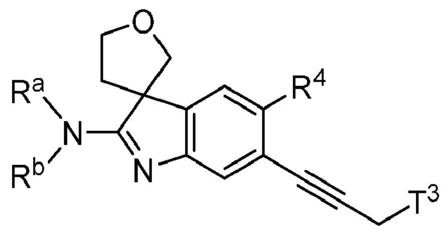 (VIIe), 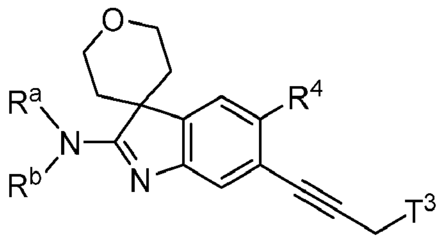 (VIIf),

or a tautomer thereof, or a pharmaceutically acceptable salt of the compound or the tautomer, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and
$R^4$ is -$Q^3$-$T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl;
each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;
each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and each $R^8$ independently is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

100. The compound of any one of the preceding clauses, being of Formula (Villa), (VIIIb), (VIIIc), (VIIId), (VIIIe), or (VIIIf):

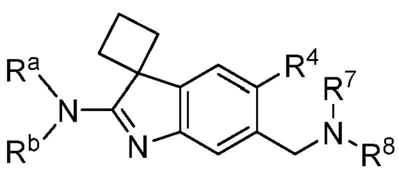 (VIIIa), 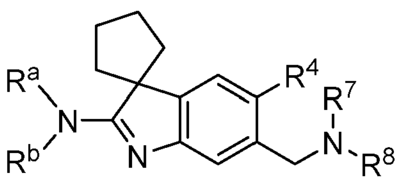 (VIIIb),

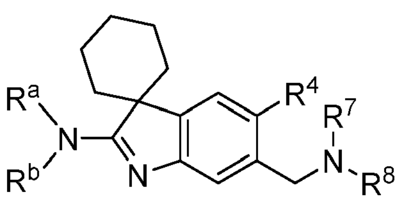 (VIIIc), 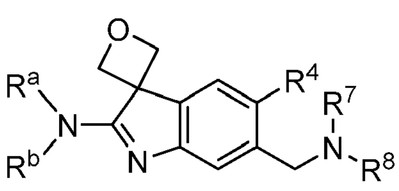 (VIIId),

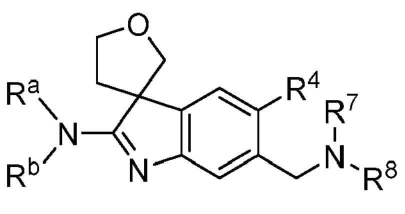 (VIIIe), 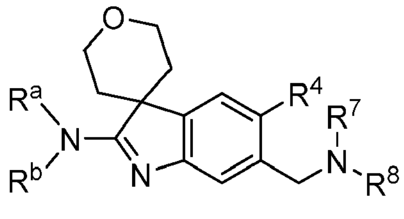 (VIIIf),

or a tautomer thereof, or a pharmaceutically acceptable salt of the compound or the tautomer, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is -$Q^3$-$T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, -$SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and each $R^8$ independently is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

101. The compound of any one of the preceding clauses, being of Formula (IXa), (IXb), (IXc), (IXd), (IXe), or (IXf):

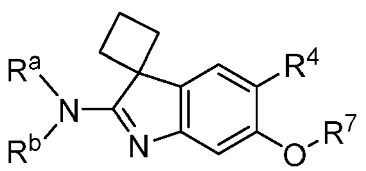 (IXa), 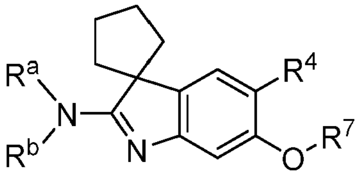 (IXb),

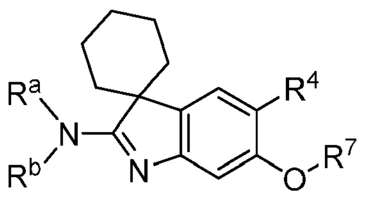 (IXc), 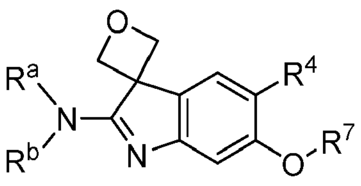 (IXd),

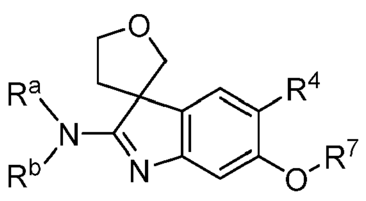 (IXe), 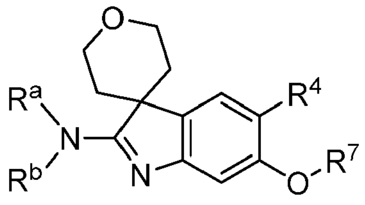 (IXf),

or a tautomer thereof, or a pharmaceutically acceptable salt of the compound or the tautomer, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is $-Q^3$-$T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

each $R^8$ independently is $-Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5$-$T^5$ is oxo.

102. The compound of any one of the preceding clauses, being of Formula (Xa), (Xb), (Xc), (Xd), (Xe), or (Xf):

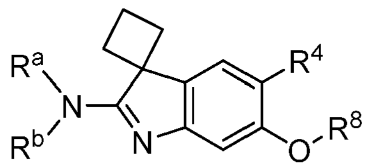 (Xa), 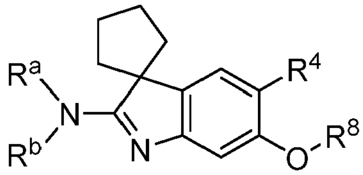 (Xb),

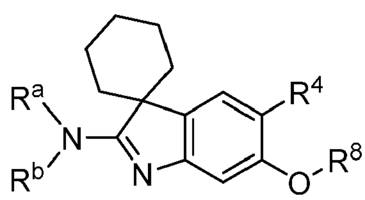 (Xc), 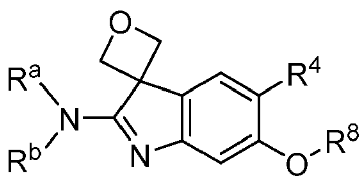 (Xd),

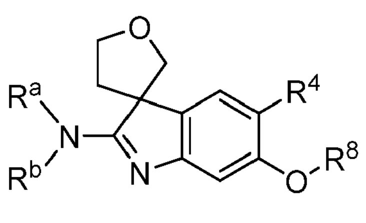 (Xe), 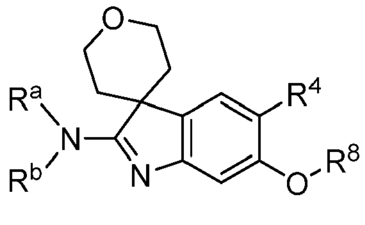 (Xf),

or a tautomer thereof, or a pharmaceutically acceptable salt of the compound or the tautomer, wherein

each of $R^a$ and $R^b$ independently is H or $R^{S5}$, or $R^a$ and $R^b$ together with the nitrogen atom to which they are attached form a 4- to 12-membered heterocycloalkyl containing 1-4 heteroatoms selected from N, O, and S; in which $R^{S5}$ is $C_1$-$C_6$ alkyl, phenyl, 5- or 6-membered heteroaryl, or 4-to 12-membered heterocycloalkyl containing 1-4 heteroatoms selected from N, O, and S, and each of $R^{S4}$, $R^{S5}$, and the heterocycloalkyl formed by $R^a$ and $R^b$ is independently optionally substituted with one or more of halo, hydroxyl, oxo, CN, amino, mono- or di-alkylamino, $C_1$-$C_6$ alkyl, $C_1$-$C_6$ alkoxyl, $C_3$-$C_{12}$ cycloalkyl, phenyl, 5- or 6-membered heteroaryl, or 4- to 12-membered heterocycloalkyl containing 1-4 heteroatoms selected from N, O, and S, or alternatively; and

$R^4$ is $-Q^3-T^3$, in which $Q^3$ is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and

each $R^8$ independently is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo.

103. The compound of any one of the preceding clauses, wherein the compound is selected from those in Table 1 and pharmaceutically acceptable salts thereof.

104. The compound of any one of the preceding clauses, wherein the compound is selected from the group consisting of Compound Nos. 1-23, 25-36, 38-39, 42-69, 77-78, 81, and 135-137, tautomers thereof, and pharmaceutically acceptable salts of the compounds and tautomers.

105. The compound of any one of the preceding clauses, wherein the compound is selected from the group consisting of Compound Nos. 24, 74-75, 82-101, 106-107, 110-134, and 141, tautomers thereof, and pharmaceutically acceptable salts of the compounds and tautomers.

106. The compound of any one of the preceding clauses, wherein the compound is selected from the group consisting of Compound Nos. 37, 40-41, 70-73, 76, 79-80, and 138-140, tautomers thereof, and pharmaceutically acceptable salts of the compounds and tautomers.

107. The compound of any one of the preceding clauses, wherein the compound inhibits a kinase with an enzyme inhibition $IC_{50}$ value of about 100 nM or greater, 1 $\mu$M or greater, 10 $\mu$M or greater, 100 $\mu$M or greater, or 1000 $\mu$M or greater.

108. The compound of any one of the preceding clauses, wherein the compound inhibits a kinase with an enzyme inhibition $IC_{50}$ value of about 1 mM or greater.

109. The compound of any one of the preceding clauses, wherein the compound inhibits a kinase with an enzyme inhibition $IC_{50}$ value of 1 μM or greater, 2 μM or greater, 5 μM or greater, or 10 μM or greater, wherein the kinase is one or more of the following: Abl, AurA, CHK1, MAP4K, IRAK4, JAK3, EphA2, FGFR3, KDR, Lck, MARK1, MNK2, PKCb2, SIK, and Src.

110. A pharmaceutical composition comprising a compound of any one of the preceding clauses or a pharmaceutically acceptable salt thereof and a pharmaceutically acceptable carrier.

111. A method of preventing or treating an EHMT-mediated disease or disorder, the method comprising administering to a subject in need thereof a therapeutically effective amount of a compound of any one of the preceding clauses.

112. A method of preventing or treating a blood disorder via inhibition of a methyltransferase enzyme selected from EHMT1 and EHMT2, the method comprising administering to a subject in need thereof a therapeutically effective amount of a compound of any one of the preceding clauses.

113. The method of any one of the preceding clauses, wherein the blood disorder is sickle cell anemia or β-thalassemia.

114. The method of any one of the preceding clauses, wherein the blood disorder is a hematological cancer.

115. The method of any one of the preceding clauses, wherein the hematological cancer is acute myeloid leukemia (AML) or chronic lymphocytic leukemia (CLL).

116. The method of any one of the preceding clauses, wherein a compound of any of Formulae (I)-(Xf) is a selective inhibitor of EHMT2.

## Claims

1. A compound of Formula (Va), (Vb), (Vc), (Vd), (Ve), or (Vf):

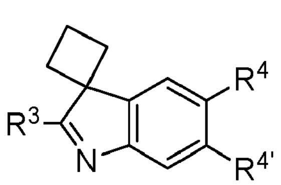 (Va), 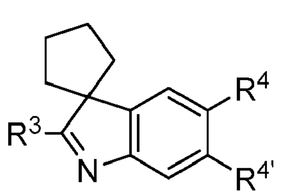 (Vb), 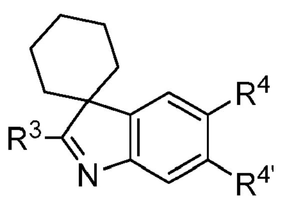 (Vc),

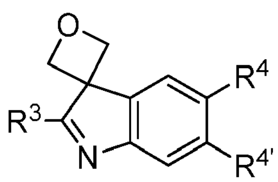 (Vd), 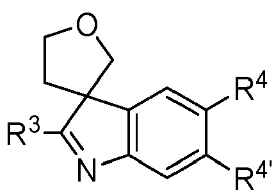 (Ve), 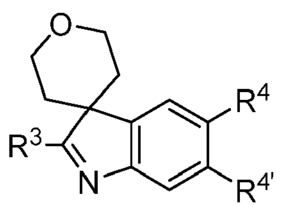 (Vf),

or a tautomer thereof, or a pharmaceutically acceptable salt of the compound or the tautomer; wherein

$R^3$ is

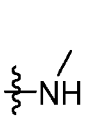, 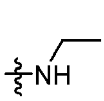, 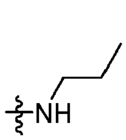, 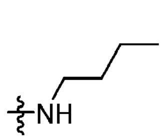, 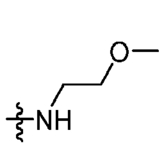, 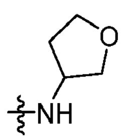, 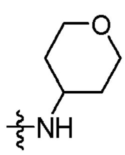,

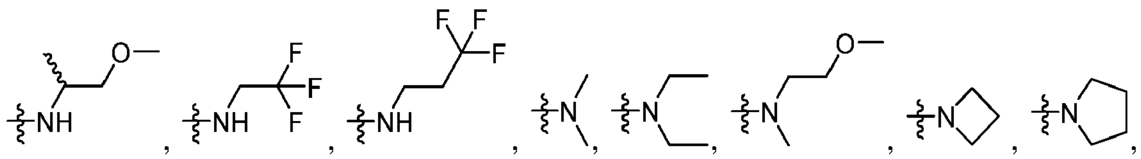

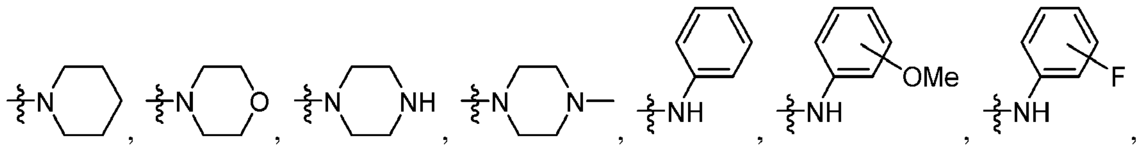

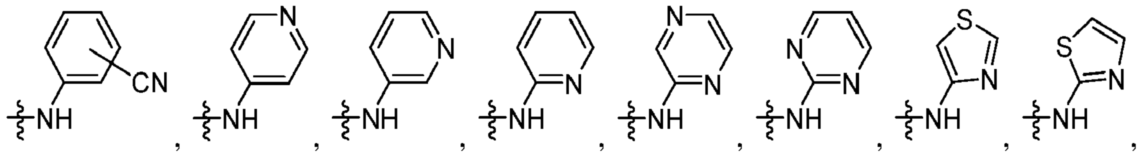

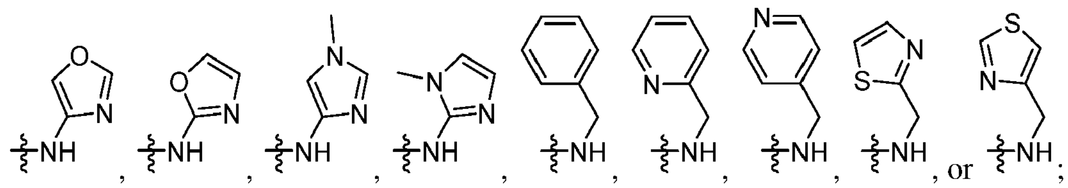

each of $R^4$ and $R^{4'}$ independently is $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and $R^8$ is $-Q^4-T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more $-Q^5-T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or $-Q^5-T^5$ is oxo;

provided that the compound is not:

(i) N-ethylspiro[cyclohexane-1,3'-indol]-2'-amine;
(ii) 5'-bromo-N-ethylspiro[cyclohexane-1,3'-indol]-2'-amine; or
(iii) 1-(2'-(ethylamino)spiro[cyclohexane-1,3'-indol]-5'-yl)ethan-1-one.

**2.** A compound of Formula (Va), (Vb), (Vd), (Ve), or (Vf):

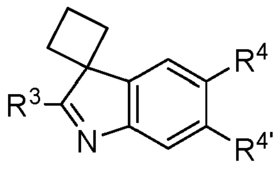 (Va), 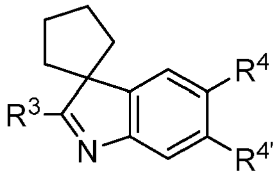 (Vb), 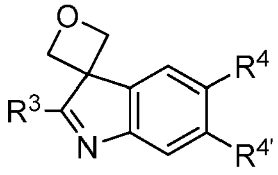 (Vd),

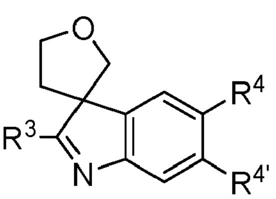 (Ve), 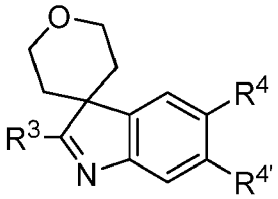 (Vf),

or a tautomer thereof, or a pharmaceutically acceptable salt of the compound or the tautomer; wherein

$R^3$ is

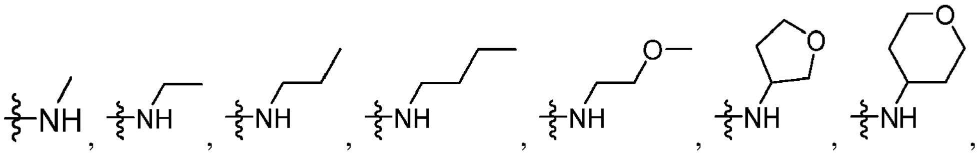

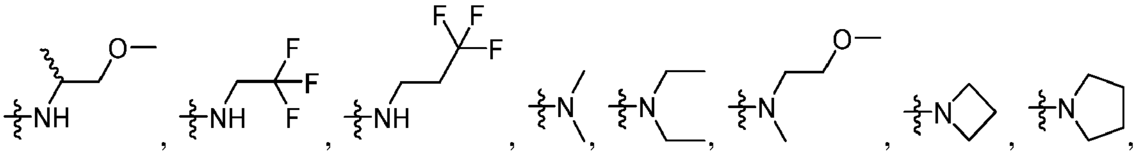

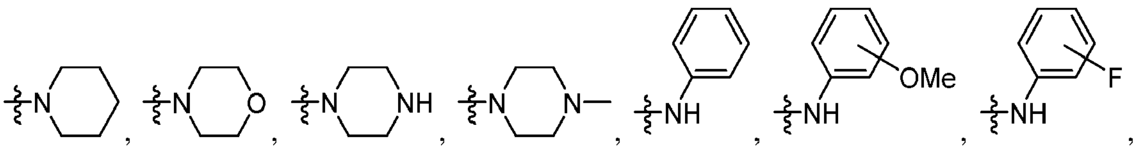

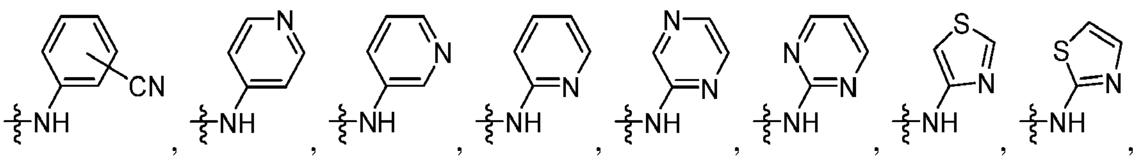

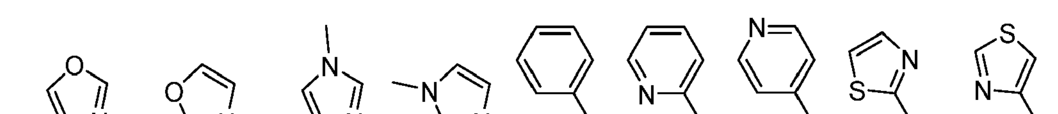

each of $R^4$ and $R^{4'}$ independently is $-Q^3-T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$;

each of $R^5$, $R^6$, and $R^7$, independently, is H or $C_1$-$C_6$ alkyl optionally substituted with one or more of halo, cyano, hydroxyl, amino, mono- or di- alkylamino, or $C_1$-$C_6$ alkoxyl; and $R^8$ is -$Q^4$-$T^4$, in which $Q^4$ is a bond or $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is H, halo, or $R^{S3}$, in which $R^{S3}$ is $C_3$-$C_{12}$ cycloalkyl, $C_6$-$C_{10}$ aryl, 4- to 12-membered heterocycloalkyl containing 1-4 heteroatoms selected from N, O and S, or a 5- to 10-membered heteroaryl, and $R^{S3}$ is optionally substituted with one or more -$Q^5$-$T^5$, wherein each $Q^5$ independently is a bond or $C_1$-$C_3$ alkylene, $C_2$-$C_3$ alkenylene, or $C_2$-$C_3$ alkynylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_{12}$ cycloalkyl, $C_6$-$C_{10}$ aryl, 4- to 7-membered heterocycloalkyl containing 1-4 heteroatoms selected from N, O, and S, 5- to 6-membered heteroaryl, $OR^c$, $C(O)R^c$, $NR^cR^d$, $C(O)NR^cR^d$, $S(O)_2R^c$, and $NR^cC(O)R^d$, each of $R^c$ and $R^d$ independently being H or $C_1$-$C_6$ alkyl optionally substituted with one or more halo; or -$Q^5$-$T^5$ is oxo.

**3.** The compound of claim 1 or claim 2, wherein each of $R^4$ and $R^{4'}$ is independently -$Q^3$-$T^3$, in which each $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di- alkylamino, or $C_1$-$C_6$ alkoxyl, and each $T^3$ independently is H, halo, $OR^7$, $OR^8$, $NR^7R^8$, $C_6$-$C_{10}$ aryl, 5- to 10-membered heteroaryl, $C_3$-$C_{12}$ cycloalkyl, or 4- to 12-membered heterocycloalkyl; and/or

wherein $R^4$ is -$Q^3$-$T^3$, in which $Q^3$ is a bond or $C_1$-$C_6$ alkylene linker, and $T^3$ is H, halo, $OR^7$, $C_6$-$C_{10}$ aryl, or 5- to 10-membered heteroaryl; and/or
wherein $R^{4'}$ is -$Q^3$-$T^3$, in which $Q^3$ independently is a bond or $C_1$-$C_6$ alkylene, $C_2$-$C_6$ alkenylene, or $C_2$-$C_6$ alkynylene linker optionally substituted with one or more of halo, cyano, hydroxyl, amino, mono- or di-alkylamino, or $C_1$-$C_6$ alkoxyl, and each $T^3$ independently is H, $OR^7$, $OR^8$, $NR^7R^8$, $C_3$-$C_{12}$ cycloalkyl, or 4- to 12-membered heterocycloalkyl; and/or
wherein at least one of $R^4$ and $R^{4'}$ is halo, $C_1$-$C_6$ alkyl, or $OR^7$; and/or
wherein $R^4$ is halo, $C_1$-$C_6$ alkyl, or $OR^7$; and/or
wherein at least one of $R^4$ and $R^{4'}$ is $C_1$-$C_6$ alkyl; and/or
wherein $R^4$ is $C_1$-$C_6$ alkyl; and/or
wherein at least one of $R^4$ and $R^{4'}$ is $CH_3$; and/or
wherein $R^4$ is $CH_3$; and/or
wherein at least one of $R^4$ and $R^{4'}$ is halo; and/or
wherein $R^4$ is halo; and/or
wherein at least one of $R^4$ and $R^{4'}$ is F or Cl; and/or
wherein $R^4$ is F or Cl; and/or
wherein at least one of $R^4$ and $R^{4'}$ is $C_6$-$C_{10}$ aryl; and/or
wherein $R^4$ is $C_6$-$C_{10}$ aryl; and/or
wherein at least one of $R^4$ and $R^{4'}$ is

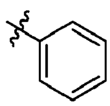

;

and/or
wherein at least one of $R^4$ and $R^{4'}$ is

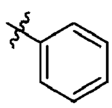

;

and/or
wherein at least one of $R^4$ and $R^{4'}$ is 5- to 10-membered heteroaryl; and/or
wherein $R^4$ is 5- to 10-membered heteroaryl; and/or
wherein at least one of $R^4$ and $R^{4'}$ is

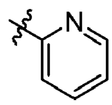, 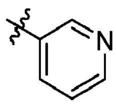, or 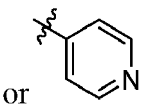;

and/or
wherein $R^4$ is

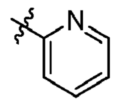, 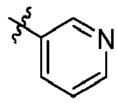, or 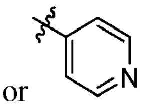.

**4.** The compound of any one of the proceeding claims, wherein at least one of $R^4$ and $R^{4'}$ is

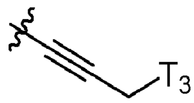,

wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$; and/or

wherein $R^{4'}$ is

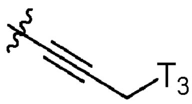,

wherein $T^3$ is H, halo, cyano, $OR^7$, $OR^8$, $C(O)R^8$, $NR^7R^8$, $C(O)NR^7R^8$, $NR^7C(O)R^8$, $C_6$-$C_{10}$ aryl, 5- to 10-membered heteroaryl, $C_3$-$C_{12}$ cycloalkyl, or 4- to 12-membered heterocycloalkyl containing 1-4 heteroatoms selected from N, O, and S, and wherein the $C_6$-$C_{10}$ aryl, 5- to 10-membered heteroaryl, $C_3$-$C_{12}$ cycloalkyl or 4- to 12-membered heterocycloalkyl is optionally substituted with one or more of halo, hydroxyl, cyano, $C_1$-$C_6$ haloalkyl, $-SO_2R^5$, $C_1$-$C_6$ alkoxyl or $C_1$-$C_6$ alkyl optionally substituted with one or more of $NR^5R^6$; and/or
wherein at least one of $R^4$ and $R^{4'}$ is

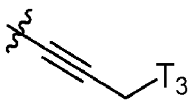,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl; and/or
wherein $R^{4'}$ is

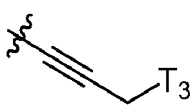,

wherein $T^3$ is 5- to 10-membered heteroaryl or 4- to 12-membered heterocycloalkyl optionally substituted with one or more of halo, hydroxyl, $C_1$-$C_6$ alkoxyl or $C_1$-$C_6$ alkyl; and/or
wherein at least one of $R^4$ and $R^{4'}$ is

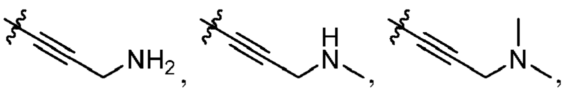, , ,

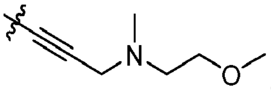, 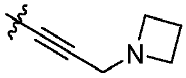, 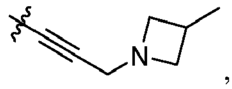, 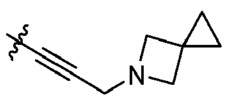, 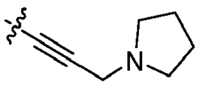,

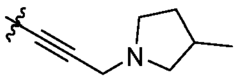, 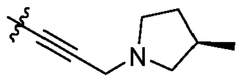, 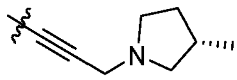, 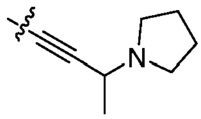, 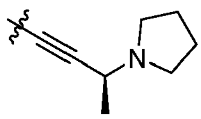,

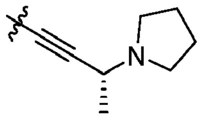, 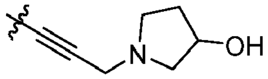, 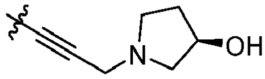, 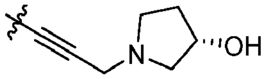,

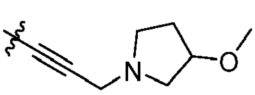, 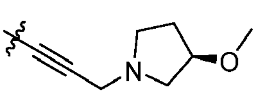, 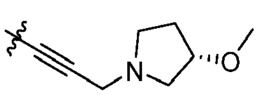, 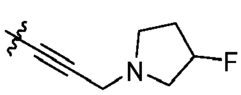,

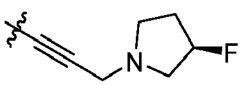, 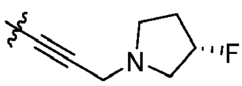, 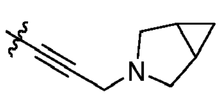, 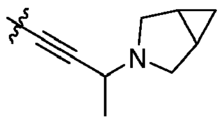, 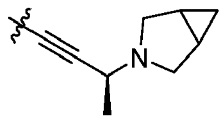,

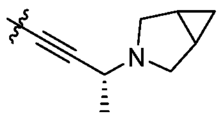, 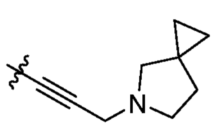, 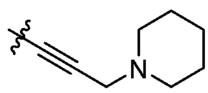, 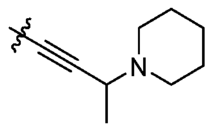, 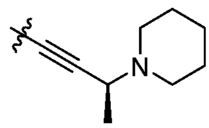,

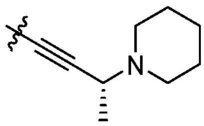, 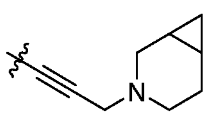, 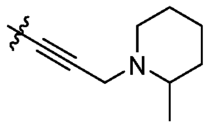, 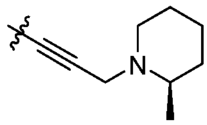, 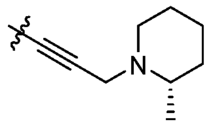,

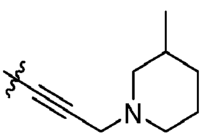, 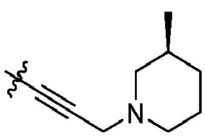, 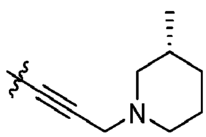, 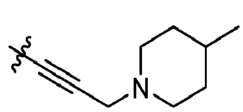, 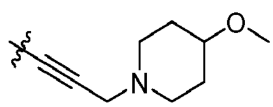,

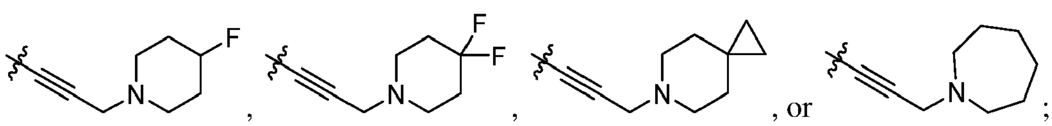, , , or ;

and/or
$R^{4'}$ is

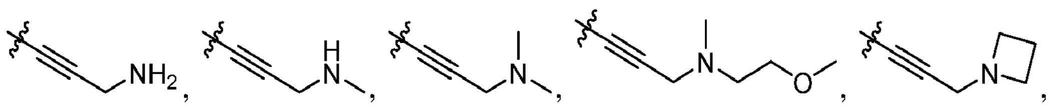

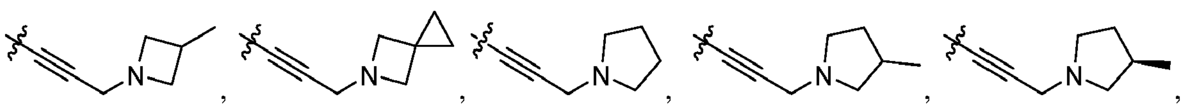

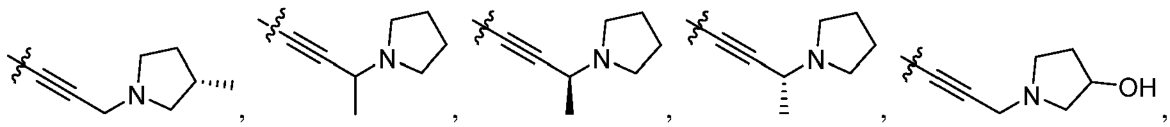

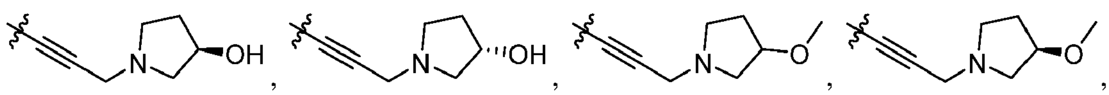

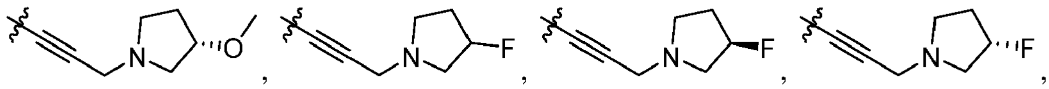

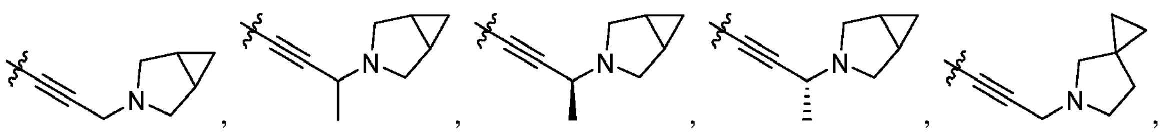

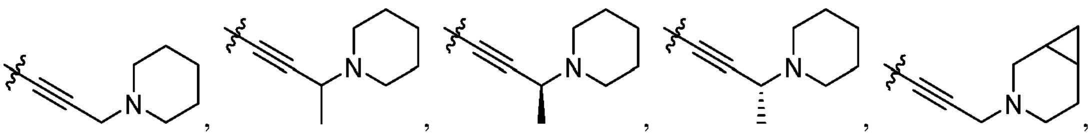

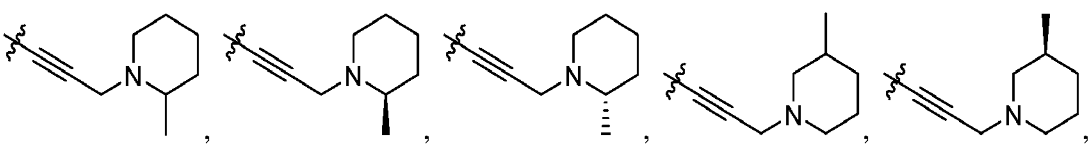

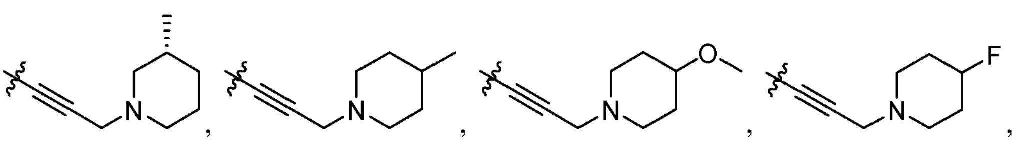

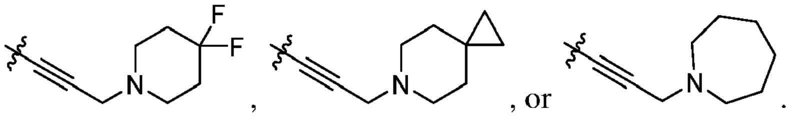

, or .

**5.** The compound of any one of the preceding claims, wherein at least one of $R^4$ and $R^{4'}$ is $OR^7$; and/or

wherein $R^4$ is $OR^7$; and/or
wherein $R^{4'}$ is $OR^7$; and/or
wherein at least one of $R^4$ and $R^{4'}$ is $OR^8$; and/or

wherein $R^{4'}$ is $OR^8$; and/or
wherein at least one of $R^4$ and $R^{4'}$ is $-CH_2-OR_8$; and/or
wherein $R^{4'}$ is $-CH_2-OR_8$; and/or
wherein at least one of $R^4$ and $R^{4'}$ is $-CH_2-NR_7R_8$; and/or
wherein $R^{4'}$ is $-CH_2-NR_7R_8$.

**6.** The compound of any one of the preceding claims, wherein $R^7$ is H or $C_1$-$C_6$ alkyl optionally substituted with one or more of hydroxyl, amino or mono- or di- alkylamino; and/or

wherein $R^8$ is $-Q^4-T^4$, in which $Q^4$ is a $C_1$-$C_6$ alkylene, $C_2$-$C_6$ alkenylene, or $C_2$-$C_6$ alkynylene linker optionally substituted with one or more of halo, cyano, hydroxyl, or $C_1$-$C_6$ alkoxyl, and $T^4$ is $C_3$-$C_{12}$ cycloalkyl, $C_6$-$C_{10}$ aryl, or 4- to 12-membered heterocycloalkyl containing 1-4 heteroatoms selected from N, O and S which is optionally substituted with one or more $-Q^5-T^5$; and/or
wherein each $Q^5$ independently is a bond or $C_1$-$C_3$ alkylene linker each optionally substituted with one or more of halo, cyano, hydroxyl, or $C_1$-$C_6$ alkoxy, and each $T^5$ independently is selected from the group consisting of H, halo, cyano, $C_1$-$C_6$ alkyl, $C_3$-$C_8$ cycloalkyl, or 4- to 7-membered heterocycloalkyl containing 1-4 heteroatoms selected from N, O, and S.

**7.** The compound of any one of the preceding claims, wherein at least one of $R^4$ and $R^{4'}$ is $C_1$-$C_6$ alkoxyl; and/or

wherein $R^4$ is $C_1$-$C_6$ alkoxyl; and/or
wherein at least one of $R^4$ and $R^{4'}$ is $-OCH_3$, $-OCH_2CH_3$, or $-OCH(CH_3)_2$; and/or
wherein $R^4$ is $-OCH_3$, $-OCH_2CH_3$, or $-OCH(CH_3)_2$; and/or
wherein at least one of $R^4$ and $R^{4'}$ is $-OCH_3$; and/or
wherein $R^4$ is $-OCH_3$; and/or
wherein at least one of $R^4$ and $R^{4'}$ is

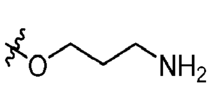, 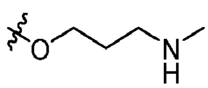, 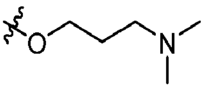,

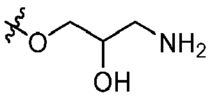, 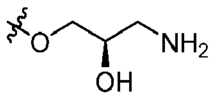, 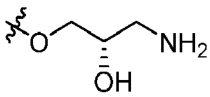, 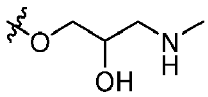, 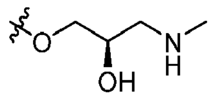,

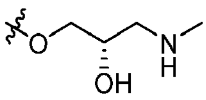, 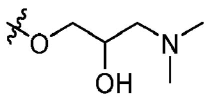, 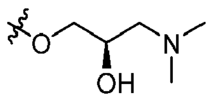, or 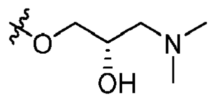;

and/or
$R^{4'}$ is

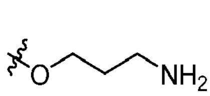, 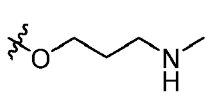, 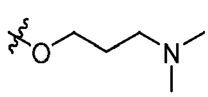, 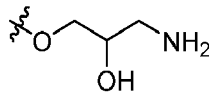, 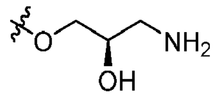,

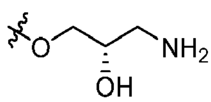, 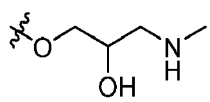, 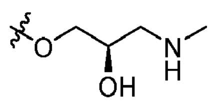, 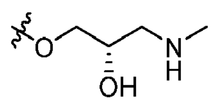, 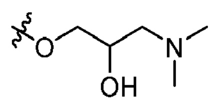,

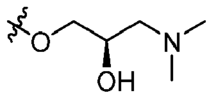, or 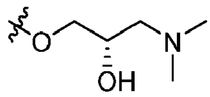;

and/or
wherein at least one of $R^4$ and $R^{4'}$ is

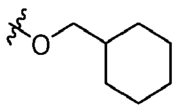, 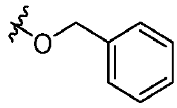, or 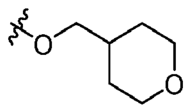;

and/or
wherein $R^{4'}$ is

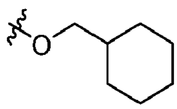, 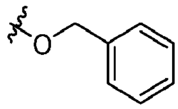, or 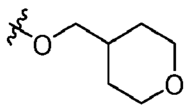;

and/or
wherein at least one of $R^4$ and $R^{4'}$ is

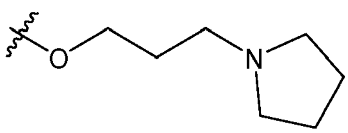, 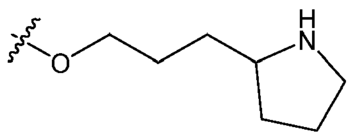,

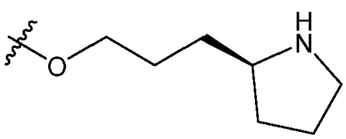, 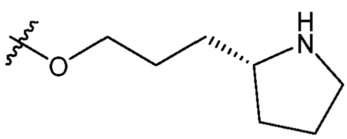, 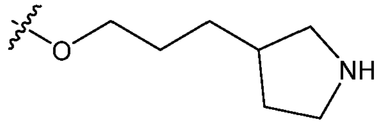,

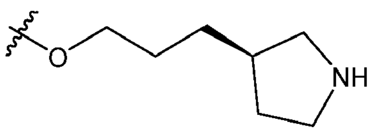, 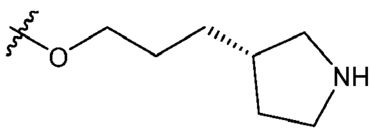, 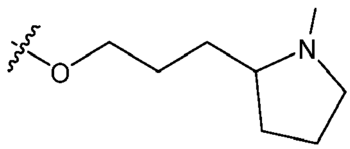,

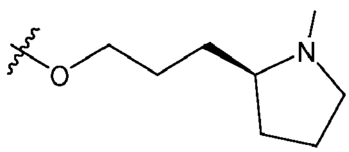, 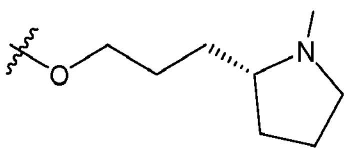, 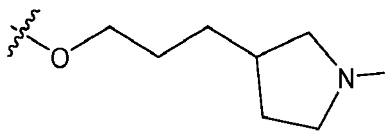,

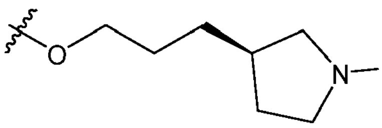, 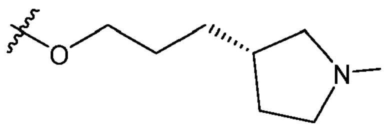, 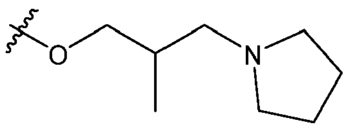,

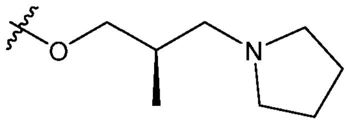, 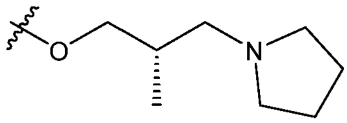, 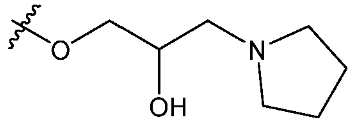,

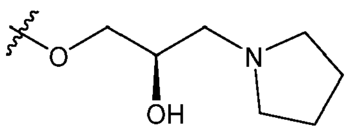, 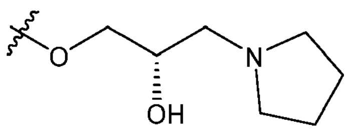, 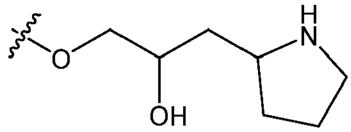,

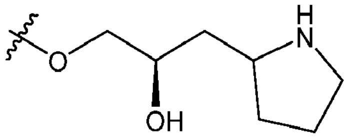, 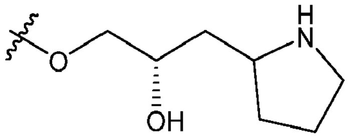, 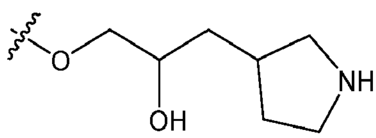,

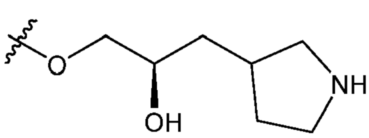, 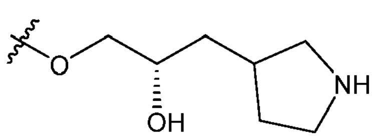, 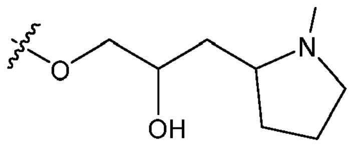,

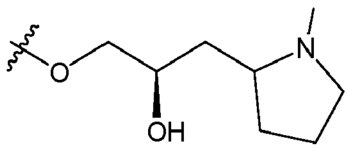, 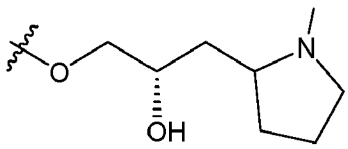, 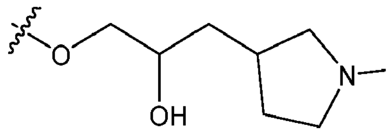,

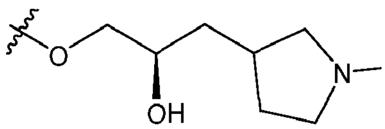, 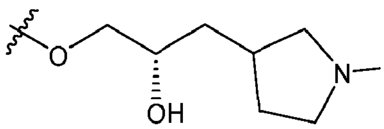, 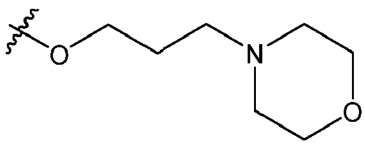,

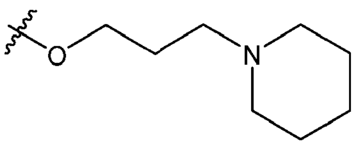, 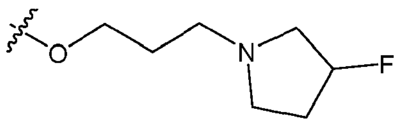, 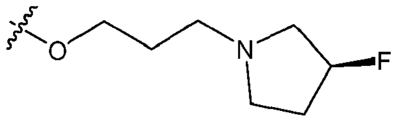,

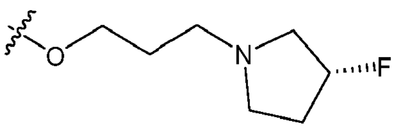, 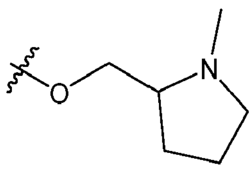, 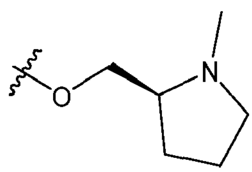, 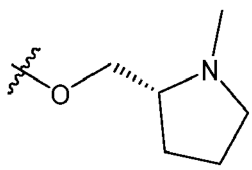,

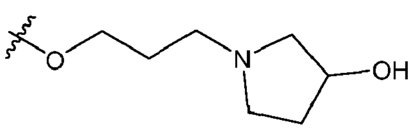, 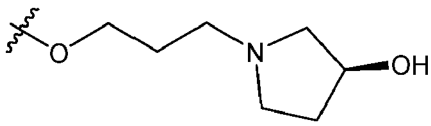,

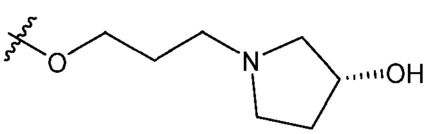, 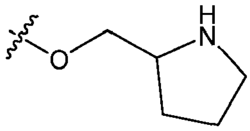, 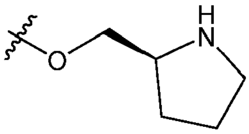, 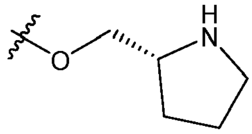,

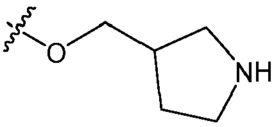, 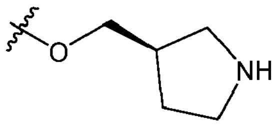, 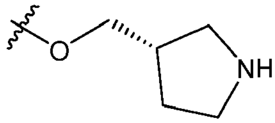, 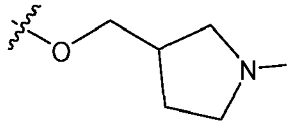,

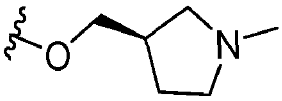, 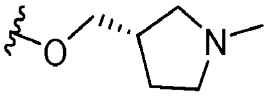, 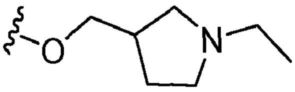, 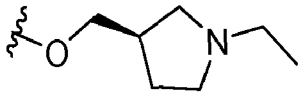,

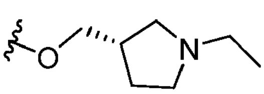, 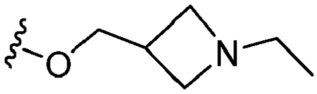, 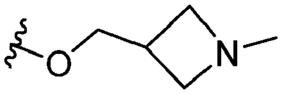, 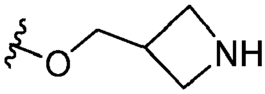,

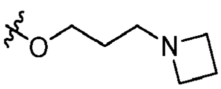, 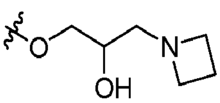, 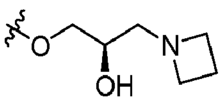, or 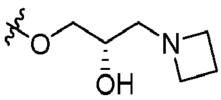;

and/or
wherein $R^{4'}$ is

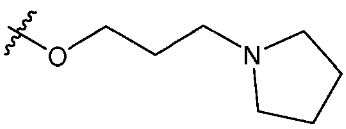, 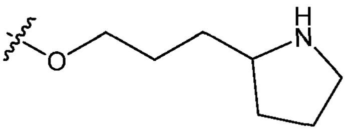,

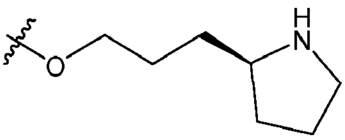, 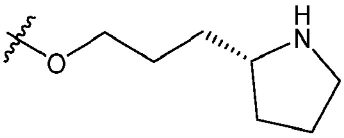, 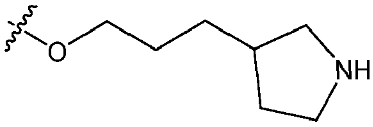,

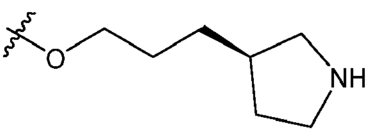, 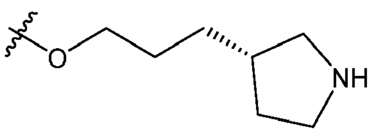, 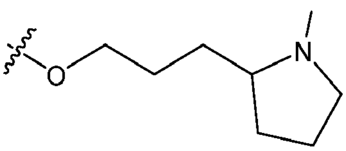,

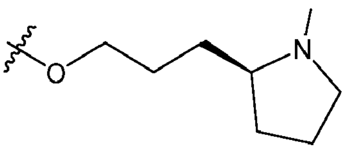, 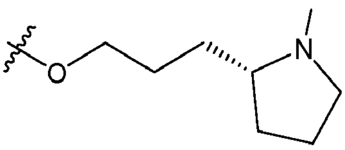, 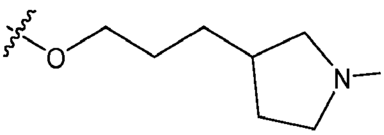,

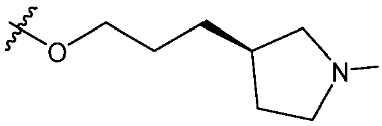, 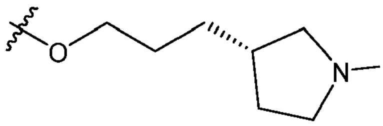, 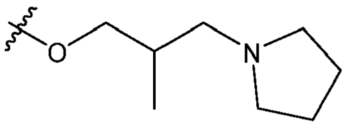,

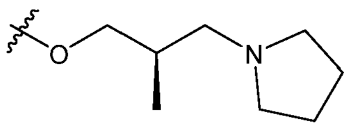, 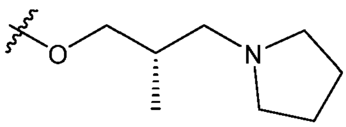, 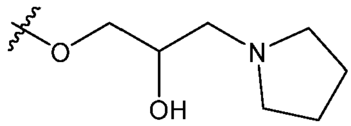,

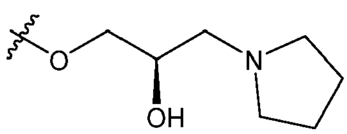, 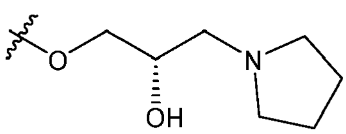, 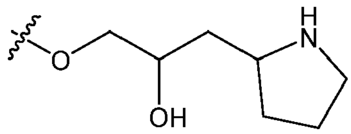,

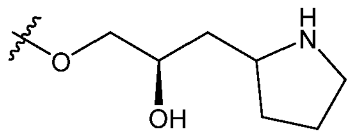, 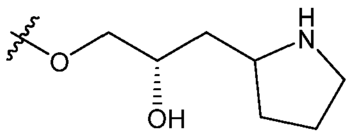, 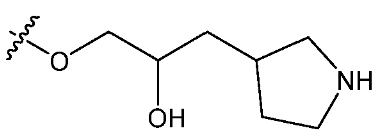,

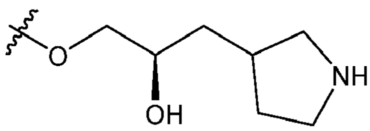, 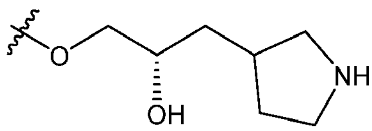, 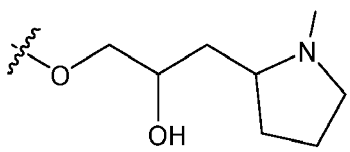,

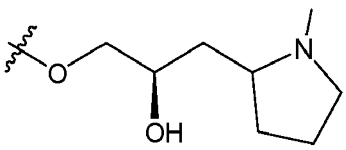, 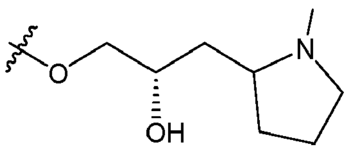, 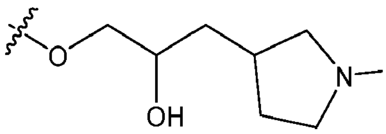,

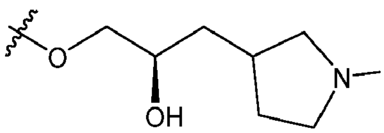, 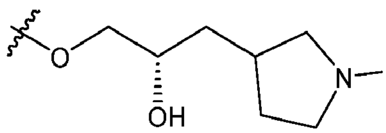, 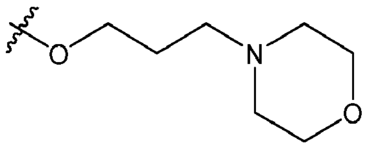,

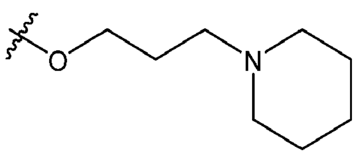, 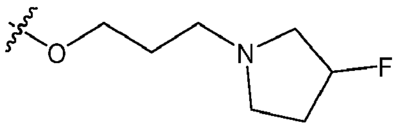, 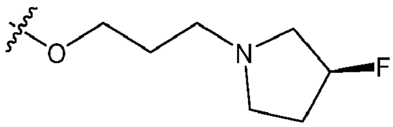,

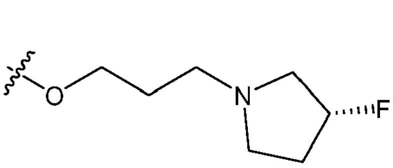, 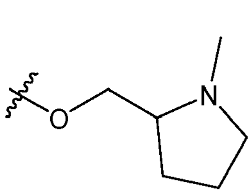, 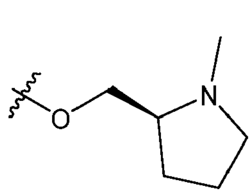, 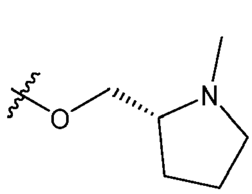,

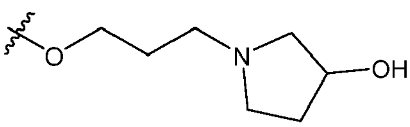, 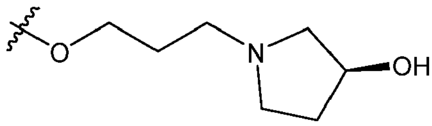,

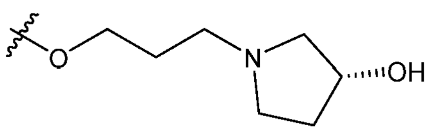, 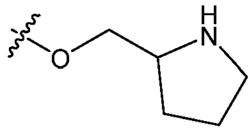, 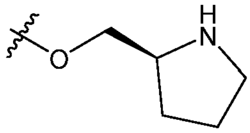, 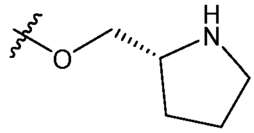

, 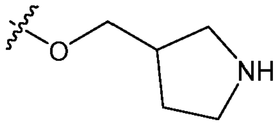, 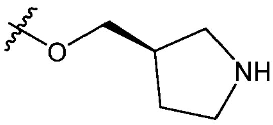, 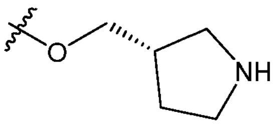, 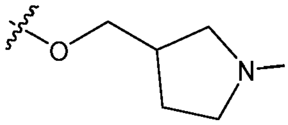,

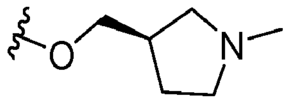, 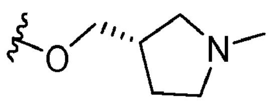, 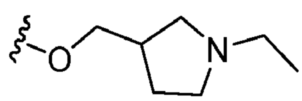, 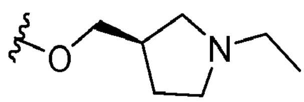,

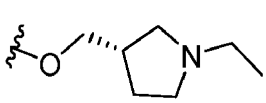, 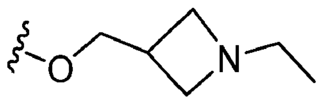, 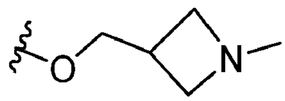, 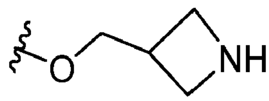,

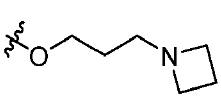, 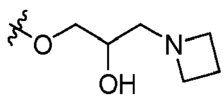, 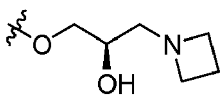, or 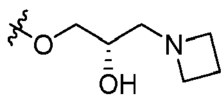;

and/or
wherein at least one of $R^4$ and $R^{4'}$ is

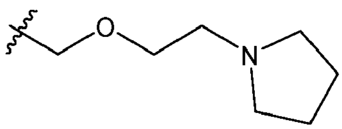;

and/or
wherein $R^{4'}$ is

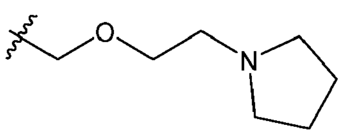;

and/or
wherein at least one of $R^4$ and $R^{4'}$ is

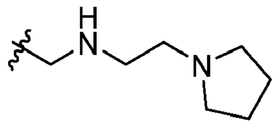, 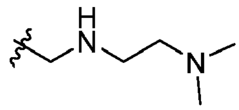,

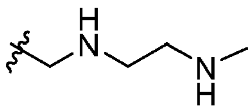, 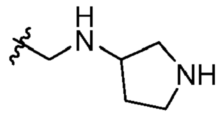, 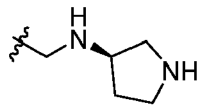, 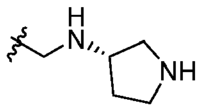, 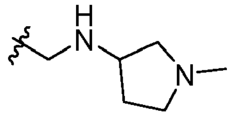,

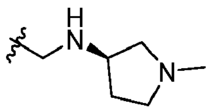, 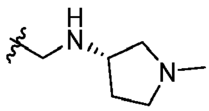, 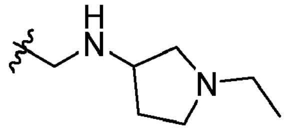, 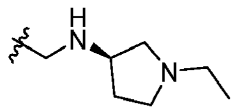, 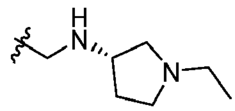,

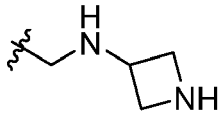, 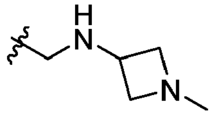, 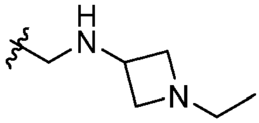, or 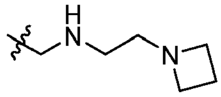;

and/or
$R^{4'}$ is

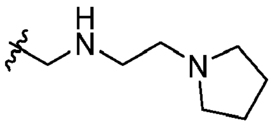, 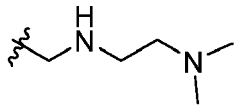, 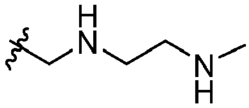, 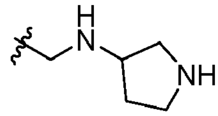,

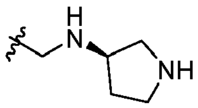, 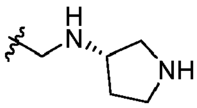, 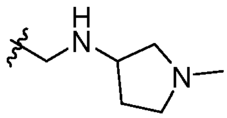, 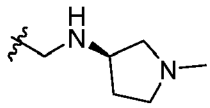, 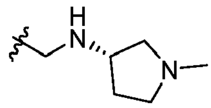,

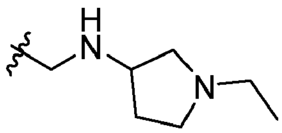, 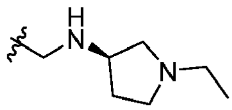, 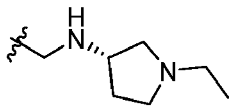, 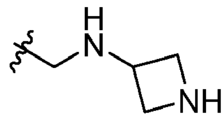,

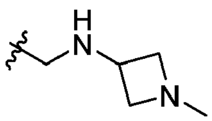, 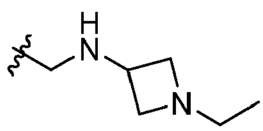, or 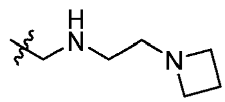.

**8.** The compound of any one of the preceding claims, wherein one of $R^4$ and $R^{4'}$ is $-OCH_3$, and the other is

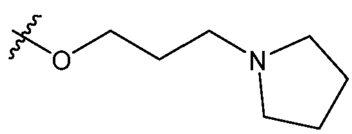;

and/or wherein $R^4$ is $-OCH_3$, and $R^{4'}$ is

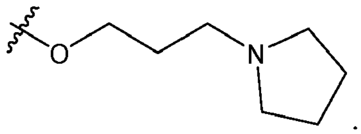

**9.** The compound of any one of the preceding claims, wherein the compound is selected from those in the following table and pharmaceutically acceptable salts thereof:

| Compound No. | Structure |
|---|---|
| 70 | 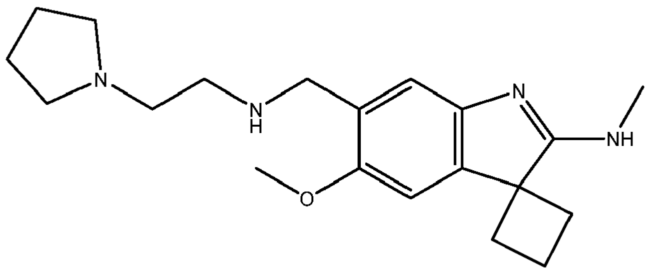 |
| 71 | 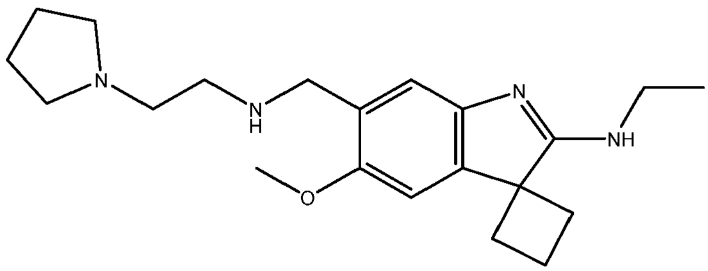 |
| 72 | 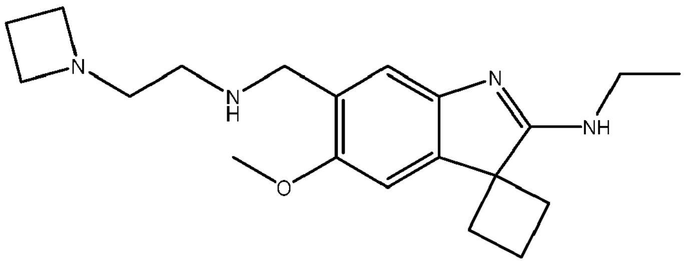 |
| 73 | 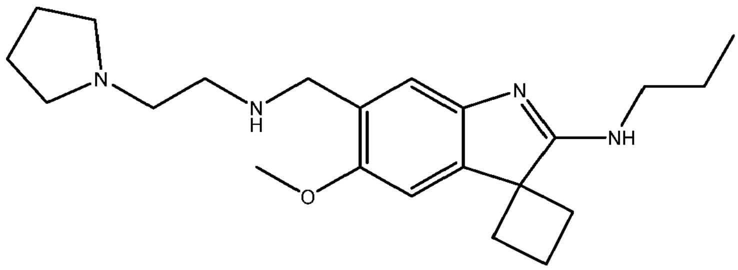 |

(continued)

| Compound No. | Structure |
|---|---|
| 74 | 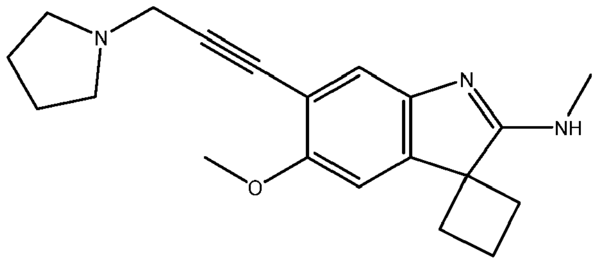 |
| 75 | 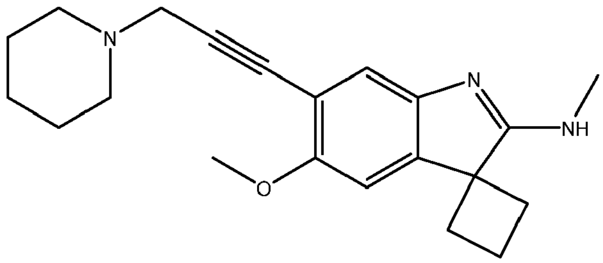 |
| 76 | 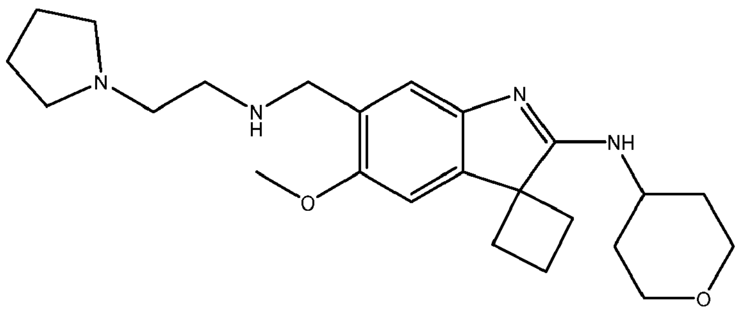 |
| 81 | 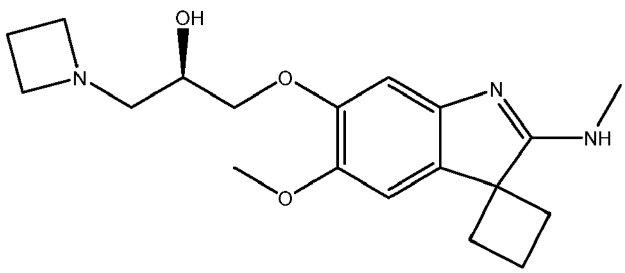 |
| 82 | 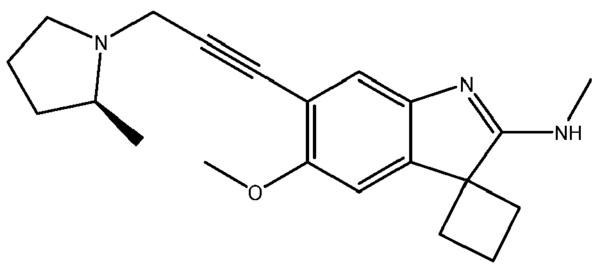 |

(continued)

| Compound No. | Structure |
|---|---|
| 83 | 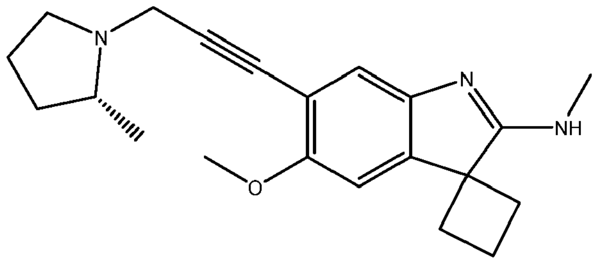 |
| 84 | 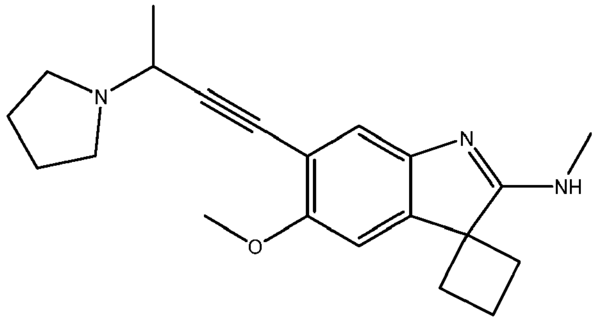 |
| 85 | 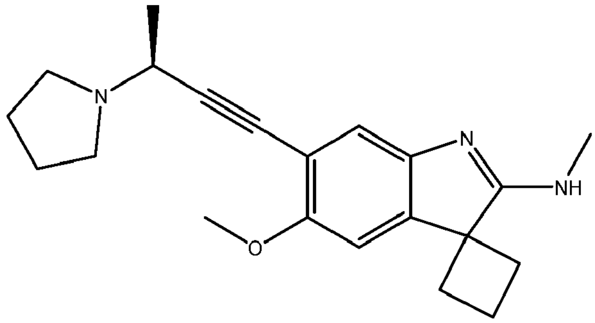 |
| 86 | 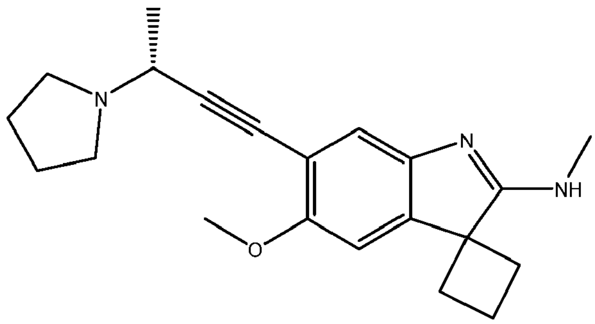 |
| 87 | 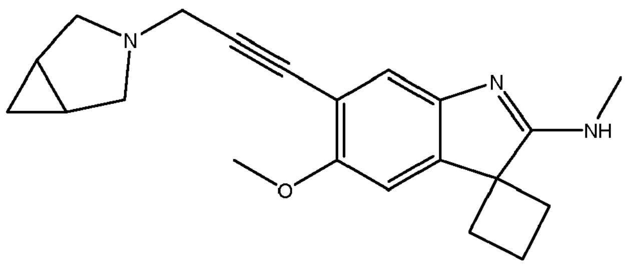 |

(continued)

| Compound No. | Structure |
|---|---|
| 88 | 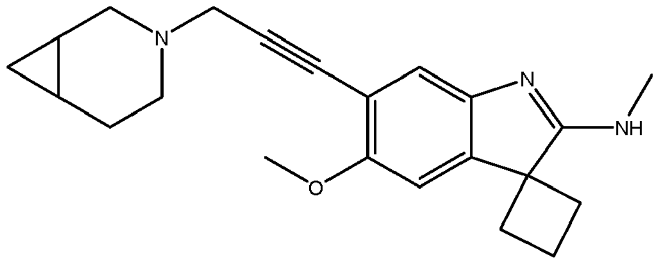 |
| 89 | 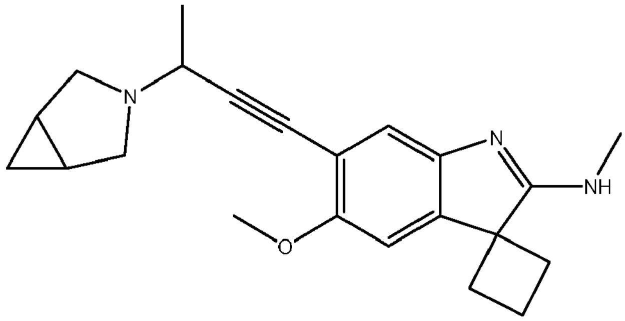 |
| 90 | 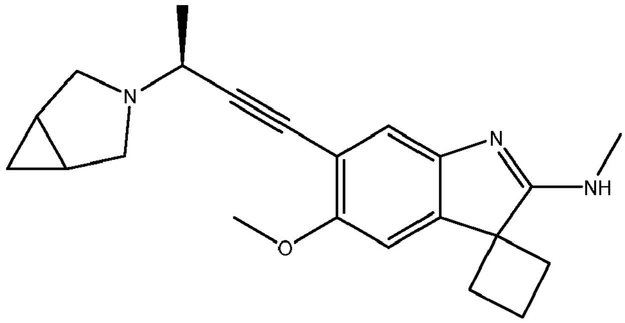 |
| 91 | 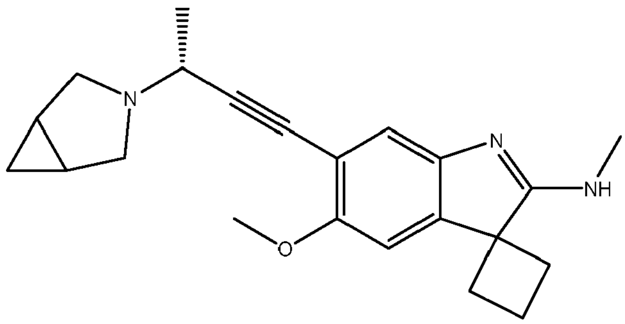 |
| 92 | 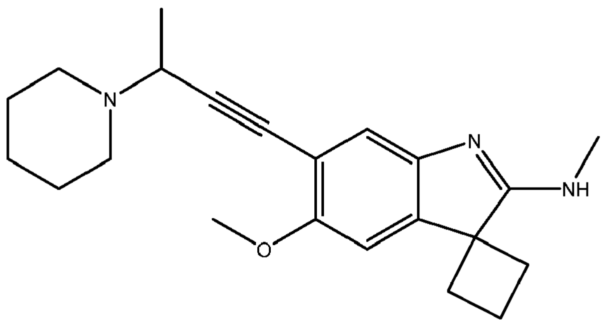 |

(continued)

| Compound No. | Structure |
|---|---|
| 93 | 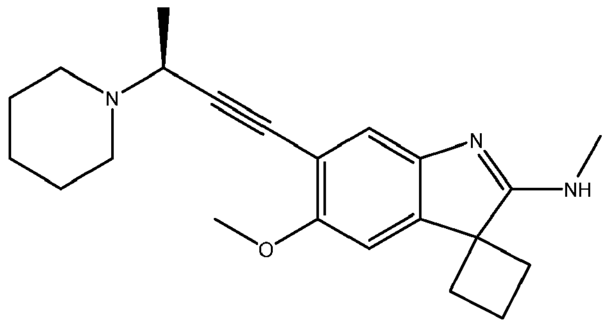 |
| 94 | 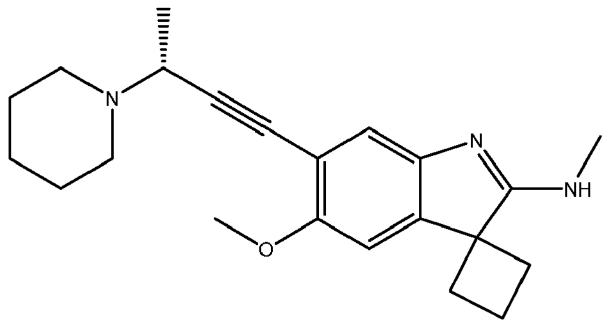 |
| 95 | 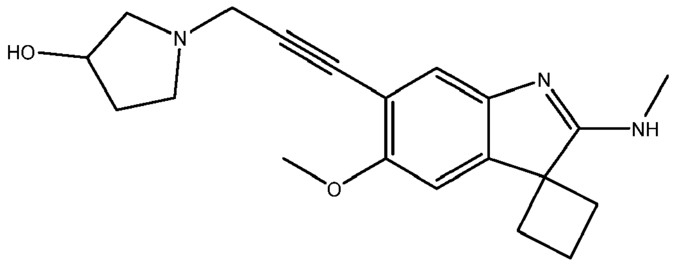 |
| 96 | 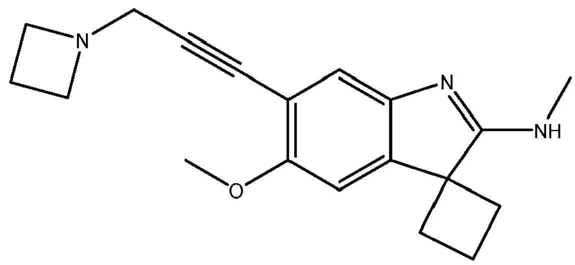 |
| 97 | 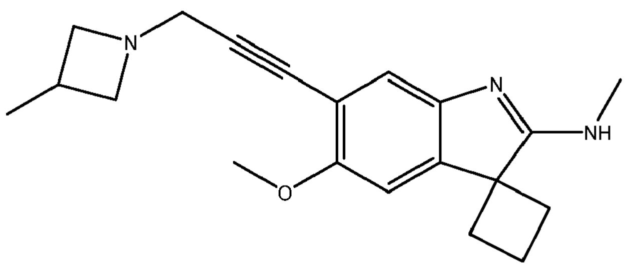 |

(continued)

| Compound No. | Structure |
|---|---|
| 98 | 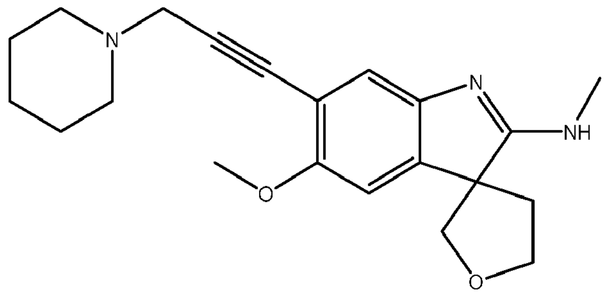 |
| 99 | 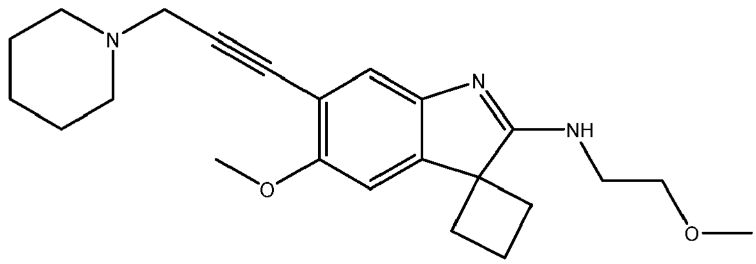 |
| 100 | 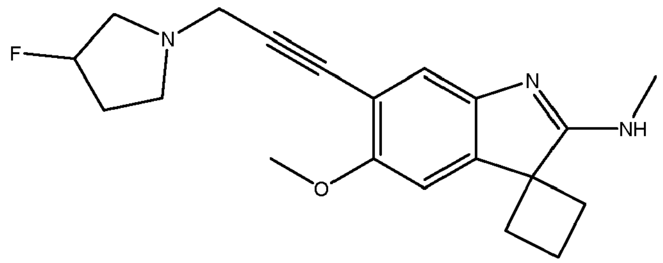 |
| 106 | 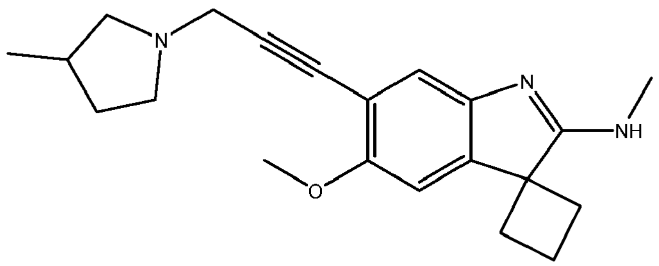 |
| 107 | 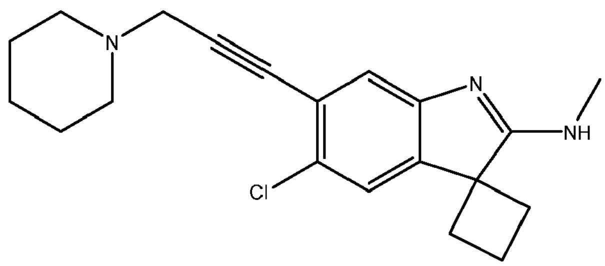 |

(continued)

| Compound No. | Structure |
|---|---|
| 110 | 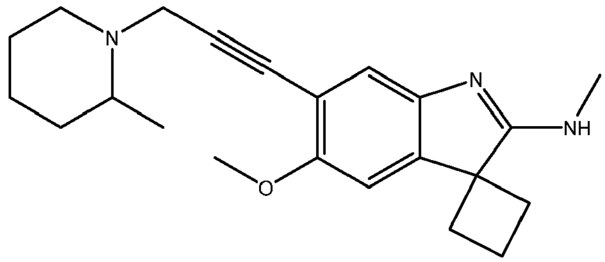 |
| 111 | 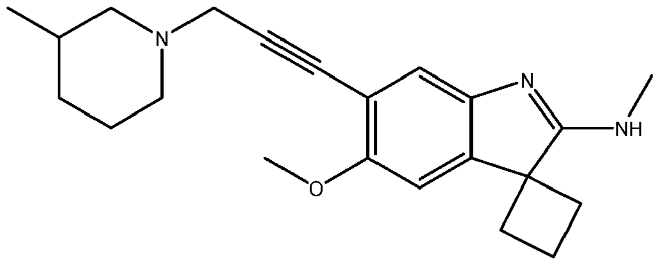 |
| 112 | 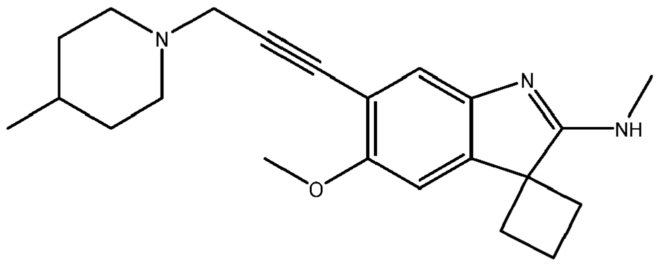 |
| 113 | 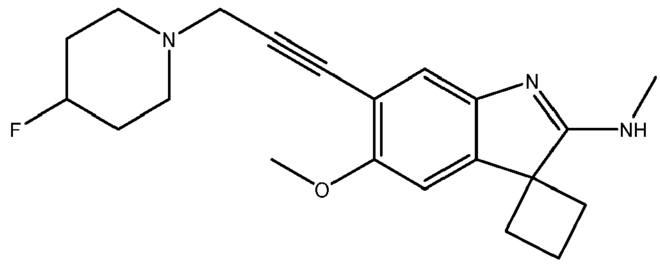 |
| 114 | 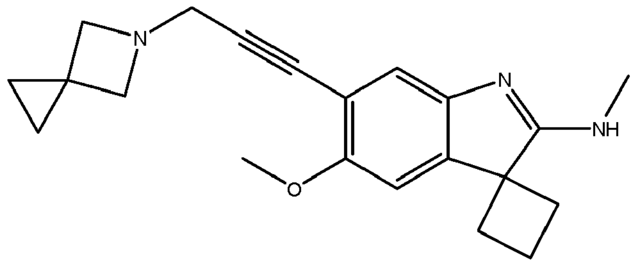 |
| 115 | 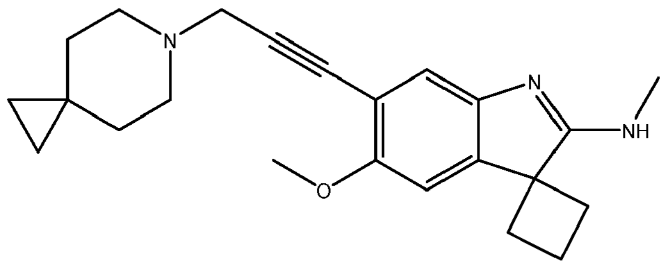 |

(continued)

| Compound No. | Structure |
|---|---|
| 116 | 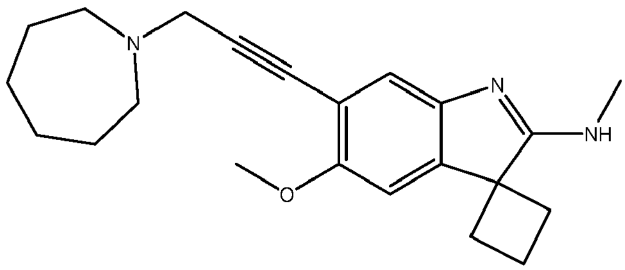 |
| 117 | 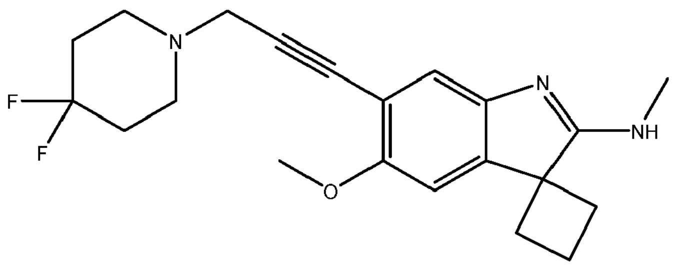 |
| 118 | 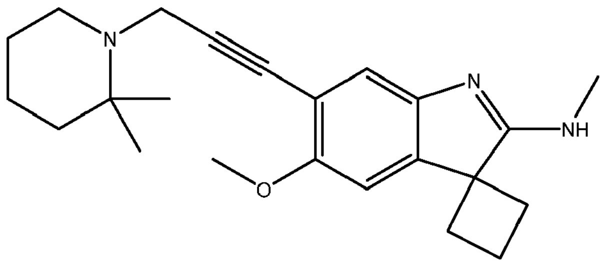 |
| 119 | 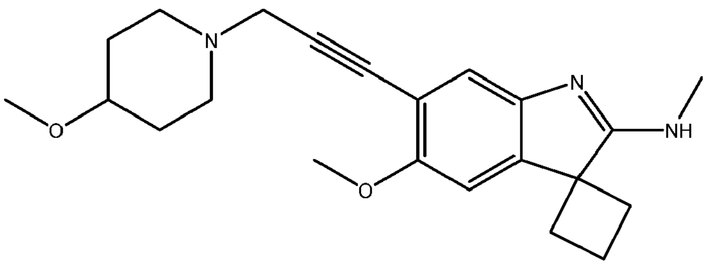 |
| 120 | 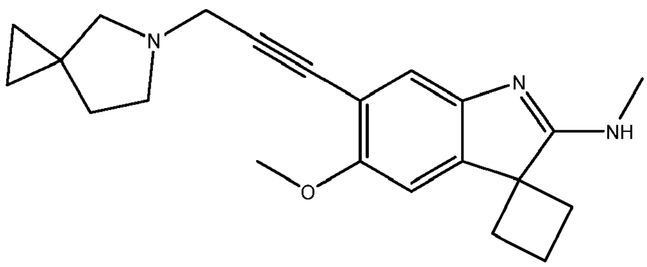 |

(continued)

| Compound No. | Structure |
|---|---|
| 121 | 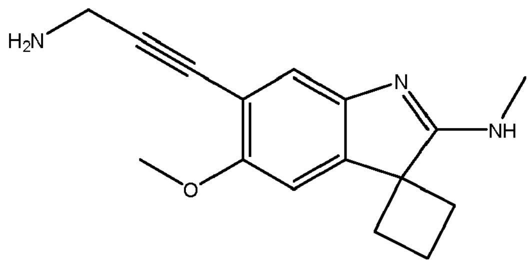 |
| 123 | 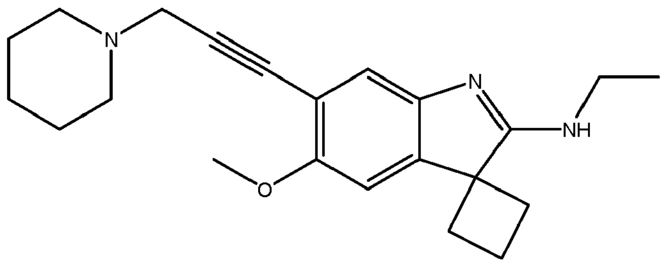 |
| 124 | 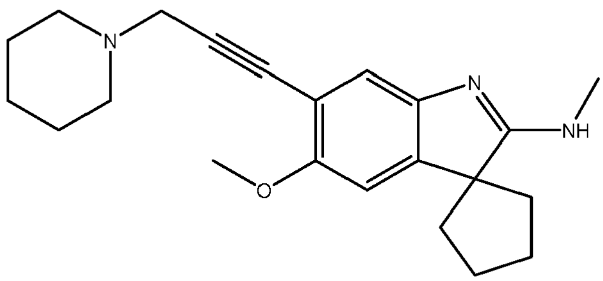 |
| 125 | 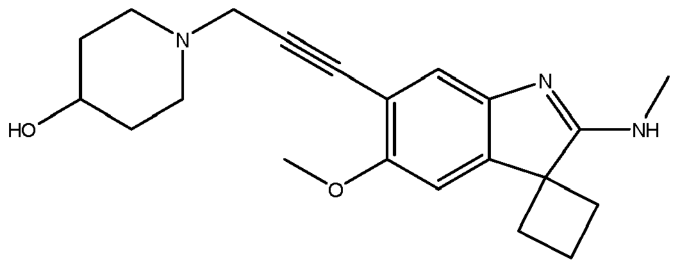 |
| 126 | 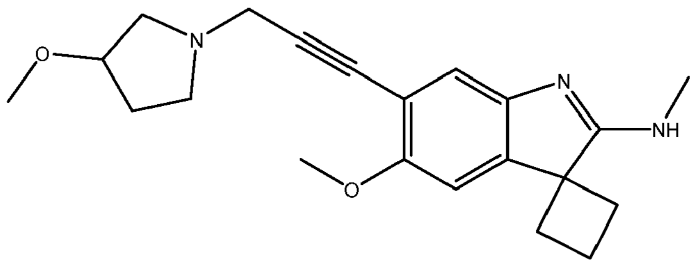 |

(continued)

| Compound No. | Structure |
|---|---|
| 127 | 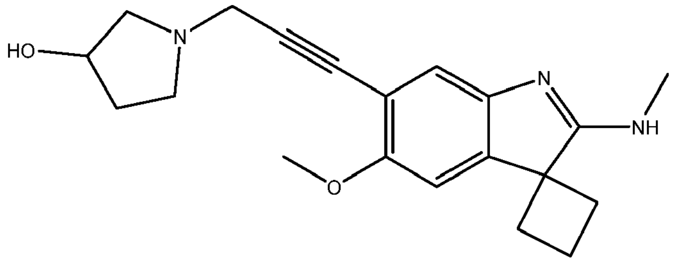 |
| 128 | 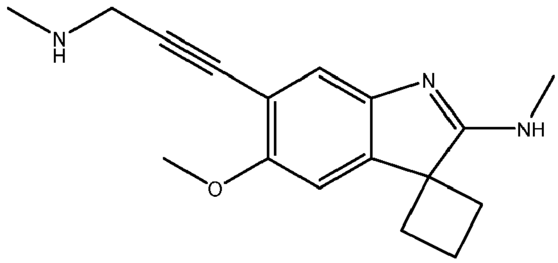 |
| 129 | 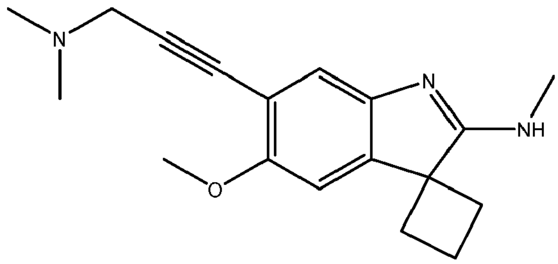 |
| 130 | 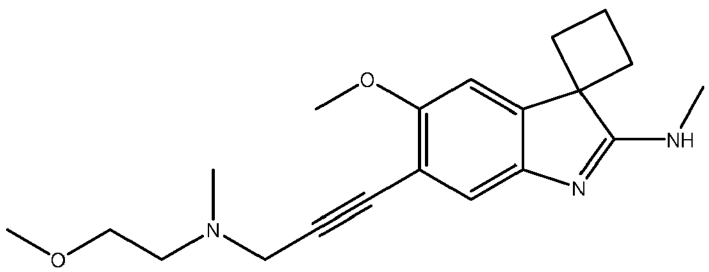 |
| 131 | 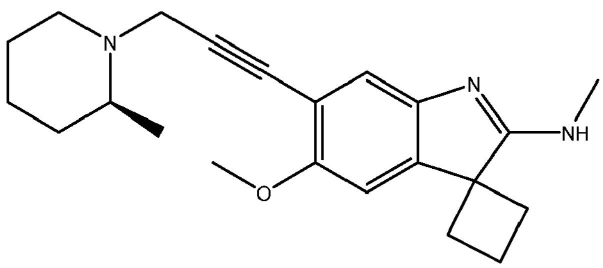 |

(continued)

| Compound No. | Structure |
|---|---|
| 132 | 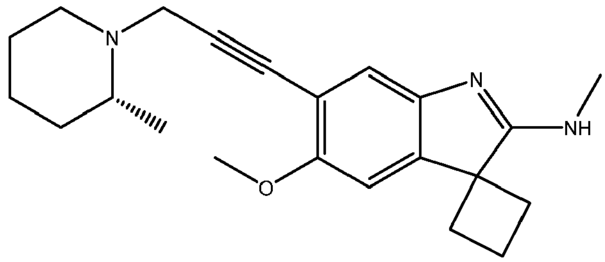 |
| 133 | 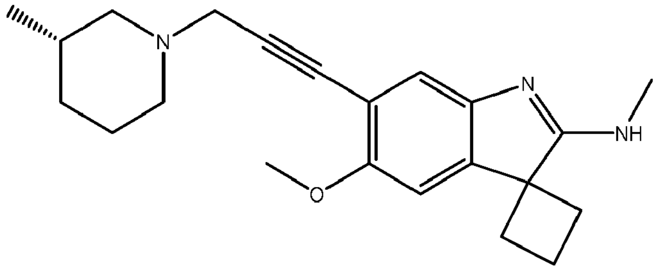 |
| 134 | 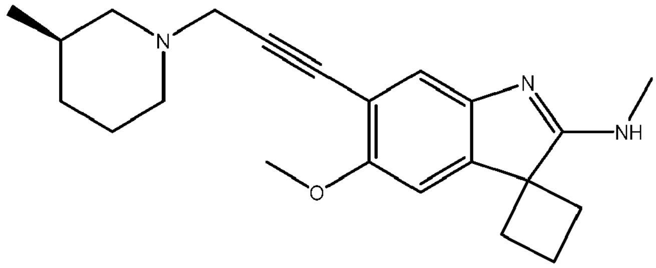 |
| 140 | 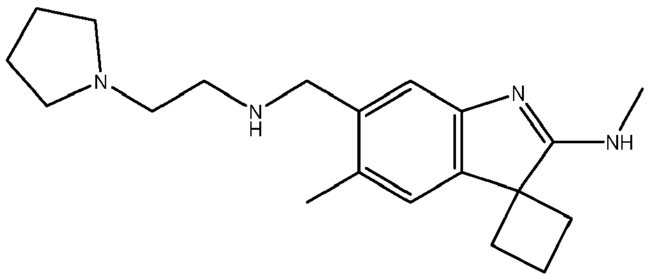 |

**10.** The compound of claim 9, wherein the compound is Compound No. 81, a tautomer thereof, and pharmaceutically acceptable salts of the compound and tautomer.

**11.** The compound of claim 9, wherein the compound is selected from the group consisting of Compound Nos. 74-75, 82-100, 106-107, 110-121, and 123-134, tautomers thereof, and pharmaceutically acceptable salts of the compounds and tautomers.

**12.** The compound of claim 9, wherein the compound is Compound No. 140, a tautomer thereof, and pharmaceutically acceptable salts of the compound and tautomer.

**13.** The compound as defined in any one of the preceding claims, wherein the compound inhibits a kinase with an enzyme inhibition $IC_{50}$ value of about 100 nM or greater, 1 $\mu$M or greater, 10 $\mu$M or greater, 100 $\mu$M or greater, or 1000 $\mu$M or greater; and/or

wherein the compound inhibits a kinase with an enzyme inhibition $IC_{50}$ value of about 1 mM or greater; and/or wherein the compound inhibits a kinase with an enzyme inhibition $IC_{50}$ value of 1 $\mu$M or greater, 2 $\mu$M or greater,

5 μM or greater, or 10 μM or greater, wherein the kinase is one or more of the following: Abl, AurA, CHK1, MAP4K, IRAK4, JAK3, EphA2, FGFR3, KDR, Lck, MARK1, MNK2, PKCb2, SIK, and Src.

**14.** A pharmaceutical composition comprising a compound as defined in any one of the preceding claims or a pharmaceutically acceptable salt thereof and a pharmaceutically acceptable carrier.

**15.** A compound as defined in any one of claims 1-13 for use as a medicament.

Europäisches Patentamt
European Patent Office
Office européen des brevets

# EUROPEAN SEARCH REPORT

Application Number
EP 22 17 3440

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WILLIAM N. PAPPANO ET AL: "The Histone Methyltransferase Inhibitor A-366 Uncovers a Role for G9a/GLP in the Epigenetics of Leukemia", PLOS ONE, vol. 10, no. 7, 6 July 2015 (2015-07-06), page e0131716, XP055431629, DOI: 10.1371/journal.pone.0131716 * figure 1 * ----- | 1-15 | INV. C07D235/30 C07D405/04 C07D471/04 C07D487/04 C07D491/10 C07D277/82 C07D209/40 A61K31/4184 A61K31/4045 A61P7/06 A61P35/02 |
| A | RAMZI F. SWEIS ET AL: "Discovery and Development of Potent and Selective Inhibitors of Histone Methyltransferase G9a", ACS MEDICINAL CHEMISTRY LETTERS, vol. 5, no. 2, 13 February 2014 (2014-02-13), pages 205-209, XP055170187, ISSN: 1948-5875, DOI: 10.1021/ml400496h * tables 1,2 * ----- | 1-15 | |
| | | | TECHNICAL FIELDS SEARCHED (IPC) |
| A | US 2015/274660 A1 (PLIUSHCHEV MARINA A [US] ET AL) 1 October 2015 (2015-10-01) * the whole document * ----- | 1-15 | C07D |
| A | G. A. GOLUBEVA ET AL: "Electrophilic substitution reactions of alkylated 2-aminoindole derivatives", CHEMISTRY OF HETEROCYCLIC COMPOUNDS, vol. 21, no. 7, 1 July 1985 (1985-07-01), pages 786-791, XP055701232, New York ISSN: 0009-3122, DOI: 10.1007/BF00519148 * compound III * ----- -/-- | 1 | |

The present search report has been drawn up for all claims

1

| Place of search | Date of completion of the search | Examiner |
|---|---|---|
| Munich | 13 September 2022 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 17 3440

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2018/118842 A1 (EPIZYME INC [US]) 28 June 2018 (2018-06-28) * compounds of formula (III0); examples 89-92,167,168,186,187,192-195,214-216,238, 277,281; compounds 283,284,288-290 * ----- | 1-3,5, 10-15 | |
| | | | TECHNICAL FIELDS SEARCHED (IPC) |

The present search report has been drawn up for all claims

1

| Place of search | Date of completion of the search | Examiner |
|---|---|---|
| Munich | 13 September 2022 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 17 3440

This annex lists the patent family members relating to the patent documents cited in the above-mentioned European search report. The members are as contained in the European Patent Office EDP file on
The European Patent Office is in no way liable for these particulars which are merely given for the purpose of information.

13-09-2022

| Patent document cited in search report | Publication date | Patent family member(s) | Publication date |
|---|---|---|---|
| US 2015274660 A1 | 01-10-2015 | NONE | |
| WO 2018118842 A1 | 28-06-2018 | AU 2017382830 A1 | 13-06-2019 |
| | | AU 2022202494 A1 | 12-05-2022 |
| | | BR 112019012140 A2 | 05-11-2019 |
| | | CA 3045032 A1 | 28-06-2018 |
| | | CL 2019001664 A1 | 06-12-2019 |
| | | CL 2020001133 A1 | 06-11-2020 |
| | | CN 110088099 A | 02-08-2019 |
| | | CO 2019006787 A2 | 30-09-2019 |
| | | EP 3555070 A1 | 23-10-2019 |
| | | IL 267090 A | 29-08-2019 |
| | | JP 2020504715 A | 13-02-2020 |
| | | JP 2022110080 A | 28-07-2022 |
| | | KR 20190092542 A | 07-08-2019 |
| | | MA 47233 A | 23-10-2019 |
| | | SG 10201913464T A | 30-03-2020 |
| | | TW 201833102 A | 16-09-2018 |
| | | US 2020039998 A1 | 06-02-2020 |
| | | US 2022235065 A1 | 28-07-2022 |
| | | WO 2018118842 A1 | 28-06-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62402863
- US 62509620 B
- EP 0356234 A
- US 5106862 A
- US 6025379 A
- US 9284272 B
- WO 2002059088 A
- WO 2015200329 A
- US 5763263 A


### Non-patent literature cited in the description


- **LIU et al.** *Journal of Medicinal Chemistry,* 2013, vol. 56, 8931-8942
- **KRIVEGA et al.** *Blood,* 2015, vol. 126 (5), 665-672
- **RENNEVILLE et al.** *Blood,* 2015, vol. 126 (16), 1930-1939
- **CAHN ; INGOLD ; PRELOG.** *Sequence Rule*
- **CAHN et al.** *Angew. Chem. Inter. Edit.,* 1966, vol. 5, 385, , 511
- **CAHN et al.** *Angew. Chem.,* 1966, vol. 78, 413
- **CAHN ; INGOLD.** *J. Chem. Soc.,* 1951, 612
- **CAHN et al.** *Experientia,* 1956, vol. 12, 81
- **CAHN.** *J. Chem. Educ.,* 1964, vol. 41, 116
- **PATANI ; LAVOIE.** *Chem. Rev.,* 1996, vol. 96, 3147-3176
- **SMITH, M. B. ; MARCH, J.** March's Advanced Organic Chemistry: Reactions, Mechanisms, and Structure. John Wiley & Sons, 2001
- **GREENE, T.W. ; WUTS, P.G. M.** Protective Groups in Organic Synthesis. John Wiley & Sons, 1999
- **R. LAROCK.** Comprehensive Organic Transformations. VCH Publishers, 1989
- **L. FIESER ; M. FIESER.** Fieser and Fieser's Reagents for Organic Synthesis. John Wiley and Sons, 1994
- Encyclopedia ofReagents for Organic Synthesis. John Wiley and Sons, 1995
- **AUSUBEL et al.** Current Protocols in Molecular Biology. John Wiley and Sons, Inc, 2005
- **SAMBROOK et al.** Molecular Cloning, A Laboratory Manual. Cold Spring Harbor Press, 2000
- **COLIGAN et al.** Current Protocols in Immunology. John Wiley & Sons
- **ENNA et al.** Current Protocols in Pharmacology. John Wiley & Sons
- **FINGL et al.** *The Pharmacological Basis of Therapeutics,* 1975
- Remington's Pharmaceutical Sciences. Mack Publishing Co, 1990
- Remington: the Science and Practice of Pharmacy. Mack Publishing Co, 1995
- **DEVLIN.** High Throughput Screening. Marcel Dekker, 1998